# EUROPÄISCHE PATENTSCHRIFT

(11) **EP 1 966 135 B1**
(45) Veröffentlichungstag und Bekanntmachung des Hinweises auf die Patenterteilung: **09.10.2013**
(21) Anmeldenummer: 06840967.1
(22) Anmeldetag: 11.12.2006
(51) Int. Cl.: C07D 209/54, C07D 307/94, C07C 233/45, C07C 61/06, A01N 43/08, A01N 43/38

(54) **3'-ALKOXY-SPIROCYCLOPENTYL SUBSTITUIERTE TETRAM- UND TETRONSÄUREN**
3'-ALKOXY-SPIROCYCLOPENTYL-SUBSTITUTED TETRAMIC AND TETRONIC ACIDS
ACIDES TÉTRAMIQUES ET TÉTRONIQUES À SUBSTITUTION 3'-ALCOXY-SPIROCYCLOPENTYL

(30) Priorität: 15.12.2005 DE 102005059891
(43) Veröffentlichungstag der Anmeldung: 10.09.2008
(73) Patentinhaber: Bayer CropScience AG, 40789 Monheim (DE)
(72) Erfinder: FISCHER, Reiner, 40789 Monheim (DE); LEHR, Stefan, 65835 Liederbach (DE); FEUCHT, Dieter, 65760 Eschborn (DE); FRANKEN, Eva-Maria, 42799 Leichlingen (DE); MALSAM, Olga, 51503 Rösrath (DE); BOJACK, Guido, 65207 Wiesbaden (DE); ARNOLD, Christian, 40764 Langenfeld (DE); HILLS, Martin, Jeffrey, 65510 Idstein (DE); KEHNE, Heinz, 65719 Hofheim (DE); ROSINGER, Christopher, Hugh, 65719 Hofheim (DE); DITTGEN, Jan, 60316 Frankfurt (DE)
(74) Vertreter: BIP Patents
(86) Internationale Anmeldenummer: PCT/EP2006/011911
(87) Internationale Veröffentlichungsnummer: WO 2007/073856

(56) Entgegenhaltungen:
- WO-A-2004/024688
- WO-A-2005/044796

## Beschreibung

Die vorliegende Erfindung betrifft neue 3'-Alkoxy-spirocyclopentyl substituierte cyclische Ketoenole, mehrere Verfahren zu ihrer Herstellung und ihre Verwendung als Schädlingsbekämpfungsmittel, Mikrobizide und/oder Herbizide. Gegenstand der Erfindung sind auch selektiv herbizide Mittel, die 3'-Alkoxy-spirocyclopentyl substituierte cyclische Ketoenole einerseits und eine die Kulturpflanzenverträglichkeit verbessernde Verbindung andererseits enthalten.

Die vorliegende Erfindung betrifft weiterhin die Steigerung der Wirkung von Pflanzenschutzmitteln enthaltend insbesondere 3'-Alkoxyspirocyclopentyl substituierte Tetram- und Tetronsäuren, durch die Zugabe von Ammonium- oder Phosphoniumsalzen und gegebenenfalls Penetrationstörderern, die entsprechenden Mittel, Verfahren zu ihrer Herstellung und ihre Anwendung im Pflanzenschutz als Insektizide und/oder Akarizide und/oder zur Verhinderung von unerwünschten Pflanzenwuchs.

1-H-Arylpyrrolidin-dion Derivate mit herbizider, insektizider oder akarizider Wirkung sind bekannt: EP-A-456 063, EP-A-521 334, EP-A-613 884, EP-A-613 885, WO 95/01358, WO 98/06721, WO 98/25928, WO 99/16748, WO 99/24437, WO 01/17972, WO 05/044791 oder WO 05/048710.

Weiterhin bekannt sind alkoxysubstituierte spirocyclische 1H-Arylpyrrolidin-dion-Derivate: EP-A-596 298, WO 95/26954, WO 95/20572, EP-A-0 668 267, WO 96/25395, WO 96/35664, WO 97/01535, WO 97/02243, WO 97/36868, WO 98/05638, WO 99/43649, WO 99/48869, WO 99/55673, WO 01/23354, WO 01/74770, WO 01/17972, WO 03/013249, WO 04/024688, WO 04/065366, WO 04/080962, WO 04/007448, WO 04/111042, WO 05/044796, WO 05/049569, WO 05/066125, WO 05/092897, WO 06/000355, WO 06/029799, WO 06/056281, WO 06/056282,WO 06/089633.

Es ist bekannt, dass bestimmte Δ³-Dihydrofuran-2-on Derivate herbizide, insektizide oder akarizide Eigenschaften aufweisen: EP-A-528 156, EP-A-647637, WO 95/26954, WO 96/20196, WO 96/25395, WO 96/35664, WO 97/01535, WO 97/02243, WO 97/36868, WO 98/05638, WO 98/06721, WO 99/16748, WO 98/25928, WO 99/43649, WO 99/48869, WO 99/55673, WO 01/23354, WO 01/74770, WO 01/17972, WO 2004/024688, WO 2004/080962, WO 04/111042, WO 05/092897, WO 06/000355, WO 06/029799, WO 06/089633.

Die herbizide und/oder akarizide und/oder insektizide Wirksamkeit und/oder Wirkungsbreite und/oder die Pflanzenverträglichkeit der bekannten Verbindungen, insbesondere gegenüber Kulturpflanzen, ist jedoch nicht immer ausreichend.

Es wurden nun neue Verbindungen der Formel (I) 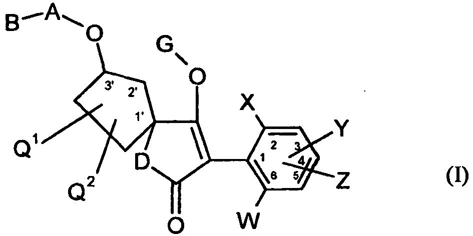 in welcher
- W: für Wasserstoff, Alkyl, Alkenyl, Alkinyl, Halogen, Alkoxy, Halogenalkyl, Halogenalkoxy oder Cyano steht,
- X: für Halogen, Alkyl, Alkenyl, Alkinyl, Alkoxy, Alkoxy-alkoxy, Halogenalkyl, Halogenalkoxy oder Cyano steht,
- Y: in für Wasserstoff, Halogen, Alkyl, Alkenyl, Alkinyl, Alkoxy, Cyano, Halogenalkyl, Halogenalkoxy, für jeweils gegebenenfalls substituiertes Phenyl oder Hetaryl steht,
- Z: für Wasserstoff, Halogen, Alkyl, Halogenalkyl, Cyano, Alkoxy oder Halogenalkoxy steht,
- A: für eine gegebenenfalls substituierte Alkandiylgruppe oder für gegebenenfalls substituiertes und/oder gegebenenfalls durch ein Heteroatom unterbrochenes Cycloalkyl steht,
- B: für Wasserstoff oder jeweils gegebenenfalls substituiertes Alkyl, Alkenyl, Alkoxy, Alkoxy-alkoxy, Phenyl, Hetaryl oder für gegebenenfalls substituiertes und/oder gegebenenfalls durch Heteroatome und/oder C=O unterbrochenes Cycloalkyl steht,
oder A für eine Bindung und B für Wasserstoff steht,
- D: für NH oder Sauerstoff steht,
- Q¹: für Wasserstoff, für jeweils gegebenenfalls substituiertes Alkyl, Alkoxy, Alkoxyalkyl oder Alkylthioalkyl, für gegebenenfalls substituiertes Cycloalkyl, worin gegebenenfalls eine Methylengruppe durch Heteroatome ersetzt ist oder für gegebenenfalls substituiertes Phenyl, Hetaryl, Phenylalkyl oder Hetarylalkyl steht,
- Q²: für Wasserstoff oder Alkyl steht,
- Q¹ und Q²: gemeinsam mit dem Kohlenstoff, an das sie gebunden sind, für einen gegebenenfalls substituierten C₃-C₆-Ring stehen, der gegebenenfalls durch ein Heteroatom unterbrochen sein kann, oder
- Q¹ und Q²: gemeinsam mit den Kohlenstoffatomen, an die sie gebunden sind, für einen gegebenenfalls substituierten C₃-C₆-Ring stehen, der gegebenenfalls durch ein Heteroatom unterbrochen sein kann,
- G: für Wasserstoff (a) oder für eine der Gruppen 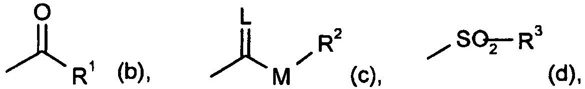 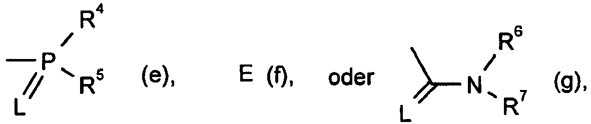 steht,
worin
E für ein Metallion oder ein Ammoniumion steht,
L für Sauerstoff oder Schwefel steht,
M für Sauerstoff oder Schwefel steht,
- R¹: für jeweils gegebenenfalls durch Halogen oder Cyano substituiertes Alkyl, Alkenyl, Alkoxyalkyl, Alkylthioalkyl oder Polyalkoxyalkyl oder für jeweils gegebenenfalls durch Halogen, Alkyl oder Alkoxy substituiertes Cycloalkyl oder Heterocyclyl oder für jeweils gegebenenfalls substituiertes Phenyl, Phenylalkyl, Hetaryl, Phenoxyalkyl oder Hetaryloxyalkyl steht,
- R²: für jeweils gegebenenfalls durch Halogen oder Cyano substituiertes Alkyl, Alkenyl, Alkoxyalkyl oder Polyalkoxyalkyl oder für jeweils gegebenenfalls substituiertes Cycloalkyl, Phenyl oder Benzyl steht,
- R³, R⁴ und R⁵: unabhängig voneinander für jeweils gegebenenfalls durch Halogen substituiertes Alkyl, Alkoxy, Alkylamino, Dialkylamino, Alkylthio, Alkenylthio oder Cycloalkylthio oder für jeweils gegebenenfalls substituiertes Phenyl, Benzyl, Phenoxy oder Phenylthio stehen,
- R⁶ und R⁷: unabhängig voneinander für Wasserstoff, für jeweils gegebenenfalls durch Halogen oder Cyano substituiertes Alkyl, Cycloalkyl, Alkenyl, Alkoxy, Alkoxyalkyl, für jeweils gegebenenfalls substituiertes Phenyl oder Benzyl stehen, oder gemeinsam mit dem N-Atom, an das sie gebunden sind, einen gegebenenfalls Sauerstoff oder Schwefel enthaltenden und gegebenenfalls substituierten Cyclus bilden.

Die Verbindungen der Formel (I) können, auch in Abhängigkeit von der Art der Substituenten, als optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Unter Einbeziehung von D für NH (1) und D für O (2) ergeben sich folgende hauptsächliche Strukturen (I-1) bis (1-2): 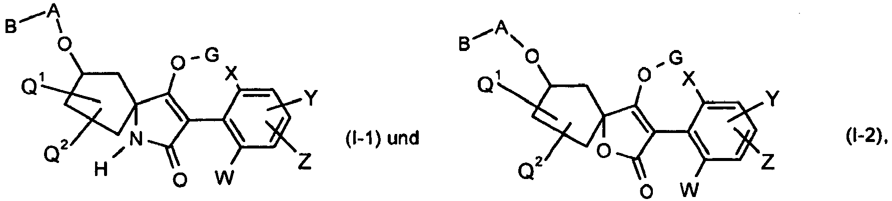 worin
A, B, G, Q¹, Q², W, X, Y und Z die oben angegebene Bedeutung haben.

Unter Einbeziehung der verschiedenen Bedeutungen (a), (b), (c), (d), (e), (f) und (g) der Gruppe G ergeben sich folgende hauptsächliche Strukturen (I-1-a) bis (I-1-g), wenn D für NH (1) steht, 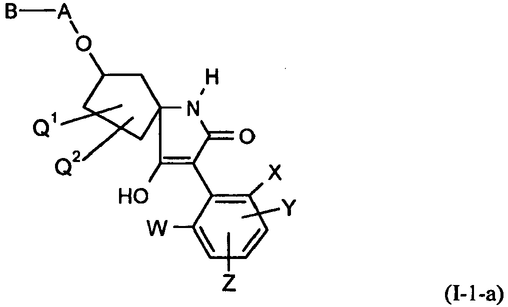 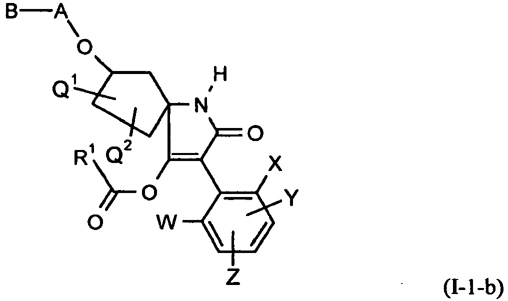 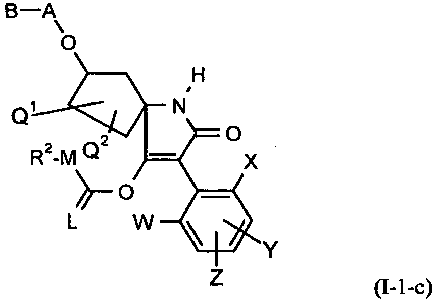 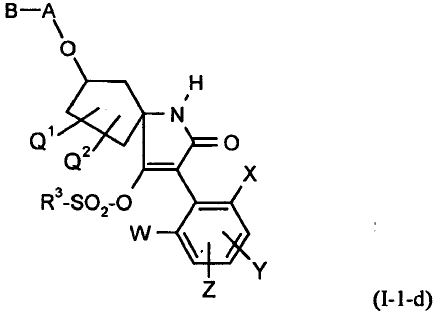 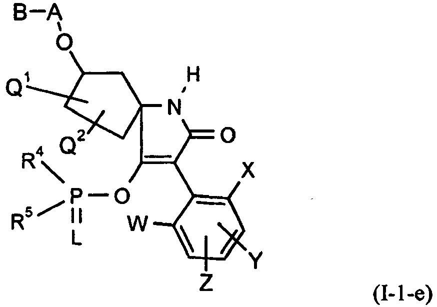 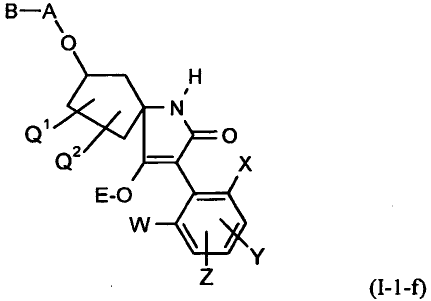 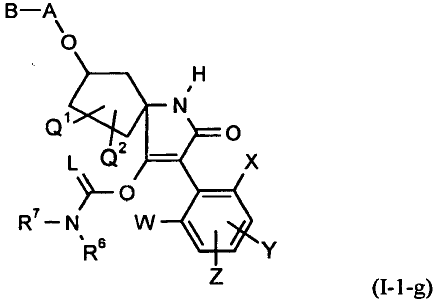 worin
A, B, E, L, M, Q¹, Q², W, X, Y, Z, R¹, R², R³, R⁴, R⁵, R⁶ und R⁷ die oben angegebenen Bedeutungen besitzen.

Unter Einbeziehung der verschiedenen Bedeutungen (a), (b), (c), (d), (e), (f) und (g) der Gruppe G ergeben sich folgende hauptsächliche Strukturen (I-2-a) bis (I-2-g), wenn D für O (2) steht, 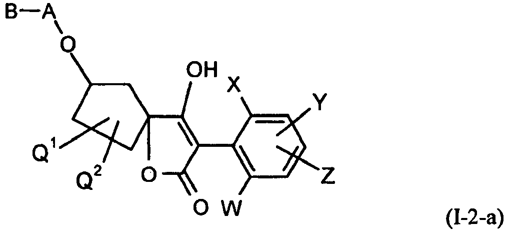 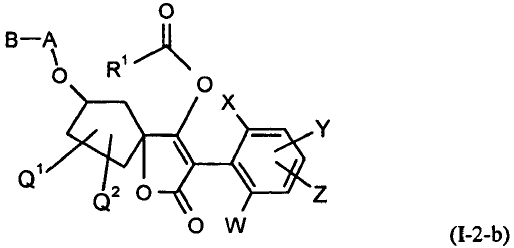 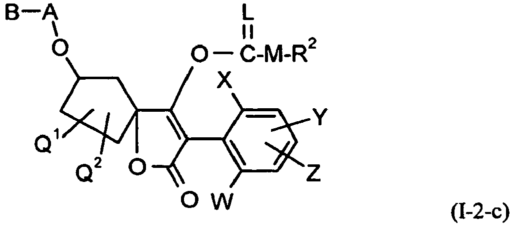 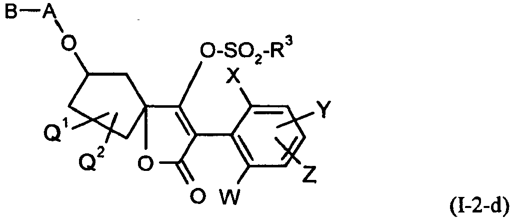 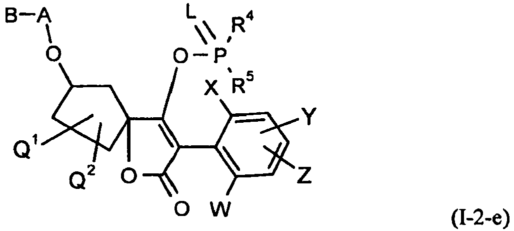 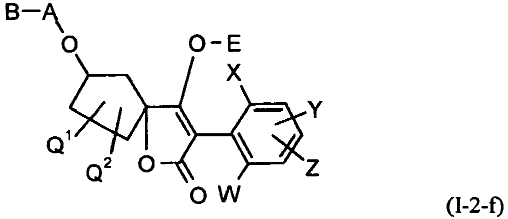 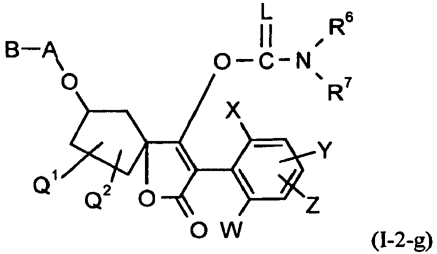 worin
A, B, E, L, M, Q¹, Q², W, X, Y, Z, R¹, R², R³, R⁴, R⁵, R⁶ und R⁷ die oben angegebene Bedeutung haben.

Weiterhin wurde gefunden, dass man die neuen Verbindungen der Formel (I) nach den im Folgenden beschriebenen Verfahren erhält:
(A) Man erhält Verbindungen der Formel (I-1-a) 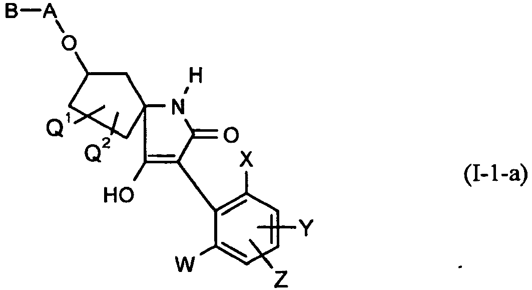 in welcher
   A, B, Q¹, Q², W, X, Y und Z die oben angegebenen Bedeutungen haben,
   wenn man
   Verbindungen der Formel (II) 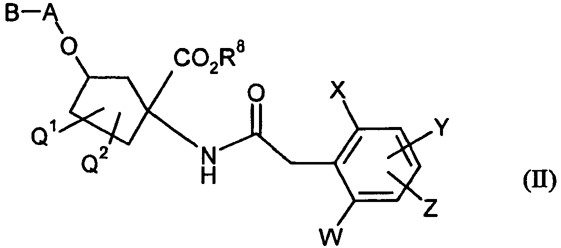 in welcher
   A, B, Q¹, Q², W, X, Y und Z die oben angegebenen Bedeutungen haben,
   und
   R⁸ für Alkyl (bevorzugt C₁-C₆₋Alkyl) steht,
   in Gegenwart eines Verdünnungsmittels und in Gegenwart einer Base intramolekular kondensiert.
(B) Außerdem wurde gefunden, dass man Verbindungen der Formel (I-2-a) 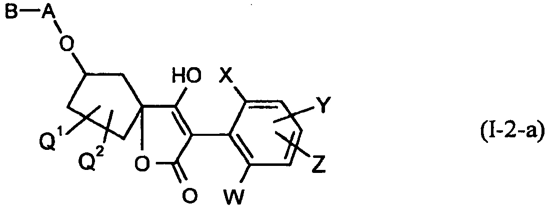 in welcher
   A, B, Q¹, Q², W, X, Y und Z die oben angegebenen Bedeutungen haben,
   erhält, wenn man
   Verbindungen der Formel (III) 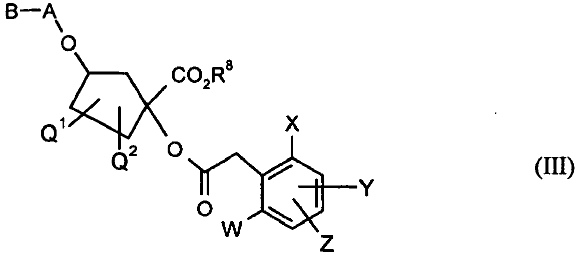 in welcher
   A, B, Q¹, Q², W, X, Y, Z und R⁸ die oben angegebenen Bedeutungen haben,
   in Gegenwart eines Verdünnungsmittels und in Gegenwart einer Base intramolekular kondensiert.
   Außerdem wurde gefunden
(C) dass man die Verbindungen der oben gezeigten Formeln (I-1-b) bis (I-2-b), in welchen R¹, A, B, Q¹, Q²,W, X, Y und Z die oben angegebenen Bedeutungen haben, erhält, wenn man Verbindungen der oben gezeigten Formeln (I-1-a) bis (I-2-a), in welchen A, B, Q¹, Q², W, X, Y und Z die oben angegebenen Bedeutungen haben, jeweils
   α) mit Verbindungen der Formel (IV) 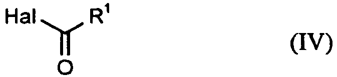 in welcher
      - R¹: die oben angegebene Bedeutung hat und
      - Hal: für Halogen (insbesondere Chlor oder Brom) steht
      oder
   ß) mit Carbonsäureanhydriden der Formel (V)

      R¹-CO-O-CO-R¹ (V)

      in welcher
      - R¹: die oben angegebene Bedeutung hat,
   gegebenenfalls in Gegenwart eines Verdünnungsmittels und gegebenenfalls in Gegenwart eines Säurebindemittels umsetzt;
(D) dass man die Verbindungen der oben gezeigten Formeln (I-1-c) bis (I-2-c), in welchen R², A, B, Q¹, Q², W, M, X, Y und Z die oben angegebenen Bedeutungen haben und L für Sauerstoff steht, erhält, wenn man Verbindungen der oben gezeigten Formeln (I-1-a) bis (I-2-a), in welchen A, B, Q¹, Q², W, X, Y und Z die oben angegebenen Bedeutungen haben, jeweils
   mit Chlorameisensäureestern oder Chlorameisensäurethioestern der Formel (VI)

   R²-M-CO-Cl (VI)

   in welcher
   R² und M die oben angegebenen Bedeutungen haben,
   gegebenenfalls in Gegenwart eines Verdünnungsmittels und gegebenenfalls in Gegenwart eines Säurebindemittels umsetzt;
(E) dass man Verbindungen der oben gezeigten Formeln (I-1-c) bis (I-2-c), in welchen R², A, B, Q¹, Q², W, M, X, Y und Z die oben angegebenen Bedeutungen haben und L für Schwefel steht, erhält, wenn man Verbindungen der oben gezeigten Formeln (I-1-a) bis (I-2-a), in welchen A, B, Q¹, Q², W, X, Y und Z die oben angegebenen Bedeutungen haben, jeweils
   mit Chlormonothioameisensäureestern oder Chlordithioameisensäureestern der Formel (VII) 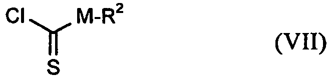 in welcher
   M und R² die oben angegebenen Bedeutungen haben,
   gegebenenfalls in Gegenwart eines Verdünnungsmittels und gegebenenfalls in Gegenwart eines Säurebindemittels umsetzt,
(F) dass man Verbindungen der oben gezeigten Formeln (I-1-d) bis (I-2-d), in welchen R³, A, B, W, Q¹, Q², X, Y und Z die oben angegebenen Bedeutungen haben, erhält, wenn man Verbindungen der oben gezeigten Formeln (I-1-a) bis (I-2-a), in welchen A, B, Q¹, Q², W, X, Y und Z die oben angegebenen Bedeutungen haben, jeweils
   mit Sulfonsäurechloriden der Formel (VIII)

   R³-SO₂-Cl (VIII)

   in welcher
   - R³: die oben angegebene Bedeutung hat,
   gegebenenfalls in Gegenwart eines Verdünnungsmittels und gegebenenfalls in Gegenwart eines Säurebindemittels umsetzt,
(G) dass man Verbindungen der oben gezeigten Formeln (I-1-e) bis (I-2-e), in welchen L, R⁴, R⁵, A, B, Q¹, Q², W, X, Y und Z die oben angegebenen Bedeutungen haben, erhält, wenn man Verbindungen der oben gezeigten Formeln (I-1-a) bis (I-2-a), in welchen A, B, Q¹, Q², W, X, Y und Z die oben angegebenen Bedeutungen haben, jeweils
   mit Phosphorverbindungen der Formel (IX) 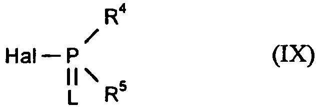 in welcher
   - L, R⁴ und R⁵: die oben angegebenen Bedeutungen haben und
   - Hal: für Halogen (insbesondere Chlor oder Brom) steht,
   gegebenenfalls in Gegenwart eines Verdünnungsmittels und gegebenenfalls in Gegenwart eines Säurebindemittels umsetzt,
(H) dass man Verbindungen der oben gezeigten Formeln (I-1-f) bis (1-2-f), in welchen E, A, B, Q¹, Q², W, X, Y und Z die oben angegebenen Bedeutungen haben, erhält, wenn man Verbindungen der oben gezeigten Formeln (I-1-a) bis (I-2-a), in welchen A, B, Q¹, Q², W, X, Y und Z die oben angegebenen Bedeutungen haben, jeweils mit Metallverbindungen oder Aminen der Formeln (X) oder (XI)

   Me(OR¹⁰)ₜ (X)

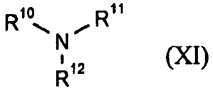

   in welchen
   - Me: für ein ein- oder zweiwertiges Metall (bevorzugt ein Alkali- oder Erdalkalimetall wie Lithium, Natrium, Kalium, Magnesium oder Calcium),
   - t: für die Zahl 1 oder 2 und
   - R¹⁰, R¹¹, R¹²: unabhängig voneinander für Wasserstoff oder Alkyl (bevorzugt C₁-C₈-Alkyl) stehen,
   gegebenenfalls in Gegenwart eines Verdünnungsmittels umsetzt,
(I) dass man Verbindungen der oben gezeigten Formeln (I-1-g) bis (I-2-g), in welchen L, R⁶, R⁷, A, B, Q¹, Q², W, X, Y und Z die oben angegebenen Bedeutungen haben, erhält, wenn man Verbindungen der oben gezeigten Formeln (I-1-a) bis (I-2-a), in welchen A, B, Q¹, Q², W, X, Y und Z die oben angegebenen Bedeutungen haben, jeweils
   α) mit Isocyanaten oder Isothiocyanaten der Formel (XII)

      R⁶-N=C=L (XII)

      in welcher
      R⁶ und L die oben angegebenen Bedeutungen haben,
      gegebenenfalls in Gegenwart eines Verdünnungsmittels und gegebenenfalls in Gegenwart eines Katalysators umsetzt oder
   ß) mit Carbamidsäurechloriden oder Thiocarbamidsäurechloriden der Formel (XIII) 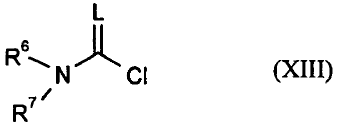 in welcher
      - L, R⁶ und R⁷: die oben angegebenen Bedeutungen haben,
   gegebenenfalls in Gegenwart eines Verdünnungsmittels und gegebenenfalls in Gegenwart eines Säurebindemittels, umsetzt.

Weiterhin wurde gefunden, dass die neuen Verbindungen der Formel (I) eine gute Wirksamkeit als Schädlingsbekämpfungsmittel, vorzugsweise als Insektizide, Akarizide und/oder Fungizide und/oder Herbizide aufweisen und darüber hinaus häufig sehr gut pflanzenverträglich, insbesondere gegenüber Kulturpflanzen, sind.

Überraschenderweise wurde nun auch gefunden, dass bestimmte substituierte, cyclische Ketoenole bei gemeinsamer Anwendung mit den im weiteren beschriebenen, die Kulturpflanzen-Verträglichkeit verbessernden Verbindungen (Safenem/Antidots) ausgesprochen gut die Schädigung der Kulturpflanzen verhindern und besonders vorteilhaft als breit wirksame Kombinationspräparate zur selektiven Bekämpfung von unerwünschten Pflanzen in Nutzpflanzenkulturen, wie z.B. in Getreide aber auch Mais, Soja und Reis, verwendet werden können.

Gegenstand der Erfindung sind auch selektiv-herbizide Mittel enthaltend einen wirksamen Gehalt an einer Wirkstoffkombination umfassend als Komponenten
(a') mindestens eine Verbindung der Formel (I), in welcher A, B, D, G, Q¹, Q², W, X, Y und Z die oben angegebene Bedeutung haben
   und
(b') zumindest eine die Kulturpflanzen-Verträglichkeit verbessernde Verbindung aus der folgenden Gruppe von Verbindungen:
   4-Dichloracetyl-1-oxa-4-aza-spiro[4.5]-decan (AD-67, MON-4660), 1-Dichloracetyl-hexahydro-3,3,8a-trimethylpyrrolo[1,2-a]-pyrimidin-6(2H)-on (Dicyclonon, BAS-145138), 4-Dichloracetyl-3,4-dihydro-3-methyl-2H-1,4-benzoxazin (Benoxacor), 5-Chlor-chinolin-8-oxy-essigsäure-(1-methyl-hexylester) (Cloquintocet-mexyl - vgl. auch verwandte Verbindungen in EP-A-86750, EP-A-94349, EP-A-191736, EP-A-492366), 3-(2-Chlor-benzyl)-1-(1-methyl-1-phenyl-ethyl)-harnstoff (Cumyluron), α-(Cyanomethoximino)-phenylacetonitril (Cyometrinil), 2,4-Dichlor-phenoxyessigsäure (2,4-D), 4-(2,4-Dichlor-phenoxy)-buttersäure (2,4-DB), 1-(1-Methyl-1-phenyl-ethyl)-3-(4-methyl-phenyl)-harnstoff (Daimuron, Dymron), 3,6-Dichlor-2-methoxy-benzoesäure (Dicamba), Piperidin-1-thiocarbonsäure-S-1-methyl-1-phenyl-ethylester (Dimepiperate), 2,2-Dichlor-N-(2-oxo-2-(2-propenylamino)-ethyl)-N-(2-propenyl)-acetamid (DKA-24), 2,2-Dichlor-N,N-di-2-propenyl-acetamid (Dichlormid), 4,6-Dichlor-2-phenyl-pyrimidin (Fenclorim), 1-(2,4-Dichlor-phenyl)-5-trichlormethyl-1H-1,2,4-triazol-3-carbonsäure-ethylester (Fenchlorazole-ethyl - vgl. auch verwandte Verbindungen in EP-A-174562 und EP-A-346620), 2-Chlor-4-trifluormethyl-thiazol-5-carbonsäure-phenylmethylester (Flurazole), 4-Chlor-N-(1,3-dioxolan-2-yl-methoxy)-α-trifluor-acetophenonoxim (Fluxofenim), 3-Dichloracetyl-5-(2-furanyl)-2,2-dimethyl-oxazolidin (Furilazole, MON-13900), Ethyl-4,5-dihydro-5,5-diphenyl-3-isoxazolcarboxylat (Isoxadifen-ethyl - vgl. auch verwandte Verbindungen in WO-A-95/07897), 1-(Ethoxycarbonyl)-ethyl-3,6-dichlor-2-methoxybenzoat (Lactidichlor), (4-Chlor-o-tolyloxy)-essigsäure (MCPA), 2-(4-Chlor-o-tolyloxy)-propionsäure (Mecoprop), Diethyl-1-(2,4-dichlor-phenyl)-4,5-dihydro-5-methyl-1H-pyrazol-3,5-di-carboxylat (Mefenpyr-diethyl - vgl. auch verwandte Verbindungen in WO-A-91/07874) 2-Dichlormethyl-2-methyl-1,3-dioxolan (MG-191), 2-Propenyl-1-oxa-4-azaspiro[4.5]decane-4-carbodithioate (MG-838), 1,8-Naphthalsäureanhydrid, α-(1,3-Dioxolan-2-yl-methoximino)-phenylacetonitril (Oxabetrinil), 2,2-Dichlor-N-(1,3-dioxolan-2-yl-methyl)-N-(2-propenyl)-acetamid (PPG-1292), 3-Dichloracetyl-2,2-dimethyl-oxazolidin (R-28725), 3-Dichloracetyl-2,2,5-trimethyl-oxazolidin (R-29148), 4-(4-Chlor-o-tolyl)-buttersäure, 4-(4-Chlor-phenoxy)-buttersäure, Diphenylmethoxyessigsäure, Diphenylmethoxyessigsäure-methylester, Diphenylmethoxyessigsäure-ethylester, 1-(2-Chlor-phenyl)-5-phenyl-1H-pyrazol-3-carbonsäure-methylester, 1-(2,4-Dichlor-phenyl) 5-methyl-1H-pyrazol-3-carbonsäure-ethylester, 1-(2,4-Dichlor-phenyl)-5-isopropyl-1H-pyrazol-3-carbonsäure-ethylester, 1-(2,4-Dichlor-phenyl)-5-(1,1-dimethyl-ethyl)-1H-pyrazol-3-carbonsäure-ethylester, 1-(2,4-Dichlor-phenyl)-5-phenyl-1H-pyrazol-3-carbonsäure-ethylester (vgl. auch verwandte Verbindungen in EP-A-269806 und EP-A-333131), 5-(2,4-Dichlor-benzyl)-2-isoxazolin-3-carbonsäure-ethylester, 5-Phenyl-2-isoxazolin-3-carbonsäure-ethylester, 5-(4-Fluor-phenyl)-5-phenyl-2-isoxazolin-3-carbonsäure-ethylester (vgl. auch verwandte Verbindungen in WO-A-91/08202), 5-Chlor-chinolin-8-oxy-essigsäure-(1,3-dimethyl-but-1-yl)-ester, 5-Chlor-chinolin-8-oxy-essigsäure-4-allyloxy-butylester, 5-Chlor-chinolin-8-oxy-essigsäure-1-allyloxy-prop-2-yl-ester, 5-Chlor-chinoxalin-8-oxy-essigsäure-methylester, 5-Chlor-chinolin-8-oxy-essigsäure-ethylester, 5-Chlor-chinoxalin-8-oxy-essigsäure-allylester, 5-Chlor-chinolin-8-oxy-essigsäure-2-oxo-prop-1-yl-ester, 5-Chlor-chinolin-8-oxy-malonsäure-diethylester, 5-Chlor-chinoxalin-8-oxy-malonsäure-di-allylester, 5-Chlor-chinolin-8-oxy-malonsäure-diethylester (vgl. auch verwandte Verbindungen in EP-A-582198), 4-Carboxy-chroman-4-yl-essigsäure (AC-304415, vgl. EP-A-613618), 4-Chlor-phenoxy-essigsäure, 3,3`-Dimethyl-4-methoxy-benzophenon, 1-Brom-4-chlormethylsulfonyl-benzol, 1-[4-(N-2-Methoxybenzoylsulfamoyl)-phenyl]-3-methyl-harnstoff (alias N-(2-Methoxy-benzoyl)-4-[(methylamino-carbonyl)-amino]-benzolsulfonamid), 1-[4-(N-2-Methoxybenzoyl-sulfamoyl)-phenyl]-3,3-dimethyl-harnstoff, 1-[4-(N-4,5-Dimethylbenzoylsulfamoyl)-phenyl]-3-methyl-harnstoff, 1-[4-(N-Naphthylsulfamoyl)-phenyl]-3,3-dimethyl-harnstoff, N-(2-Methoxy-5-methyl-benzoyl)-4-(cyclopropylaminocarbonyl)-benzolsulfonamid,
   und/oder eine der folgenden durch allgemeine Formeln definierten Verbindungen
      der allgemeinen Formel (IIa) 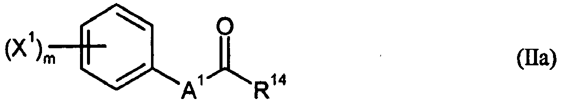 oder der allgemeinen Formel (IIb) 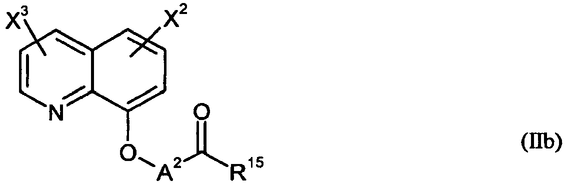 oder der Formel (IIc) 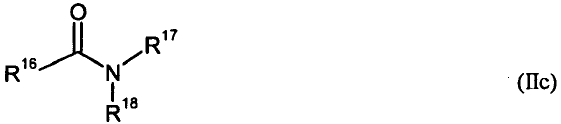 wobei
      - m: für eine Zahl 0, 1, 2, 3, 4 oder 5 steht,
      - A¹: für eine der nachstehend skizzierten divalenten heterocyclischen Gruppierungen steht, 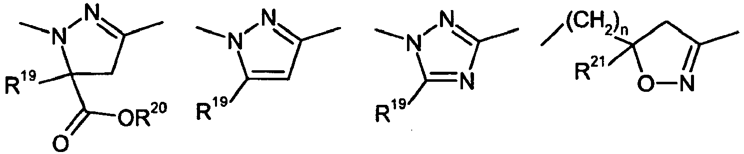
      - n: für eine Zahl 0, 1, 2, 3, 4 oder 5 steht,
      - A²: für gegebenenfalls durch C₁-C₄-Alkyl und/oder C₁-C₄-Alkoxy-carbonyl und/oder C₁-C₄-Alkenyloxy-carbonyl substituiertes Alkandiyl mit 1 oder 2 Kohlenstoffatomen steht,
      - R¹⁴: für Hydroxy, Mercapto, Amino, C₁-C₆-Alkoxy, C₁-C₆-Alkylthio, C₁-C₆-Alkylamino oder Di-(C₁-C₄-alkyl)-amino steht,
      - R¹⁵: für Hydroxy, Mercapto, Amino, C₁-C₇-Alkoxy, C₁-C₆-Alkenyloxy, C₁-C₆-Alkenyloxy-C₁-C₆-alkoxy, C₁-C₆-Alkylthio, C₁-C₆-Alkylamino oder Di-(C₁-C₄-alkyl)-amino steht,
      - R¹⁶: für gegebenenfalls durch Fluor, Chlor und/oder Brom substituiertes C₁-C₄-Alkyl steht,
      - R¹⁷: für Wasserstoff, jeweils gegebenenfalls durch Fluor, Chlor und/oder Brom substituiertes C₁-C₆-Alkyl, C₂-C₆-Alkenyl oder C₂-C₆-Alkinyl, C₁-C₄-Alkoxy-C₁-C₄-alkyl, Dioxolanyl-C₁-C₄-alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl steht,
      - R¹⁸: für Wasserstoff, jeweils gegebenenfalls durch Fluor, Chlor und/oder Brom substituiertes C₁-C₆-Alkyl, C₂-C₆-Alkenyl oder C₂-C₆-Alkinyl, C₁-C₄-Alkoxy-C₁-C₄-alkyl, Dioxolanyl-C₁-C₄-alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl steht, R¹⁷ und R¹⁸ auch gemeinsam für jeweils gegebenenfalls durch C₁-C₄-Alkyl, Phenyl, Furyl, einen annellierten Benzolring oder durch zwei Substituenten, die gemeinsam mit dem C-Atom, an das sie gebunden sind, einen 5- oder 6-gliedrigen Carboxyclus bilden, substituiertes C₃-C₆-Alkandiyl oder C₂-C₅-Oxaalkandiyl steht,
      - R¹⁹: für Wasserstoff, Cyano, Halogen, oder für jeweils gegebenenfalls durch Fluor, Chlor und/oder Brom substituiertes C₁-C₄-Alkyl, C₃-C₆-Cycloalkyl oder Phenyl steht,
      - R²⁰: für Wasserstoff, jeweils gegebenenfalls durch Hydroxy, Cyano, Halogen oder C₁-C₄-Alkoxy substituiertes C₁-C₆-Alkyl, C₃-C₆-Cycloalkyl oder Tri-(C₁-C₄-alkyl)-silyl steht,
      - R²¹: für Wasserstoff, Cyano, Halogen, oder für jeweils gegebenenfalls durch Fluor, Chlor und/oder Brom substituiertes C₁-C₄-Alkyl, C₃-C₆-Cycloalkyl oder Phenyl steht,
      - X¹: für Nitro, Cyano, Halogen, C₁-C₄-Alkyl, C₁-C₄-Halogenalkyl, C₁-C₄-Alkoxy oder C₁-C₄-Halogenalkoxy steht,
      - X²: für Wasserstoff, Cyano, Nitro, Halogen, C₁-C₄-Alkyl, C₁-C₄-Halogenalkyl, C₁-C₄-Alkoxy oder C₁-C₄-Halogenalkoxy steht,
      - X³: für Wasserstoff, Cyano, Nitro, Halogen, C₁-C₄-Alkyl, C₁-C₄-Halogenalkyl, C₁-C₄-Alkoxy oder C₁-C₄-Halogenalkoxy steht,
      und/oder die folgenden durch allgemeine Formeln definierten Verbindungen
      der allgemeinen Formel (IId) 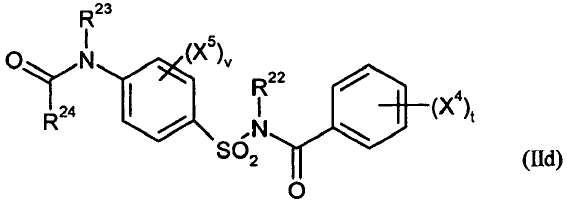 oder der allgemeinen Formel (IIe) 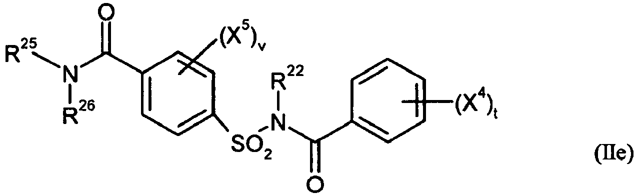 wobei
      - t: für eine Zahl 0, 1, 2, 3, 4 oder 5 steht,
      - v: für eine Zahl 0, 1, 2, 3, 4 oder 5 steht,
      - R²²: für Wasserstoff oder C₁-C₄-Alkyl steht,
      - R²³: für Wasserstoff oder C₁-C₄-Alkyl steht,
      - R²⁴: für Wasserstoff, jeweils gegebenenfalls durch Cyano, Halogen oder C₁-C₄-Alkoxy substituiertes C₁-C₆-Alkyl, C₁-C₆-Alkoxy, C₁-C₆-Alkylthio, C₁-C₆-Alkylamino oder Di-(C₁-C₄-alkyl)-amino, oder jeweils gegebenenfalls durch Cyano, Halogen oder C₁-C₄-Alkyl substituiertes C₃-C₆-Cycloalkyl, C₃-C₆-Cycloalkyloxy, C₃-C₆-Cycloalkylthio oder C₃-C₆-Cycloalkylamino steht,
      - R²⁵: für Wasserstoff, gegebenenfalls durch Cyano, Hydroxy, Halogen oder C₁-C₄-Alkoxy substituiertes C₁-C₆-Alkyl, jeweils gegebenenfalls durch Cyano oder Halogen substituiertes C₃-C₆-Alkenyl oder C₃-C₆-Alkinyl, oder gegebenenfalls durch Cyano, Halogen oder C₁-C₄-Alkyl substituiertes C₃-C₆-Cycloalkyl steht,
      - R²⁶: für Wasserstoff, gegebenenfalls durch Cyano, Hydroxy, Halogen oder C₁-C₄-Alkoxy substituiertes C₁-C₆-Alkyl, jeweils gegebenenfalls durch Cyano oder Halogen substituiertes C₃-C₆-Alkenyl oder C₃-C₆-Alkinyl, gegebenenfalls durch Cyano, Halogen oder C₁-C₄-Alkyl substituiertes C₃-C₆-Cycloalkyl, oder gegebenenfalls durch Nitro, Cyano, Halogen, C₁-C₄-Alkyl, C₁-C₄-Halogenalkyl, C₁-C₄-Alkoxy oder C₁-C₄-Halogenalkoxy substituiertes Phenyl steht, oder zusammen mit R²⁵ für jeweils gegebenenfalls durch C₁-C₄-Alkyl substituiertes C₂-C₆-Alkandiyl oder C₂-C₆-Oxaalkandiyl steht,
      - X⁴: für Nitro, Cyano, Carboxy, Carbamoyl, Formyl, Sulfamoyl, Hydroxy, Amino, Halogen, C₁-C₄-Alkyl, C₁-C₄-Halogenalkyl, C₁-C₄-Alkoxy oder C₁-C₄-Halogenalkoxy steht, und
      - X⁵: für Nitro, Cyano, Carboxy, Carbamoyl, Formyl, Sulfamoyl, Hydroxy, Amino, Halogen, C₁-C₄-Alkyl, C₁-C₄-Halogenalkyl, C₁-C₄-Alkoxy oder C₁-C₄-Halogenalkoxy steht.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im Folgenden erläutert:
- W: steht bevorzugt für Wasserstoff, C₁-C₆-Alkyl, C₂-C₆-Alkenyl, C₂-C₆-Alkinyl, Halogen, C₁-C₆-Alkoxy, C₁-C₄-Halogenalkyl, C₁-C₄-Halogenalkoxy oder Cyano,
- X: steht bevorzugt für Halogen, C₁-C₆-Alkyl, C₂-C₆-Alkenyl, C₂-C₆-Alkinyl, C₁-C₆-Alkoxy, C₁-C₆-Alkoxy-C₁-C₄-alkoxy, C₁-C₄-Halogenalkyl, C₁-C₄-Halogenalkoxy oder Cyano,
- Y: steht bevorzugt für Wasserstoff, Halogen, C₁-C₆-Alkyl, C₂-C₆-Alkenyl, C₂-C₆-Alkinyl, C₁-C₆-Alkoxy, Cyano, C₁-C₄-Halogenalkyl, Halogenalkoxy, für durch V¹ und V² substituierten Phenyl oder Pyridyl,
- V¹: steht bevorzugt für Halogen, C₁-C₁₂-Alkyl, C₁-C₆-Alkoxy, C₁-C₄-Halogenalkyl, C₁-C₄-Halogenalkoxy, Cyano oder Nitro,
- V²: steht bevorzugt für Wasserstoff, Halogen, C₁-C₆-Alkyl, C₁-C₆-Alkoxy, C₁-C₄-Halogen-alkyl,
- V¹ und V²: stehen gemeinsam bevorzugt für C₃-C₄-Alkandiyl, welches gegebenenfalls durch Halogen und/oder C₁-C₂-Alkyl substituiert sein kann und welches gegebenenfalls durch ein oder zwei Sauerstoffatome unterbrochen sein kann,
- Z: steht bevorzugt für Wasserstoff, Halogen, C₁-C₆-Alkyl, C₁-C₄-Halogenalkyl, Cyano, C₁-C₆-Alkoxy oder C₁-C₄-Halogenalkoxy,
- A: steht bevorzugt für eine gegebenenfalls durch C₁-C₄-Alkyl substituierte C₁-C₄-Alkandiylgruppe oder für gegebenenfalls durch C₁-C₄-Alkyl substituiertes C₅-C₈-Cycloalkyl, in welchem gegebenenfalls eine Methylengruppe durch Sauerstoff ersetzt ist,
- B: steht bevorzugt für Wasserstoff oder für jeweils gegebenenfalls durch Halogen substituiertes C₁-C₈-Alkyl, C₂-C₈-Alkenyl, C₁-C₆-Alkoxy, C₁-C₆-Alkoxy-C₁-C₄-alkoxy, C₁-C₄-Alkoxy-bis-C₁-C₄-alkoxy, für gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl, für gegebenenfalls durch Halogen, C₁-C₄-Alkyl oder C₁-C₂-Halogenalkyl substituiertes Pyridyl, Pyrimidyl, Thiazolyl oder Thienyl oder für gegebenenfalls durch Halogen, C₁-C₄-Alkyl, C₁-C₄-Alkoxy oder C₁-C₂-Halogenalkyl substituiertes C₃-C₈-Cycloalkyl, in welchem gegebenenfalls eine oder zwei nicht direkt benachbarte Methylengruppen durch Sauerstoff ersetzt, zwei Methylengruppen durch den Rest -O-CO- oder drei Methylengruppen durch den Rest -O-CO-O- ersetzt sind,
oder A bevorzugt für eine Bindung und B für Wasserstoff steht,
- D: steht bevorzugt für NH oder Sauerstoff,
- Q¹: steht bevorzugt für Wasserstoff oder für jeweils gegebenenfalls durch Halogen substituiertes C₁-C₆₋Alkyl, C₁-C₆-Alkoxy, C₁-C₄-Alkoxy-C₁-C₄-alkyl, C₁-C₄-Alkylthio-C₁-C₄-alkyl oder für gegebenenfalls durch Halogen, C₁-C₄-Alkyl oder C₁-C₄-Alkoxy substituiertes C₃-C₆-Cycloalkyl worin gegebenenfalls eine Methylengruppe durch Sauerstoff ersetzt ist oder für jeweils gegebenenfalls einfach bis zweifach durch Halogen, C₁-C₄-Alkyl, C₁-C₄-Alkoxy, C₁-C₄-Halogenalkyl oder C₁-C₄-Halogenalkoxy substituiertes Phenyl, Phenyl-C₁-C₂-alkyl oder Hetaryl,
- Q²: steht bevorzugt für Wasserstoff oder C₁-C₆-Alkyl, oder
- Q¹ und Q²: stehen gemeinsam mit dem Kohlenstoff, an das sie gebunden sind bevorzugt für einen gegebenenfalls einfach bis zweifach durch Fluor, Chlor, C₁-C₄-Alkyl, C₁-C₄-Alkoxy oder Trifluormethyl substituierten C₃-C₆-Ring, worin gegebenenfalls eine Methylengruppe durch Sauerstoff ersetzt sein kann, oder
- Q¹ und Q²: stehen gemeinsam mit den Kohlenstoffatomen, an die sie gebunden sind, bevorzugt für einen gegebenenfalls einfach bis zweifach durch Fluor, Chlor, C₁-C₄-Alkyl, C₁-C₄-Alkoxy oder Trifluormethyl substituierten C₃-C₆-Ring, worin gegebenenfalls eine Methylengruppe durch Sauerstoff ersetzt sein kann.
- G: steht bevorzugt für Wasserstoff (a) oder für eine der Gruppen 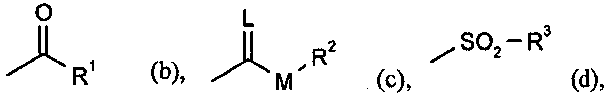 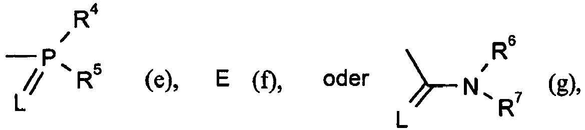 in welchen
E für ein Metallion oder ein Ammoniumion steht,
L für Sauerstoff oder Schwefel steht und
M für Sauerstoff oder Schwefel steht.
- R¹: steht bevorzugt für jeweils gegebenenfalls durch Halogen oder Cyano substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl oder Poly-C₁-C₈-alkoxy-C₁-C₈-alkyl oder für gegebenenfalls durch Halogen, C₁-C₆-Alkyl oder C₁-C₆-Alkoxy substituiertes C₃-C₈-Cycloalkyl, in welchem gegebenenfalls eine oder zwei nicht direkt benachbarte Methylengruppen durch Sauerstoff und/oder Schwefel ersetzt sind,
für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkylsulfonyl substituiertes Phenyl,
für gegebenenfalls durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls durch Halogen oder C₁-C₆-Alkyl substituiertes 5- oder 6-gliedriges Hetaryl mit ein oder zwei Heteroatomen aus der Reihe Sauerstoff, Schwefel und Stickstoff,
für gegebenenfalls durch Halogen oder C₁-C₆-Alkyl substituiertes Phenoxy-C₁-C₆-alkyl oder
für gegebenenfalls durch Halogen, Amino oder C₁-C₆-Alkyl substituiertes 5- oder 6-gliedriges Hetaryloxy-C₁-C₆-alkyl mit ein oder zwei Heteroatomen aus der Reihe Sauerstoff, Schwefel und Stickstoff.
- R²: steht bevorzugt für jeweils gegebenenfalls durch Halogen oder Cyano substituiertes C₁-C₂₀-Alkyl, C₂-C₂₀-Alkenyl, C₁-C₈-Alkoxy-C₂-C₈-alkyl oder Poly-C₁-C₈-alkoxy-C₂-C₈-alkyl,
für gegebenenfalls durch Halogen, C₁-C₆-Alkyl oder C₁-C₆-Alkoxy substituiertes C₃-C₈-Cycloalkyl oder
für jeweils gegebenenfalls durch Halogen, Cyano, Nitro, C₁-C₆-Alkyl, C₁-C₆-Alkoxy, C₁-C₆-Halogenalkyl oder C₁-C₆-Halogenalkoxy substituiertes Phenyl oder Benzyl.
- R³: steht bevorzugt für gegebenenfalls durch Halogen substituiertes C₁-C₈-Alkyl oder jeweils gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Benzyl.
- R⁴ und R⁵: stehen unabhängig voneinander bevorzugt für jeweils gegebenenfalls durch Halogen substituiertes C₁-C₈-Alkyl, C₁-C₈-Alkoxy, C₁-C₈-Alkylamino, Di-(C₁-C₈-alkyl)amino, C₁-C₈-Alkylthio oder C₃-C₈-Alkenylthio oder für jeweils gegebenenfalls durch Halogen, Nitro, Cyano, C₁-C₄-Alkoxy, C₁-C₄-Halogenalkoxy, C₁-C₄-Alkylthio, C₁-C₄-Halogen-alkylthio, C₁-C₄-Alkyl oder C₁-C₄-Halogenalkyl substituiertes Phenyl, Phenoxy oder Phenylthio.
- R⁶ und R⁷: stehen unabhängig voneinander bevorzugt für Wasserstoff, für jeweils gegebenenfalls durch Halogen oder Cyano substituiertes C₁-C₈-Alkyl, C₃-C₈-Cycloalkyl, C₁-C₈-Alkoxy, C₃-C₈-Alkenyl oder C₁-C₈-Alkoxy-C₂-C₈-alkyl, für jeweils gegebenenfalls durch Halogen, C₁-C₈-Alkyl, C₁-C₈-Halogenalkyl oder C₁-C₈-Alkoxy substituiertes Phenyl oder Benzyl oder zusammen für einen gegebenenfalls durch C₁-C₆-Alkyl substituierten C₃-C₆-Alkylenrest, in welchem gegebenenfalls eine Methylengruppe durch Sauerstoff oder Schwefel ersetzt ist.

In den als bevorzugt genannten Restedefinitionen steht Halogen für Fluor, Chlor, Brom und Iod, insbesondere für Fluor, Chlor und Brom.
- W: steht besonders bevorzugt für Wasserstoff, Chlor, Brom, Iod, C₁-C₄-Alkyl, C₂-C₄-Alkenyl, C₂-C₄-Alkinyl, C₁-C₄-Alkoxy, C₁-C₂-Halogenalkyl oder C₁-C₂-Halogenalkoxy,
- X: steht besonders bevorzugt für Chlor, Brom, Iod, C₁-C₄-Alkyl, C₂-C₄-Alkenyl, C₂-C₄-Alkinyl, C₁-C₄-Alkoxy, C₁-C₄-Alkoxy-C₁-C₃-alkoxy, C₁-C₂-Halogenalkyl, C₁-C₂-Halogenalkoxy oder Cyano,
- Y: steht besonders bevorzugt in der 4-Position für Wasserstoff, Fluor, Chlor, Brom, Iod, Methoxy, Ethoxy, Cyano, Trifluormethyl, Difluormethoxy oder Trifluormethoxy,
- Z: steht besonders bevorzugt für Wasserstoff.
- W: steht auch besonders bevorzugt für Wasserstoff, Chlor, Brom oder C₁-C₄-Alkyl,
- X: steht auch besonders bevorzugt für Chlor, Brom, C₁-C₄-Aikyl, C₁-C₄-Alkoxy, C₁-C₂-Halogenalkyl, C₁-C₂-Halogenalkoxy oder Cyano,
- Y: steht auch besonders bevorzugt in der 4-Position für C₂-C₄-Alkenyl, C₂-C₄-Alkinyl oder für den Rest 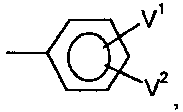
- Z: steht auch besonders bevorzugt für Wasserstoff,
- V¹: steht auch besonders bevorzugt für Fluor, Chlor, C₁-C₄-Alkyl, C₁-C₄-Alkoxy, C₁-C₂-Halogenalkyl oder C₁-C₂-Halogenalkoxy,
- V²: steht auch besonders bevorzugt für Wasserstoff, Fluor, Chlor, C₁-C₄-Alkyl, C₁-C₄-Alkoxy oder C₁-C₂-Halogenalkyl,
- V¹ und V²: stehen gemeinsam auch besonders bevorzugt für-O-CH₂-O- und -O-CF₂-O-.
- W: steht ebenfalls besonders bevorzugt für Wasserstoff, Chlor, Brom oder C₁-C₄-Alkyl,
- X: steht ebenfalls besonders bevorzugt für Chlor, Brom, C₁-C₄-Alkyl oder C₁-C₂-Halogenalkyl,
- Y: steht ebenfalls besonders bevorzugt in der 5-Position für C₂-C₄-Alkenyl, C₂-C₄-Alkinyl, für den Rest 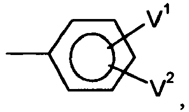
- Z: steht ebenfalls besonders bevorzugt in der 4-Position für Wasserstoff, C₁-C₄-Alkyl oder Chlor,
- V¹: steht ebenfalls besonders bevorzugt für Fluor, Chlor, C₁-C₄-Alkyl, C₁-C₄-Alkoxy, C₁-C₂-Halogenalkyl oder C₁-C₂-Halogenalkoxy,
- V²: steht ebenfalls besonders bevorzugt für Wasserstoff, Fluor, Chlor, C₁-C₄-Alkyl, C₁-C₄-Alkoxy oder C₁-C₂-Halogenalkyl,
- V¹ und V²: stehen gemeinsam ebenfalls besonders bevorzugt für-O-CH₂-O- oder -O-CF₂-O-.
- W: steht außerdem besonders bevorzugt für Wasserstoff, C₁-C₄-Alkyl, C₂-C₄-Alkenyl, C₂-C₄-Alkinyl, C₁-C₄-Alkoxy, Chlor, Brom, Iod oder Trifluormethyl,
- X: steht außerdem besonders bevorzugt für Chlor, Brom, Iod, C₁-C₄-Alkyl, C₂-C₄-Alkenyl, C₂-C₄-Alkinyl, C₁-C₄-Alkoxy, C₁-C₄-Alkoxy-C₁-C₃-alkoxy, C₁-C₂-Halogenalkyl, C₁-C₂-Halogenalkoxy oder Cyano,
- Y: steht außerdem besonders bevorzugt in der 4-Position für C₁-C₄-Alkyl,
- Z: steht außerdem besonders bevorzugt für Wasserstoff.
- W: steht weiterhin besonders bevorzugt für Wasserstoff, Chlor, Brom, Iod, C₁-C₄-Alkyl oder C₁-C₄-Alkoxy,
- X: steht weiterhin besonders bevorzugt für Chlor, Brom, Iod, C₁-C₄-Alkyl, C₁-C₄-Alkoxy, C₁-C₂-Halogenalkyl, C₁-C₂-Halogenalkoxy oder Cyano,
- Y: steht weiterhin besonders bevorzugt in der 4-Position für Wasserstoff, Chlor, Brom, C₁-C₄-Alkyl, C₁-C₂-Halogenalkyl oder C₁-C₂-Halogenalkoxy,
- Z: steht weiterhin besonders bevorzugt in der 3- oder 5-Position für Fluor, Chlor, Brom, Iod, C₁-C₄-Alkyl, C₁-C₂-Halogenalkyl, C₁-C₄-Alkoxy oder C₁-C₂-Halogenalkoxy.
- A: steht besonders bevorzugt für eine gegebenenfalls durch C₁-C₂-Alkyl substituierte C₁-C₃-Alkandiylgruppe oder für C₅-C₆-Cycloalkyl in welchen gegebenenfalls eine Methylengruppe durch Sauerstoff ersetzt ist.
- B: steht besonders bevorzugt für Wasserstoff oder jeweils gegebenenfalls einfach bis dreifach durch Fluor oder Chlor substituiertes C₁-C₆-Alkyl, C₂-C₆-Alkenyl, C₁-C₄-Alkoxy, C₁-C₄-Alkoxy-C₁-C₃-alkoxy, C₁-C₄-Alkoxy-bis-C₁-C₃-alkoxy, für gegebenenfalls einfach bis dreifach durch Fluor, Chlor, Brom, C₁-C₄-Alkyl, C₁-C₄-Alkoxy, C₁-C₂-Halogenalkyl, C₁-C₂-Halogenalkoxy, Cyano oder Nitro substituiertes Phenyl, für gegebenenfalls einfach bis zweifach durch Fluor, Chlor, Brom, Methyl, Ethyl oder Trifluormethyl substituiertes Pyridyl, Pyrimidyl, Thiazolyl oder Thienyl oder für gegebenenfalls einfach bis zweifach durch Fluor, Chlor, Methyl, Methoxy oder Trifluormethyl substituiertes C₃-C₆-Cycloalkyl, in welchem gegebenenfalls eine Methylengruppe oder zwei nicht direkt benachbarte Methylengruppen durch Sauerstoff ersetzt sind,
oder A besonders bevorzugt für eine Bindung und B für Wasserstoff steht.
- D: steht besonders bevorzugt für NH oder Sauerstoff.
- Q¹: steht besonders bevorzugt für Wasserstoff, für gegebenenfalls einfach bis dreifach durch Fluor substituiertes C₁-C₄-Alkyl,
- Q²: steht besonders bevorzugt für Wasserstoff oder C₁-C₄-Alkyl.
- Q¹ und Q²: stehen gemeinsam mit dem Kohlenstoffatom, an das sie gebunden sind, besonders bevorzugt für einen gegebenenfalls einfach durch Fluor, Methyl, Methoxy oder Trifluor-methyl substituierten C₃-C₆-Ring, worin gegebenenfalls eine Methylengruppe durch Sauerstoff ersetzt sein kann, oder
- Q¹ und Q²: stehen gemeinsam mit den Kohlenstoffatomen, an die sie gebunden sind, besonders bevorzugt für einen gegebenenfalls einfach durch Fluor, Methyl, Methoxy oder Trifluor-methyl substituierten C₃-C₆-Ring, worin gegebenenfalls eine Methylengruppe durch Sauerstoff ersetzt sein kann.
- G: steht besonders bevorzugt für Wasserstoff (a) oder für eine der Gruppen 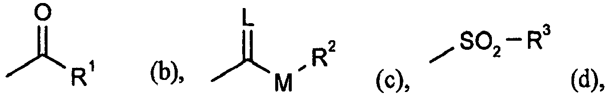 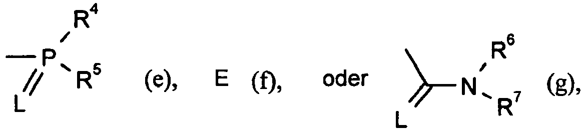 in welchen
E für ein Metallion oder ein Ammoniumion steht,
L für Sauerstoff oder Schwefel steht und
M für Sauerstoff oder Schwefel steht.
- R¹: steht besonders bevorzugt für jeweils gegebenenfalls einfach bis dreifach durch Fluor oder Chlor substituiertes C₁-C₁₆-Alkyl, C₂-C₁₆₋Alkenyl, C₁-C₆-Alkoxy-C₁-C₄-alkyl, C₁-C₆-Alkylthio-C₁-C₄-alkyl oder Poly-C₁-C₆-alkoxy-C₁-C₄-alkyl oder für gegebenenfalls einfach bis zweifach durch Fluor, Chlor, C₁-C₅-Alkyl oder C₁-C₅-Alkoxy substituiertes C₃-C₇-Cycloalkyl, in welchem gegebenenfalls eine oder zwei nicht direkt benachbarte Methylengruppen durch Sauerstoff und/oder Schwefel ersetzt sind,
für gegebenenfalls einfach bis dreifach durch Fluor, Chlor, Brom, Cyano, Nitro, C₁-C₄-Alkyl, C₁-C₄-Alkoxy, C₁-C₃-Halogenalkyl, C₁-C₃-Halogenalkoxy, C₁-C₄-Alkylthio oder C₁-C₄-Alkylsulfonyl substituiertes Phenyl,
für gegebenenfalls einfach bis zweifach durch Fluor, Chlor, Brom, C₁-C₄-Alkyl, C₁-C₄-Alkoxy, C₁-C₃-Halogenatkyl oder C₁-C₃-Halogenalkoxy substituiertes Phenyl-C₁-C₄-alkyl,
für jeweils gegebenenfalls einfach bis zweifach durch Fluor, Chlor, Brom oder C₁-C₄-Alkyl substituiertes Pyrazolyl, Thiazolyl, Pyridyl, Pyrimidyl, Furanyl oder Thienyl,
für gegebenenfalls einfach bis zweifach durch Fluor, Chlor, Brom oder C₁-C₄-Alkyl substituiertes Phenoxy-C₁-C₅-alkyl oder
für jeweils gegebenenfalls einfach bis zweifach durch Fluor, Chlor, Brom, Amino oder C₁-C₄-Alkyl substituiertes Pyridyloxy-C₁-C₅-alkyl, Pyrimidyloxy-C₁-C₅-alkyl oder Thiazolyloxy-C₁-C₅-alkyl.
- R²: steht besonders bevorzugt für jeweils gegebenenfalls einfach bis dreifach durch Fluor oder Chlor substituiertes C₁-C₁₆-Alkyl, C₂-C₁₆₋Alkenyl, C₁-C₆-Alkoxy-C₂-C₆-alkyl oder Poly-C₁-C₆-alkoxy-C₂-C₆-alkyl,
für gegebenenfalls einfach bis zweifach durch Fluor, Chlor, C₁-C₄-Alkyl oder C₁-C₄-Alkoxy substituiertes C₃-C₇-Cycloalkyl oder
für jeweils gegebenenfalls einfach bis dreifach durch Fluor, Chlor, Brom, Cyano, Nitro, C₁-C₄-Alkyl, C₁-C₃-Alkoxy, C₁-C₃-Halogenalkyl oder C₁-C₃-Halogenalkoxy substituiertes Phenyl oder Benzyl.
- R³: steht besonders bevorzugt für gegebenenfalls einfach bis dreifach durch Fluor oder Chlor substituiertes C₁-C₆-Alkyl oder jeweils gegebenenfalls einfach bis zweifach durch Fluor, Chlor, Brom, C₁-C₄-Alkyl, C₁-C₄-Alkoxy, C₁-C₂-Halogenalkoxy, C₁-C₂-Halogenalkyl, Cyano oder Nitro substituiertes Phenyl oder Benzyl.
- R⁴ und R⁵: stehen unabhängig voneinander besonders bevorzugt für jeweils gegebenenfalls einfach bis dreifach durch Fluor oder Chlor substituiertes C₁-C₆-Alkyl, C₁-C₆-Alkoxy, C₁-C₆-Alkylamino, Di-(C₁-C₆-alkyl)amino, C₁-C₆-Alkylthio oder C₃-C₄-Alkenylthio oder für jeweils gegebenenfalls einfach bis zweifach durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl oder C₁-C₃-Halogenalkyl substituiertes Phenyl, Phenoxy oder Phenylthio.
- R⁶ und R⁷: stehen unabhängig voneinander besonders bevorzugt für Wasserstoff, für jeweils gegebenenfalls einfach bis dreifach durch Fluor oder Chlor substituiertes C₁-C₆-Alkyl, C₃-C₆-Cycloalkyl, C₁-C₆-Alkoxy, C₃-C₆-Alkenyl oder C₁-C₆-Alkoxy-C₂-C₆-alkyl, für jeweils gegebenenfalls einfach bis dreifach durch Fluor, Chlor, Brom, C₁-C₅-Halo-genalkyl, C₁-C₅-Alkyl oder C₁-C₅-Alkoxy substituiertes Phenyl oder Benzyl, oder zusammen für einen gegebenenfalls durch C₁-C₄-Alkyl substituierten C₃-C₆-Alkylenrest, in welchem gegebenenfalls eine Methylengruppe durch Sauerstoff oder Schwefel ersetzt ist.

In den als besonders bevorzugt genannten Restedefinitionen steht Halogen für Fluor, Chlor und Brom, insbesondere für Fluor und Chlor.
- W: steht ganz besonders bevorzugt für Wasserstoff, Chlor, Brom, Iod, Methyl, Ethyl, Methoxy, Ethoxy oder Trifluormethyl,
- X: steht ganz besonders bevorzugt für Chlor, Brom, Iod, Methyl, Ethyl, Propyl, Methoxy, Ethoxy, Propoxy, Methoxy-ethoxy, Ethoxy-ethoxy, Trifluormethyl, Difluormethoxy, Trifluormethoxy oder Cyano,
- Y: steht ganz besonders bevorzugt in der 4-Position für Wasserstoff, Chlor, Brom, lod, Trifluormethyl oder Trifluormethoxy,
- Z: steht ganz besonders bevorzugt für Wasserstoff.
- W: steht auch ganz besonders bevorzugt für Wasserstoff, Chlor, Brom, Methyl oder Ethyl,
- X: steht auch ganz besonders bevorzugt für Chlor, Brom, Methyl, Ethyl, Propyl, Methoxy, Trifluormethyl, Difluormethoxy oder Cyano,
- Y: steht auch ganz besonders bevorzugt in der 4-Position für Vinyl, Ethinyl, Propinyl oder für den Rest 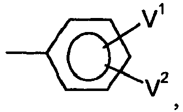
- Z: steht auch ganz besonders bevorzugt für Wasserstoff,
- V¹: steht auch ganz besonders bevorzugt für Fluor, Chlor, Methyl, Methoxy, Trifluormethyl oder Trifluormethoxy,
- V²: steht auch ganz besonders bevorzugt für Wasserstoff, Fluor, Chlor, Methyl, Methoxy oder Trifluormethyl.
- W: steht ebenfalls ganz besonders bevorzugt für Wasserstoff, Chlor oder Methyl,
- X: steht ebenfalls ganz besonders bevorzugt für Chlor, Methyl oder Trifluormethyl,
- Y: steht ebenfalls ganz besonders bevorzugt in der 5-Position für Vinyl, Ethinyl, Propinyl oder für den Rest 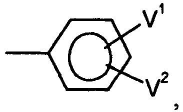
- Z: steht ebenfalls ganz besonders bevorzugt in der 4-Position für Wasserstoff oder Methyl,
- V¹: steht ebenfalls ganz besonders bevorzugt für Fluor, Chlor, Methyl, Methoxy, Trifluor-methyl oder Trifluormethoxy,
- V²: steht ebenfalls ganz besonders bevorzugt für Wasserstoff, Fluor, Chlor, Methyl, Methoxy oder Trifluormethyl.
- W: steht außerdem ganz besonders bevorzugt für Wasserstoff, Methyl, Ethyl, Methoxy, Ethoxy, Chlor, Brom oder Iod,
- X: steht außerdem ganz besonders bevorzugt für Chlor, Brom, Iod, Methyl, Ethyl, Propyl, Methoxy, Ethoxy, Propoxy, Methoxy-ethoxy, Ethoxy-ethoxy, Trifluormethyl, Difluor-methoxy, Trifluormethoxy oder Cyano,
- Y: steht außerdem ganz besonders bevorzugt in der 4-Position für Methyl oder Ethyl,
- Z: steht außerdem ganz besonders bevorzugt für Wasserstoff.
- W: steht weiterhin ganz besonders bevorzugt für Wasserstoff, Chlor, Brom, Iod, Methyl oder Ethyl,
- X: steht weiterhin ganz besonders bevorzugt für Chlor, Brom, Iod, Methyl, Ethyl, Methoxy, Trifluormethyl, Difluormethoxy oder Trifluormethoxy,
- Y: steht weiterhin ganz besonders bevorzugt in der 4-Position für Wasserstoff, Chlor, Brom, Iod, Methyl oder Ethyl,
- Z: steht weiterhin ganz besonders bevorzugt in der 3- oder 5-Position für Fluor, Chlor, Brom, Iod, Methyl, Ethyl, Trifluormethyl oder Trifluormethoxy.
- A: steht ganz besonders bevorzugt für -CH₂-, -CHCH₃-, -CH₂-CH₂-, -CH₂-CHCH₃-, -CH₂-CH₂-CH₂-.
- B: steht ganz besonders bevorzugt für Wasserstoff, Methyl, Ethyl, Propyl, iso-Propyl, Butyl, iso-Butyl, C₂-C₄-Alkenyl, Methoxy, Ethoxy, Propoxy, Isopropoxy, Butoxy, Isobutoxy, Methoxy-ethoxy, Ethoxy-ethoxy, für gegebenenfalls einfach bis zweifach durch Fluor, Chlor, Brom, Methyl, Methoxy, Trifluormethyl, Trifluormethoxy, Cyano oder Nitro substituiertes Phenyl, für Cyclopropyl, für Cyclopentyl oder Cyclohexyl, in welchem gegebenenfalls eine Methylengruppe durch Sauerstoff ersetzt ist,
oder A ganz besonders bevorzugt für eine Bindung und B für Wasserstoff steht.
- D: steht ganz besonders bevorzugt für NH oder Sauerstoff.
- Q¹: steht ganz besonders bevorzugt für Wasserstoff, Methyl oder Ethyl.
- Q²: steht ganz besonders bevorzugt für Wasserstoff, Methyl oder Ethyl.
- Q¹ und Q²: stehen ganz besonders bevorzugt gemeinsam mit dem Kohlenstoffatom, an das sie gebunden sind für Cyclopropyl, Cyclopentyl oder Cyclohexyl, oder
- Q¹ und Q²: stehen ganz besonders bevorzugt gemeinsam mit den Kohlenstoffatomen, an die sie gebunden sind, für einen gegebenenfalls durch Sauerstoff unterbrochenen C₅-C₆-Ring.
- G: steht ganz besonders bevorzugt für Wasserstoff (a) oder für eine der Gruppen 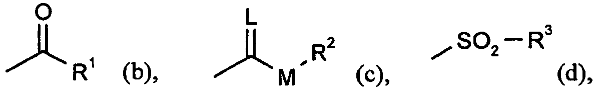 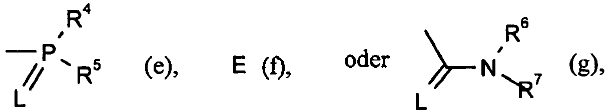 in welchen
E für ein Metallion oder ein Ammoniumion steht,
L für Sauerstoff oder Schwefel steht und
M für Sauerstoff oder Schwefel steht.
- R¹: steht ganz besonders bevorzugt für jeweils gegebenenfalls einfach bis dreifach durch Fluor oder Chlor substituiertes C₁-C₁₀-Alkyl, C₂-C₁₀-Alkenyl, C₁-C₄-Alkoxy-C₁-C₂-alkyl, C₁-C₄-Alkylthio-C₁-C₂-alkyl oder für gegebenenfalls einfach durch Fluor, Chlor, Methyl, Ethyl oder Methoxy substituiertes C₃-C₆-Cycloalkyl,
für gegebenenfalls einfach bis zweifach durch Fluor, Chlor, Brom, Cyano, Nitro, Methyl, Ethyl, n-Propyl, i-Propyl, Methoxy, Ethoxy, Trifluormethyl oder Trifluormethoxy substituiertes Phenyl,
für jeweils gegebenenfalls einfach durch Chlor, Brom oder Methyl substituiertes Furanyl, Thienyl oder Pyridyl.
- R²: steht ganz besonders bevorzugt für jeweils gegebenenfalls einfach bis dreifach durch Fluor oder Chlor substituiertes C₁-C₁₀-Alkyl, C₂-C₁₀-Alkenyl oder C₁-C₄-Alkoxy-C₂-C₄-alkyl,
für Cyclopentyl oder Cyclohexyl
oder für jeweils Gegebenenfalls einfach bis zweifach durch Fluor, Chlor, Cyano, Nitro, Methyl, Ethyl, Methoxy, Trifluormethyl oder Trifluormethoxy substituiertes Phenyl oder Benzyl.
- R³: steht ganz besonders bevorzugt für jeweils gegebenenfalls einfach bis dreifach durch Fluor oder Chlor substituiertes Methyl, Ethyl, Propyl oder iso-Propyl, oder gegebenenfalls einfach durch Fluor, Chlor, Brom, Methyl, Ethyl, iso-Propyl, tert.-Butyl, Methoxy, Ethoxy, iso-Propoxy, Trifluormethyl, Trifluormethoxy, Cyano oder Nitro substituiertes Phenyl.
- R⁴ und R⁵: stehen unabhängig voneinander ganz besonders bevorzugt für C₁-C₄-Alkoxy oder C₁-C₄-Alkylthio oder für jeweils gegebenenfalls einfach durch Fluor, Chlor, Brom, Nitro, Cyano, Methyl, Methoxy, Trifluormethyl oder Trifluormethoxy substituiertes Phenyl, Phenoxy oder Phenylthio.
- R⁶ und R⁷: stehen unabhängig voneinander ganz besonders bevorzugt für Wasserstoff, für C₁-C₄-Alkyl, C₃-C₆-Cycloalkyl, C₁-C₄-Alkoxy, C₃-C₄-Alkenyl oder C₁-C₄-Alkoxy-C₂-C₄-alkyl, für gegebenenfalls einfach bis zweifach durch Fluor, Chlor, Brom, Methyl, Methoxy oder Trifluormethyl substituiertes Phenyl, oder zusammen für einen C₅-C₆-Alkylenrest, in welchem gegebenenfalls eine Methylengruppe durch Sauerstoff oder Schwefel ersetzt ist.
- W: steht insbesondere bevorzugt für Methyl, Ethyl oder Methoxy,
- X: steht insbesondere bevorzugt für Chlor, Methyl, Ethyl oder Methoxy,
- Y: steht insbesondere bevorzugt in der 4-Position für Chlor oder Brom,
- Z: steht insbesondere bevorzugt für Wasserstoff.
- W: steht ebenfalls insbesondere bevorzugt für Wasserstoff,
- X: steht ebenfalls insbesondere bevorzugt für Methyl,
- Y: steht ebenfalls insbesondere bevorzugt in der 5-Position für den Rest, 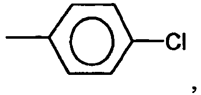
- Z: steht insbesondere bevorzugt in der 4-Position für Wasserstoff.
- W: steht außerdem insbesondere bevorzugt für Methyl oder Ethyl,
- X: steht außerdem insbesondere bevorzugt für Chlor, Brom oder Methyl,
- Y: steht außerdem insbesondere bevorzugt in der 4-Position für Methyl,
- Z: steht außerdem insbesondere bevorzugt für Wasserstoff.
- A: steht insbesondere bevorzugt für -CH₂- oder -CH₂-CH₂-,
- B: steht insbesondere bevorzugt für Wasserstoff, Methyl, Ethyl, Propyl, Methoxy oder Cyclopropyl,
oder A insbesondere bevorzugt für eine Bindung und B für Wasserstoff steht.
- D: steht insbesondere bevorzugt für NH.
- Q¹: steht insbesondere bevorzugt für Wasserstoff.
- Q²: steht insbesondere bevorzugt für Wasserstoff.
- G: steht insbesondere bevorzugt für Wasserstoff (a) oder für eine der Gruppen 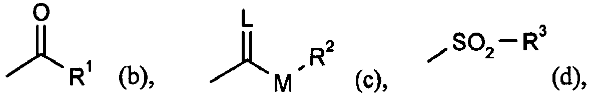 E (f), oder 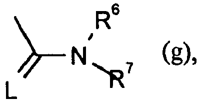 hervorgehoben bevorzugt sind Wasserstoff, die Gruppen (b) und (c),
in welchen
E für ein Metallion steht,
L für Sauerstoff steht und
M für Sauerstoff steht.
- R¹: steht insbesondere bevorzugt für C₁-C₁₀-Alkyl,
für gegebenenfalls einfach durch Chlor substituiertes Phenyl,
- R²: steht insbesondere bevorzugt für C₁-C₁₀-Alkyl oder C₂-C₁₀-Alkenyl,
- R³: steht insbesondere bevorzugt für Methyl.
- R⁶ und R⁷: stehen insbesondere bevorzugt zusammen für einen C₅-C₆-Alkylenrest, in welchem gegebenenfalls eine Methylengruppe durch Sauerstoff ersetzt ist.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Erfindungsgemäß insbesondere bevorzugt werden die Verbindungen der Formel (I), in welchen eine Kombination der vorstehend als insbesondere bevorzugt aufgeführten Bedeutungen vorliegt.

Gesättigte oder ungesättigte Kohlenwasserstoffreste wie Alkyl, Alkandiyl oder Alkenyl können, auch in Verbindung mit Heteroatomen, wie z.B. in Alkoxy, soweit möglich, jeweils geradkettig oder verzweigt sein.

Gegebenenfalls substituierte Reste können, sofern nicht anderes angegeben ist, einfach oder mehrfach substituiert sein, wobei bei Mehrfachsubstitutionen die Substituenten gleich oder verschieden sein können.

Im einzelnen seien außer den bei den Herstellungsbeispielen genannten Verbindungen die folgenden Verbindungen der Formel (I-I-a) genannt:

**Tabelle 1**

| | | | | | |
|---|---|---|---|---|---|
| 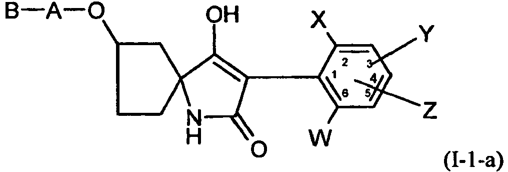 | | | | | |

| **A** | **B** | **X** | **W** | **Y** | **Z** |
|---|---|---|---|---|---|
| CH₂ | H | CH₃ | H | H | H |
| CH₂ | H | Br | H | H | H |
| CH₂ | H | Cl | H | H | H |
| CH₂ | H | CF₃ | H | H | H |
| CH₂ | H | OCH₃ | H | H | H |
| CH₂ | H | OC₂H₅ | H | H | H |
| CH₂ | H | Br | H | 4-Cl | H |
| CH₂ | H | Cl | H | 4-Br | H |
| CH₂ | H | Cl | H | 4-Cl | H |
| CH₂ | H | Cl | H | 4-CH₃ | H |
| CH₂ | H | CH₃ | H | 4-Cl | H |
| CH₂ | H | CH₃ | H | 4-CH₃ | H |
| CH₂ | H | Cl | Cl | H | H |
| CH₂ | H | Cl | OCH₃ | H | H |
| CH₂ | H | Cl | CH₃ | H | H |
| CH₂ | H | Cl | OC₂H₅ | H | H |
| CH₂ | H | OCH₃ | OCH₃ | H | H |
| CH₂ | H | CH₃ | CH₃ | H | H |
| CH₂ | H | Br | CH₃ | 4-Br | H |
| CH₂ | H | Cl | Cl | 4-CH₃ | H |
| CH₂ | H | CH₃ | Br | 4-CH₃ | H |
| CH₂ | H | CH₃ | Cl | 4-CH₃ | H |
| CH₂ | H | OCH₃ | CH₃ | 4-CH₃ | H |
| CH₂ | H | OC₂H₅ | CH₃ | 4-CH₃ | H |
| CH₂ | H | OC₃H₇ | CH₃ | 4-CH₃ | H |
| CH₂ | H | CH₃ | CH₃ | 4-CH₃ | H |
| CH₂ | H | Br | Br | 4-CH₃ | H |
| CH₂ | H | CH₃ | CH₃ | 4-Br | H |
| CH₂ | H | C₂H₅ | CH₃ | H | H |
| CH₂ | H | C₂H₅ | C₂H₅ | H | H |
| CH₂ | H | OCH₃ | C₂H₅ | 4-CH₃ | H |
| CH₂ | H | CH₃ | CH₃ | 4-OCH₃ | H |
| CH₂ | H | Br | Cl | 4-CH₃ | H |
| CH₂ | H | Br | CH₃ | 4-Cl | H |
| CH₂ | H | Cl | CH₃ | 4-Br | H |
| CH₂ | H | CH₃ | CH₃ | 4-Cl | H |
| CH₂ | H | C₂H₅ | CH₃ | 4-CH₃ | H |
| CH₂ | H | C₂H₅ | CH₃ | 4-C₂H₅ | H |
| CH₂ | H | C₂H₅ | C₂H₅ | 4-CH₃ | H |
| CH₂ | H | C₂H₅ | C₂H₅ | 4-C₂H₅ | H |
| CH₂ | H | C₂H₅ | CH₃ | 4-Cl | H |
| CH₂ | H | C₂H₅ | C₂H₅ | 4-Cl | H |
| CH₂ | H | C₂H₅ | CH₃ | 4-Br | H |
| CH₂ | H | C₂H₅ | C₂H₅ | 4-Br | H |
| CH₂ | H | C₂H₅ | Cl | 4-CH₃ | H |
| CH₂ | H | C₂H₅ | Br | 4-CH₃ | H |
| CH₂ | H | C₂H₅ | Cl | 4-Cl | H |
| CH₂ | H | C₂H₅ | Br | 4-Br | H |
| CH₂ | H | C₂H₅ | Cl | 4-Br | H |
| CH₂ | H | C₂H₅ | Br | 4-Cl | H |
| CH₂ | H | OCH₃ | CH₃ | 4-Cl | H |
| CH₂ | H | OCH₃ | C₂H₅ | 4-Cl | H |
| CH₂ | H | OC₂H₅ | CH₃ | 4-Cl | H |
| CH₂ | H | OC₂H₅ | C₂H₅ | 4-Cl | H |
| CH₂ | H | Cl | OCH₃ | 4-CH₃ | H |
| CH₂ | H | Cl | OC₂H₅ | 4-CH₃ | H |
| CH₂ | H | Cl | Cl | 4-Cl | H |
| CH₂ | H | Cl | H | 4-Cl | 5-Cl |
| CH₂ | H | CH₃ | H | 4-CH₃ | 5-CH₃ |
| CH₂ | H | CH₃ | H | 4-Cl | 5-CH₃ |
| CH₂ | H | Br | H | 4-Cl | 5-CH₃ |
| CH₂ | H | Br | H | 4-CH₃ | 5-CH₃ |
| CH₂ | H | Cl | H | 4-Br | 5-CH₃ |
| CH₂ | H | Cl | H | 4-Cl | 5-CH₃ |
| CH₂ | H | CH₃ | H | 4-Br | 5-CH₃ |
| CH₂ | H | Cl | H | 4-CH₃ | 5-Cl |
| CH₂ | H | CH₃ | H | H | 5-CH₃ |
| CH₂ | H | Cl | H | H | 5-CH₃ |
| CH₂ | H | Br | H | H | 5-CH₃ |
| CH₂ | H | CH₃ | H | H | 5-Cl |
| CH₂ | H | CH₃ | H | H | 5-Br |
| CH₂ | H | CH₃ | CH₃ | 4-CH₃ | 5-CH₃ |
| CH₂ | H | CH₃ | CH₃ | 4-CH₃ | 5-Cl |
| CH₂ | H | CH₃ | CH₃ | 4-CH₃ | 5-Br |
| CH₂ | H | CH₃ | CH₃ | H | 3-Cl |
| CH₂ | H | CH₃ | CH₃ | H | 3-Br |
| CH₂ | H | Cl | Cl | H | 3-Br |
| CH₂ | H | CH₃ | CH₃ | 4-(4-Cl-C₆H₄) | H |
| CH₂ | H | C₂H₅ | CH₃ | 4-(4-Cl-C₆H₄) | H |
| CH₂ | H | C₂H₅ | C₂H₅ | 4-(4-Cl-C₆H₄) | H |
| CH₂ | H | Cl | CH₃ | 4-(4-Cl-C₆H₄) | H |
| CH₂ | H | Cl | C₂H₅ | 4-(4-Cl-C₆H₄) | H |
| CH₂ | H | CH₃ | H | 5-(4-Cl-C₆H₄) | H |
| CH₂ | H | CH₃ | CH₃ | 5-(4-Cl-C₆H₄) | H |
| CH₂ | H | CH₃ | H | 5-(4-Cl-C₆H₄) | 4-CH₃ |
| CH₂ | H | CH₃ | CH₃ | 5-(4-Cl-C₆H₄) | 4-CH₃ |
| CH₂ | H | Cl | H | 5-(4-Cl-C₆H₄) | H |
| CH₂ | H | O-(CH₂)₂-OCH₃ | CH₃ | 4-Cl | H |
| CH₂ | H | O-(CH₂)₂-OCH₃ | C₂H₅ | 4-Cl | H |
| CH₂ | H | O-CH₃ | CH₃ | 4-Br | H |
| CH₂ | H | O-CH₃ | C₂H₅ | 4-Br | H |
| CH₂ | H | O-C₂H₅ | CH₃ | 4-Br | H |
| CH₂ | H | O-C₂H₅ | C₂H₅ | 4-Br | H |
| CH₂ | H | J | H | H | H |
| CH₂ | H | J | H | 4-CH₃ | H |
| CH₂ | H | J | CH₃ | H | H |
| CH₂ | H | J | C₂H₅ | H | H |
| CH₂ | H | CH₃ | H | H | 5-J |
| CH₂ | H | CH₃ | H | 4-CH₃ | 5-J |
| CH₂ | H | J | CH₃ | 4-CH₃ | H |
| CH₂ | H | J | C₂H₅ | 4-CH₃ | H |
| CH₂ | H | J | CH₃ | 4-Cl | H |
| CH₂ | H | J | C₂H₅ | 4-Cl | H |
| CH₂ | H | J | Cl | 4- CH₃ | H |
| CH₂ | H | J | H | 4- CH₃ | 5- CH₃ |
| CH₂ | H | CH₃ | H | 4-J | H |
| CH₂ | H | C₂H₅ | H | 4-J | H |
| CH₂ | H | CH₃ | CH₃ | 4-J | H |
| CH₂ | H | C₂H₅ | CH₃ | 4-J | H |
| CH₂ | H | C₂H₅ | C₂H₅ | 4-J | H |
| CH₂ | H | Cl | CH₃ | 4-J | H |
| CH₂ | H | Cl | C₂H₅ | 4-J | H |
| CH₂ | H | CH₃ | H | 4-J | 5-CH₃ |
| CH₂ | H | CH₃ | CH₃ | H | 3-J |
| CH₂ | H | J | H | H | 5-CH₃ |

**Tabelle 2:**

| | |
|---|---|
| | A, W, X, Y und Z wie in Tabelle 1 angegeben |
| | B = CH₃ |

**Tabelle 3:**

| | |
|---|---|
| | A, W, X, Y und Z wie in Tabelle 1 angegeben |
| | B = C₂H₅ |

**Tabelle 4:**

| | |
|---|---|
| | A, W, X, Y und Z wie in Tabelle 1 angegeben |
| | B = C₃H₇ |

**Tabelle 5:**

| | |
|---|---|
| | A, W, X, Y und Z wie in Tabelle 1 angegeben |
| | B = i-C₃H₇ |

**Tabelle 6:**

| | |
|---|---|
| A, W, X, Y und Z wie in Tabelle 1 angegeben | |
| B = | 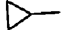 |

**Tabelle 7:**

| | |
|---|---|
| A, W, X, Y und Z wie in Tabelle 1 angegeben | |
| B = | 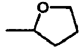 |

**Tabelle 8:**

| | |
|---|---|
| | A, W, X, Y und Z wie in Tabelle 1 angegeben |
| | A = -CH₂-CH₂-; B = OCH₃ |

**Tabelle 9:**

| | |
|---|---|
| | A, W, X, Y und Z wie in Tabelle 1 angegeben |
| | A = -CH₂-CH₂-_{;} B = OC₂H₅ |

Im einzelnen seien außer den bei den Herstellungsbeispielen genannten Verbindungen die folgenden Verbindungen der Formel (I-2-a) genannt:

**Tabelle 10**

| | | | | | |
|---|---|---|---|---|---|
| 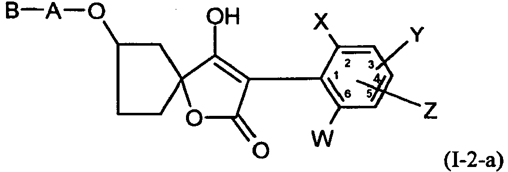 | | | | | |

| **A** | **B** | **X** | **W** | **Y** | **Z** |
|---|---|---|---|---|---|
| CH₂ | H | CH₃ | H | H | H |
| CH₂ | H | Br | H | H | H |
| CH₂ | H | Cl | H | H | H |
| CH₂ | H | CF₃ | H | H | H |
| CH₂ | H | OCH₃ | H | H | H |
| CH₂ | H | Br | H | 4-Cl | H |
| CH₂ | H | Cl | H | 4-Br | H |
| CH₂ | H | Cl | H | 4-Cl | H |
| CH₂ | H | Cl | H | 4-CH₃ | H |
| CH₂ | H | CH₃ | H | 4-Cl | H |
| CH₂ | H | CH₃ | H | 4-CH₃ | H |
| CH₂ | H | Cl | Cl | H | H |
| CH₂ | H | Cl | OCH₃ | H | H |
| CH₂ | H | Cl | CH₃ | H | H |
| CH₂ | H | Cl | OC₂H₅ | H | H |
| CH₂ | H | OCH₃ | OCH₃ | H | H |
| CH₂ | H | CH₃ | CH₃ | H | H |
| CH₂ | H | C₂H₅ | CH₃ | H | H |
| CH₂ | H | C₂H₅ | C₂H₅ | H | H |
| CH₂ | H | Br | CH₃ | 4-Br | H |
| CH₂ | H | Cl | Cl | 4-CH₃ | H |
| CH₂ | H | CH₃ | Br | 4-CH₃ | H |
| CH₂ | H | CH₃ | Cl | 4-CH₃ | H |
| CH₂ | H | OCH₃ | CH₃ | 4-CH₃ | H |
| CH₂ | H | OCH₃ | C₂H₅ | 4-CH₃ | H |
| CH₂ | H | OC₂H₅ | CH₃ | 4-CH₃ | H |
| CH₂ | H | OC₃H₇ | CH₃ | 4-CH₃ | H |
| CH₂ | H | CH₃ | CH₃ | 4-CH₃ | H |
| CH₂ | H | Br | Br | 4-CH₃ | H |
| CH₂ | H | Cl | Cl | 4-CH₃ | H |
| CH₂ | H | CH₃ | CH₃ | 4-Br | H |
| CH₂ | H | CH₃ | CH₃ | 4-OCH₃ | H |
| CH₂ | H | Br | Cl | 4-CH₃ | H |
| CH₂ | H | Br | CH₃ | 4-Cl | H |
| CH₂ | H | Cl | CH₃ | 4-Br | H |
| CH₂ | H | CH₃ | CH₃ | 4-Cl | H |
| CH₂ | H | C₂H₅ | CH₃ | 4-CH₃ | H |
| CH₂ | H | C₂H₅ | C₂H₅ | 4-CH₃ | H |
| CH₂ | H | C₂H₅ | CH₃ | 4-C₂H₅ | H |
| CH₂ | H | C₂H₅ | C₂H₅ | 4-C₂H₅ | H |
| CH₂ | H | C₂H₅ | CH₃ | 4-Cl | H |
| CH₂ | H | C₂H₅ | C₂H₅ | 4-Cl | H |
| CH₂ | H | C₂H₅ | CH₃ | 4-Br | H |
| CH₂ | H | C₂H₅ | C₂H₅ | 4-Br | H |
| CH₂ | H | C₂H₅ | Cl | 4-CH₃ | H |
| CH₂ | H | C₂H₅ | Br | 4-CH₃ | H |
| CH₂ | H | C₂H₅ | Cl | 4-Cl | H |
| CH₂ | H | C₂H₅ | Br | 4-Br | H |
| CH₂ | H | C₂H₅ | Cl | 4-Br | H |
| CH₂ | H | C₂H₅ | Br | 4-Cl | H |
| CH₂ | H | OCH₃ | CH₃ | 4-Cl | H |
| CH₂ | H | OCH₃ | C₂H₅ | 4-Cl | H |
| CH₂ | H | OC₂H₅ | CH₃ | 4-Cl | H |
| CH₂ | H | OC₂H₅ | C₂H₅ | 4-Cl | H |
| CH₂ | H | Cl | OCH₃ | 4-CH₃ | H |
| CH₂ | H | Cl | OC₂H₅ | 4-CH₃ | H |
| CH₂ | H | CH₃ | CH₃ | 4-Cl | H |
| CH₂ | H | Cl | H | 4-Cl | 5-Cl |
| CH₂ | H | CH₃ | H | 4-CH₃ | 5-CH₃ |
| CH₂ | H | CH₃ | H | 4-Cl | 5-CH₃ |
| CH₂ | H | Br | H | 4-Cl | 5-CH₃ |
| CH₂ | H | Br | H | 4-CH₃ | 5-CH₃ |
| CH₂ | H | Cl | H | 4-Br | 5-CH₃ |
| CH₂ | H | Cl | H | 4-Cl | 5-CH₃ |
| CH₂ | H | CH₃ | H | 4-Br | 5-CH₃ |
| CH₂ | H | Cl | H | 4-CH₃ | 5-Cl |
| CH₂ | H | CH₃ | H | H | 5-CH₃ |
| CH₂ | H | Cl | H | H | 5-CH₃ |
| CH₂ | H | Br | H | H | 5-CH₃ |
| CH₂ | H | CH₃ | H | H | 5-Cl |
| CH₂ | H | CH₃ | H | H | 5-Br |
| CH₂ | H | CH₃ | CH₃ | 4-CH₃ | 5-CH₃ |
| CH₂ | H | CH₃ | CH₃ | 4-CH₃ | 5-Cl |
| CH₂ | H | CH₃ | CH₃ | 4-CH₃ | 5-Br |
| CH₂ | H | CH₃ | CH₃ | H | 3-Cl |
| CH₂ | H | CH₃ | CH₃ | H | 3-Br |
| CH₂ | H | Cl | Cl | H | 3-Br |
| CH₂ | H | CH₃ | CH₃ | 4-(4-Cl-C₆H₄) | H |
| CH₂ | H | C₂H₅ | CH₃ | 4-(4-Cl-C₆H₄) | H |
| CH₂ | H | C₂H₅ | C₂H₅ | 4-(4-Cl-C₆H₄) | H |
| CH₂ | H | Cl | CH₃ | 4-(4-Cl-C₆H₄) | H |
| CH₂ | H | Cl | C₂H₅ | 4-(4-Cl-C₆H₄) | H |
| CH₂ | H | CH₃ | H | 5-(4-Cl-C₆H₄) | H |
| CH₂ | H | CH₃ | CH₃ | 5-(4-Cl-C₆H₄) | H |
| CH₂ | H | CH₃ | H | 5-(4-Cl-C₆H₄) | 4-CH₃ |
| CH₂ | H | CH₃ | CH₃ | 5-(4-Cl-C₆H₄) | 4-CH₃ |
| CH₂ | H | Cl | H | 5-(4-Cl-C₆H₄) | H |
| CH₂ | H | J | H | H | H |
| CH₂ | H | J | H | 4-CH₃ | H |
| CH₂ | H | J | CH₃ | H | H |
| CH₂ | H | J | C₂H₅ | H | H |
| CH₂ | H | CH₃ | H | H | 5-J |
| CH₂ | H | CH₃ | H | 4-CH₃ | 5-J |
| CH₂ | H | J | CH₃ | 4-CH₃ | H |
| CH₂ | H | J | C₂H₅ | 4-CH₃ | H |
| CH₂ | H | J | CH₃ | 4-Cl | H |
| CH₂ | H | J | C₂H | 4-Cl | H |
| CH₂ | H | J | Cl | 4-CH₃ | H |
| CH₂ | H | J | H | 4-CH₃ | 5-CH₃ |
| CH₂ | H | CH₃ | H | 4-J | H |
| CH₂ | H | C₂H₅ | H | 4-J | H |
| CH₂ | H | CH₃ | CH₃ | 4-J | H |
| CH₂ | H | C₂H₅ | CH₃ | 4-J | H |
| CH₂ | H | C₂H₅ | C₂H₅ | 4-J | H |
| CH₂ | H | Cl | CH₃ | 4-J | H |
| CH₂ | H | Cl | C₂H₅ | 4-J | H |
| CH₂ | H | CH₃ | H | 4-J | 5-CH₃ |
| CH₂ | H | CH₃ | CH₃ | H | 3-J |
| CH₂ | H | J | H | H | 5-CH₃ |

**Tabelle 11:**

| | |
|---|---|
| | A, W, X, Y und Z wie in Tabelle 10 angegeben |
| | B = CH₃ |

**Tabelle 12:**

| | |
|---|---|
| | A, W, X, Y und Z wie in Tabelle 10 angegeben |
| | B = C₂H₅ |

**Tabelle 13:**

| | |
|---|---|
| | A, W, X, Y und Z wie in Tabelle 10 angegeben |
| | B = C₃H₇ |

**Tabelle 14:**

| | |
|---|---|
| | A, W, X, Y und Z wie in Tabelle 10 angegeben |
| | B = i-C₃H₇ |

**Tabelle 15:**

| | |
|---|---|
| A, W, X, Y und Z wie in Tabelle 10 angegeben | |
| B= | 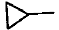 |

**Tabelle 16:**

| | |
|---|---|
| A, W, X, Y und Z wie in Tabelle 10 angegeben | |
| B= | 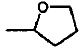 |

**Tabelle 17:**

| | |
|---|---|
| | W, X, Y und Z wie in Tabelle 10 angegeben |
| | A = -CH₂-CH₂-; B = OCH₃ |

**Tabelle 18:**

| | |
|---|---|
| | W, X, Y und Z wie in Tabelle 10 angegeben |
| | A = -CH₂-CH₂-; B = OC₂H₅ |

Bevorzugte Bedeutungen der oben in Zusammenhang mit den die Kultutpflanzen-Verträglichkeit verbessernden Verbindungen ("Herbizid-Safenern") der Formeln (IIa), (IIb), (IIc), (IId) und (IIe) aufgeführten Gruppen werden im Folgenden definiert.
- m: steht bevorzugt für die Zahlen 0, 1, 2, 3 oder 4.
- A¹: steht bevorzugt für eine der nachstehend skizzierten divalenten heterocyclischen Gruppierungen 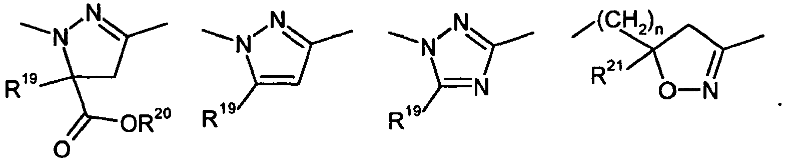
- n: steht bevorzugt for die Zahlen 0, 1, 2, 3 oder 4.
- A²: steht bevorzugt für jeweils gegebenenfalls durch Methyl, Ethyl, Methoxycarbonyl, Ethoxycarbonyl oder Alkyloxycarbonyl substituiertes Methylen oder Ethylen.
- R¹⁴: steht bevorzugt für Hydroxy, Mercapto, Amino, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Dimethylamino oder Diethylamino.
- R¹⁵: steht bevorzugt für Hydroxy, Mercapto, Amino, Methoxy, Ethoxy, n- oder i-Propoxy, n-, i-, s- oder t-Butoxy, 1-Methyl-hexyloxy, Allyloxy, 1-Allyloxymethyl-ethoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Dimethylamino oder Diethylamino.
- R¹⁶: steht bevorzugt für jeweils gegebenenfalls durch Fluor, Chlor und/oder Brom substituiertes Methyl, Ethyl, n- oder i-Propyl.
- R¹⁷: steht bevorzugt für Wasserstoff, jeweils gegebenenfalls durch Fluor und/oder Chlor substituiertes Methyl, Ethyl, n- oder i-Propyl, n-, i-, s- oder t-Butyl, Propenyl, Butenyl, Propinyl oder Butinyl, Methoxymethyl, Ethoxymethyl, Methoxyethyl, Ethoxyethyl, Dioxolanylmethyl, Furyl, Furylmethyl, Thienyl, Thiazolyl, Piperidinyl, oder gegebenenfalls durch Fluor, Chlor, Methyl, Ethyl, n- oder i-Propyl, n-, i-, s- oder t-Butyl substituiertes Phenyl.
- R¹⁸: steht bevorzugt für Wasserstoff, jeweils gegebenenfalls durch Fluor und/oder Chlor substituiertes Methyl, Ethyl, n- oder i-Propyl, n-, i-, s- oder t-Butyl, Propenyl, Butenyl, Propinyl oder Butinyl, Methoxymethyl, Ethoxymethyl, Methoxyethyl, Ethoxyethyl, Dioxolanylmethyl, Furyl, Furylmethyl, Thienyl, Thiazolyl, Piperidinyl, oder gegebenenfalls durch Fluor, Chlor, Methyl, Ethyl, n- oder i-Propyl, n-, i-, s- oder t-Butyl substituiertes Phenyl, oder zusammen mit R¹⁷ für einen der Reste -CH₂-O-CH₂-CH₂- und -CH₂-CH₂-O-CH₂-CH₂-, die gegebenenfalls substituiert sind durch Methyl, Ethyl, Furyl, Phenyl, einen annellierten Benzolring oder durch zwei Substituenten, die gemeinsam mit dem C-Atom, an das sie gebunden sind, einen 5- oder 6-gliedrigen Carbocyclus bilden.
- R¹⁹: steht bevorzugt für Wasserstoff, Cyano, Fluor, Chlor, Brom, oder für jeweils gegebenenfalls durch Fluor, Chlor und/oder Brom substituiertes Methyl, Ethyl, n- oder i-Propyl, Cyclopropyl, Cyclobutyl, Cyclopentyl, Cyclohexyl oder Phenyl.
- R²⁰: steht bevorzugt für Wasserstoff, jeweils gegebenenfalls durch Hydroxy, Cyano, Fluor, Chlor, Methoxy, Ethoxy, n- oder i-Propoxy substituiertes Methyl, Ethyl, n- oder i-Propyl, n-, i-, s- oder t-Butyl.
- R²¹: steht bevorzugt für Wasserstoff, Cyano, Fluor, Chlor, Brom, oder für jeweils gegebenenfalls durch Fluor, Chlor und/oder Brom substituiertes Methyl, Ethyl, n- oder i-Propyl, n-, i-, s- oder t-Butyl, Cyclopropyl, Cyclobutyl, Cyclopentyl, Cyclohexyl oder Phenyl.
- X¹: steht bevorzugt für Nitro, Cyano, Fluor, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluormethyl, Fluordichlormethyl, Methoxy, Ethoxy, n- oder i-Propoxy, Difluormethoxy oder Trifluormethoxy.
- X²: steht bevorzugt für Wasserstoff, Nitro, Cyano, Fluor, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluormethyl, Fluordichlormethyl, Methoxy, Ethoxy, n- oder i-Propoxy, Difluormethoxy oder Trifluormethoxy.
- X³: steht bevorzugt für Wasserstoff, Nitro, Cyano, Fluor, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluormethyl, Fluordichlormethyl, Methoxy, Ethoxy, n- oder i-Propoxy, Difluormethoxy oder Trifluormethoxy.
- t: steht bevorzugt für die Zahlen 0, 1, 2, 3 oder 4.
- v: steht bevorzugt für die Zahlen 0, 1, 2, 3 oder 4.
- R²²: steht bevorzugt für Wasserstoff, Methyl, Ethyl, n- oder i-Propyl.
- R²³: steht bevorzugt für Wasserstoff, Methyl, Ethyl, n- oder i-Propyl.
- R²⁴: steht bevorzugt für Wasserstoff, jeweils gegebenenfalls durch Cyano, Fluor, Chlor, Methoxy, Ethoxy, n- oder i-Propoxy substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, n-, i-, s- oder t-Butylthio, , Methylamino, Ethylamino, n- oder i-Propylamino, n-, i-, s- oder t-Butylamino, Dimethylamino oder Diethylamino, oder jeweils gegebenenfalls durch Cyano, Fluor, Chlor, Brom, Methyl, Ethyl, n- oder i-Propyl substituiertes Cyclopropyl, Cyclobutyl, Cyclopentyl, Cyclohexyl, Cyclopropyloxy, Cyclobutyloxy, Cyclopentyloxy, Cyclohexyloxy, Cyclopropylthio, Cyclobutylthio, Cyclopentylthio, Cyclohexylthio, Cyclopropylamino, Cyclobutylamino, Cyclopentylamino oder Cyclohexylamino.
- R²⁵: steht bevorzugt für Wasserstoff, jeweils gegebenenfalls durch Cyano, Hydroxy, Fluor, Chlor, Methoxy, Ethoxy, n- oder i-Propoxy substituiertes Methyl, Ethyl, n- oder i-Propyl, n-, i- oder s-Butyl, jeweils gegebenenfalls durch Cyano, Fluor, Chlor oder Brom substituiertes Propenyl, Butenyl, Propinyl oder Butinyl, oder jeweils gegebenenfalls durch Cyano, Fluor, Chlor, Brom, Methyl, Ethyl, n- oder i-Propyl substituiertes Cyclopropyl, Cyclobutyl, Cyclopentyl oder Cyclohexyl.
- R²⁶: steht bevorzugt für Wasserstoff, jeweils gegebenenfalls durch Cyano, Hydroxy, Fluor, Chlor, Methoxy, Ethoxy, n- oder i-Propoxy substituiertes Methyl, Ethyl, n- oder i-Propyl, n-, i- oder s-Butyl, jeweils gegebenenfalls durch Cyano, Fluor, Chlor oder Brom substituiertes Propenyl, Butenyl, Propinyl oder Butinyl, jeweils gegebenenfalls durch Cyano, Fluor, Chlor, Brom, Methyl, Ethyl, n- oder i-Propyl substituiertes Cyclopropyl, Cyclobutyl, Cyclopentyl oder Cyclohexyl, oder gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Di-fluormethoxy oder Trifluormethoxy substituiertes Phenyl, oder zusammen mit R²⁵ für jeweils gegebenenfalls durch Methyl oder Ethyl substituiertes Butan-1,4-diyl (Trimethylen), Pentan-1,5-diyl, 1-Oxa-butan-1,4-diyl oder 3-Oxa-pentan-1,5-diyl.
- X⁴: steht bevorzugt für Nitro, Cyano, Carboxy, Carbamoyl, Formyl, Sulfamoyl, Hydroxy, Amino, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy.
- X⁵: steht bevorzugt für Nitro, Cyano, Carboxy, Carbamoyl, Formyl, Sulfamoyl, Hydroxy, Amino, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy.

Beispiele für die als erfindungsgemäße Herbizid-Safener ganz besonders bevorzugten Verbindungen der Formel (IIa) sind in der nachstehenden Tabelle 19 aufgeführt.

**Tabelle 19: Beispiele für die Verbindungen der Formel (IIa)**

| | | | |
|---|---|---|---|
| | 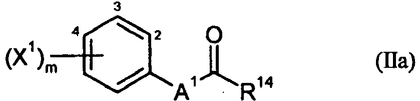 | | |

| **Beispiel-Nr.** | **(Positionen) (X¹)m** | **A¹** | **R¹⁴** |
|---|---|---|---|
| IIa-1 | (2) Cl, (4) Cl | 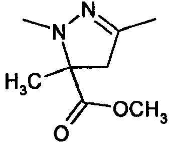 | OCH₃ |
| IIa-2 | (2) Cl, (4) Cl | 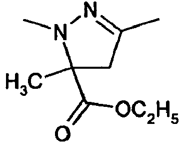 | OCH₃ |
| IIa-3 | (2) Cl, (4) Cl | 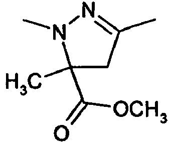 | OC₂H₅ |
| IIa-4 | (2) Cl, (4) Cl | 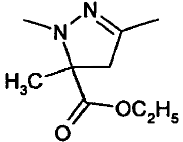 | OC₂H₅ |
| IIa-5 | (2) Cl | 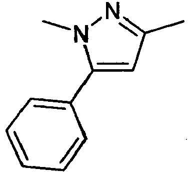 | OCH₃ |
| IIa-6 | (2) Cl, (4) Cl | 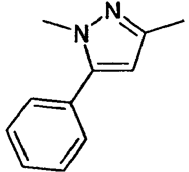 | OCH₃ |
| IIa-7 | (2) F | 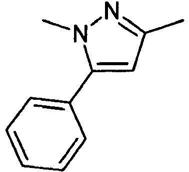 | OCH₃ |
| IIa-8 | (2) F | 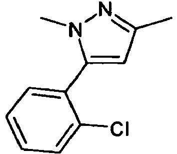 | OCH₃ |
| IIa-9 | (2) Cl, (4) Cl | 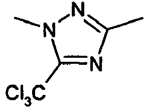 | OC₂H₅ |
| IIa-10 | (2) Cl, (4) CF₃ | 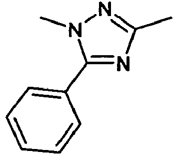 | OCH₃ |
| IIa-11 | (2) Cl | 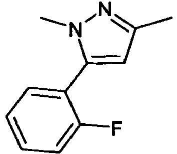 | OCH₃ |
| IIa-12 | - | 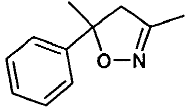 | OC₂H₅ |
| IIa-13 | (2) Cl, (4) Cl | 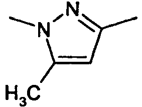 | OC₂H₅ |
| IIa-14 | (2) Cl, (4) Cl | 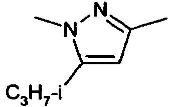 | OC₂H₅ |
| IIa-15 | (2) Cl, (4) Cl | 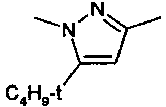 | OC₂H₅ |
| IIa-16 | (2) Cl, (4) Cl | 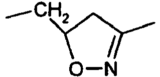 | OC₂H₅ |
| IIa-17 | (2) Cl, (4) Cl | 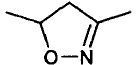 | OC₂H₅ |
| IIa-18 | - | 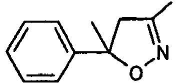 | OH |

Beispiele für die als erfindungsgemäße Herbizid-Safener ganz besonders bevorzugten Verbindungen der Formel (IIb) sind in der nachstehenden Tabelle 20 aufgeführt.

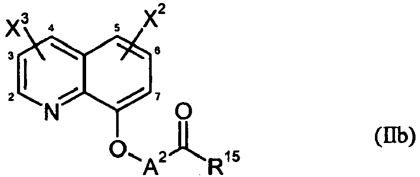

**Tabelle 20: Beispiele für die Verbindungen der Formel (IIb)**

| **Beispiel-Nr.** | **(Position) X²** | **(Position) X³** | **A²** | **R¹⁵** |
|---|---|---|---|---|
| IIb-1 | (5) Cl | - | CH₂ | OH |
| IIb-2 | (5) Cl | - | CH₂ | OCH₃ |
| IIb-3 | (5) Cl | - | CH₂ | OC₂H₅ |
| IIb-4 | (5) Cl | - | CH₂ | OC₃H₇-n |
| IIb-5 | (5) Cl | - | CH₂ | OC₃H₇-i |
| IIb-6 | (5) Cl | - | CH₂ | OC₄H₉-n |
| IIb-7 | (5) Cl | - | CH₂ | OCH(CH₃)C₅H₁₁-n |
| IIb-8 | (5) Cl | (2) F | CH₂ | OH |
| IIb-9 | (5) Cl | (2) Cl | CH₂ | OH |
| IIb-10 | (5) Cl | - | CH₂ | OCH₂CH=CH₂ |
| IIb-11 | (5) Cl | - | CH₂ | OC₄H₉-i |
| IIb-12 | (5) Cl | - | CH₂ | 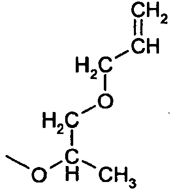 |
| IIb-13 | (5) Cl | - | 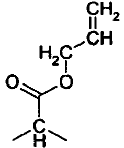 | OCH₂CH=CH₂ |
| IIb-14 | (5) Cl | - | 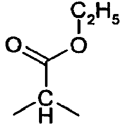 | OC₂H₅ |
| IIb-15 | (5) Cl | - | 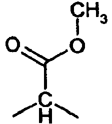 | OCH3 |

Beispiele für die als erfindungsgemäße Herbizid-Safener ganz besonders bevorzugten Verbindungen der Formel (IIc) sind in der nachstehenden Tabelle 21 aufgeführt.

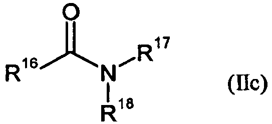

**Tabelle 21: Beispiele für die Verbindungen der Formel (IIc)**

| **Beispiel-Nr.** | **R¹⁶** | **N(R¹⁷,R¹⁸)** |
|---|---|---|
| IIc-1 | CHCl₂ | N(CH₂CH=CH₂)₂ |
| IIc-2 | CHCl₂ | 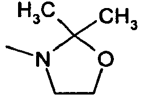 |
| IIc-3 | CHCl₂ | 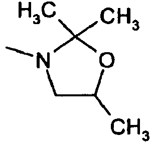 |
| IIc-4 | CHCl₂ | 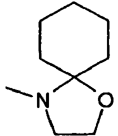 |
| IIc-5 | CHCl₂ | 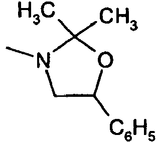 |
| IIc-6 | CHCl₂ | 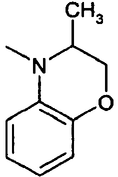 |
| IIc-7 | CHCl₂ | 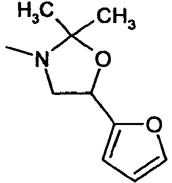 |

Beispiele für die als erfindungsgemäße Herbizid-Safener ganz besonders bevorzugten Verbindungen der Formel (IId) sind in der nachstehenden Tabelle 22 aufgeführt.

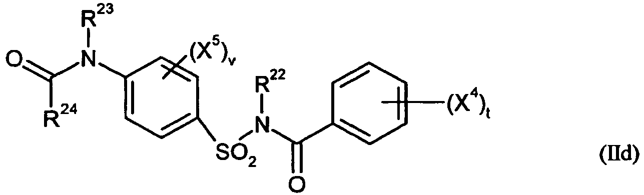

**Tabelle 22: Beispiele für die Verbindungen der Formel (IId)**

| **Beispiel- Nr.** | **R²²** | **R²³** | **R²⁴** | **(Positionen) (X⁴)ₜ** | **(Positionen) (X⁵)ᵥ** |
|---|---|---|---|---|---|
| IId-1 | H | H | CH₃ | (2) OCH₃ | - |
| IId-2 | H | H | C₂H₅ | (2) OCH₃ | - |
| IId-3 | H | H | C₃H₇-n | (2) OCH₃ | - |
| IId-4 | H | H | C₃H₇-i | (2) OCH₃ | - |
| IId-5 | H | H | 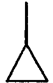 | (2) OCH₃ | - |
| IId-6 | H | H | CH₃ | (2) OCH₃ | - |
| | | | | (5) CH₃ | |
| IId-7 | H | H | C₂H₅ | (2) OCH₃ | - |
| | | | | (5) CH₃ | |
| IId-8 | H | H | C₃H₇-n | (2) OCH₃ | - |
| | | | | (5) CH₃ | |
| IId-9 | H | H | C₃H₇-i | (2) OCH₃ | - |
| | | | | (5) CH₃ | |
| IId-10 | H | H | 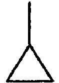 | (2) OCH₃ | - |
| | | | | (5) CH₃ | |
| IId-11 | H | H | OCH₃ | (2) OCH₃ | - |
| | | | | (5) CH₃ | |
| IId-12 | H | H | OC₂H₅ | (2) OCH₃ | - |
| | | | | (5) CH₃ | |
| IId-13 | H | H | OC₃H₇-i | (2) OCH₃ | - |
| | | | | (5) CH₃ | |
| IId-14 | H | H | SCH₃ | (2) OCH₃ | - |
| | | | | (5) CH₃ | |
| IId-15 | H | H | SC₂H₅ | (2) OCH₃ | - |
| | | | | (5) CH₃ | |
| IId-16 | H | H | SC₃H₇-i | (2) OCH₃ | - |
| | | | | (5) CH₃ | |
| IId-17 | H | H | NHCH₃ | (2) OCH₃ | - |
| | | | | (5) CH₃ | |
| IId-18 | H | H | NHC₂H₅ | (2) OCH₃ | - |
| | | | | (5) CH₃ | |
| IId-19 | H | H | NHC₃H₇-i | (2) OCH₃ | - |
| | | | | (5) CH₃ | |
| IId-20 | H | H | 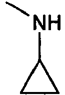 | (2) OCH₃ | - |
| | | | | (5) CH₃ | |
| IId-21 | H | H | NHCH₃ | (2) OCH₃ | - |
| IId-22 | H | H | NHC₃H₇-i | (2) OCH₃ | - |
| IId-23 | H | H | N(CH₃)₂ | (2) OCH₃ | - |
| IId-24 | H | H | N(CH₃)₂ | (3) CH₃ | - |
| | | | | (4) CH₃ | |
| IId-25 | H | H | CH₂-O-CH₃ | (2) OCH3 | - |

Beispiele für die als erfindungsgemäße Herbizid-Safener ganz besonders bevorzugten Verbindungen der Formel (IIe) sind in der nachstehenden Tabelle 23 aufgeführt.

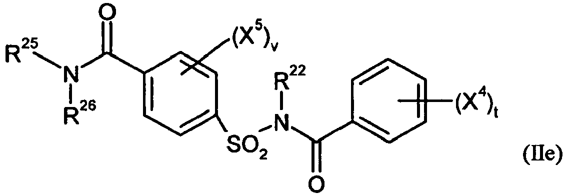

**Tabelle 23: Beispiele für die Verbindungen der Formel (IIe)**

| **Beispiel-Nr.** | **R²²** | **R²⁵** | **R²⁶** | **(Positionen) (X⁴)ₜ** | **(Positionen) (X⁵)ᵥ** |
|---|---|---|---|---|---|
| IIe-1 | H | H | CH₃ | (2) OCH₃ | - |
| IIe-2 | H | H | C₂H₅ | (2) OCH₃ | - |
| IIe-3 | H | H | C₃H₇-n | (2) OCH₃ | - |
| IIe-4 | H | H | C₃H₇-i | (2) OCH₃ | - |
| IIe-5 | H | H | 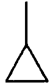 | (2) OCH₃ | - |
| IIe-6 | H | CH₃ | CH₃ | (2) OCH₃ | - |
| IIe-7 | H | H | CH₃ | (2) OCH₃ | - |
| | | | | (5) CH₃ | |
| IIe-8 | H | H | C₂H₅ | (2) OCH₃ | - |
| | | | | (5) CH₃ | |
| IIe-9 | H | H | C₃H₇-n | (2) OCH₃ | - |
| | | | | (5) CH₃ | |
| IIe-10 | H | H | C₃H₇-i | (2) OCH₃ | - |
| | | | | (5) CH₃ | |
| IIe-11 | H | H | 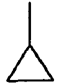 | (2) OCH₃ | - |
| | | | | (5) CH₃ | |
| IIe-12 | H | CH₃ | CH₃ | (2) OCH₃ | - |
| | | | | (5) CH₃ | |

Als die die Kulturpflanzen-Verträglichkeit verbessernde Verbindung [Komponente (b')] sind Cloquintocet-mexyl, Fenchlorazol-ethyl, Isoxadifen-ethyl, Mefenpyr-diethyl, Furilazole, Fenclorim, Cumyluron, Dymron, Dimepiperate und die Verbindungen Ile-5 (Cyprosulfamide) und IIe-11 am meisten bevorzugt, wobei Cloquintocet-mexyl und Mefenpyr-diethyl, aber auch Isoxadifen-ethyl sowie Cyprosulfamide besonders hervorgehoben seien.

Die als Safener erfindungsgemäß zu verwendenden Verbindungen der allgemeinen Formel (IIa) sind bekannt und/oder können nach an sich bekannten Verfahren hergestellt werden (vgl. WO-A-91/07874, WO-A-95/07897).

Die als Safener erfindungsgemäß zu verwendenden Verbindungen der allgemeinen Formel (IIb) sind bekannt und/oder können nach an sich bekannten Verfahren hergestellt werden (vgl. EP-A-191736).

Die als Safener erfindungsgemäß zu verwendenden Verbindungen der allgemeinen Formel (IIc) sind bekannt und/oder können nach an sich bekannten Verfahren hergestellt werden (vgl. DE-A-2218097, DE-A-2350547).

Die als Safener erfindungsgemäß zu verwendenden Verbindungen der allgemeinen Formel (IId) sind bekannt und/oder können nach an sich bekannten Verfahren hergestellt werden (vgl. DE-A-19621522/US-A-6235680).

Die als Safener erfindungsgemäß zu verwendenden Verbindungen der allgemeinen Formel (IIe) sind bekannt und können nach an sich bekannten Verfahren hergestellt werden (vgl. WO-A-99/66795/US-A-6251827).

Beispiele für die erfindungsgemäßen selektiv herbiziden Kombinationen aus jeweils einem Wirkstoff der Formel (I) und jeweils einem der oben definierten Safener sind in der nachstehenden Tabelle 24 aufgeführt.

**Tabelle 24: Beispiele für die erfindungsgemäßen Kombinationen**

| **Wirkstoffe der Formel (I)** | **Safener** |
|---|---|
| I-1-a | Cloquintocet-mexyl |
| I-1-a | Fenchlorazole-ethyl |
| I-1-a | Isoxadifen-ethyl |
| I-1-a | Mefenpyr-diethyl |
| I-1-a | Furilazole |
| I-1-a | Fenclorim |
| I-1-a | Cumyluron |
| I-1-a | Daimuron /Dymron |
| I-1-a | Dimepiperate |
| I-1-a | IIe-11 |
| I-1-a | IIe-5 |
| I-1-b | Cloquintocet-mexyl |
| I-1-b | Fenchlorazole-ethyl |
| I-1-b | Isoxadifen-ethyl |
| I-1-b | Mefenpyr-diethyl |
| I-1-b | Furilazole |
| I-1-b | Fenclorim |
| I-1-b | Cumyluron |
| I-1-b | Daimuron /Dymron |
| I-1-b | Dimepiperate |
| I-1-b | IIe-11 |
| I-1-b | IIe-5 |
| I-1-c | Cloquintocet-mexyl |
| I-1-c | Fenchlorazole-ethyl |
| I-1-c | Isoxadifen-ethyl |
| I-1-c | Mefenpyr-diethyl |
| I-1-c | Furilazole |
| I-1-c | Fenclorim |
| I-1-c | Cumyluron |
| I-1-c | Daimuron /Dymron |
| I-1-c | Dimepiperate |
| I-1-c | IIe-5 |
| I-1-c | IIe-11 |
| I-1-d | Cloquintocet-mexyl |
| I-1-d | Fenchlorazole-ethyl |
| I-1-d | Isoxadifen-ethyl |
| I-1-d | Mefenpyr-diethyl |
| I-1-d | Furilazole |
| I-1-d | Fenclorim |
| I-1-d | Cumyluron |
| I-1-d | Daimuron /Dymron |
| I-1-d | Dimepiperate |
| I-1-d | IIe-11 |
| I-1-d | IIe-5 |
| I-1-e | Cloquintocet-mexyl |
| I-1-e | Fenchlorazole-ethyl |
| I-1-e | Isoxadifen-ethyl |
| I-1-e | Mefenpyr-diethyl |
| I-1-e | Furilazole |
| I-1-e | Fenclorim |
| I-1-e | Cumyluron |
| I-1-e | Daimuron /Dymron |
| I-1-e | Dimepiperate |
| I-1-e | IIe-5 |
| I-1-e | IIe-11 |
| I-1-f | Cloquintocet-mexyl |
| I-1-f | Fenchlorazole-ethyl |
| I-1-f | Isoxadifen-ethyl |
| I-1-f | Mefenpyr-diethyl |
| I-1-f | Furilazole |
| I-1-f | Fenclorim |
| I-1-f | Cumyluron |
| I-1-f | Daimuron /Dymron |
| I-1-f | Dimepiperate |
| I-1-f | IIe-5 |
| I-1-f | IIe-11 |
| I-1-g | Cloquintocet-mexyl |
| I-1-g | Fenchlorazole-ethyl |
| I-1-g | Isoxadifen-ethyl |
| I-1-g | Mefenpyr-diethyl |
| I-1-g | Furilazole |
| I-1-g | Fenclorim |
| I-1-g | Cumyluron |
| I-1-g | Daimuron /Dymron |
| I-1-g | Dimepiperate |
| I-1-g | IIe-5 |
| I-1-g | IIe-11 |

**Tabelle 25: Beispiele für die erfindungsgemäßen Kombinationen**

| **Wirkstoffe der Formel (I)** | **Safener** |
|---|---|
| I-2-a | Cloquintocet-mexyl |
| I-2-a | Fenchlorazole-ethyl |
| I-2-a | Isoxadifen-ethyl |
| I-2-a | Mefenpyr-diethyl |
| I-2-a | Furilazole |
| I-2-a | Fenclorim |
| I-2-a | Cumyluron |
| I-2-a | Daimuron /Dymron |
| I-2-a | Dimepiperate |
| I-2-a | IIe-11 |
| I-2-a | IIe-5 |
| I-2-b | Cloquintocet-mexyl |
| I-2-b | Fenchlorazole-ethyl |
| I-2-b | Isoxadifen-ethyl |
| I-2-b | Mefenpyr-diethyl |
| I-2-b | Furilazole |
| I-2-b | Fenclorim |
| I-2-b | Cumyluron |
| I-2-b | Daimuron /Dymron |
| I-2-b | Dimepiperate |
| I-2-b | IIe-11 |
| I-2-b | IIe-5 |
| I-2-c | Cloquintocet-mexyl |
| I-2-c | Fenchlorazole-ethyl |
| I-2-c | Isoxadifen-ethyl |
| I-2-c | Mefenpyr-diethyl |
| I-2-c | Furilazole |
| I-2-c | Fenclorim |
| I-2-c | Cumyluron |
| I-2-c | Daimuron /Dymron |
| I-2-c | Dimepiperate |
| I-2-c | IIe-5 |
| I-2-c | IIe-11 |
| I-2-d | Cloquintocet-mexyl |
| I-2-d | Fenchlorazole-ethyl |
| I-2-d | Isoxadifen-ethyl |
| I-2-d | Mefenpyr-diethyl |
| I-2-d | Furilazole |
| I-2-d | Fenclorim |
| I-2-d | Cumyluron |
| I-2-d | Daimuron /Dymron |
| I-2-d | Dimepiperate |
| I-2-d | IIe-11 |
| I-2-d | IIe-5 |
| I-2-e | Cloquintocet-mexyl |
| I-2-e | Fenchlorazole-ethyl |
| I-2-e | Isoxadifen-ethyl |
| I-2-e | Mefenpyr-diethyl |
| I-2-e | Furilazole |
| I-2-e | Fenclorim |
| I-2-e | Cumyluron |
| I-2-e | Daimuron /Dymron |
| I-2-e | Dimepiperate |
| I-2-e | IIe-5 |
| I-2-e | IIe-11 |
| I-2-f | Cloquintocet-mexyl |
| I-2-f | Fenchlorazole-ethyl |
| I-2-f | Isoxadifen-ethyl |
| I-2-f | Mefenpyr-diethyl |
| I-2-f | Furilazole |
| I-2-f | Fenclorim |
| I-2-f | Cumyluron |
| I-2-f | Daimuron /Dymron |
| I-2-f | Dimepiperate |
| I-2-f | IIe-5 |
| I-2-f | IIe-11 |
| I-2-g | Cloquintocet-mexyl |
| I-2-g | Fenehlorazole-ethyl |
| I-2-g | Isoxadifen-ethyl |
| I-2-g | Mefenpyr-diethyl |
| I-2-g | Furilazole |
| I-2-g | Fenclorim |
| I-2-g | Cumyluron |
| I-2-g | Daimuron /Dymron |
| I-2-g | Dimepiperate |
| I-2-g | IIe-5 |
| I-2-g | IIe-11 |

Es wurde nun überraschend gefunden, dass die oben definierten Wirkstoffkombinationen aus substituierten cyclischen Ketoenole der allgemeinen Formel (I) und Safenern (Antidots) aus der oben aufgeführten Gruppe (b') bei sehr guter Nutzpflanzen-Verträglichkeit eine besonders hohe herbizide Wirksamkeit aufweisen und in verschiedenen Kulturen, insbesondere in Getreide (vor allem Weizen), aber auch in Soja, Kartoffeln, Mais und Reis zur selektiven Unkrautbekämpfung verwendet werden können.

Dabei ist es als überraschend anzusehen, dass aus einer Vielzahl von bekannten Safenern oder Antidots, die befähigt sind, die schädigende Wirkung eines Herbizids auf die Kulturpflanzen zu antagonisieren, gerade die oben aufgeführten Verbindungen der Gruppe (b') geeignet sind, die schädigende Wirkung von substituierten cyclischen Ketoenolen auf die Kulturpflanzen annähernd vollständig aufzuheben, ohne dabei die herbizide Wirksamkeit gegenüber den Unkräutern maßgeblich zu beeinträchtigen.

Hervorgehoben sei hierbei die besonders vorteilhafte Wirkung der besonders und am meisten bevorzugten Kombinationspartner aus der Gruppe (b'), insbesondere hinsichtlich der Schonung von Getreidepflanzen, wie z.B. Weizen, Gerste und Roggen, aber auch Mais und Reis, als Kulturpflanzen.

In der Literatur wurde bereits beschrieben, dass sich die Wirkung verschiedener Wirkstoffe durch Zugabe von Ammoniumsalzen steigern lässt. Dabei handelt es sich jedoch um als Detergens wirkende Salze (z.B. WO 95/017817) bzw. Salze mit längeren Alkyl- und / oder Arylsubstituenten, die permeabilisierend wirken oder die Löslichkeit des Wirkstoffs erhöhen (z.B. EP-A 0 453 086, EP-A 0 664 081, FR-A 2 600 494, US 4 844 734, US 5 462 912, US 5 538 937, US-A 03/0224939, US-A 05/0009880, US-A 05/0096386). Weiterhin beschreibt der Stand der Technik die Wirkung nur für bestimmte Wirkstoffe und / oder bestimmte Anwendungen der entsprechenden Mittel. In wieder anderen Fällen handelt es sich um Salze von Sulfonsäuren, bei denen die Säuren selber paralysierend auf Insekten wirken (US 2 842 476). Eine Wirkungssteigerung z.B. durch Ammoniumsulfat ist beispielsweise für die Herbizide Glyphosat und Phosphinothricin beschrieben (US 6 645 914, EP-A2 0 036 106). Eine entsprechende Wirkung bei Insektiziden wird durch diesen Stand der Technik weder offenbart noch nahegelegt.

Auch der Einsatz von Ammoniumsulfat als Formulierhilfsmittel ist für bestimmte Wirkstoffe und Anwendungen beschrieben (WO 92/16108), es dient dort aber zur Stabilisierung der Formulierung, nicht zur Wirkungssteigerung.

Es wurde nun völlig überraschend gefunden, dass sich die Wirkung von Insektiziden und/oder Akariziden und/oder Herbiziden aus der Klasse der 3'-Alkoxy-spirocyclopentyl substituierter Tetram- und Tetronsäuren durch den Zusatz von Ammonium- oder Phosphoniumsalzen zur Anwendungslösung oder durch den Einbau dieser Salze in eine Formulierung enthaltend 3'-Alkoxy-spirocyclopentyl substituierter Tetram- und Tetronsäuren, deutlich steigern lässt. Gegenstand der vorliegenden Erfindung ist also die Verwendung von Ammonium- oder Phosphoniumsalzen zur Wirkungssteigerung von Pflanzenschutzmitteln, die insektizid und / oder akarizid wirksame 3'-Alkoxyspiro-cyclopentyl substituierter Tetram- und Tetronsäuren als Wirkstoff enthalten. Gegenstand der Erfindung sind ebenfalls Mittel, die insektizid wirksame 3'-Alkoxyspirocyclopentyl substituierter Tetram- und Tetronsäuren und die Wirkung steigernde Ammonium- oder Phosphoniumsalze enthalten und zwar sowohl formulierte Wirkstoffe als auch anwendungsfertige Mittel (Spritzbrühen). Gegenstand der Erfindung ist schließlich weiterhin die Verwendung dieser Mittel zur Bekämpfung von Schadinsekten und/oder Spinnmilben und/oder unerwünschten Pflanzenwuchs. Diese Zusammensetzungen können auch die oben genannten die Kulturpflanzen-Vertäglichkeit verbessernde Verbindungen enthalten.

Die Wirkstoffe können in den erfindungsgemäßen Zusammensetzungen in einem breiten Konzentrationsbereich eingesetzt werden. Die Konzentration der Wirkstoffe in der Formulierung beträgt dabei üblicherweise 0,1 - 50 Gew.-%.

Ammonium- und Phosphoniumsalze, die erfindungsgemäß die Wirkung von Pflanzenschutzmitteln enthaltend Fettsäure-Biosynthese-Inhibitoren steigern, werden durch Formel (III') definiert 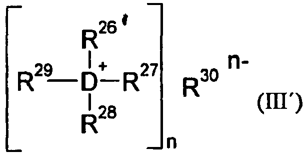 in welcher
- D: für Stickstoff oder Phosphor steht,
- D: bevorzugt für Stickstoff steht,
- R^{26'}, R²⁷, R²⁸ und R²⁹: unabhängig voneinander für Wasserstoff oder jeweils gegebenenfalls substituiertes C₁-C₈-Alkyl oder einfach oder mehrfach ungesättigtes, gegebenenfalls substituiertes C₁-C₈-Alkylen stehen, wobei die Substituenten aus Halogen, Nitro und Cyano ausgewählt sein können,
- R^{26'}, R²⁷, R²⁸ und R²⁹: bevorzugt unabhängig voneinander für Wasserstoff oder jeweils gegebenenfalls substituiertes C₁-C₄-Alkyl stehen, wobei die Substituenten aus Halogen, Nitro und Cyano ausgewählt sein können,
- R^{26'}, R²⁷, R²⁸ und R²⁹: besonders bevorzugt unabhängig voneinander für Wasserstoff, Methyl, Ethyl, n-Propyl, i-Propyl, n-Butyl, i-Butyl, s-Butyl oder t-Butyl stehen,
- R^{26'}, R²⁷, R²⁸ und R²⁹: ganz besonders bevorzugt für Wasserstoff stehen,
- n: für 1, 2, 3 oder 4 steht,
- n: bevorzugt für 1 oder 2 steht,
- R³⁰: für ein anorganisches oder organisches Anion steht,
- R³⁰: bevorzugt für Hydrogencarbonat, Tetraborat, Fluorid, Bromid, Jodid, Chlorid, Monohydrogenphosphat, Dihydrogenphosphat, Hydrogensulfat, Tartrat, Sulfat, Nitrat, Thiosulfat, Thiocyanat, Formiat, Laktat, Acetat, Propionat, Butyrat, Pentanoat oder Oxalat steht,
- R³⁰: besonders bevorzugt für Laktat, Sulfat, Nitrat, Thiosulfat, Thiocyanat, Oxalat oder Formiat steht.
- R³⁰: ganz besonders bevorzugt für Sulfat steht.

Die Ammonium- und Phosphoniumsalze der Formel (III') können in einem breiten Konzentrationsbereich zur Steigerung der Wirkung von Pflanzenschutzmitteln enthaltend Ketoenole eingesetzt werden. Im Allgemeinen werden die Ammonium- oder Phosphoniumsalze im anwendungsfertigen Pflanzenschutzmittel in einer Konzentration von 0,5 bis 80 mmol/l, bevorzugt 0,75 bis 37,5 mmol/l, besonders bevorzugt 1,5 bis 25 mmol/l eingesetzt. Im Fall eines formulierten Produktes wird die Ammonium- und/oder Phosphoniumsalzkonzentration in der Formulierung so gewählt, dass sie nach Verdünnung der Formulierung auf die gewünschte Wirkstoffkonzentration in diesen angegebenen allgemeinen, bevorzugten oder besonders bevorzugten Bereichen liegt. Die Konzentration des Salzes in der Formulierung beträgt dabei üblicherweise 1 - 50 Gew.-%.

In einer bevorzugten Ausführungsform der Erfindung wird den Pflanzenschutzmitteln zur Wirkungssteigerung nicht nur ein Ammonium- und/oder Phosphoniumsalz, sondern zusätzlich ein Penetrationsförderer zugegeben. Es ist als völlig überraschend zu bezeichnen, dass selbst in diesen Fällen eine noch weiter gehende Wirkungssteigerung zu beobachten ist. Gegenstand der vorliegenden Erfindung ist also ebenfalls die Verwendung einer Kombination von Penetrationsförderer und Ammonium- und/oder Phosphoniumsalzen zur Wirkungssteigerung von Pflanzenschutzmitteln, die insektizid wirksame 3'-Alkoxyspiro-cyclopentyl substituierter Tetram- und Tetronsäuren als Wirkstoff enthalten. Gegenstand der Erfindung sind ebenfalls Mittel, die insektizid wirksame 3'-Alkoxyspiro-cyclopentyl substituierter Tetram- und Tetronsäuren, Penetrationsförderer und Ammonium- und/oder Phosphoniumsalze enthalten und zwar sowohl formulierte Wirkstoffe als auch anwendungsfertige Mittel (Spritzbrühen). Gegenstand der Erfindung ist schließlich weiterhin die Verwendung dieser Mittel zur Bekämpfung von Schadinsekten.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der wässerigen Spritzbrühe und/oder aus dem Spritzbelag in die Kutikula der Pflanze eindringen und dadurch die Stoffbeweglichkeit (Mobilität) von Wirkstoffen in der Kutikula erhöhen können. Die in der Literatur (Baur et al., 1997, Pesticide Science 51, 131-152) beschriebene Methode kann zur Bestimmung dieser Eigenschaft eingesetzt werden.

Als Penetrationsförderer kommen beispielsweise Alkanol-alkoxylate in Betracht. Erfindungsgemäße Penetrationsförderer sind Alkanol-alkoxylate der Formel (IV')

R-O-(-AO)ᵥ-R' (IV')

in welcher
- R: für geradkettiges oder verzweigtes Alkyl mit 4 bis 20 Kohlenstoffatomen steht,
- R': für Wasserstoff, Methyl, Ethyl, n-Propyl, i-Propyl, n-Butyl, i-Butyl, t-Butyl, n-Pentyl oder n-Hexyl steht,
- AO: für einen Ethylenoxid-Rest, einen Propylenoxid-Rest, einen Butylenoxid-Rest oder für Gemische aus Ethylenoxid- und Propylenoxid-Resten oder Butylenoxid-Resten steht und
- v: für Zahlen von 2 bis 30 steht.

Eine bevorzugte Gruppe von Penetrationsförderern sind Alkanolalkoxylate der Formel

R-O-(-EO-)ₙ-R' (IV'-a)

in welcher
- R: die oben angegebene Bedeutung hat,
- R': die oben angegebene Bedeutung hat,
- EO: für -CH₂-CH₂-O- steht und
- n: für Zahlen von 2 bis 20 steht.

Eine weitere bevorzugte Gruppe von Penetrationsförderern sind Alkanol-alkoxylate der Formel

R-O-(-EO-)ₚ-(-PO-)_{q}-R' (IV'-b)

in welcher
- R: die oben angegebene Bedeutung hat,
- R': die oben angegebene Bedeutung hat,
- EO: für -CH₂-CH₂-O- steht,

- PO: für 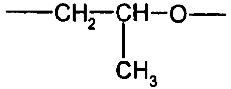 steht,
- p: für Zahlen von 1 bis 10 steht und
- q: für Zahlen von 1 bis 10 steht.

Eine weitere bevorzugte Gruppe von Penetrationsförderern sind Alkanol-Alkoxylate der Formel

R-O-(-PO-)ᵣ-(EO-)ₛ-R' (IV'-c)

in welcher
- R: die oben angegebene Bedeutung hat,
- R': die oben angegebene Bedeutung hat,
- EO: für -CH₂-CH₂-O- steht,
- PO: für 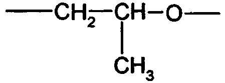 steht,
- r: für Zahlen von 1 bis 10 steht und
- s: für Zahlen von 1 bis 10 steht.

Eine weitere bevorzugte Gruppe von Penetrationsförderern sind Alkanol-alkoxylate der Formel

R-O-(-EO-)ₚ-(-BO-)_{q}-R' (IV'-d)

in welcher
- R und R': die oben angegebenen Bedeutungen haben,
- EO: für CH₂-CH₂-O- steht,
- BO: für 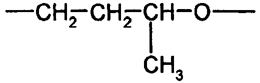 steht,
- p: für Zahlen von 1 bis 10 steht und
- q: für Zahlen von 1 bis 10 steht.

Eine weitere bevorzugte Gruppe von Penetrationsförderern sind Alkanol-alkoxylate der Formel

R-O-(-BO-)ᵣ-(-EO-)ₛ-R' (IV'-e)

in welcher
- R und R': die oben angegebenen Bedeutungen haben,
- BO: für 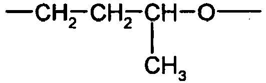 steht,
- EO: für CH₂-CH₂-O- steht,
- r: für Zahlen von 1 bis 10 steht und
- s: für Zahlen von 1 bis 10 steht.

Eine weitere bevorzugte Gruppe von Penetrationsförderern sind Alkanol-Alkoxylate der Formel

CH₃-(CH₂)ₜ-CH₂-O-(-CH₂-CH₂-O-)ᵤ-R' (IV'-f)

in welcher
- R': die oben angegebene Bedeutung hat,
- t: für Zahlen von 8 bis 13 steht
- u: für Zahlen von 6 bis 17 steht.

In den zuvor angegebenen Formeln steht
- R: vorzugsweise für Butyl, i-Butyl, n-Pentyl, i-Pentyl, Neopentyl, n-Hexyl, i-Hexyl, n-Octyl, i-Octyl, 2-Ethyl-hexyl, Nonyl, i-Nonyl, Decyl, n-Dodecyl, i-Dodecyl, Lauryl, Myristyl, i-Tridecyl, Trimethyl-nonyl, Palmityl, Stearyl oder Eicosyl.

Als Beispiel für ein Alkanol-Alkoxylat der Formel (IV-c) sei 2-Ethyl-hexyl-alkoxylat der Formel 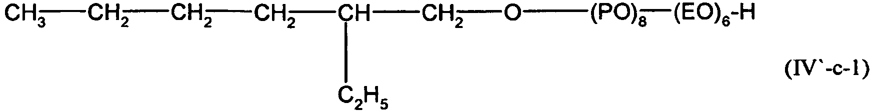 in welcher
- EO: für -CH₂-CH₂-O- steht,
- PO: für 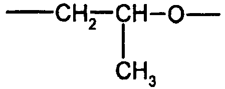 steht und
die Zahlen 8 und 6 Durchschnittswerte darstellen, genannt.

Als Beispiel für ein Alkanol-Alkoxylat der Formel (IV-d) sei die Formel

CH₃-(CH₂)₁₀-O-(-EO-)₆-(-BO-)₂-CH₃ (IV'-d-1)

in welcher
- EO: für CH₂-CH₂-O- steht,
- BO: für 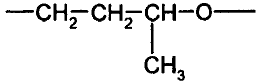 steht und
die Zahlen 10, 6 und 2 Durchschnittswerte darstellen, genannt.

Besonders bevorzugte Alkanol-Alkoxylate der Formel (IV'-f) sind Verbindungen dieser Formel, in denen
- t: für Zahlen von 9 bis 12 und
- u: für Zahlen von 7 bis 9
steht.

Ganz besonders bevorzugt genannt sei Alkanol-Alkoxylat der Formel (IV'-f-1)

CH₃-(CH₂)ₜ-CH₂-O-(-CH₂-CH₂-O-)ᵤ-H (IV'-f-1)

in welcher
- t: für den Durchschnittswert 10,5 steht und
- u: für den Durchschnittswert 8,4 steht.

Die Alkanol-Alkoxylate sind durch die obigen Formeln allgemein definiert. Bei diesen Substanzen handelt es sich um Gemische von Stoffen des angegebenen Typs mit unterschiedlichen Kettenlängen. Für die Indices errechnen sich deshalb Durchschnittswerte, die auch von ganzen Zahlen abweichen können.

Die Alkanol-Alkoxylate der angegebenen Formeln sind bekannt und sind teilweise kommerziell erhältlich oder lassen sich nach bekannten Methoden herstellen (vgl. WO 98/35 553, WO 00/35 278 und EP-A 0 681 865).

Als Penetrationsförderer kommen beispielsweise auch Substanzen in Betracht, die die Verfügbarkeit der Verbindungen der Formel (I) im Spritzbelag fördern. Dazu gehören beispielsweise mineralische oder vegetabile Öle. Als Öle kommen alle üblicherweise in agrochemischen Mitteln einsetzbaren mineralischen oder vegetabilen - gegebenenfalls modifizierte - Öle in Frage. Beispielhaft genannt seien Sonnenblumenöl, Rapsöl, Olivenöl, Rizinusöl, Rüböl, Maiskernöl, Baumwollsaatöl und Sojabohnenöl oder die Ester der genannten Öle. Bevorzugt sind Rapsöl, Sonnenblumenöl und deren Methyl- oder Ethylester.

Die Konzentration an Penetrationsförderer kann in den erfindungsgemäßen Mitteln in einem weiten Bereich variiert werden. Bei einem formulierten Pflanzenschutzmittel liegt sie im allgemeinen bei 1 bis 95 Gew.-%, bevorzugt bei 1 bis 55 Gew.-%, besonders bevorzugt bei 15 - 40 Gew.-%. In den anwendungsfertigen Mitteln (Spritzbrühen) liegen die Konzentration im allgemeinen zwischen 0,1 und 10 g/l, bevorzugt zwischen 0,5 und 5 g/l.

Erfindungsgemäße Pflanzenschutzmittel können auch weitere Komponente, beispielsweise Tenside bzw. Dispergierhilfsmittel oder Emulgatoren enthalten.

Als nicht-ionische Tenside bzw. Dispergierhilfsmittel kommen alle üblicherweise in agrochemischen Mitteln einsetzbaren Stoffe dieses Typs in Betracht. Vorzugsweise genannt seien Polyethylenoxid-polypropylenoxid-Blockcopolymere, Polyethylenglykolether von linearen Alkoholen, Umsetzungsprodukte von Fettsäuren mit Ethylenoxid und/oder Propylenoxid, ferner Polyvinylalkohol, Polyvinylpyrrolidon, Mischpolymerisate aus Polyvinylalkohol und Polyvinylpyrrolidon sowie Copolymerisate aus (Meth)acrylsäure und (Meth)acrylsäureestern, weiterhin Alkylethoxylate und Alkylarylethoxylate, die gegebenenfalls phosphatiert und gegebenenfalls mit Basen neutralisiert sein können, wobei Sorbitolethoxylate beispielhaft genannt seien, sowie Polyoxyalkylenamin-Derivate.

Als anionische Tenside kommen alle üblicherweise in agrochemischen Mitteln einsetzbaren Substanzen dieses Typs in Frage. Bevorzugt sind Alkalimetall- und Erdalkalimetall-Salze von Alkylsulfonsäuren oder Alkylarylsulfonsäuren.

Eine weitere bevorzugte Gruppe von anionischen Tensiden bzw. Dispergierhilfsmitteln sind in Pflanzenöl wenig lösliche Salze von Polystyrolsulfonsäuren, Salze von Polyvinylsulfonsäuren, Salze von Naphthalinsulfonsäure-Formaldehyd-Kondensationsprodukten, Salze von Kondensationsprodukten aus Naphthalinsulfonsäure, Phenolsulfonsäure und Formaldehyd sowie Salze von Ligninsulfonsäure.

Als Zusatzstoffe, die in den erfindungsgemäßen Formulierungen enthalten sein können, kommen Emulgatoren, schaumhemmende Mittel, Konservierungsmittel, Antioxydantien, Farbstoffe und inerte Füllmaterialien in Betracht.

Bevorzugte Emulgatoren sind ethoxylierte Nonylphenole, Umsetzungsprodukte von Alkylphenolen mit Ethylenoxid und/oder Propylenoxid, ethoxylierte Arylalkylphenole, weiterhin ethoxylierte und propoxylierte Arylalkylphenole, sowie sulfatierte oder phosphatierte Arylalkylethoxylate bzw. -ethoxy-propoxylate, wobei Sorbitan-Derivate, wie Polyethylenoxid-Sorbitan-Fettsäureester und Sorbitan-Fettsäureester, beispielhaft genannt seien.

Verwendet man beispielsweise gemäß Verfahren (A) N-[(4-Chlor-2,6-dimethyl)-phenylacetyl]-1-amino-3-methoxy-cyclopentancarbonsäureethylester als Ausgangsstoff, so kann der Verlauf des erfindungsgemäßen Verfahrens durch folgendes Reaktionsschema wiedergegeben werden:

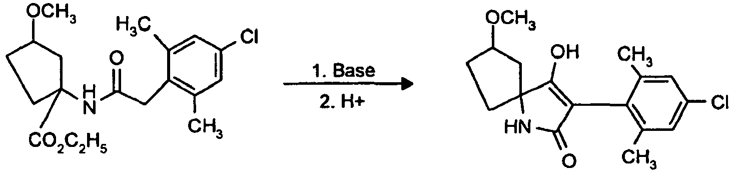

Verwendet man beispielsweise gemäß Verfahren (B) O-[(2-Chlor-6-methyl)-phenylacetyl]-1-hydroxy-3-ethoxy-cyclopentancarbonsäureethylester, so kann der Verlauf des erfindungsgemäßen Verfahrens durch folgendes Reaktionsschema wiedergegeben werden:

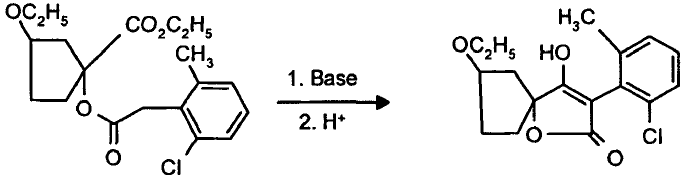

Verwendet man beispielsweise gemäß Verfahren (Cα) 7-Butoxy-3-[(4-chlor-2,6-dimethyl)-phenyl]-1-azaspiro[4,4]nonan-2,4-dion und Pivaloylchlorid als Ausgangsstoffe, so kann der Verlauf des erfindungsgemäßen Verfahrens durch folgendes Reaktionsschema wiedergegeben werden:

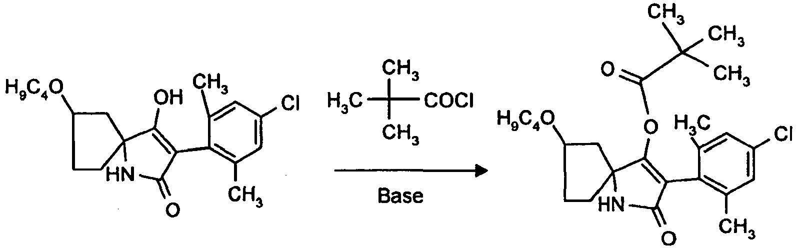

Verwendet man beispielsweise gemäß Verfahren (C) (Variante β) 7-Ethoxy-3-[(2,4-dichlor)-phenyl]-1-oxaspiro-[4,4]-nonan-2,4-dion und Acetanhydrid als Ausgangsverbindungen, so kann der Verlauf des erfindungsgemäßen Verfahrens durch folgendes Reaktionsschema wiedergegeben werden:

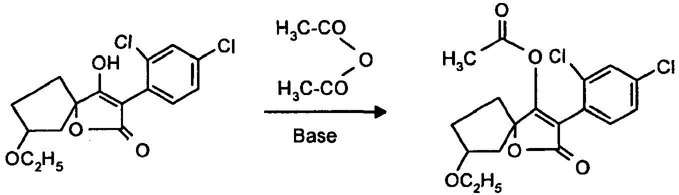

Verwendet man beispielsweise gemäß Verfahren (D) 7-Methoxy-3-[(2,4-dichlor-6-methyl)-phenyl]-1-azaspiro[4,4]nonan-2,4-dion und Chlorameisensäureethylester als Ausgangsverbindungen, so kann der Verlauf des erfindungsgemäßen Verfahrens durch folgendes Reaktionsschema wiedergegeben werden:

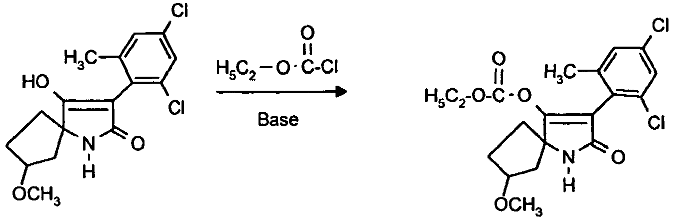

Verwendet man beispielsweise gemäß Verfahren (E) 7-Ethoxy-3-[(2,4,6-trimethyl)-phenyl]-1-oxaspiro[4,4]nonan-2,4-dion und Chlormonothioameisensäuremethylester als Ausgangsprodukte, so kann der Reaktionsverlauf folgendermaßen wiedergegeben werden:

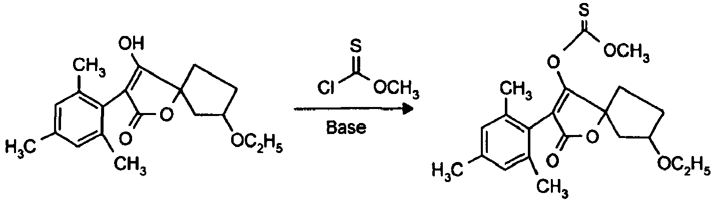

Verwendet man beispielsweise gemäß Verfahren (F) 7-Butoxy-3-[(2,4,6-trimethyl)-phenyl]-1-azaspiro[4,4]nonan-2,4-dion und Methansulfonsäurechlorid als Ausgangsprodukte, so kann der Reaktionsverlauf durch folgendes Reaktionsschema wiedergegeben werden:

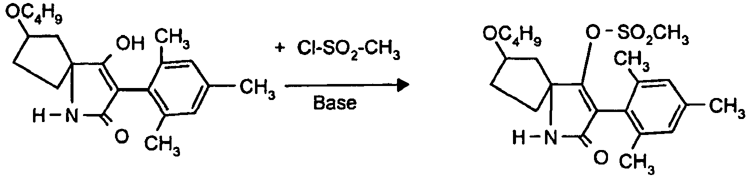

Verwendet man beispielsweise gemäß Verfahren (G) 7-Methoxy-3-[(2,4-dichlor-6-methyl)-phenyl]-1-oxaspiro[4,4]nonan-2,4-dion und Methanthio-phosphonsäurechlorid-(2,2,2-trifluorethyl-ester) als Ausgangsprodukte, so kann der Reaktionsverlauf durch folgendes Reaktionsschema wiedergegeben werden:

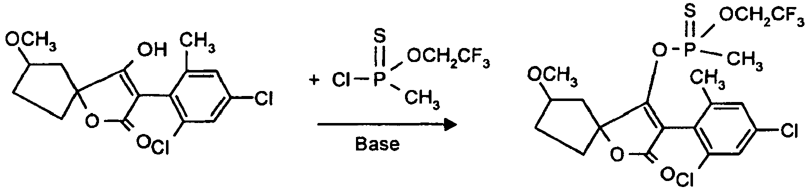

Verwendet man beispielsweise gemäß Verfahren (H) 7-Methoxy-3-[(2,3,4,6-tetramethylphenyl]-1-azaspiro[4,4]nonan-2,4-dion und NaOH als Komponenten, so kann der Verlauf des erfindungsgemäßen Verfahrens durch folgendes Reaktionsschema wiedergegeben werden:

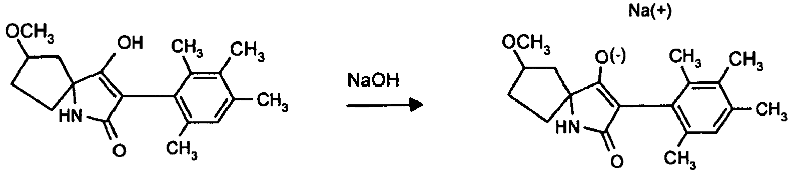

Verwendet man beispielsweise gemäß Verfahren (I) (Variante α) 7-Ethoxy-3-[(2,4,5-trimethyl)-phenyl]-1-oxaspiro[4,4]nonan-2,4-dion und Ethylisocyanat als Ausgangsprodukte, so kann der Reaktionsverlauf durch folgendes Reaktionsschema wiedergegeben werden:

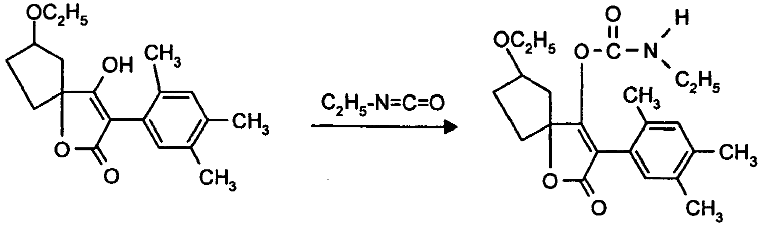

Verwendet man beispielsweise gemäß Verfahren (I) (Variante β) 7-Butoxy-3-[(2,4,6-trimethyl)-phenyl]-1-azaspiro[4,4]nonan-2,4-dion und Dimethylcarbamidsäurechlorid als Ausgangsprodukte, so kann der Reaktionsverlauf durch folgendes Schema wiedergegeben werden:

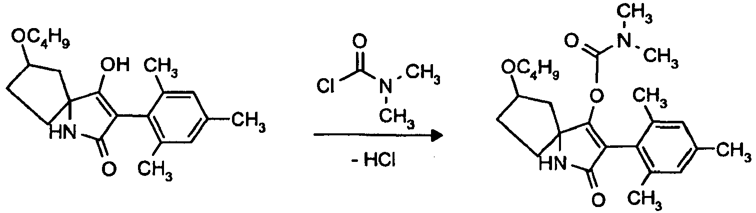

Die beim erfindungsgemäßen Verfahren (A) als Ausgangsstoffe benötigten Verbindungen der Formel (II) 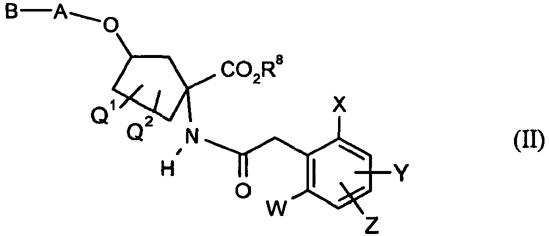 in welcher
- A, B, Q¹, Q², W, X, Y, Z und R⁸: die oben angegebenen Bedeutungen haben,
sind neu.

Man erhält die Acylaminosäureester der Formel (II) beispielsweise, wenn man Aminosäurederivate der Formel (XIV) 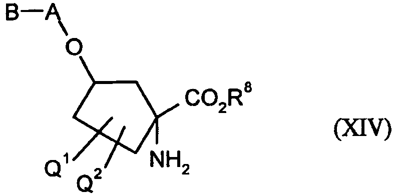 in welcher
- A, B, Q¹ und Q² und R⁸: die oben angegebene Bedeutung haben,
mit substituierten Phenylessigsäurederivaten der Formel (XV) 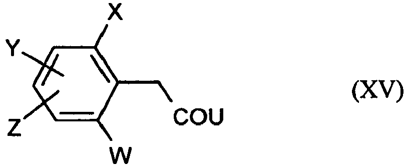 in welcher
- W, X, Y und Z: die oben angegebenen Bedeutungen haben und
- U: für eine durch Carbonsäureaktivierungsreagenzien wie Carbonyldiimidazol, Carbonyldiimide (wie z.B. Dicyclohexylcarbondiimid), Phosphorylierungsreagenzien (wie z.B. POCl₃, BOP-Cl), Halogenierungnsmittel, wie z.B. Thionylchlorid, Oxalylchlorid, Phosgen oder Chlorameisensäureester eingeführte Abgangsgruppe steht,
acyliert (Chem. Reviews 52, 237-416 (1953); Bhattacharya, Indian J. Chem. 6, 341-5, 1968)
oder wenn man Acylaminosäuren der Formel (XVI) 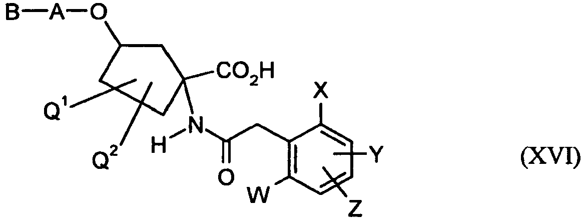 in welcher
- A, B, Q¹, Q², W, X, Y und Z: die oben angegebenen Bedeutungen haben,
verestert (Chem. Ind. (London) 1568 (1968)).

Die Verbindungen der Formel (XVI) 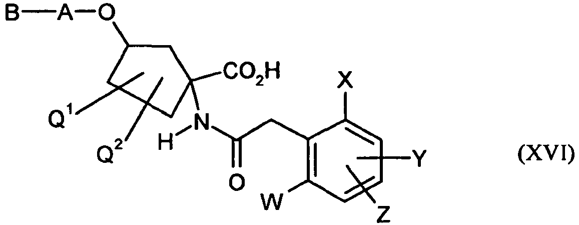 in welcher
- A, B, Q¹, Q², W, X, Y und Z: die oben angegebenen Bedeutungen haben,
sind neu.

Man erhält die Verbindungen der Formel (XVI) beispielsweise, wenn man 1-Amino-cyclohexancarbonsäuren der Formel (XVII) 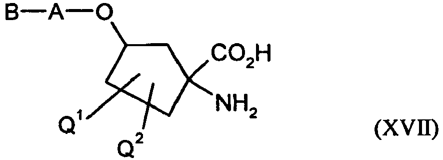 in welcher
- A, B, Q¹ und Q²: die oben angegebenen Bedeutungen haben
mit substituierten Phenylessigsäurederivaten der Formel (XV) 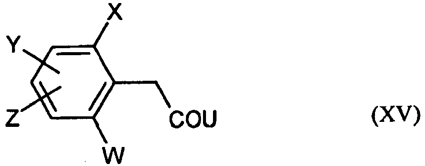 in welcher
- U, W, X, Y und Z: die oben angegebenen Bedeutungen haben
nach Schotten-Baumann acyliert (Organikum, VEB Deutscher Verlag der Wissenschaften, Berlin 1977, S. 505).

Die Verbindungen der Formel (XV) sind teilweise bekannt und/oder lassen sich nach den bekannten Verfahren in den eingangs zitierten Offenlegungsschriften herstellen.

Die Verbindungen der Formel (XIV) und (XVII) sind teilweise neu und lassen sich nach bekannten Verfahren darstellen (Tetrahedron Assymetry, 8, 825 ff(1997) und WO 02/46128).

Die bei dem erfindungsgemäßen Verfahren (B) als Ausgangstoffe benötigten Verbindungen der Formel (III) 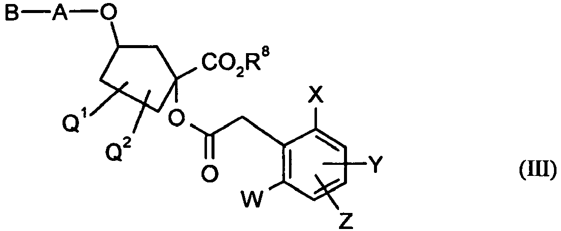 in welcher
- A, B, Q¹, Q², W, X, Y, Z und R⁸: die oben angegebenen Bedeutungen haben,
sind neu.

Sie lassen sich nach im Prinzip bekannten Methoden in einfacher Weise herstellen.

Man erhält die Verbindungen der Formel (III) beispielsweise, wenn man
1-Hydroxy-cyclohexan-carbonsäureester der Formel (XVIII) 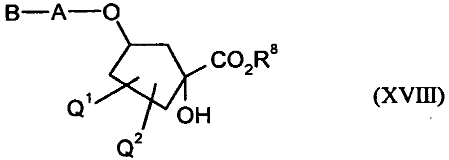 in welcher
- A, B, Q¹, Q² und R⁸: die oben angegebenen Bedeutungen haben,
mit substituierten Phenylessigsäurederivaten der Formel (XV) 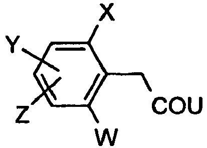 in welcher
U, W, X, Y und Z die oben angegebenen Bedeutungen haben,
acyliert (Chem. Reviews 52, 237-416 (1953)).

Die 1-Hydroxy-3-alkoxy-cyclopentyl-carbonsäureester der Formel (XVIII) sind neu. Man erhält sie beispielsweise, indem man substituierte 1-Hydroxy-3-alkoxy-cyclopentan-carbonsäurenitrile in Gegenwart von Säuren, z.B. nach Pinner mit Alkoholen umsetzt. Das Cyanhydrin erhält man beispielsweise durch Umsetzung von substituierten 3-Alkoxy-cyclopentan-1-onen mit Blausäure.

Die zur Durchführung der erfindungsgemäßen Verfahren (C), (D), (E), (F), (G), (H) und (I) außerdem als Ausgangsstoffe benötigten Säurehalogenide der Formel (IV), Carbonsäureanhydride der Formel (V), Chlorameisensäureester oder Chlorameisensäurethioester der Formel (VI), Chlormonothioameisensäureester oder Chlordithioameisensäureester der Formel (VII), Sulfonsäurechloride der Formel (VIII), Phosphorverbindungen der Formel (IX) und Metallhydroxide, Metallalkoxide oder Amine der Formel (X) und (XI) und Isocyanate der Formel (XII) und Carbamidsäurechloride der Formel (XIII) sind allgemein bekannte Verbindungen der organischen bzw. anorganischen Chemie.

Die Verbindungen der Formel (XV) sind darüber hinaus aus den eingangs zitierten Patentanmeldungen bekannt und/oder lassen sich nach den dort angegebenen Methoden herstellen.

Das Verfahren (A) ist dadurch gekennzeichnet, dass man Verbindungen der Formel (II), in welcher A, B, Q¹, Q², W, X, Y, Z und R⁸ die oben angegebenen Bedeutungen haben, in Gegenwart eines Verdünnungsmittels und in Gegenwart einer Base einer intramolekularen Kondensation unterwirft.

Als Verdünnungsmittel können bei dem erfindungsgemäßen Verfahren (A) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (=Methyltrialkyl(C₈-C₁₀)ammoniumchlorid) oder TDA 1 (=Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natriummethylat, Natriumethylat und Kalium-tert.-butylat einsetzbar.

Die Reaktionstemperatur kann bei der Durchführung des erfindungsgemäßen Verfahrens (A) innerhalb eines größeren Bereiches variiert werden. Im Allgemeinen arbeitet man bei Temperaturen zwischen -75°C und 200°C, vorzugsweise zwischen -50°C und 150°C.

Das erfindungsgemäße Verfahren (A) wird im Allgemeinen unter Normaldruck durchgeführt.

Bei der Durchführung des erfindungsgemäßen Verfahrens (A) setzt man die Reaktionskomponente der Formel (II) und die deprotonierende Base im Allgemeinen in äquimolaren bis etwa doppeltäquimolaren Mengen ein. Es ist jedoch auch möglich, die eine oder andere Komponente in einem größeren Überschuss (bis zu 3 Mol) zu verwenden.

Das Verfahren (B) ist dadurch gekennzeichnet, dass man Verbindungen der Formel (III), in welcher A, B, Q¹, Q², W, X, Y, Z und R⁸ die oben angegebenen Bedeutungen haben, in Gegenwart eines Verdünnungsmittels und in Gegenwart einer Base intramolekular kondensiert.

Als Verdünnungsmittel können bei dem erfindungsgemäßen Verfahren (B) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, Iso-Propanol, Butanol, Iso-Butanol und tert.-Butanol eingesetzt werden.

Als Base (Deprotonierungsmittel) können bei der Durchführung des erfindungsgemäßen Verfahrens (B) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)(ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natriummethylat, Natriumethylat und Kalium-tert.-butylat einsetzbar.

Die Reaktionstemperatur kann bei der Durchführung des erfindungsgemäßen Verfahrens (B) innerhalb eines größeren Bereiches variiert werden. Im Allgemeinen arbeitet man bei Temperaturen zwischen -75°C und 200°C, vorzugsweise zwischen -50°C und 150°C.

Das erfindungsgemäße Verfahren (B) wird im Allgemeinen unter Normaldruck durchgeführt.

Bei der Durchführung des erfindungsgemäßen Verfahrens (B) setzt man die Reaktionskomponenten der Formel (III) und die deprotonierenden Basen im Allgemeinen in etwa äquimolaren Mengen ein. Es ist jedoch auch möglich, die eine oder andere Komponente in einem größeren Überschuss (bis zu 3 Mol) zu verwenden.

Das Verfahren (C_{α}) ist dadurch gekennzeichnet, dass man Verbindungen der Formeln (I-1-a) bis (I-2-a) jeweils mit Carbonsäurehalogeniden der Formel (IV) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (C_{α}) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylformamid, Dimethylsulfoxid und Sulfolan. Wenn die Hydrolysestabilität des Säurehalogenids es zulässt, kann die Umsetzung auch in Gegenwart von Wasser durchgeführt werden.

Als Säurebindemittel kommen bei der Umsetzung nach dem erfindungsgemäßen Verfahren (C_{α}) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperatur kann bei dem erfindungsgemäßen Verfahren (C_{α}) innerhalb eines größeren Bereiches variiert werden. Im Allgemeinen arbeitet man bei Temperaturen zwischen -20°C und +150°C, vorzugsweise zwischen 0°C und 100°C.

Bei der Durchführung des erfindungsgemäßen Verfahrens (C_{α}) werden die Ausgangsstoffe der Formeln (I-1-a) bis (I-2-a) und das Carbonsäurehalogenid der Formel (IV) im Allgemeinen jeweils in angenähert äquivalenten Mengen verwendet. Es ist jedoch auch möglich, das Carbonsäurehalogenid in einem größeren Überschuss (bis zu 5 Mol) einzusetzen. Die Aufarbeitung erfolgt nach üblichen Methoden.

Das Verfahren (C_{β}) ist dadurch gekennzeichnet, dass man Verbindungen der Formeln (I-1-a) bis (I-2-a) jeweils mit Carbonsäureanhydriden der Formel (V) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (C_{β}) vorzugsweise diejenigen Verdünnungsmittel verwendet werden, die auch bei der Verwendung von Säurehalogeniden vorzugsweise in Betracht kommen. Im übrigen kann auch ein im Überschuss eingesetztes Carbonsäureanhydrid gleichzeitig als Verdünnungsmittel fungieren.

Als gegebenenfalls zugesetzte Säurebindemittel kommen beim Verfahren (C_{β}) vorzugsweise diejenigen Säurebindemittel in Frage, die auch bei der Verwendung von Säurehalogeniden vorzugsweise in Betracht kommen.

Die Reaktionstemperatur kann bei dem erfindungsgemäßen Verfahren (C_{β}) innerhalb eines größeren Bereiches variiert werden. Im Allgemeinen arbeitet man bei Temperaturen zwischen -20°C und +150°C, vorzugsweise zwischen 0°C und 100°C.

Bei der Durchführung des erfindungsgemäßen Verfahrens (C_{β}) werden die Ausgangsstoffe der Formeln (I-1-a) bis (I-2-a) und das Carbonsäureanhydrid der Formel (V) im Allgemeinen in jeweils angenähert äquivalenten Mengen verwendet. Es ist jedoch auch möglich, das Carbonsäureanhydrid in einem größeren Überschuss (bis zu 5 Mol) einzusetzen. Die Aufarbeitung erfolgt nach üblichen Methoden.

Im Allgemeinen geht man so vor, dass man Verdünnungsmittel und im Überschuss vorhandenes Carbonsäureanhydrid sowie die entstehende Carbonsäure durch Destillation oder durch Waschen mit einem organischen Lösungsmittel oder mit Wasser entfernt.

Das Verfahren (D) ist dadurch gekennzeichnet, dass man Verbindungen der Formeln (I-1-a) bis (I-2-a) jeweils mit Chlorameisensäureestern oder Chlorameisensäurethiolestern der Formel (VI) gegebenenfalls in Gegenwart eines Verdünnungsmittels und gegebenenfalls in Gegenwart eines Säurebindemittels umsetzt.

Als Säurebindemittel kommen bei dem erfindungsgemäßen Verfahren (D) alle üblichen Säureakzeptoren in Betracht. Vorzugsweise verwendbar sind tertiäre Amine, wie Triethylamin, Pyridin, DABCO, DBU, DBN, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (D) alle gegenüber den Chlorameisensäureestern bzw. Chlorameisensäurethiolestern inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, außerdem Nitrile wie Acetonitril und auch stark polare Solventien, wie Dimethylformamid, Dimethylsulfoxid und Sulfolan.

Die Reaktionstemperatur kann bei der Durchführung des erfindungsgemäßen Verfahrens (D) innerhalb eines größeren Bereiches variiert werden. Die Reaktionstemperatur liegt im Allgemeinen zwischen -20°C und +100°C, vorzugsweise zwischen 0°C und 50°C.

Das erfindungsgemäße Verfahren (D) wird im Allgemeinen unter Normaldruck durchgeführt.

Bei der Durchführung des erfindungsgemäßen Verfahrens (D) werden die Ausgangsstoffe der Formeln (I-1-a) bis (I-2-a) und der entsprechende Chlorameisensäureester bzw. Chlorameisensäurethiolester der Formel (VI) im Allgemeinen jeweils in angenähert äquivalenten Mengen verwendet. Es ist jedoch auch möglich, die eine oder andere Komponente in einem größeren Überschuss (bis zu 2 Mol) einzusetzen. Die Aufarbeitung erfolgt nach üblichen Methoden. Im Allgemeinen geht man so vor, dass man ausgefallene Salze entfernt und das verbleibende Reaktionsgemisch durch Abziehen des Verdünnungsmittels einengt.

Das erfindungsgemäße Verfahren (E) ist dadurch gekennzeichnet, dass man Verbindungen der Formeln (I-1-a) bis (I-2-a) jeweils mit Verbindungen der Formel (VII) in Gegenwart eines Verdünnungsmittels und gegebenenfalls in Gegenwart eines Säurebindemittels umsetzt.

Beim Herstellungsverfahren (E) setzt man pro Mol Ausgangsverbindung der Formeln (I-1-a) bis (I-2-a) ca. 1 Mol Chlormonothioameisensäureester bzw. Chlordithioameisensäureester der Formel (VII) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Ether, Amide, Sulfone, Sulfoxide, aber auch Halogenalkane.

Vorzugsweise werden Dimethylsulfoxid, Tetrahydrofuran, Dimethylformamid, Essigsäureethylester oder Methylenchlorid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindungen (I-1-a) bis (I-2-a) dar, kann auf den weiteren Zusatz von Säurebindemitteln verzichtet werden.

Als Basen können beim Verfahren (E) alle üblichen Protonenakzeptoren eingesetzt werden. Vorzugsweise verwendbar sind Alkalimetallhydride, Alkalimetallalkoholate, Alkali- oder Erdalkalimetallcarbonate oder -hydrogencarbonate oder Stickstoffbasen. Genannt seien beispielsweise Natriumhydrid, Natriummethanolat, Natriumhydroxid, Calciumhydroxid, Kaliumcarbonat, Natriumhydrogencarbonat, Triethylamin, Dibenzylamin, Diisopropylamin, Pyridin, Chinolin, Diazabicyclooctan (DABCO), Diazabicyclononen (DBN) und Diazabicycloundecen (DBU).

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (F) ist dadurch gekennzeichnet, dass man Verbindungen der Formeln (I-1-a) bis (I-2-a) jeweils mit Sulfonsäurechloriden der Formel (VIII) gegebenenfalls in Gegenwart eines Verdünnungsmittels und gegebenenfalls in Gegenwart eines Säurebindemittels umsetzt.

Beim Herstellungsverfahren (F) setzt man pro Mol Ausgangsverbindung der Formel (I-1-a) bis (I-2-a) ca. 1 Mol Sulfonsäurechlorid der Formel (VIII) bei -20 bis 150°C, vorzugsweise bei 0 bis 70°C um.

Das Verfahren (F) wird vorzugsweise in Gegenwart eines Verdünnungsmittels durchgeführt.

Als Verdünnungsmittel kommen alle inerten polaren organischen Lösungsmittel in Frage wie Ether, Amide, Ketone, Carbonsäureester, Nitrile, Sulfone, Sulfoxide oder halogenierte Kohlenwasserstoffe wie Methylenchlorid.

Vorzugsweise werden Dimethylsulfoxid, Tetrahydrofuran, Dimethylformamid, Essigsäureethylester, Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindungen (I-1-a) bis (I-2-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin und Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (G) ist dadurch gekennzeichnet, dass man Verbindungen der Formeln (I-1-a) bis (I-2-a) jeweils mit Phosphorverbindungen der Formel (IX) gegebenenfalls in Gegenwart eines Verdünnungsmittels und gegebenenfalls in Gegenwart eines Säurebindemittels umsetzt.

Beim Herstellungsverfahren (G) setzt man zum Erhalt von Verbindungen der Formeln (I-1-e) bis (I-2-e) auf 1 Mol der Verbindungen (I-1-a) bis (I-2-a), 1 bis 2, vorzugsweise 1 bis 1,3 Mol der Phosphorverbindung der Formel (IX) bei Temperaturen zwischen -40°C und 150°C, vorzugsweise zwischen -10 und 110°C um.

Das Verfahren (G) wird vorzugsweise in Gegenwart eines Verdünnungsmittels durchgeführt.

Als Verdünnungsmittel kommen alle inerten, polaren organischen Lösungsmittel in Frage wie Ether, Carbonsäureester, halogenierte Kohlenwasserstoffe, Ketone, Amide, Nitrile, Sulfone, Sulfoxide etc.

Vorzugsweise werden Acetonitril, Dimethylsulfoxid, Tetrahydrofuran, Dimethylformamid, Methylenchlorid eingesetzt.

Als gegebenenfalls zugesetzte Säurebindemittel kommen übliche anorganische oder organische Basen in Frage wie Hydroxide, Carbonate oder Amine. Beispielhaft seien Natriumhydroxid, Natriumcarbonat, Kaliumcarbonat, Pyridin und Triethylamin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der Organischen Chemie. Die Endprodukte werden vorzugsweise durch Kristallisation, chromatographische Reinigung oder durch sogenanntes "Andestillieren", d.h. Entfernung der flüchtigen Bestandteile im Vakuum gereinigt.

Das Verfahren (H) ist dadurch gekennzeichnet, dass man Verbindungen der Formeln (I-1-a) bis (I-2-a) jeweils mit Metallhydroxiden bzw. Metallalkoxiden der Formel (X) oder Aminen der Formel (XI), gegebenenfalls in Gegenwart eines Verdünnungsmittels, umsetzt.

Als Verdünnungsmittel können bei dem erfindungsgemäßen Verfahren (H) vorzugsweise Ether wie Tetrahydrofuran, Dioxan, Diethylether oder aber Alkohole wie Methanol, Ethanol, Isopropanol, aber auch Wasser eingesetzt werden. Das erfindungsgemäße Verfahren (H) wird im Allgemeinen unter Normaldruck durchgeführt. Die Reaktionstemperatur liegt im Allgemeinen zwischen -20°C und 100°C, vorzugsweise zwischen 0°C und 50°C.

Das erfindungsgemäße Verfahren (I) ist dadurch gekennzeichnet, dass man Verbindungen der Formeln (I-1-a) bis (I-2-a) jeweils mit (Iα) Verbindungen der Formel (XII) gegebenenfalls in Gegenwart eines Verdünnungsmittels und gegebenenfalls in Gegenwart eines Katalysators oder (Iß) mit Verbindungen der Formel (XIII) gegebenenfalls in Gegenwart eines Verdünnungsmittels und gegebenenfalls in Gegenwart eines Säurebindemittels umsetzt.

Bei Herstellungsverfahren (Iα) setzt man pro Mol Ausgangsverbindung der Formeln (I-1-a) bis (I-2-a) ca. 1 Mol Isocyanat der Formel (XII) bei 0 bis 100°C, vorzugsweise bei 20 bis 50°C um.

Das Verfahren (Iα) wird vorzugsweise in Gegenwart eines Verdünnungsmittels durchgeführt.

Als Verdünnungsmittel kommen alle inerten organischen Lösungsmittel in Frage, wie aromatische Kohlenwasserstoffe, halogenierte Kohlenwasserstoffe, Ether, Amide, Nitrile, Sulfone oder Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden.

Es wird vorzugsweise bei Normaldruck gearbeitet.

Beim Herstellungsverfahren (Iß) setzt man pro Mol Ausgangsverbindung der Formeln (I-1-a) bis (I-2-a) ca. 1 Mol Carbamidsäurechlorid der Formel (XIII) bei 0 bis 150°C, vorzugsweise bei 20 bis 70°C um.

Als gegebenenfalls zugesetzte Verdünnungsmittel kommen alle inerten polaren organischen Lösungsmittel in Frage wie Ether, Carbonsäureester, Nitrile, Ketone, Amide, Sulfone, Sulfoxide oder halogenierte Kohlenwasserstoffe.

Vorzugsweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung (I-1-a) bis (I-2-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Triethylamin oder Pyridin genannt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Anoplura (Phthiraptera) z.B. Damalinia spp., Haematopinus spp., Linognathus spp., Pediculus spp., Trichodectes spp.
Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.
Aus der Klasse der Bivalva z.B. Dreissena spp.
Aus der Ordnung der Chilopoda z.B. Geophilus spp., Scutigera spp.
Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp.
Aus der Ordnung der Collembola z.B. Onychiurus armatus.
Aus der Ordnung der Dermaptera z.B. Forficula auricularia.
Aus der Ordnung der Diplopoda z.B. Blaniulus guttulatus.
Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.
Aus der Klasse der Gastropoda z.B. Arion spp., Biomphalaria spp., Bulinus spp., Deroceras spp., Galba spp., Lymnaea spp., Oncomelania spp., Succinea spp.
Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

Aus der Ordnung der Hymenoptera z.B. Diprion spp., Hoplocampa spp., Lasius spp., Monomorium pharaonis, Vespa spp.

Aus der Ordnung der Isopoda z.B. Armadillidium vulgare, Oniscus asellus, Porcellio scaber.

Aus der Ordnung der Isoptera z.B. Reticulitermes spp., Odontotermes spp.

Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp.

Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella immaculata.

Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp.

Aus der Ordnung der Thysanura z.B. Lepisma saccharina.

Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp.

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgerührt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoffimprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP- POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Die Formulierungen enthalten im Allgemeinen zwischen 0,01 und 98 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen.

Besonders günstige Mischpartner sind z.B. die folgenden:

### Fungizide:

Inhibitoren der Nucleinsäure Synthese
   Benalaxyl, Benalaxyl-M, Bupirimat, Chiralaxyl, Clozylacon, Dimethirimol, Ethirimol,
   Furalaxyl, Hymexazol, Metalaxyl, Metalaxyl-M, Ofurace, Oxadixyl, Oxolinsäure
Inhibitoren der Mitose und Zellteilung
   Benomyl, Carbendazim, Diethofencarb, Fuberidazole, Pencycuron, Thiabendazol, Thiophanat-methyl, Zoxamid
Inhibitoren der Atmungskette Komplex I
   Diflumetorim
Inhibitoren der Atmungskette Komplex II
   Boscalid, Carboxin, Fenfuram, Flutolanil, Furametpyr, Mepronil, Oxycarboxin, Penthiopyrad, Thifluzamid
Inhibitoren der Atmungskette Komplex III
   Azoxystrobin, Cyazofamid, Dimoxystrobin, Enestrobin, Famoxadon, Fenamidon, Fluoxastrobin, Kresoximmethyl, Metominostrobin, Orysastrobin, Pyraclostrobin, Picoxystrobin, Trifloxystrobin
Entkoppler
   Dinocap, Fluazinam
Inhibitoren der ATP Produktion
   Fentinacetat, Fentinchlorid, Fentinhydroxid, Silthiofam
Inhibitoren der Aminosäure- und Proteinbiosynthese
   Andoprim, Blasticidin-S, Cyprodinil, Kasugamycin, Kasugamycinhydrochlorid Hydrat, Mepanipyrim, Pyrimethanil
Inhibitoren der Signal-Transduktion
   Fenpiclonil, Fludioxonil, Quinoxyfen
Inhibitoren der Fett- und Membran Synthese
   Chlozolinat, Iprodion, Procymidon, Vinclozolin
   Ampropylfos, Kalium-Ampropylfos, Edifenphos, Iprobenfos (IBP), Isoprothiolan, Pyrazophos
   Tolclofos-methyl, Biphenyl
   Iodocarb, Propamocarb, Propamocarb hydrochlorid
Inhibitoren der Ergosterol Biosynthese
   Fenhexamid,
   Azaconazol, Bitertanol, Bromuconazol, Cyproconazol, Diclobutrazol, Difenoconazol, Diniconazol, Diniconazol-M, Epoxiconazol, Etaconazol, Fenbuconazol, Fluquinconazol, Flusilazol, Flutriafol, Furconazol, Furconazol-cis, Hexaconazol, Imibenconazol, Ipconazol, Metconazol, Myclobutanil, Paclobutrazol, Penconazol, Propiconazol, Prothioconazol, Simeconazol, Tebuconazol, Tetraconazol, Triadimefon, Triadimenol, Triticonazol, Uniconazol, Voriconazol, Imazalil, Imazalilsulfat, Oxpoconazol, Fenarimol, Flurprimidol, Nuarimol, Pyrifenox, Triforin, Pefurazoat, Prochloraz, Triflumizol, Viniconazol,
   Aldimorph, Dodemorph, Dodemorphacetat, Fenpropimorph, Tridemorph, Fenpropidin, Spiroxamin,
   Naftifin, Pyributicarb, Terbinafin
Inhibitoren der Zellwand Synthese
   Benthiavalicarb, Bialaphos, Dimethomorph, Flumorph, Iprovalicarb, Polyoxins, Polyoxorim, Validamycin A
Inhibitoren der Melanin Biosynthese
   Capropamid, Diclocymet, Fenoxanil, Phtalid, Pyroquilon, Tricyclazol
Resistenzinduktion
   Acibenzolar-S-methyl, Probenazol, Tiadinil
Multisite
   Captafol, Captan, Chlorothalonil, Kupfersalze wie: Kupferhydroxid, Kupfernaphthenat, Kupferoxychlorid, Kupfersulfat, Kupferoxid, Oxin-Kupfer und Bordeaux Mischung, Dichlofluanid, Dithianon, Dodin, Dodin freie Base, Ferbam, Folpet, Fluorofolpet, Guazatin, Guazatinacetat, Iminoctadin, Iminoctadinalbesilat, Iminoctadintriacetat, Mankupfer, Mancozeb, Maneb, Metiram, Metiram Zink, Propineb, Schwefel und Schwefelpräparate enthaltend Calciumpolysulphid, Thiram, Tolylfluanid, Zineb, Ziram
Unbekannter Mechanismus
   Amibromdol, Benthiazol, Bethoxazin, Capsimycin, Carvon, Chinomethionat, Chloropicrin, Cufraneb, Cyflufenamid, Cymoxanil, Dazomet, Debacarb, Diclomezine, Dichlorophen, Dicloran, Difenzoquat, Difenzoquat Methylsulphat, Diphenylamin, Ethaboxam, Ferimzon, flumetover, Flusulfamid, Fluopicolid, Fluoroimid, Hexachlorobenzol, 8-Hydroxy-chinolinsulfat, Irumamycin, Methasulphocarb, Metrafenon, Methyl Isothiocyanat, Mildiomycin, Natamycin, Nickel dimethyldithiocarbamat, Nitrothal-isopropyl, Octhilinon, Oxamocarb, Oxyfenthiin, Pentachlorophenol und Salze, 2-Phenylphenol und Salze, Piperalin, Propanosin -Natrium, Proquinazid, Pyrrolnittin, Quintozen, Tecloftalam, Tecnazen, Triazoxid, Trichlamid, Zarilamid und 2,3,5,6-Tetrachlor-4-(methylsulfonyl)-pyridin, N-(4-Chlor-2-nitrophenyl)-N-ethyl-4-methyl-benzenesulfonamid, 2-Amino-4-methyl-N-phenyl-5-thiazolecarboxamid, 2-Chlor-N-(2,3-dihydro-1,1,3-trimethyl-1H-inden-4-yl)-3-pyridincarboxamid, 3-[5-(4-Chlorphenyl)-2,3-dimethylisoxazolidin-3-yl]pyridin, cis-1-(4-Chlorphenyl)-2-(1H-1,2,4-triazol-1-yl)-cycloheptanol, 2,4-Dihydro-5-methoxy-2-methyl-4-[[[[1-[3-(trifluoromethyl)-phenyl]-ethyliden]-amino]-oxy]-methyl]-phenyl]-3H-1,2,3-triazol-3-on (185336-79-2), Methyl 1-(2,3-dihydro-2,2-dimethyl-1H-inden-1-yl)-1H-imidazole-5-carboxylat, 3,4,5-Trichlor-2,6-pyridindicarbonitril, Methyl 2-[[[cyclopropyl[(4-methoxyphenyl) imino]methyl]thio]methyl]-.alpha.-(methoxymethylen)- benzacetat, 4-Chlor-alpha-propinyloxy-N-[2-[3-methoxy-4-(2-propinyloxy)phenyl]ethyl]-benzacetamide, (2S)-N-[2-[4-[[3-(4-chlorophenyl)-2-propinyl]oxy]-3-methoxyphenyl]ethyl]-3-methyl-2-[(methyl-sulfonyl)amino]-butanamid, 5-Chlor-7-(4-methylpiperidin-1-yl)-6-(2,4,6-trifluorophenyl)-[1,2,4]triazolo[1,5-a]pyrimidin, 5-Chlor-6-(2,4,6-trifluorophenyl)-N-[(1R)-1,2,2-trimethyl-propyl][1,2,4]triazolo[1,5-a]pyrimidin-7-amin, 5-Chlor-N-[(R)-1,2-dimethylpropyl]-6-(2,4,6-trifluorophenyl) [1,2,4]triazolo[1,5-a]pyrimidin-7-amine, N-[1-(5-Brom-3-chloro-pyridin-2-yl)ethyl]-2,4-dichloronicotinamid, N-(5-Brom-3-chlorpyridin-2-yl)methyl-2,4-dichlornicotinamid, 2-Butoxy-6-iod-3-propyl-benzopyranon-4-on, N-{(Z)-[(cyclopropyl-methoxy)imino][6-(difluormethoxy)-2,3-difluorphenyl]methyl}-2-benzacetamid, N-(3-Ethyl-3,5,5-trimethyl-cyclohexyl)-3-formylamino-2-hydroxy-benzamid, 2-[[[[1-[3(1Fluor-2-phenyl-ethyl)oxy] phenyl]ethyliden]amino]oxy]methyl]-alpha-(methoxyimino)-N-methyl-alphaE-benzacetamid, N-{2-[3-Chlor-5-(trifluormethyl)pyridin-2-yl]ethyl}-2-(trifluoromethyl)-benzamid, N-(3',4'-dichlor-5-fluorbiphenyl-2-yl)-3-(difluormethyl)-1-methyl-1H-pyrazol-4-carboxamid, N-(6-Methoxy-3-pyridinyl)-cyclopropan carboxamid, 1-[(4-Methoxyphenoxy)-methyl]-2,2-dimethylpropyl-1H-imidazol-1- carbonsäure, O-[1-[(4-Methoxyphenoxy) methyl]-2,2-dimethylpropyl]-1H-imidazol- 1- carbothioic acid, 2-(2-{[6-(3-Chlor-2-methyl-phenoxy)-5-fluorpyrimidin-4-yl]oxy}phenyl)-2-(methoxyimino)-N-methylacetamid

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Acetylcholinesterase (AChE) Inhibitoren
   Carbamate,
      zum Beispiel Alanycarb, Aldicarb, Aldoxycarb, Allyxycarb, Aminocarb, Bendiocarb, Benfuracarb, Bufencarb, Butacarb, Butocarboxim, Butoxycarboxim, Carbaryl, Carbofuran, Carbosulfan, Cloethocarb, Dimetilan, Ethiofencarb, Fenobucarb, Fenothiocarb, Formetanate, Furathiocarb, Isoprocarb, Metam-sodium, Methiocarb, Methomyl, Metolcarb, Oxamyl, Pirimicarb, Promecarb, Propoxur, Thiodicarb, Thiofanox, Trimethacarb, XMC, Xylylcarb, Triazamate
   Organophosphate,
      zum Beispiel Acephate, Azamethiphos, Azinphos (-methyl, -ethyl), Bromophos-ethyl, Bromfenvinfos (-methyl), Butathiofos, Cadusafos, Carbophenothion, Chlorethoxyfos, Chlorfenvinphos, Chlormephos, Chlorpyrifos (-methyl/-ethyl), Coumaphos, Cyanofenphos, Cyanophos, Chlorfenvinphos, Demeton-S-methyl, Demeton-S-methylsulphon, Dialifos, Diazinon, Dichlofenthion, Dichlorvos/DDVP, Dicrotophos, Dimethoate, Dimethylvinphos, Dioxabenzofos, Disulfoton, EPN, Ethion, Ethoprophos, Etrimfos, Famphur, Fenamiphos, Fenitrothion, Fensulfothion, Fenthion, Flupyrazofos, Fonofos, Formothion, Fosmethilan, Fosthiazate, Heptenophos, Iodofenphos, Iprobenfos, Isazofos, Isofenphos, Isopropyl O-salicylate, Isoxathion, Malathion, Mecarbam, Methacrifos, Methamidophos, Methidathion, Mevinphos, Monocrotophos, Naled, Omethoate, Oxydemeton-methyl, Parathion (-methyl/-ethyl), Phenthoate, Phorate, Phosalone, Phosmet, Phosphamidon, Phosphocarb, Phoxim, Pirimiphos (-methyl/-ethyl), Profenofos, Propaphos, Propetamphos, Prothiofos, Prothoate, Pyraclofos, Pyridaphenthion, Pyridathion, Quinalphos, Sebufos, Sulfotep, Sulprofos, Tebupirimfos, Temephos, Terbufos, Tetrachlorvinphos, Thiometon, Triazophos, Triclorfon, Vamidothion
Natrium-Kanal-Modulatoren / Spannungsabhängige Natrium-Kanal-Blocker
   Pyrethroide,
      zum Beispiel Acrinathrin, Allethrin (d-cis-trans, d-trans), Beta-Cyfluthrin, Bifenthrin, Bioallethrin, Bioallethrin-S-cyclopentyl-isomer, Bioethanomethrin, Biopermethrin, Bioresmethrin, Chlovaporthrin, Cis-Cypermethrin, Cis-Resmethrin, Cis-Permethrin, Clocythrin, Cycloprothrin, Cyfluthrin, Cyhalothrin, Cypermethrin (alpha-, beta-, theta-, zeta-), Cyphenothrin, Deltamethrin, Empenthrin (1R-isomer), Esfenvalerate, Etofenprox, Fenfluthrin, Fenpropathrin, Fenpyrithrin, Fenvalerate, Flubrocythrinate, Flucythrinate, Flufenprox, Flumethrin, Fluvalinate, Fubfenprox, Gamma-Cyhalothrin, Imiprothrin, Kadethrin, Lambda- Cyhalothrin, Metofluthrin, Permethrin (cis-, trans-), Phenothrin (1R-trans isomer), Prallethrin, Profluthrin, Protrifenbute, Pyresmethrin, Resmethrin, RU 15525, Silafluofen, Tau-Fluvalinate, Tefluthrin, Terallethrin, Tetramethrin (-1R- isomer), Tralomethrin, Transfluthrin, ZXI 8901, Pyrethrins (pyrethrum)
   DDT
   Oxadiazine,
      zum Beispiel Indoxacarb
   Semicarbazone,
      zum Beispiel Metaflumizone (BAS 320 1)
Acetylcholin-Rezeptor-Agonisten/-Antagonisten
   Chloronicotinyle,
      zum Beispiel Acetamiprid, Clothianidin, Dinotefuran, Imidacloprid, Nitenpyram, Nithiazine, Thiacloprid, Thiamethoxam
   Nicotine, Bensultap, Cartap
Acetylcholin-Rezeptor-Modulatoren
   Spinosyne,
      zum Beispiel Spinosad
GABA-gesteuerte Chlorid-Kanal-Antagonisten
   Organochlorine,
      zum Beispiel Camphechlor, Chlordane, Endosulfan, Gamma-HCH, HCH, Heptachlor, Lindane, Methoxychlor
   Fiprole,
      zum Beispiel Acetoprole, Ethiprole, Fipronil, Pyrafluprole, Pyriprole, Vaniliprole
Chlorid-Kanal-Aktivatoren
   Mectine,
      zum Beispiel Avermectin, Emamectin, Emamectin-benzoate, Ivermectin, Milbemycin
Juvenilhormon-Mimetika,
   zum Beispiel Diofenolan, Epofenonane, Fenoxycarb, Hydroprene, Kinoprene, Methoprene, Pyriproxifen, Triprene
Ecdysonagonisten/disruptoren
   Diacylhydrazine,
      zum Beispiel Chromafenozide, Halofenozide, Methoxyfenozide, Tebufenozide Inhibitoren der Chitinbiosynthese
   Benzoylharnstoffe,
      zum Beispiel Bistrifluron, Chlofluazuron, Diflubenzuron, Fluazuron, Flucycloxuron, Flufenoxuron, Hexaflumuron, Lufenuron, Novaluron, Noviflumuron, Penfluron, Teflubenzuron, Triflumuron
   Buprofezin
   Cyromazine
Inhibitoren der oxidativen Phosphorylierung, ATP-Disruptoren
   Diafenthiuron
   Organozinnverbindungen,
      zum Beispiel Azocyclotin, Cyhexatin, Fenbutatin-oxide
Entkoppler der oxidativen Phoshorylierung durch Unterbrechung des H-Protongradienten
   Pyrrole,
      zum Beispiel Chlorfenapyr
   Dinitrophenole,
      zum Beispiel Binapacyrl, Dinobuton, Dinocap, DNOC
Seite-I-Elektronentransportinhibitoren
   METI's,
      zum Beispiel Fenazaquin, Fenpyroximate, Pyrimidifen, Pyridaben, Tebufenpyrad, Tolfenpyrad
   Hydramethylnon
   Dicofol
Seite-II-Elektronentransportinhibitoren
   Rotenone
Seite-III-Elektronentransportinhibitoren
   Acequinocyl, Fluacrypyrim
Mikrobielle Disruptoren der Insektendarmmembran
   Bacillus thuringiensis-Stämme
Inhibitoren der Fettsynthese
   Tetronsäuren,
   zum Beispiel Spirodiclofen, Spiromesifen
   Tetramsäuren,
   zum Beispiel Spirotetramat
Carboxamide,
   zum Beispiel Flonicamid
Oktopaminerge Agonisten,
   zum Beispiel Amitraz
Inhibitoren der Magnesium-stimulierten ATPase,
   Propargite
Ryanodinrezeptor-Effektoren
   a) Benzoesäuredicarboxamide,
      zum Beispiel Flubendiamide
   b) Anthranilamide, z.B.
      Rynaxapyr (3-bromo-N-{4-chloro-2-methyl-6-[(methylamino)carbonyl]phenyl}-1-(3-chloropyridin-2-yl)-1H-pyrazole-5-carboxamide)
Nereistoxin-Analoge,
   zum Beispiel Thiocyclam hydrogen oxalate, Thiosultap-sodium Biologika, Hormone oder Pheromone
   Azadirachtin, Bacillus spec., Beauveria spec., Codlemone, Metarrhizium spec., Paecilomyces spec., Thuringiensin, Verticillium spec.
Wirkstoffe mit unbekannten oder nicht spezifischen Wirkmechanismen
   Begasungsmittel,
      zum Beispiel Aluminium phosphide, Methyl bromide, Sulfuryl fluoride
   Fraßhemmer,
      zum Beispiel Cryolite, Flonicamid, Pymetrozine
   Milbenwachstumsinhibitoren,
      zum Beispiel Clofentezine, Etoxazole, Hexythiazox
   Amidoflumet, Benclothiaz, Benzoximate, Bifenazate, Bromopropylate, Buprofezin, Chinomethionat, Chlordimeform, Chlorobenzilate, Chloropicrin, Clothiazoben, Cycloprene, Cyflumetofen, Dicyclanil, Fenoxacrim, Fentrifanil, Flubenzimine, Flufenerim, Flutenzin, Gossyplure, Hydramethylnone, Japonilure, Metoxadiazone, Petroleum, Piperonyl butoxide, Potassium oleate, Pyridalyl, Sulfluramid, Tetradifon, Tetrasul, Triarathene,Verbutin

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.% Wirkstoff, vorzugsweise zwischen 0,00001 und 1 Gew.% liegen.

Die Anwendung geschieht in einer den Anwendungsformen angepaßten üblichen Weise.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp.
Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp.
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp.
Aus der Ordnung der Siphonapterida z.B. Pulex spp., Ctenocephalides spp., Xenopsylla spp., Ceratophyllus spp.
Aus der Ordnung der Heteropterida z.B. Cimex spp., Triatoma spp., Rhodnius spp., Panstrongylus spp.
Aus der Ordnung der Blattarida z.B. Blatta orientalis, Periplaneta americana, Blattela germanica, Supella spp.
Aus der Unterklasse der Acari (Acarina) und den Ordnungen der Meta- sowie Mesostigmata z.B. Argas spp., Omithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp.
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so dass durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, dass die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirexjuvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Borstenschwänze wie Lepisma saccharina.
Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.
Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.
Aus der Ordnung der Araneae z.B. Aviculariidae, Araneidae.
Aus der Ordnung der Opiliones z.B. Pseudoscorpiones chelifer, Pseudoscorpiones cheiridium, Opiliones phalangium.
Aus der Ordnung der Isopoda z.B. Oniscus asellus, Porcellio scaber.
Aus der Ordnung der Diplopoda z.B. Blaniulus guttulatus, Polydesmus spp.
Aus der Ordnung der Chilopoda z.B. Geophilus spp.
Aus der Ordnung der Zygentoma z.B. Ctenolepisma spp., Lepisma saccharina, Lepismodes inquilinus.
Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.
Aus der Ordnung der Saltatoria z.B. Acheta domesticus.
Aus der Ordnung der Dermaptera z.B. Forficula auricularia.
Aus der Ordnung der Isoptera z.B. Kalotermes spp., Reticulitermes spp.
Aus der Ordnung der Psocoptera z.B. Lepinatus spp., Liposcelis spp.
Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.
Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.
Aus der Ordnung der Lepidoptera z.B. Achroia grisella, Galleria mellonella, Plodia interpunctella, Tinea cloacella, Tinea pellionella, Tineola bisselliella.
Aus der Ordnung der Siphonaptera z.B. Ctenocephalides canis, Ctenocephalides felis, Pulex irritans, Tunga penetrans, Xenopsylla cheopis.
Aus der Ordnung der Hymenoptera z.B. Camponotus herculeanus, Lasius fuliginosus, Lasius niger, Lasius umbratus, Monomorium pharaonis, Paravespula spp., Tetramorium caespitum.
Aus der Ordnung der Anoplura z.B. Pediculus humanus capitis, Pediculus humanus corporis, Pemphigus spp., Phylloera vastatrix, Phthirus pubis.
Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neonicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propeller Verdampfern, energielosen bzw. passiven Verdampfungssystemen, Mottenpapieren, Mottensäckchen und Mottengelen, als Granulate oder Stäube, in Streuködern oder Köderstationen.

Die erfindungsgemäßen Wirkstoffe/Wirkstoffkombinationen können auch als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe/Wirkstoffkombinationen können z.B. bei den folgenden Pflanzen verwendet werden:
Dikotyle Unkräuter der Gattungen: Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.
Dikotyle Kulturen der Gattungen: Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia.
Monokotyle Unkräuter der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.
Monokotyle Kulturen der Gattungen: Allium, Ananas, Asparagus, Avena, Hordeum, Oryza, Panicum, Saccharum, Secale, Sorghum, Triticale, Triticum, Zea.

Die Verwendung der erfindungsgemäßen Wirkstoffe/Wirkstoffkombinationen ist jedoch keineswegs auf diese Gattungen beschränkt, sondern erstreckt sich in gleicher Weise auch auf andere Pflanzen.

Die erfindungsgemäßen Wirkstoffe/Wirkstoffkombinationen eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuss-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Verbindungen der Formel (I)/Wirkstoffkombinationen zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf-Verfahren.

Die erfindungsgemäßen Wirkstoffe/Wirkstoffkombinationen können in bestimmten Konzentrationen bzw. Aufwandmengen auch zur Bekämpfung von tierischen Schädlingen und pilzlichen oder bakteriellen Pflanzenkrankheiten verwendet werden. Sie lassen sich gegebenenfalls auch als Zwischen- oder Vorprodukte für die Synthese weiterer Wirkstoffe einsetzen.

Die Wirkstoffe/Wirkstoffkombinationen können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im Allgemeinen zwischen 0,1 und 95 Gewichtsprozent Wirkstoff, vorzugsweise zwischen 0,5 und 90 %.

Die erfindungsgemäßen Wirkstoffe/Wirkstoffkombinationen können als solche oder in ihren Formulierungen auch in Mischung mit bekannten Herbiziden und/oder mit Stoffen, welche die Kulturpflanzen-Verträglichkeit verbessern ("Safenern") zur Unkrautbekämpfung verwendet werden, wobei Fertigformulierungen oder Tankmischungen möglich sind. Es sind also auch Mischungen mit Unkrautbekämpfungsmitteln möglich, welche ein oder mehrere bekannte Herbizide und einen Safener enthalten.

Für die Mischungen kommen bekannte Herbizide infrage, beispielsweise
Acetochlor, Acifluorfen (-sodium), Aclonifen, Alachlor, Alloxydim (-sodium), Ametryne, Amicarbazone, Amidochlor, Amidosulfuron, Aminopyralid, Anilofos, Asulam, Atrazine, Azafenidin, Azimsulfuron, Beflubutamid, Benazolin (-ethyl), Benfuresate, Bensulfuron (-methyl), Bentazon, Benzcarbazone, Benzfendizone, Benzobicyclon, Benzofenap, Benzoylprop (-ethyl), Bialaphos, Bifenox, Bispyribac (-sodium), Bromobutide, Bromofenoxim, Bromoxynil, Butachlor, Butafenacil (-allyl), Butroxydim, Butylate, Cafenstrole, Caloxydim, Carbetamide, Carfentrazone (-ethyl), Chlomethoxyfen, Chloramben, Chloridazon, Chlorimuron (-ethyl), Chlomitrofen, Chlorsulfuron, Chlortoluron, Cinidon (-ethyl), Cinmethylin, Cinosulfuron, Clefoxydim, Clethodim, Clodinafop (-propargyl), Clomazone, Clomeprop, Clopyralid, Clopyrasulfuron (-methyl), Cloransulam (-methyl), Cumyluron, Cyanazine, Cybutryne, Cycloate, Cyclosulfamuron, Cycloxydim, Cyhalofop (-butyl), 2,4-D, 2,4-DB, Desmedipham, Diallate, Dicamba, Dichlorprop (-P), Diclofop (-methyl), Diclosulam, Diethatyl (-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epropodan, EPTC, Esprocarb, Ethalfluralin, Ethametsulfuron (-methyl), Ethofumesate, Ethoxyfen, Ethoxysulfuron, Etobenzanid, Fenoxaprop (-P-ethyl), Fentrazamide, Flamprop (-isopropyl, -isopropyl-L, -methyl), Flazasulfuron, Florasulam, Fluazifop (-P-butyl), Fluazolate, Flucarbazone (-sodium), Flufenacet, Flumetsulam, Flumiclorac (-pentyl), Flumioxazin, Flumipropyn, Flumetsulam, Fluometuron, Fluorochloridone, Fluoroglycofen (-ethyl), Flupoxam, Flupropacil, Flurpyrsulfuron (-methyl, -sodium), Flurenol (-butyl), Fluridone, Fluroxypyr (-butoxypropyl, -meptyl), Flurprimidol, Flurtamone, Fluthiacet (-methyl), Fluthiamide, Fomesafen, Foramsulfuron, Glufosinate (-ammonium), Glyphosate (-isopropylammonium), Halosafen, Haloxyfop (-ethoxyethyl, -P-methyl), Hexazinone, HOK-201, Imazamethabenz -methyl), Imazamethapyr, Imazamox, Imazapic, Imazapyr, Imazaquin, Imazethapyr, Imazosulfuron, Iodosulfuron (-methyl, -sodium), Ioxynil, Isopropalin, Isoproturon, Isouron, Isoxaben, Isoxachlortole, Isoxaflutole, Isoxapyrifop, Lactofen, Lenacil, Linuron, MCPA, Mecoprop, Mefenacet, Mesosulfurone, Mesotrione, Metamifop, Metamitron, Metazachlor, Methabenzthiazuron, Metobenzuron, Metobromuron, (alpha-) Metolachlor, Metosulam, Metoxuron, Metribuzin, Metsulfuron (-methyl), Molinate, Monolinuron, Naproanilide, Napropamide, Neburon, Nicosulfuron, Norflurazon, Orbencarb, Orthosulfamuron, Oryzalin, Oxadiargyl, Oxadiazon, Oxasulfuron, Oxaziclomefone, Oxyfluorfen, Paraquat, Pelargonsäure, Pendimethalin, Pendralin, Penoxsulam, Pentoxazone, Phenmedipham, Picolinafen, Pinoxaden, Piperophos, Pretilachlor, Primisulfuron (-methyl), Profluazol, Prometryn, Propachlor, Propanil, Propaquizafop, Propisochlor, Propoxycarbazone (-sodium), Propyzamide, Prosulfocarb, Prosulfuron, Pyraflufen (-ethyl), Pyrasulfotole, Pyrazogyl, Pyrazolate, Pyrazosulfuron (-ethyl), Pyrazoxyfen, Pyribenzoxim, Pyributicarb, Pyridate, Pyridatol, Pyriftalid, Pyriminobac (-methyl), Pyrimisulfan, Pyrithiobac (-sodium), Pyroxsulam, Pyroxasulfone, Quinchlorac, Quinmerac, Quinoclamine, Quizalofop (-P-ethyl, -P-tefuryl), Rimsulfuron, Sethoxydim, Simazine, Simetryn, Sulcotrione, Sulfentrazone, Sulfometuron (-methyl), Sulfosate, Sulfosulfuron, Tebutam, Tebuthiuron, Tembotrione, Tepraloxydim, Terbuthylazine, Terbutryn, Thenylchlor, Thiafluamide, Thiazopyr, Thidiazimin, Thiencarbazone-methyl, Thifensulfuron (-methyl), Thiobencarb, Tiocarbazil, Topramezone, Tralkoxydim, Triallate, Triasulfuron, Tribenuron (-methyl), Triclopyr, Tridiphane, Trifluralin, Trifloxysulfuron, Triflusulfuron (-methyl), Tritosulfuron und

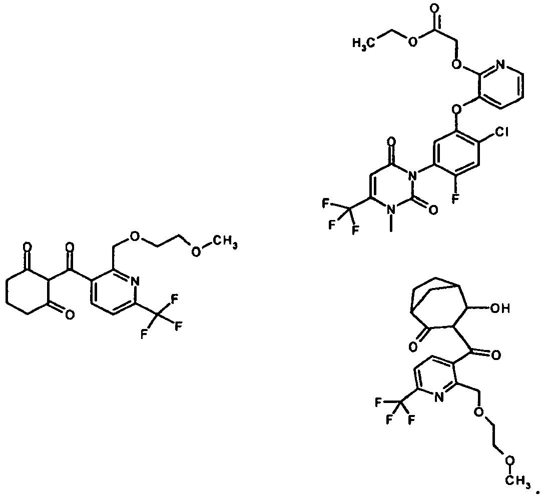

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe bzw. Wirkstoffkombinationen können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe bzw. Wirkstoffkombinationen können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im Allgemeinen liegen die Aufwandmengen zwischen 1 g und 10 kg Wirkstoff pro Hektar Bodenfläche, vorzugsweise zwischen 5 g und 5 kg pro ha.

Der vorteilhafte Effekt der Kulturpflanzen-Verträglichkeit der erfindungsgemäßen Wirkstoffkombinationen ist bei bestimmten Konzentrationsverhältnissen besonders stark ausgeprägt. Jedoch können die Gewichtsverhältnisse der Wirkstoffe in den Wirkstoffkombinationen in relativ großen Bereichen variiert werden. Im Allgemeinen entfallen auf 1 Gewichtsteil Wirkstoff der Formel (I) Salzen 0,001 bis 1000 Gewichtsteile, vorzugsweise 0,01 bis 100 Gewichtsteile, besonders bevorzugt 0,05 bis 20 Gewichtsteile einer der oben unter (b') genannten, die Kulturpflanzen Verträglichkeit verbessernden Verbindungen (Antidots/Safener).

Die erfindungsgemäßen Wirkstoffkombinationen werden im Allgemeinen in Form von Fertigformulierungen zur Anwendung gebracht. Die in den Wirkstoffkombinationen enthaltenen Wirkstoffe können aber auch in Einzelformulierungen bei der Anwendung gemischt, d.h. in Form von Tankmischungen zur Anwendung gebracht werden.

Für bestimmte Anwendungszwecke, insbesondere im Nachauflauf-Verfahren, kann es ferner vorteilhaft sein, in die Formulierungen als weitere Zusatzstoffe pflanzenverträgliche mineralische oder vegetabilische Öle (z.B. das Handelspräparat "Rako Binol") oder Ammoniumsalze wie z.B. Ammoniumsulfat oder Ammoniumrhodanid aufzunehmen.

Die neuen Wirkstoffkombinationen können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Stäuben oder Streuen.

Die Aufwandmengen der erfindungsgemäßen Wirkstoffkombinationen können in einem gewissen Bereich variiert werden; sie hängen u.a. vom Wetter und von den Bodenfaktoren ab. Im Allgemeinen liegen die Aufwandmengen zwischen 0,001 und 5 kg pro ha, vorzugsweise zwischen 0,005 und 2 kg pro ha, besonders bevorzugt zwischen 0,01 und 0,5 kg pro ha.

Die erfindungsgemäßen Wirkstoffkombinationen können vor und nach dem Auflaufen der Pflanzen appliziert werden, also im Vorauflauf und Nachauflauf-Verfahren.

Die erfindungsgemäß einzusetzenden Safener können je nach ihren Eigenschaften zur Vorbehandlung des Saatgutes der Kulturpflanze (Beizung der Samen) verwendet werden oder vor der Saat in die Saatfurchen eingebracht oder vor dem Herbizid separat angewendet werden oder zusammen mit dem Herbizid vor oder nach dem Ablaufen der Pflanzen angewendet werden.

Fungizide lassen sich im Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder
Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustitago nuda oder Ustilago avenae;
Pellicularia-Arten, wie beispielsweise Pellicularia sasakii;
Pyriculari-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die erfindungsgemäßen Wirkstoffe weisen auch eine starke stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokolation mit unerwünschten Mikroorgansimen weitgehende Resistenz gegen diese Mirkroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im Allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im Allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel die oben gannten Verbindungen (Fungizide, Bakterizide, Insektizide, Akarizide, Nematizide) in Frage.

Auch eine Mischung mit anderen bekannten Wirkstoffen, wie Herbiziden oder mit Düngemitteln und Wachstumsregulatoren ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sproßpilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes,

Microsporon-Spezies wie Microsporon canis und audouinii. Die Aufzählung dieser Pilze stellt keinesfalls eine Beschränkung des erfaßbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im Allgemeinen zwischen 0,1 und 10.000 g/ha, vorzugsweise zwischen 10 und 1.000 g/ha. Bei der Saatgutbehandlung liegen die Aufwandmengen an Wirkstoff im Allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im Allgemeinen zwischen 0,1 und 10.000 g/ha, vorzugsweise zwischen 1 und 5.000 g/ha.

Der Begriff Wirkstoff umfasst die genannten Wirkstoffkombinationen ebenso, sowie die formulierten Zusammensetzungen enthaltend Ammonium- und/oder Phosphoniumsalze und gegebenenfalls Penetrationsförderer.

Die Herstellung und die Verwendung der erfindungsgemäßen Wirkstoffe geht aus den nachfolgenden Beispielen hervor.

### Herstellungsbeispiele

### Beispiel I-1-a-1

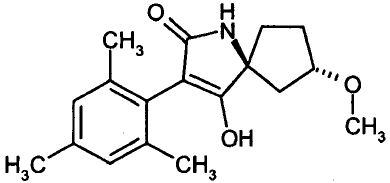

Kalium-tert.-butylat wird in 2 ml Dimethylacetamid vorgelegt und auf 100°C erwärmt. Man gibt bei dieser Temperatur in 10 Portionen innerhalb von 1 h 3 ml einer Lösung gemäß Beispiel II-1 Dimethylacetamid zu. Man lässt 2 h bei 100°C rühren und versetzt anschließend mit 20 ml Wasser und stellt mit konzentrierter Salzsäure auf pH = 1 ein. Anschließend engt man ein und nimmt in 50 ml Dichlormethan auf, trocknet mit Natriumsulfat und engt ein. Säulenchromatographische Reinigung (Gradient (n-Heptan/Essigsäure-ethylester 4:1 nach Essigsäure-ethylester) liefert 80 mg an Zielprodukt (Ausbeute: 42 % der Theorie) mit Fp.: 209 - 217°C.

In Analogie zu Beispiel (I-1-a-1) und gemäß den allgemeinen Angaben zur Herstellung erhält man folgende Verbindungen der Formel (1-1-a)

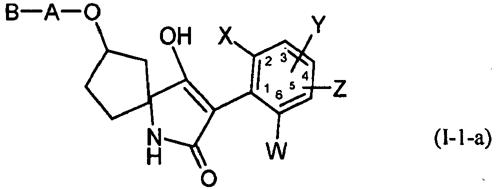

| **Bsp.-Nr.** | **W** | **X** | **Y** | **Z** | **A** | **B** | **Fp.°C** | **Isomer** |
|---|---|---|---|---|---|---|---|---|
| I-1-a-2 | C₂H₅ | Br | 4-CH₃ | H | CH₂ | H | erstarrter Schaum | cis |
| | | | | | | | *2.41 (m, 2H, Ar-CH₂) | |
| | | | | | | | 3.23 (s, 3 H, OCH₃) | |
| | | | | | | | 3.92 (m, 1H, CHOCH₃) | |
| I-1-a-3 | C₂H₅ | Br | 4-CH3 | H | CH₂ | H | Öl | trans |
| | | | | | | | *2.41 (m, 2H, Ar-CH₂) | |
| | | | | | | | 3.20 (s, 3H, OCH₃) | |
| | | | | | | | 4.01 (m, 1H, CHOCH₃) | |
| I-1-a-4 | OCH₃ | C₂H₅ | 4-Cl | H | CH₂ | H | erstarrtes Glas | trans |
| | | | | | | | *2.45 (m, 2H, Ar-CH₂) | |
| | | | | | | | 3.27 (s, 3H, CHOCH₃) | |
| | | | | | | | 4.01, 4.06 (2m, ∑ 1H, CHOCH₃) | |
| I-1-a-5 | OCH₃ | C₂H₅ | 4-Cl | H | CH₂ | H | 171-178 | cis |
| I-1-a-6 | CH₃ | CH₃ | 4-CH₃ | H | CH₂ | H | 232 | cis |
| I-1-a-7 | C₂H₅ | Br | 4-CH₃ | H | CH₂ | CH₃ | 182-186 | cis |
| I-1-a-8 | OCH₃ | C₂H₅ | 4-Cl | H | - | H | zähes Öl | cis |
| | | | | | | | *1.00, 1.09 (dt, 3H, Ar-CH₂-CH₃) | |
| | | | | | | | 4.14, 4.24(2 m, ∑, 1H, CHOH) | |
| I-1-a-9 | CH₃ | CH₃ | 4-CH₃ | H | CH₂ | CH₃ | 199-206 | cis |
| I-1-a-10 | CH₃ | CH₃ | 4-Br | H | CH₂ | H | 242 | cis |
| I-1-a-11 | CH₃ | Cl | 4-CH₃ | H | CH₂ | H | 243 | cis |
| I-1-a-12 | C₂H₅ | Br | 4-CH₃ | H | CH₂ | C₃H₇ | 70-82 | cis |
| I-1-a-13 | CH₃ | CH₃ | 4-CH₃ | H | CH₂ | C₃H₇ | 180-188 | cis |
| I-1-a-14 | C₂H₅ | Br | 4-CH₃ | H | CH₂ | C₃H₇ | 69-76 | trans |
| I-1-a-15 | CH₃ | CH₃ | 4-CH₃ | H | CH₂ | C₃H₇ | Öl | trans |
| | | | | | | | *6.84 ppm (s, 2H, Ar-H), 4.10 ppm (m, 1H, CH-O), 3.37 ppm (t, 2H, CH-OCH₂), | |
| I-1-a-16 | H | CH₃ | 5-(4-Cl-Ph) | H | CH₂ | H | 231-233 | cis |
| I-1-a-17 | CH₃ | CH₃ | 4-CH₃ | H | CH₂ | C₂H₅ | 224 | cis |
| I-1-a-18 | CH₃ | CH₃ | 4-CH₃ | H | CH₂ | C₂H₅ | erstarrter Schaum | trans |
| | | | | | | | *6.84 ppm (s, 2H, Ar-H), 4.11 ppm (m, 1H, CH-O), 3.33 ppm (t, 2H, CH-OCH₂), | |
| I-1-a-19 | C₂H₅ | Br | 4-CH₃ | H | CH₂ | C₂H₅ | 172-174 | cis |
| I-1-a-20 | C₂H₅ | Br | 4-CH₃ | H | CH₂ | C₂H₅ | erstarrter Schaum | cis |
| | | | | | | | *7.30 und 7.02 ppm (je s, 1H, Ar-H), 4.10 ppm (m, 1H, CH-O), 3.32 ppm (t, 2H, CH-OCH₂), | |
| I-1-a-21 | C₂H₅ | Br | 4-CH₃ | H | CH₂ | 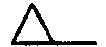 | erstarrter Schaum | cis |
| | | | | | | | *4.04 ppm (m, 1H, CH-O), 3.21 ppm (d, 2H, CH-OCH₂), 2.30 ppm (s, 3H, Ar-CH₃), | |
| I-1-a-22 | C₂H₅ | Br | 4-CH₃ | H | CH₂ | 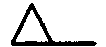 | erstarrter Schaum | trans |
| | | | | | | | *4.15 ppm (m, 1H, CH-O), 3.21 ppm (d, 2H, CH-OCH₂), 2.30 ppm (s, 3H, Ar-CH₃), | |
| I-1-a-23 | CH₃ | CH₃ | 4-CH₃ | H | CH₂ | 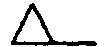 | 217-221 | cis |
| I-1-a-24 | CH₃ | CH₃ | 4-CH₃ | H | CH₂ | 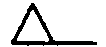 | erstarrter Schaum | trans |
| | | | | | | | *6.84 ppm (s, 2H, Ar-H), 4.15 ppm (m, 1H, CH-O), 3.22 ppm (d, 2H, CH-OCH₂), | |
| I-1-a-25 | H | CH₃ | 5-(4-Cl-Ph) | H | CH₂ | 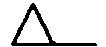 | 211 | cis |
| I-1-a-26 | H | CH₃ | 5-(4-Cl-Ph) | H | CH₂ | 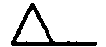 | erstarrter Schaum | trans |
| | | | | | | | *4.33 ppm (m, 1H, CH-O), 3.24 ppm (d, 2H, CH-OCH₂), 2.23 ppm (s, 3H, Ar-CH₃), | |
| I-1-a-27 | C₂H₅ | Br | 4-CH₃ | H | -(CH₂)₂- | OCH₃ | erstarrter Schaum | cis |
| | | | | | | | *4.04 ppm (m, 1H, CH-O), 3.27 ppm (s, 3H, OCH₃), 2.29 ppm (s, 3H, Ar-CH₃), | |
| I-1-a-28 | C₂H₅ | Br | 4-CH₃ | H | -(CH₂)₂- | OCH₃ | erstarrter Schaum | trans |
| | | | | | | | *4.15 ppm (m, 1H, CH-O), 3.25 ppm (s, 3H, OCH₃), 2.29 ppm (s, 3H, Ar-CH₃), | |
| I-1-a-29 | CH₃ | CH₃ | 4-CH₃ | H | -(CH₂)₂- | OCH₃ | erstarrter Schaum | trans |
| | | | | | | | *6.85 ppm (s, 2H, Ar-H), 4.15 ppm (m, 1H, CH-O), 3.25 ppm (s, 3H, OCH₃), | |
| I-1-a-30 | CH₃ | CH₃ | 4-CH₃ | H | -(CH₂)₂- | OCH₃ | erstarrter Schaum | cis |
| | | | | | | | *6.84 ppm (s, 2H, Ar-H), 4.05 ppm (m, 1H, CH-O), 3.27 ppm (s, 3H, OCH₃), | |
| I-1-a-31 | C₂H₅ | Cl | 4-Cl | H | CH₂ | 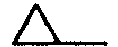 | erstarrter Schaum | cis |
| | | | | | | | **7.44 und 7.28 ppm (je s, 1H, Ar-H), 4.04 ppm (m, 1H, (CH-O), 0.47 und 0.18 ppm (je m, 2H, CH-Cyclopropyl) | |
| I-1-a-32 | C₂H₅ | Cl | 4-Cl | H | CH₂ | 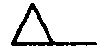 | erstarrter Schaum | trans |
| | | | | | | | **7.44 und 7.28 ppm (je s, 1H, Ar-H), 4.14 ppm (m, 1H, CH-O), 0.47 und 0.18 ppm (je m, 2H, CH-Cyclopropyl), | |

| | | | | | | | | |
|---|---|---|---|---|---|---|---|---|
| * ¹H-NMR (400 MHz, d₆-DMSO): Verschiebung δ in ppm ** ¹H-NMR(300 MHz, d₆-DMSO): Verschiebung δ in ppm Ph = Phenyl | | | | | | | | |

### Beispiel 1-1-b-1

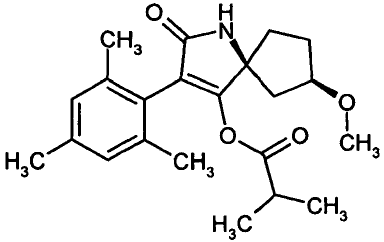

0,18 g der Verbindung gemäß Beispiel I-1-a-6 werden in 8 ml Essigsäureethylester vorgelgt, 0,1 ml Triethylamin und 1,5 mg 4-N,N'-Dimethylaminopyridin zugegeben und auf 60°C erhitzt. Eine Lösung von 0,07 g Isobuttersäurechlorid in 2 ml Essigsäureethylester werden in 7 Portionen innerhalb von 60 min zugegeben und 6 h bei 60°C gerührt. Nach Stehenlassen über Nacht wird mit halbkonzentrierter Natriumchloridlösung versetzt, die organische Phase abgetrennt und säulenchromatographisch (Gradient EtOAc/n-Heptan 1:9 nach Essigsäureethylester/n-Heptan 100:0) an Kieselgel gereinigt. Man erhält 85 mg eines farblosen Feststoffes (38 % Ausbeute der Theorie). Fp. 126-134°C

In Analogie zu Beispiel (I-I-b-1) und gemäß den allgemeinen Angaben zur Herstellung erhält man folgende Verbindungen der Formel (I-I-b)

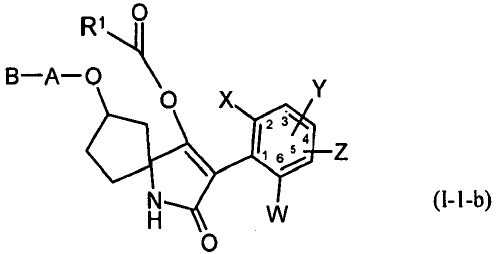

| **Bsp.-Nr.** | **W** | **X** | **Y** | **Z** | **A** | **B** | **R¹** | **Fp.°C** | **Isomer** |
|---|---|---|---|---|---|---|---|---|---|
| I-1-b-2 | C₂H₅ | Br | 4-CH₃ | H | CH₂ | H | i-C₃H₇ | zähes Harz | cis |
| | | | | | | | | *4.00 ppm (m, 1H, CH-OCH₃), 2.27 ppm (s, 3H, Ar-CH₃) 1.04 ppm (m, 6H, CH(CH₃)₂) | |
| I-1-b-3 | C₂H₅ | OCH₃ | 4-Cl | H | CH₂ | H | i-C₃H₇ | 163 | cis |
| I-I-b-4 | C₂H₅ | Br | 4-CH₃ | H | CH₂ | C₃H₇ | i-C₃H₇ | Öl | cis |
| | | | | | | | | *7.28 und 7.01 ppm (je s, 1H, Ar-H), 4.07 ppm (m, 1H, CH-OCH₂), 3.41 ppm (t, 2H, CH-OCH₂). 1.05 ppm (m, 6H, CH(CH₃)₂) | |
| I-1-b-5 | CH₃ | CH₃ | 4-CH3 | H | CH₂ | C₃H₇ | i-C₃H₇ | 76-85 | cis |
| I-1-b-6 | C₂H₅ | Br | 4-CH₃ | H | CH₂ | 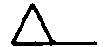 | i-C₃H₇ | Öl | cis |
| | | | | | | | | *4.13 ppm (m, 1H, CH-O), 2.29 ppm (s, 3 H, Ar-CH₃), 1.04 ppm (m, 6H, CH(CH₃)₂), 0.56 ppm (m, 2H, CH-Cyclopropyl) | |
| I-1-b-7 | C₂H₅ | Br | 4-CH₃ | H | CH₂ | 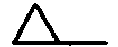 | i-C₃H₇ | Öl | trans |
| | | | | | | | | *4.16 ppm (m, 1H, CH-O), 2.29 ppm (s, 3 H, Ar-CH₃), 1.05 ppm (m, 6H, CH(CH₃)₂, 0.52 ppm (m, 2H, CH-Cyclopropyl) | |
| I-1-b-8 | CH₃ | CH₃ | 4-CH₃ | H | CH₂ | 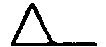 | i-C₃H₇ | 105 | cis |
| I-1-b-9 | CH₃ | CH₃ | 4-CH₃ | H | CH₂ | 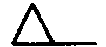 | i-C₃H₇ | 142 | trans |
| I-1-b-10 | H | CH₃ | 5-(4-Cl-Ph) | H | CH₂ | 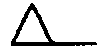 | i-C₃H₇ | 136 | cis |
| I-1-b-11 | H | CH₃ | 5-(4-Cl-Ph) | H | CH₂ | 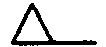 | i-C₃H₇ | 154-156 | trans |
| I-1-b-12 | C₂H₅ | Br | 4-CH₃ | H | -(CH₂)₂- | OCH₃ | i-C₃H₇ | Öl | cis |
| | | | | | | | | *4.15 ppm (m, 1H, CH-O), 3.43 ppm (d, 3H, OCH₃), 2.30 ppm (s, 3H, Ar-CH₃), 1.03 ppm (m, 6H, CH(CH₃)₂) | |
| I-1-b-13 | C₂H₅ | Br | 4-CH₃ | H | -(CH₂)₂- | OCH₃ | i-C₃H₇ | Öl | trans |
| | | | | | | | | *4.18 ppm (m, 1 H, CH-O), 3.37 ppm (d, 3H, OCH₃), 2.29 ppm (s, 3H, Ar-CH₃), 1.05 ppm (m, 6H, CH(CH₃)₂) | |
| I-1-b-14 | CH₃ | CH₃ | 4-CH₃ | H | -(CH₂)₂- | OCH₃ | i-C₃H₇ | Wachs | cis |
| | | | | | | | | *6.82 ppm (s, 2H, Ar-H), 4.15 ppm (m, 1H, CH-O), 3.42 ppm (d, 3H, OCH₃), 1.00 ppm (m, 6H, CH(CH₃)₂) | |
| I-1-b-15 | CH₃ | CH₃ | 4-CH₃ | H | -(CH₂)₂- | OCH₃ | i-C₃H₇ | Wachs | trans |
| | | | | | | | | *6.84 ppm (s, 2H, Ar-H), 4.17 ppm (m, 1H, CH-O), 3.37 ppm (d, 3H, OCH₃), 1.01 ppm (m, 6H, CH(CH₃)₂) | |
| I-1-b-16 | C₂H₅ | Br | 4-CH₃ | H | -(CH₂)₂- | OCH₃ | 2-Cl-Ph | 127-147 | cis |

| | | | | | | | | | |
|---|---|---|---|---|---|---|---|---|---|
| * ¹H-NMR (400 MHz, CDCl₃): Verschiebung δ in ppm Ph = Phenyl | | | | | | | | | |

### Beispiel I-1-c-1

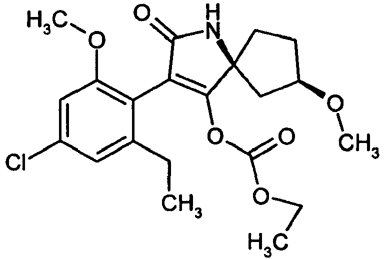

0,077 g gemäß Beispiel I-1-a-5 (0,219 mmol) werden in 3 ml Dichlormethan gelöst mit 0,04 ml Triethylamin (1.2 eq) versetzt, 10 min bei Raumtemperatur gerührt. Abschließend werden 0,02 ml Chlorameisensäureethylester (1.1 eq) zugegeben und bei Raumtemperatur über Nacht gerührt. Nach Extraktion mit 4 %iger Na₂CO₃-Lösung wird die organische Phase getrocknet, eingeengt und säulenchromatographisch an Kieselgel gereinigt (Gradient n-Heptan/Essigsäure-ethylester 9:1 nach Essigsäure-ethylester). Man erhält 44 mg Produkt als erstarrtes Glas (Ausbeute: 47 % der Theorie).
¹H-NMR (400 MHz, CDCl₃) δ = 3.32 (s, 3H, CH-OCH₃), 3.76 (s, 3H, Ar-OCH₃), 4.05 (q, 2H, OCH₂) ppm.

In Analogie zu Beispiel (I-1-c-1) erhält man Beispiel (I-1-c-2). 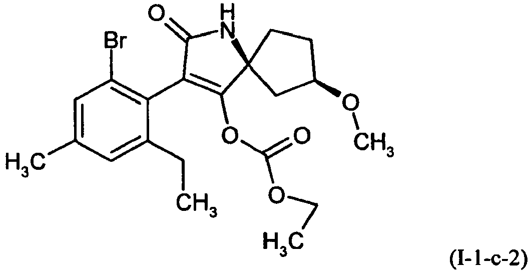 ¹H-NMR (400 MHz, CDCl₃) δ = 3.32 (s, 3H, CH-O**CH**₃), 2.58 (m, 2H, Ar-CH₂), 4.07 (q, 2H, OCH₂) ppm.

In Analogie zu Beispiel (I-1-c-1), (I-1-c-2) und gemäß den allgemeinen Angaben zur Herstellung erhält man folgende Verbindungen der Formel (I-1-c)

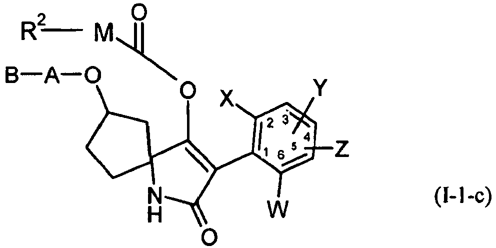

| **Bsp.-Nr.** | **W** | **X** | **Y** | **Z** | **A** | **B** | **M** | **R¹** | **Fp.°C** | **Isomer** |
|---|---|---|---|---|---|---|---|---|---|---|
| I-1-c-3 | CH₃ | CH₃ | 4-CH₃ | H | CH₂ | H | O | C₂H₅ | 114-117 | cis |
| I-1-c-4 | CH₃ | CH₃ | 4-CH₃ | H | CH₂ | H | O | C₂H₅ | 174-178 | trans |
| I-1-c-5 | CH₃ | Br | 4-CH3 | H | CH₂ | H | O | C₂H₅ | Öl | cis |
| | | | | | | | | | *7.24 und 6.99 ppm (je s, 1H, Ar-H), 4.07 ppm (q, 2H, CH₂-O), 4.00 ppm (m, 1H, CH-OCH₃) | |
| I-1-c-6 | CH₃ | Cl | 4-CH₃ | H | CH₂ | H | O | C₂H₅ | 119-123 | cis |
| I-1-c-7 | C₂H₅ | CH₃ | 4-Br | H | CH₂ | C₃H₇ | O | C₂H₅ | Öl | cis |
| | | | | | | | | | *7.24 und 7.01 ppm (je s, 1H, Ar-H), 4.07 ppm (m, 3H, CH₂-O und CH-OCH₂), 3.41 ppm (t, 2H, CH-OCH₂) | |
| I-1-c-8 | CH₃ | CH₃ | 4-CH₃ | H | CH₂ | C₃H₇ | O | C₂H₅ | Öl | cis |
| | | | | | | | | | *6.86 ppm (s, 2H, Ar-H), 4.08 ppm (m, 1H, CH-O), 4.01 ppm (q, 2H, CH₂-O), 3.42 ppm (t, 2H, CH-OCH₂) | |
| I-1-c-9 | C₂H₅ | Br | 4-CH₃ | H | CH₂ | C₃H₇ | O | C₂H₅ | Öl | trans |
| | | | | | | | | | *7.24 und 7.01 ppm (je s, 1H, Ar-H), 4.10 ppm (m, 3H, CH₂-O und CH-OCH₂) 3.37 ppm (t, 2H, CH-OCH₂) | |
| I-1-c-10 | CH₃ | CH₃ | 4-CH₃ | H | CH₂ | C₃H₇ | O | C₂H₅ | 126-129 | trans |
| I-1-c-11 | C₂H₅ | Br | 4-CH₃ | H | CH₂ | CH₃ | O | C₂H₅ | 94-96 | cis |
| I-1-c-12 | C₂H₅ | Br | 4-CH₃ | H | CH₂ | CH₃ | O | C₂H₅ | Öl | trans |
| | | | | | | | | | *7.28 und 7.01 ppm (je s, 1H, Ar-H), 4.13 ppm (m, 1H, CH-OCH₂), 4.07 (q, 2H, CH₂-O), 3.45 ppm (t, 2H, CH-OCH₂) | |
| I-1-c-13 | CH₃ | CH₃ | 4-CH₃ | H | CH₂ | CH₃ | O | C₂H₅ | Öl | cis |
| | | | | | | | | | *6.87 ppm (s, 2H, Ar-H), 4.13 ppm (m, 1H, CH-O), 4.01 ppm (q, 2H, CH₂-O), 3.47 ppm (t, 2H, CH-OCH₂) | |
| I-1-c-14 | CH₃ | CH₃ | 4-CH₃ | H | CH₂ | CH₃ | O | C₂H₅ | 120-125 | trans |
| I-1-c-15 | H | CH₃ | 5-(4-Cl-Ph) | H | CH₂ | H | O | C₂H₅ | zähes Öl | cis |
| | | | | | | | | | **4.03 ppm (m, 3H, CH-OCH₃, CH₂-O), 2.32 (s, 3H, Ar-CH₃) | |
| I-1-c-16 | H | CH₃ | 5-(4-Cl-Ph) | H | CH₂ | H | O | C₂H₅ | 167-171 | trans |
| I-1-c-17 | CH₃ | CH₃ | 4-CH₃ | H | CH₂ | C₂H₅ | O | C₂H₅ | Öl | cis |
| | | | | | | | | | *6.87 ppm (s, 2H, Ar-H), 4.08 ppm (m, 1H, CH-O), 4.01 ppm (q, 2H, CH₂-O), 3.37 ppm (t, 2H, CH-OCH₂) | |
| I-1-c-18 | C₂H₅ | Br | 4-CH₃ | H | CH₂ | C₂H₅ | O | C₂H₅ | 102-105 | cis |
| I-1-c-19 | CH₃ | CH₃ | 4-CH₃ | H | CH₂ | C₂H₅ | O | C₂H₅ | 110-113 | trans |
| I-1-c-20 | C₂H₅ | Br | 4-CH₃ | H | CH₂ | C₂H₅ | O | C₂H₅ | Öl | trans |
| | | | | | | | | | *7.24 und 7.01 ppm (je s, 1H, Ar-H), 4.08 ppm (m, 3H, CH-O und CH₂-O), 3.37 ppm (t, 2H, CH-OCH₂) | |
| I-1-c-21 | C₂H₅ | Br | 4-CH₃ | H | CH₂ | Δ_ | O | C₂H₅ | 127-129 | cis |
| I-1-c-22 | CH₃ | CH₃ | 4-CH₃ | H | CH₂ | 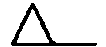 | O | C₂H₅ | 113 | cis |
| I-1-c-23 | H | CH₃ | 5-(4-Cl-Ph) | H | CH₂ | 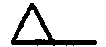 | O | C₂H₅ | erstarrter Schaum | cis |
| | | | | | | | | | *4.15 ppm (m, 1H, CH-O), 4.01 ppm (q, 2H, CH₂-O), 3.27 ppm (d, 2H, CH-OCH₂), 2.34 ppm (s, 3H, Ar-CH₃) | |
| I-1-c-24 | C₂H₅ | Br | 4-CH₃ | H | CH₂ | 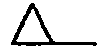 | O | C₂H₅ | Öl | trans |
| | | | | | | | | | *4.15 ppm (m, 1H, CH-O), 4.05 ppm (q, 2H, CH₂-O), 3.23 ppm (d, 2H, CH-OCH₂), 2.30 ppm (s, 3H, Ar-CH₃) | |
| I-1-c-25 | CH₃ | CH₃ | 4-CH₃ | H | CH₂ | 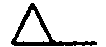 | O | C₂H₅ | erstarrter Schaum | trans |
| | | | | | | | | | *6.86 ppm (s, 2H, Ar-H), 4.15 ppm (m, 1H, CH-O), 4.03 ppm (q, 2H, CH₂-O), 3.23 ppm (d, 2H, CH-OCH₂) | |
| I-1-c-26 | H | CH₃ | 5-(4-Cl-Ph) | H | CH₂ | 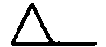 | O | C₂H₅ | erstarrter Schaum | trans |
| | | | | | | | | | *4.18 ppm (m, 1H, CH-O), 4.01 ppm (q, 2H, CH₂-O), 3.23 ppm (d, 2H, CH-OCH₂), 2.31 ppm (s, 3H, Ar-CH₃) | |
| I-1-c-27 | C₂H₅ | Br | 4-CH₃ | H | -(CH₂)₂- | OCH₃ | O | C₂H₅ | Öl | cis |
| | | | | | | | | | *4.16 ppm (m, 1H, CH-O), 4.05 ppm (q, 2H, CH₂-O), 3.42 ppm (s, 3H, OCH₃), 2.30 ppm (s, 3H, Ar-CH₃), | |
| I-1-c-28 | C₂H₅ | Br | 4-CH₃ | H | -(CH₂)₂- | OCH₃ | O | C₂H₅ | Öl | trans |
| | | | | | | | | | *4.18 ppm (m, 1H, CH-O), 4.05 ppm (q, 2H, CH₂-O), 3.38 ppm (s, 3H, OCH₃), 2.30 ppm (s, 3H, Ar-CH₃), | |
| I-1-c-29 | CH₃ | CH₃ | 4-CH₃ | H | -(CH₂)₂- | OCH₃ | O | C₂H₅ | erstarrter Schaum | cis |
| | | | | | | | | | *6.85 ppm (s, 2H, Ar-H), 4.15 ppm (m, 1H, CH-O), 4.01 ppm (q, 2H, CH₂-O), 3.42 ppm (s, 3H, OCH₃) | |
| I-1-c-30 | CH₃ | CH₃ | 4-CH₃ | H | -(CH₂)₂- | OCH₃ | O | C₂H₅ | Öl | trans |
| | | | | | | | | | *6.86 ppm (s, 2H, Ar-H), 4.17 ppm (m, 1H, CH-O), 4.01 ppm (q, 2H, CH₂-O), 3.37 ppm (s, 3H, CH-O-CH₃ | |
| I-1-c-31 | C₂H₅ | Br | 4-CH₃ | H | -(CH₂)₂- | OCH₃ | O | CH₂=CH-CH₂ | Öl | cis |
| | | | | | | | | | *4.48 ppm (d, 2H, CH₂=CH-CH₂O), 4.16 ppm (m, 1H, CH-O), 3.42 ppm (d, 3H, OCH₃), 2.30 ppm (s, 3H, Ar-CH₃), | |
| I-1-c-32 | C₂H₅ | Cl | 4-Cl | H | CH₂ | 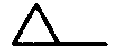 | O | C₂H₅ | Öl | cis |
| | | | | | | | | | *7.29 und 7.18 ppm (je s, 1H, Ar-H), 4.15 ppm (m, 1H, CH-O), 4.07 ppm (q, 2H, C(=O)OCH₂), 1.04 ppm (m, 1H, CH-Cyclopropyl) | |
| I-1-c-33 | C₂H₅ | Cl | 4-Cl | H | CH₂ | 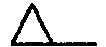 | O | C₂H₅ | Öl | trans |
| | | | | | | | | | *7.29 und 7.18 ppm (je s, 1 H, Ar-H), 4.17 ppm (m, 1H, CH-O), 4.08 ppm (q, 2H, C(=O)OCH₂), 1.04 ppm (m, 1H, CH-Cyclopropyl) | |

| | | | | | | | | | | |
|---|---|---|---|---|---|---|---|---|---|---|
| * ¹H-NMR (400 MHz, CDCl₃): Verschiebung δ in ppm ** ¹H-NMR (300 MHz, CDCl₃): Verschiebung δ in ppm Ph = Phenyl | | | | | | | | | | |

### Beispiel I-1-d-1

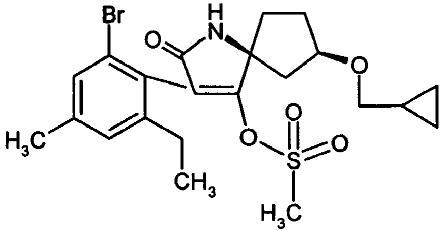

0,156 g der Verbindung gemäß Bsp. I-1-a-21 werden in 10 ml Dichlormethan gelöst und 0,06 ml Triethlamin zugegeben. Zu dieser Lösung werden noch 0,032 ml Methansulfonsäurechlorid gegeben und 20 h bei Raumtemperatur gerührt. Anschließend wird mit 5 ml 5 %iger Natriumhydrogencarbonat-Lösung 0,5 h verrührt, die organische Lösung abgetrennt, mit Natriumsulfat getrocknet, einrotiert und der erhaltene Rückstand säulenchromatographisch gereinigt (Gradient n-Heptan + Essigsäureethylester 9:1 nach Essigsäureethylester).
Ausbeute: 0,14 g (76 % d. Theorie)
¹H-NMR (CDCl₃, 400 MHz): δ=4,16 ppm (m, 1H, CH-O), 2,62 ppm (s, 3H, SO₂CH₃), 2,32 ppm, (s, 3H, Ar-CH₃), 1,03 ppm (m, 1H, CH-cyclopropyl).

### Beispiel I-1-f-1

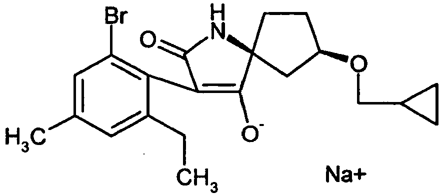

0,1 g der Verbindung gemäß Bsp. I-1-a-21 werden in 7 ml wasserfreiem Methanol gelöst und 0,045 ml Natriummethylat-Lösung 30 %ig zugegeben. Nach 2 h wird die Lösung einrotiert, noch 2 mal mit je 5 ml wasserfreiem Methanol abrotiert und am Hochvakuum getrocknet.
Ausbeute: 0,095 g (90 % d. Theorie)
¹H-NMR (d6-DMSO, 400 MHz): δ=5,01 ppm (m, 1H, CH-O), 2,21 ppm (s, 3H, Ar-CH₃), 0,45 und 0,16 ppm (je m, 2H, CH-cyclopropyl).

### Beispiel I-1-g-1

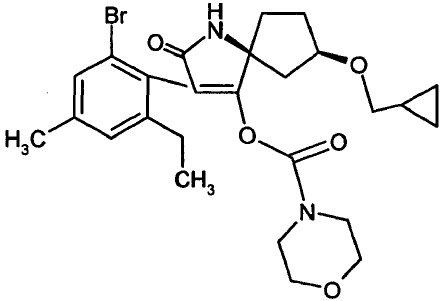

0,158 g der Verbindung gemäß Bsp. I-1-a-21 werden in 10 ml Chloroform gelöst und 0,08 ml Diisopropylethylamin und 2 mg DMAP zugegeben. Zu dieser Lösung wird noch 0,048 ml Morpholin-N-carbonsäurechlorid gegeben und 20 h bei Raumtemperatur gerührt. Anschließend wird mit 5 ml 5 %iger Natriumhydrogencarbonat-Lösung 0,5 h verrührt, die organische Lösung abgetrennt, mit Natriumsulfat getrocknet, einrotiert und der erhaltene Rückstand säulenchromatographisch gereingit (Gradient n-Heptan + Essigsäureethylester 9:1 nach Essigsäureethylester).
Ausbeute: 0,14 g (63 % d. Theorie)
¹H-NMR (CDCl₃, 400 MHz): δ=4,14 ppm (m, 1H, CH-O), 36 - 31 ppm (mehrere Multiplets, 10H, CH₂O und CH₂N Signale), 2,32 ppm (s, 3H, Ar-CH₃).

### Beispiel II-1

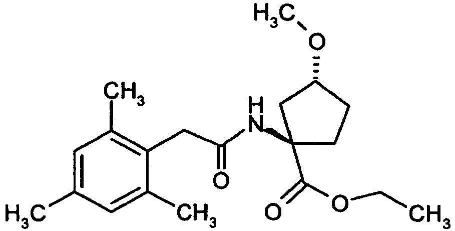

0,41 g gemäß Beispiel Il-10 (1,23 mmol) werden in 15 ml Dichlormethan gelöst. Man gibt 0,29 g 1,8-Bisdimethylaminonaphthalin (1.1 eq) und 0,20 g Trimethyloxoniumtetrafluoroborat (1.1 eq) hinzu und lässt bei Raumtemperatur rühren. Anschließend wird nach jeweils 4 Stunden noch einmal je 0.3 eq Trimethyloxoniumtetrafluoroborat und 1,8-Bismethylaminonaphthalin zugegeben und erneut über Nacht gerührt. Man setzt anschließend 20 ml 5 %ige Zitronensäure zu, rührt 45 min, trennt die organische Phase ab, und trocknet mit Natriumsulfat und reinigt chromatographisch an Kieselgel (Gradient n-Heptan/Essigsäure-ethylester 4:1 nach Essigsäure-ethylester).

Man erhält 0,27 g an Produkt (63 % Ausbeute der Theorie).
¹H-NMR (400 MHz, CDCl₃) δ = 4.16 (q, 2H, CH-OCH₂), 3.93 (m, 1H, **CH**-OCH₃), 3.56 (s, 2H, CH₂-Ar), 3.26 (s, 3H, OCH₃) ppm.

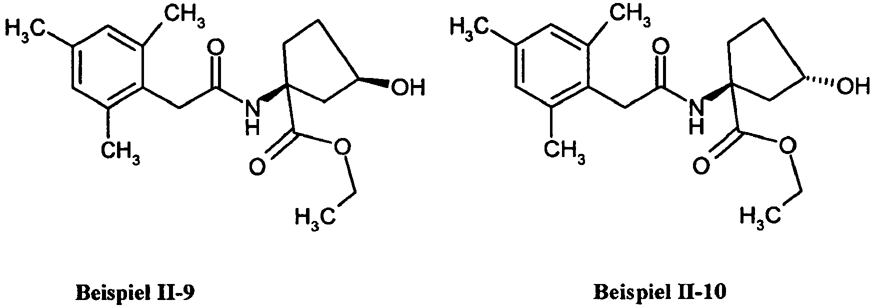

1,05 g Mesitylenessigsäure (5.89 mmol) werden mit 6 ml Thionylchlorid und zwei Tropfen N,N-Dimethylformamid unter Rückfluss zum Sieden erhitzt. Nach beendeter Gasentwicklung wird eingeengt und in 20 ml Dichlormethan aufgenommen (Lösung 1). Zu einer Lösung von 3-Hydroxy-1-amino-cyclopentan-carbonsäure-ethylester (5,89 mmol) werden 1,25 g Triethylamin (2.1 eq) zugegeben und 10 min gerührt. Lösung 1 wird innerhalb 20 min bei Raumtemperatur zugetropft. Man lässt über Nacht bei Raumtemperatur rühren. Man wäscht den Ansatz mit 15 ml Wasser, trocknet die organische Phase mit Natriumsulfat und reinigt säulenchromatographisch an Kieselgel (Gradienten Essigsäure-ethylester/n-Heptan 0:100 nach 100:0). Man erhält 0,95 g an cis-Isomer (Ausbeute 48 % der Theorie) und 0,43 g trans-Isomere (Ausbeute 22 % der Theorie).
trans-Isomer Bsp. II-10
   ¹H-NMR (400 MHz, CDCl₃) δ = 4.38 (m, 1H, CHOH), 3.53 (s, 2H, Ar-CH₂) ppm
cis-Isomer Bsp. II-9
   ¹H-NMR (400 MHz, CDCl₃) δ = 4.21 ppm (m, 1H, CHOH), 3.60 (s, 2H, Ar-CH₂) ppm

### Beispiel II-34

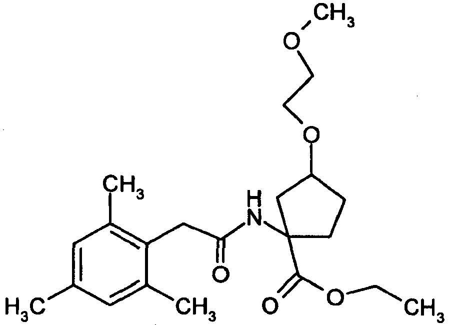

1,87 g Mesitylessigsäure und 6,25 g Thionylchlorid werden vorgelegt und mit 2 Tropfen Dimethylformamid versetzt. Anschließend wird bis zum Ende der Gasentwicklung erhitzt, eingeengt und in 25 ml Dichlormethan gelöst (Lösung 1). 2,49 der Verbindung gemäß Beispiel (XIV-2) wird in 25 ml Dichlormethan gelöst und mit 3,06 ml Triethylamin vesetzt, anschließend tropft man innerhalb von 30 min Lösung 1 zu und rührt über Nacht bei Raumtemperatur. Es wird mit halbkonzentrierter Natriumchloridlösung versetzt, die Phasen getrennt, die organische Phase eingeengt, mit Natriumsulfat getrocknet und säulenchromatographisch aufgereinigt (Gradient Essigsäureethylester/Heptan 5:95 nach 70:30 Essigsäureethylester).

| | |
|---|---|
| Ausbeute: 1,91 g (38 % der Theorie). | |
| | |
| ¹H-NMR (400 MHz, CDCl₃) δ = | 6,89 ppm (d, 1H, Ar-H) |
| | 3,35 ppm (d, 3H, OCH₃) |
| | 1,24 ppm (t, 3H, CH₃-CH₂O). |

In Analogie zu den Beispielen (II-1), (II-9), (II-10) und (II-34) und gemäß den allgemeinen Angaben zur Herstellung erhält man folgende Verbindungen der Formel (II)

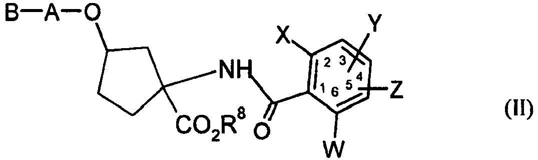

| **Bsp.-Nr.** | **W** | **X** | **Y** | **Z** | **A** | **B** | **R⁸** | **Fp.°C** | **Isomer** |
|---|---|---|---|---|---|---|---|---|---|
| II-2 | OCH₃ | C₂H₅ | 4-Cl | H | CH₂ | H | C₂H₅ | **Öl** | cis |
| | | | | | | | | *3.07 (s, 3H, CHOCH₃) | |
| | | | | | | | | 3.55 (s, 2H, ArCH₂-CO) | |
| | | | | | | | | 3.84 (m, 1H, CH-OCH₃) | |
| | | | | | | | | und s, 3H, Ar-OCH₃) | |
| | | | | | | | | 4.13 (q, 2H, O-CH₂-CH₃) | |
| II-3 | CH₃ | CH₃ | 4-CH3 | H | CH₂ | H | C₂H₅ | Öl | cis |
| | | | | | | | | *3.01 (s, 3H, CH-OCH₃) | |
| | | | | | | | | 3.52 (s, 2H, Ar-CH₂-CO) | |
| | | | | | | | | 3.81 (m, 1H, CH-OCH₃) | |
| | | | | | | | | 4.16 (q, 2H, O-CH₂-CH₃) | |
| II-4 | C₂H₅ | Br | 4-CH₃ | H | CH₂ | H | C₂H₅ | Öl | cis |
| | | | | | | | | *3.05 (m, 3H, CHOCH₃) | |
| | | | | | | | | 3.76 (s, 2H, Ar-CH₂-CO) | |
| | | | | | | | | 3.84, (m, 1 H, CHOCH₃) | |
| | | | | | | | | 4.16 (q, 2H, O-CH₂-CH₃) | |
| II-5 | C₂H₅ | Br | 4-CH₃ | H | CH₂ | H | C₂H₅ | Öl | trans |
| | | | | | | | | *3.29 (s, 3H, CH-OCH₃) | |
| | | | | | | | | 3.75 (s, 2H, Ar-CH₂-CO) | |
| | | | | | | | | 4.03, (m, 1 H, CH-OCH₃) | |
| | | | | | | | | 4.16 (q, 1H, O-CH₂-CH₃) | |
| II-6 | OCH₃ | C₂H₅ | 4-Cl | H | CH₂ | H | C₂H₅ | Öl | trans |
| | | | | | | | | wurde ohne weitere Charakterisierung zu Bsp. Nr. I-1-a-4 umgesetzt | |
| II-7 | C₂H₅ | Br | 4-CH₃ | H | CH₂ | CH₃ | C₂H₅ | Öl | cis |
| | | | | | | | | *3.29 (m, 2H, CH-OCH₂-CH₃) | |
| | | | | | | | | 3.76 (s, 2H, Ar-CH₂-CO) | |
| | | | | | | | | 3.95, (m, 1H, CH-O-CH₂CH₃) | |
| | | | | | | | | 4.15 (q, 2H, O-CH₂-CH₃) | |
| II-8 | C₂H₅ | Br | 4-CH₃ | H | CH₂ | CH₃ | C₂H₅ | Öl | trans |
| | | | | | | | | *3.43 (m, 2H, CH-OCH₂- CH₃) | |
| | | | | | | | | 3.75 (s, 2H, Ar-CH₂-CO) | |
| | | | | | | | | 4.14 (m, 2H + 1H, CH-OCH₂CH₃ und O-CH₂-CH₃) | |
| II-9 | CH₃ | CH₃ | 4-CH₃ | H | - | H | C₂H₅ | siehe Vorschrift | cis |
| II-10 | CH₃ | CH₃ | 4-CH₃ | H | - | H | C₂H₅ | siehe Vorschrift | trans |
| II-11 | C₂H₅ | Br | 4-CH₃ | H | - | H | C₂H₅ | Öl | cis |
| | | | | | | | | *3.80 (s, 2H, Ar-CH₂-CO) | |
| | | | | | | | | 4.36 (m, 1H CH-OH) | |
| II-12 | C₂H₅ | Br | 4-CH₃ | H | - | H | C₂H₅ | Öl | trans |
| | | | | | | | | *3.76 (s, 2H, Ar-CH₂CO) | |
| | | | | | | | | 4.43 (m, 1H CH-OH) | |
| II-13 | OCH₃ | C₂H₅ | 4-Cl | H | - | H | C₂H₅ | Öl | cis |
| | | | | | | | | *3.60 (s, 2H, Ar-CH₂-CO) | |
| | | | | | | | | 4.34 (m, 1H CH-OH) | |
| II-14 | OCH₃ | C₂H₅ | 4-Cl | H | - | H | C₂H₅ | Öl | trans |
| | | | | | | | | *3.55 (s, 2H, Ar-CH₂-CO) | |
| | | | | | | | | 4.43 (m, 1 H CHOH) | |
| II-15 | CH₃ | CH₃ | 4-CH₃ | H | CH₂ | CH₃ | C₂H₅ | Öl | cis |
| | | | | | | | | *6.86 ppm (je s, 1H, Ar-H), 1,25 und 0,99 ppm (je t, 3H, CH₃-CH₂O) | |
| II-16 | CH₃ | CH₃ | 4-CH₃ | H | CH₂ | CH₃ | C₂H₅ | Öl | trans |
| | | | | | | | | *6.86 ppm (je s, 1H, Ar-H), 1,24 und 1,15 ppm (je t, 3H, CH₃-CH₂O) | |
| H-17 | CH₃ | Br | 4-CH₃ | H | - | H | C₂H₅ | Öl | cis |
| | | | | | | | | *7.28 und 6.99 ppm (je s, 1H, Ar-H), 4,36 ppm (m, 1H, CH-O), 4.15 ppm (m, 2H, CH₂-O) | |
| II-18 | CH₃ | Br | 4-CH₃ | H | - | H | C₂H₅ | Öl | trans |
| | | | | | | | | *7.28 und 6.99 ppm (je s, 1H, Ar-H), 4,44 ppm (m, 1H, CH-O), 4.16 ppm (m, 2H, CH₂-O) | |
| II-19 | CH₃ | Br | 4-CH₃ | H | CH₂ | H | C₂H₅ | Öl | cis |
| | | | | | | | | *7.24 und 6.99 ppm (je s, 1H, Ar-H), 4,16 ppm (m, 2H, CH₂-O), 3.84 ppm (m, 1H, CH-OCH₃) | |
| II-20 | CH₃ | Cl | 4-CH₃ | H | - | H | C₂H₅ | Öl | cis |
| | | | | | | | | *7.13 und 6.96 ppm (je s, 1H, Ar-H), 4,35 ppm (m, 1H, CH-O), 4.16 ppm (m, 2H, CH₂-O) | |
| II-21 | CH₃ | Cl | 4-CH₃ | H | - | H | C₂H₅ | erstarrter Schaum | trans |
| | | | | | | | | *7.13 und 6.96 ppm (je s, 1 H, Ar-H), 4,45 ppm (m, 1H, CH₂-O), 4.16 ppm (m, 2H, CH₂-O) | |
| II-22 | CH₃ | Cl | 4-CH₃ | H | CH₂ | H | C₂H₅ | 95-99 | cis |
| II-23 | CH₃ | CH₃ | 4-CH₃ | H | CH₂ | C₃H₇ | C₂H₅ | Öl | Gemisch cis/trans |
| | | | | | | | | *6.91 und 6.88 ppm (je s, Summe 2H, Ar-H), 4,16 ppm (m, 2H, CH₂-O), 4.00 und 3.92 ppm (je m, Summe 1H, CH-O) | |
| II-24 | C₂H₅ | Br | 4-CH₃ | H | CH₂ | C₃H₇ | C₂H₅ | Öl | Gemisch cis/trans |
| | | | | | | | | *7.30 und 7.27 ppm (je s, Summe 1H, Ar-H), 7,00 und 6.99 ppm (je s, Summe 1H, AR-H), 4.16 ppm (m, 2H, CH₂-O), 4.08 und 3.95 ppm (je m, Summe 1 H, CH-O) | |
| II-25 | H | CH₃ | 5-(4-Cl-Ph) | H | - | H | C₂H₅ | erstarrter Schaum | trans |
| | | | | | | | | **4.43 ppm (m, 1 H, CH-CO) 4.16 (m, 2H CH₂-O), 2,34 (s, 3H, Ar-CH₃) | |
| II-26 | H | CH₃ | 5-(4-Cl-Ph) | H | - | H | C₂H₅ | erstarrter Schaum | cis |
| | | | | | | | | **4.37 ppm (m, 1 H, CH-CO) 4.16 (m, 2H CH₂-O), 2,34 (s, 3H, Ar-CH₃) | |
| II-27 | H | CH₃ | 5-(4-Cl-Ph) | H | CH₂ | H | C₂H₅ | erstarrter Schaum | trans |
| | | | | | | | | **4.16 (m, 2H, CH₂-O) 4.03 ppm (m, 1H CH-O), 2,36 (s, 3H, Ar-CH₃) | |
| II-28 | H | CH₃ | 5-(4-Cl-Ph) | H | CH₂ | H | C₂H₅ | Öl | cis |
| | | | | | | | | **4.16 (m, 2H, CH₂-O) 3.82 ppm (m, 1H CH-O), 2,37 (s, 3H, Ar-CH₃) | |
| II-29 | CH₃ | CH₃ | 4-CH₃ | H | CH₂ | C₂H₅ | C₂H₅ | Öl | Gemisch |
| | | | | | | | | *6.91 und 6.88 ppm (je s, Summe 2H, Ar-H), 4.16 ppm (m, 2H, CH₂-O), 4.01 und 3.90 ppm (je m, Summe 1H, CH-O) | cis/trans |
| II-30 | C₂H₅ | Br | 4-CH₃ | H | CH₂ | C₂H₅ | C₂H₅ | Öl | Gemisch cis/trans |
| | | | | | | | | *7.30 und 7.27 ppm (je s, Summe 1H, Ar-H), 7,00 und 6.99 ppm (je s, Summe 1H, AR-H), 4.16 ppm (m, 2H, CH₂-O), 4.09 und 3.93 ppm (je m, Summe 1H, CH-O) | |
| II-31 | CH₃ | CH₃ | 4-CH₃ | H | CH₂ | 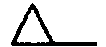 | C₂H₅ | Öl | Gemisch cis/trans |
| | | | | | | | | *6.88 ppm (d, 1H, Ar-H), 1,25 ppm (t, 3H, CH₃-CH₂O), 0.51 ppm (m, 2H, CH(Cyclopropyl) | |
| II-32 | C₂H₅ | Br | 4-CH₃ | H | CH₂ | 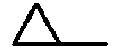 | C₂H₅ | Öl | Gemisch cis/trans |
| | | | | | | | | *2.30 ppm (d, 2H, Ar-CH₃), 1,25 ppm (m, 6H, CH₃-CH₂O) und Ar-CH₂-CH₃), 0.49 ppm (m, 2H, CH(Cyclopropyl) | |
| II-33 | H | CH₃ | 5-(4-Cl-Ph) | H | CH₂ | 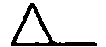 | C₂H₅ | Öl | Gemisch cis/trans |
| | | | | | | | | *2.35 ppm (d, 2H, Ar-CH₃), 1,25 ppm (m, 3H, CH₃-CH₂O) 0.50 und 0.40 ppm (je m, zusammen 2H, CH (Cyclopropyl)) | |
| II-34 | CH₃ | CH₃ | 4-CH₃ | H | -(CH₂)₂- | OCH₃ | C₂H₅ | Öl | Gemisch cis/trans |
| | | | | | | | | *6.89 ppm (d, 1H, Ar-H), 3,35 ppm (d, 3H, OCH₃), 1.24 ppm (t, 3H, CH₃-CH₂O) | |
| II-35 | C₂H₅ | Br | 4-CH₃ | H | -(CH₂)₂- | OCH₃ | C₂H₅ | Öl | Gemisch cis/trans |
| | | | | | | | | **3.37 ppm (s, 3H, OCH₃), 2,30 ppm (d, 3H, Ar-CH₃), 1.24 ppm (m, 6H, CH₃-CH₂O und Ar-CH₂CH₃) | |
| II-36 | CH₃ | CH₃ | 4-Br | H | - | H | C₂H₅ | Wachs | trans |
| | | | | | | | | *4.41 ppm (m, 1H, CH-OH), 4.18 ppm (m, 2H, CH₂-O), 3.52 (s, 2H, Ar-CH₂) | |
| II-37 | CH₃ | CH₃ | 4-Br | H | - | H | C₂H₅ | Wachs | cis |
| | | | | | | | | *4.37 ppm (m, 1H, CH-OH), 4.15 ppm (m, 2H, CH₂-O) | |

| | | | | | | | | | |
|---|---|---|---|---|---|---|---|---|---|
| * ¹H-NMR (400 MHz, CDCl₃): Verschiebung δ in ppm ** ¹H-NMR (300 MHz, CDCl₃): Verschiebung δ in ppm Ph = Phenyl | | | | | | | | | |

### 3-Hydroxy-1-amino-1-cyclopentancarbonsäure-ethylester

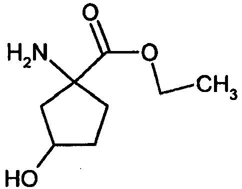

analog WO 02/46128

9.188 g Natriumhydrid (60 %ig, 2.2. eq) werden in 400 ml wasserfreiem Tetrahydrofuran suspendiert und 27.914 g des N-(Diphenylmethylen-glycin-ethylester (1 e.q.) in 100 ml Tetrahydrofuran gelöst innerhalb von 30 min zugetropft. Man lässt 30 min nachrühren und gibt dann in 100 ml Tetrahydrofuran gelöst Dibromid A* innerhalb von 30 min hinzu. Man rührt zunächst 4 h unter Rückfluss und anschließend über Nacht bei Raumtemperatur.

Anschließend versetzt man die Lösung mit 0,6 1 Wasser und 1,2 1 Essigsäure und lässt 8 h bei 55°C Innentemperatur rühren. Nach Stehenlassen über Nacht wird der Ansatz eingeengt, mit 0,6 1 Wasser versetzt, mit Natriumhydrogencarbonat auf pH = 7 eingestellt und erneut eingeengt. Der erhaltene Rückstand wird dreimal mit je 0,4 1 Dichlormethan versetzt und die vereinigten organischen Phasen mit Natriumsulfat getrocknet. Man erhält 9,8 g an Produkt als zähes Öl. (54,2 % Ausbeute der Theorie)
¹H-NMR (CDCl₃, 400 MHz): δ = 4.20 (m, 2H, OCH₂), 4.35 und 4.50 (je m, zusammen 1H, CH-OH) ppm.
*Dibromid A = 1,4-Dibrom-2-(2'-tetrahydropyranyl)-oxy-butan

### 3-Methoxy-1-amino-1-cyclopentancsrbonsäure-ethylester (XIV-1)

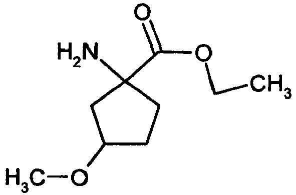

analog A. Börner et. al, Chem.Ber. 128, 767 (1995)
Ma, Dawei et.al., Tetrahedron Assymetry 8, 825 (1997)

0.637 g Natriumhydrid (60 %ig, 2.2. eq) werden in 20 ml wasserfreiem Tetrahydrofuran suspendiert und 1.935 g des N-(Diphenylmethylen-glycin-ethylester (1 e.q.) in 100 ml Tetrahydrofuran gelöst innerhalb von 30 min zugetropft. Man lässt 30 min nachrühren und gibt dann in 10 ml Tetrahydrofuran gelöst Bismesylat A* innerhalb von 30 min hinzu. Man rührt zunächst 4 h bei Rückfluss und anschließend über Nacht bei Raumtemperatur.

Anschließend versetzt man die Lösung mit 40 ml Wasser und 80 ml Essigsäure und lässt 8 h bei 55°C Innentemperatur rühren. Nach Stehenlassen über Nacht wird der Ansatz eingeengt, mit 50 ml Wasser vesetzt, mit Natriumhydrogencarbonat auf pH = 7 eingestellt und erneut eingeengt. Der erhaltene Rückstand wird dreimal mit je 0,1 L Dichlormethan versetzt und die vereinigten organischen Phasen mit Natriumsulfat getrocknet. Man erhält 0,75 g an Produkt als zähes Öl. (52,7 % Ausbeute der Theorie).
*Bismesylat A = 2-Methoxy-1,4-butandiol-bismesylat

In Analogie zu Beispiel (XIV-1) und gemäß den allgemeinen Angaben zur Herstellung erhält man folgende Verbindungen der Formel (XIV)

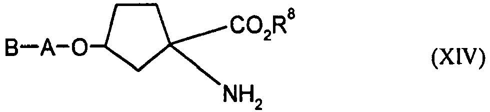

| **Bsp.-Nr.** | **A** | **B** | **R⁸** | **Fp.°C** | **Isomer** |
|---|---|---|---|---|---|
| XIV-2 | (CH₂)₂ | OCH₃ | C₂H₅ | Öl | Gemisch cis/trans |
| | | | | wurde direkt weiter umgesetzt | |
| XIV-3 | CH₂ | 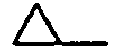 | C₂H₅ | Öl | Gemisch cis/trans |
| | | | | 0.52 (m, 1H, CH-Cyclopropyl, 1.28 ppm (m, 3H, O-CH₂-CH₃), 3.24 ppm (m, 2H, O-CH₂-Cyclopropyl) | |
| XIV-4 | CH₂ | CH₃ | C₂H₅ | Öl | Gemisch cis/trans |
| | | | | wurde direkt weiter umgesetzt | |
| XIV-5 | CH₂ | C₂H₅ | C₂H₅ | Öl | Gemisch cis/trans |
| | | | | 4.18 ppm (m, 2H, CH₂-O), 4.18 und 4.01 ppm (je m, Summe 1H, CH-O), 3.39 ppm (m, 2H, OCH₂) | |
| XIV-6 | CH₂ | C₃H₇ | C₂H₅ | Öl | Gemisch cis/trans |
| | | | | 4.18 ppm (m, 2H, CH₂-O), 4.18 und 4.01 ppm (je m, Summe 1H, CH-O), 3.39 ppm (m, 2H, OCH₂) | |

| | | | | | |
|---|---|---|---|---|---|
| * ¹H-NMR (300 MHz, CDCl₃): Verschiebung δ in ppm | | | | | |

### Anwendunizsbeispiele

### Beispiel 1

### Phaedon -Test (PHAECO Spritzbehandlung)

| | |
|---|---|
| Lösungsmittel: | 78 Gewichtsteile Aceton |
| | 1,5 Gewichtsteile Dimethylformamid |
| | |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis)* werden mit einer Wirkstoffzubereitung der gewünschten Konzentration gespritzt und nach dem Abtrocknen mit Larven des Meerrettichblattkäfers *(Phaedon cochleariae)* besetzt.

Nach der gewünschten Zeit wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigt z.B. die folgende Verbindung der Herstellungsbeispiele eine Wirkung von ≥ 80 % bei einer Aufwandmenge von 500 g/ha: I-1-a-1, I-1-a-2, I-1-a-6, I-1-a-9, I-1-a-10, I-1-a-13, I-1-a-16, I-1-a-17, I-1-a-18, I-1-b-5, I-1-c-4, I-1-c-5, I-1-c-6, I-1-c-10, I-1-c-13, I-1-c-14, I-1-c-15, 1-I-c-16, I-1-c-17, I-1-c-20.

### Beispiel 2

### Myzus-Test (MYZUPE Spritzbehandlung)

| | |
|---|---|
| Lösungsmittel: | 78 Gewichtsteile Aceton |
| | 1,5 Gewichtsteile Dimethylformamid |
| | |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis)*, die von allen Stadien der Grünen Pfirsichblattlaus *(Myzus persicae)* befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z.B. die folgenden Verbindungen der Herstellungsbeispiele eine Wirkung von ≥ 80 % bei einer Aufwandmenge von 500 g/ha: I-1-a-1, I-1-a-2, I-1-a-3, I-1-a-6, I-1-a-7, I-1-a-9, I-1-a-10, I-1-a-11, I-1-a-12, I-1-a-13, I-1-a-15, I-1-a-16, I-1-a-17, I-1-a-18, I-1-a-19, I-1-a-21, 1-1-a-22, I-1-a-24, I-1-a-25, I-1-a-26, I-1-b-1, I-1-b-2, I-1-b-5, I-1-c-2, I-1-c-3, I-1-c-4, I-1-c-5, I-1-c-6, I-1-c-8, I-1-c-14, I-1-c-15, I-1-c-16, I-1-c-17, I-1-c-19, I-1-c-20, I-1-c-22, I-1-c-23, I-1-c-24, I-1-c-25, I-1-c-26, I-1-c-27, I-1-c-28, I-1-c-29.

### Beispiel 3

### Spodoptera frugiperda-Test (SPODFR Spritzbebandlung)

| | |
|---|---|
| Lösungsmittel: | 78 Gewichtsteile Aceton |
| | 1,5 Gewichtsteile Dimethylformamid |
| | |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach der gewünschten Zeit wird die Wirkung in % bestimmt. Dabei bedeutet 100 %, dass alle Raupen abgetötet wurden; 0 % bedeutet, dass keine Raupe abgetötet wurde.

Bei diesem Test zeigen z.B. die folgenden Verbindungen der Herstellungsbeispiele eine Wirkung von ≥ 80 % bei einer Aufwandmenge von 500 g/ha: I-1-a-1, I-1-a-10, I-1-a-11, I-1-a-16, I-1-a-25, I-1-a-26, I-1-b-5, I-1-c-6, I-1-c-15, I-1-c-16, I-1-c-20.

### Beispiel 4

### Tetranychus -Test, OP-resistent ( TETRUR Spritzbehandlung)

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenpflanzen *(Phaseolus vulgaris)*, die stark von allen Stadien der Gemeinen Spinnmilbe *(Tetranychus urticae)* befallen sind, werden in eine Wirkstoffzubereitung der gewünschten Konzentration getaucht.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z.B. die folgenden Verbindungen der Herstellungsbeispiele eine Wirkung von ≥ 80 % bei einer Aufwandmenge von 100 g/ha: I-1-a-10 I-1-a-21, I-1-a-22, I-1-a-24, I-1-a-25, I-1-a-26, I-1-b-1, I-1-b-4, I-1-b-5, I-1-c-3, I-1-c-4, I-1-c-5, I-1-c-13, I-1-c-14, I-1-c-15, I-1-c-16, 1-1-c-20, I-1-c-21, I-1-c-22, I-1-c-23, I-1-c-24, I-1-c-25, I-1-c-26, I-1-c-27, I-1-c-28, I-1-c-29.

### Beispiel 5

### Herbizide Wirkung im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten Testverbindungen werden dann als wässrige Suspension mit einer Wasseraufwandmenge von umgerechnet 800 1/ha unter Zusatz von 0,2 % Netzmittel auf die Oberfläche der Abdeckerde appliziert

Nach Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100 % Wirkung = Pflanzen sind abgestorben, 0 % Wirkung = wie Kontrollpflanzen).

Folgende Verbindungen zeigen im Vorauflauf mit 320 g/ha a.i. gegen Lolium multiflorum und Setaria viridis eine Wirkung von ≥ 80%: I-1-a-2, I-1-a-8.

Folgende Verbindungen zeigen im Vorauflauf mit 320 g/ha a.i. gegen Lolium multiflorum und Echinohcloa crus-gali eine Wirkung von ≥ 80%: I-I-a-2, I-1-a-3, I-1-a-5, I-1-a-6, I-1-a-7, I-1-a-10, I-1-a-11, I-1-a-12, I-1-a-19, I-1-a-20, I-1-b-2, I-1-b-3, I-1-b-4, I-1-c-1, I-1-c-2, I-1-c-3, I-1-c-7, I-1-c-9, I-1-c-10, I-1-c-11, I-1-c-15, I-1-c-16, I-1-c-18, I-1-c-20.

### Herbizide Wirkung im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten Testverbindungen werden dann als wässrige Suspension mit einer Wasseraufwandmenge von umgerechnet 800 1/ha unter Zusatz von 0,2 % Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu behandelten Kontrollen bonitiert (herbizide Wirkung in Prozent (%): 100 % Wirkung = Pflanzen sind abgestorben, 0 % Wirkung = wie Kontrollpflanzen).

Folgende Verbindungen zeigen im Nachauflauf mit 320 g/ha a.i. gegen Avena fatua, Lolium multiflorum und Setaria viridis und Echinochloa crus-galli eine Wirkung von ≥ 80 %: I-1-a-2, I-1-a-3, I-1-a-7, I-1-a-8, I-1-a-12, I-1-a-20, I-1-b-2, I-1-b-3, I-1-c-1, I-1-c-2, I-1-c-11, I-1-c-20.

### Beispiel 6

### Herbizide Wirkung im Nachauflauf

Samen von mono- bzw. dikotylen Unkraut- bzw. Kulturpflanzen werden in Holzfasertöpfen oder in Plastiktöpfen in sandigem Lehmboden ausgelegt, mit Erde abgedeckt und im Gewächshaus, während der Vegetationsperiode auch im Freien ausserhalb des Gewächshauses, unter guten Wachstumsbedingungen angezogen. 2 bis 3 Wochen nach der Aussaat werden die Versuchspflanzen im Ein- bis Dreiblattstadium behandelt. Die als Spritzpulver (WP) oder Flüssigkeit (EC) formulierten Testverbindungen werden in verschiedenen Dosierungen mit einer Wasseraufwandmenge von umgerechnet 300 1/ha unter Zusatz von Netzmittel (0,2 bis 0,3 %) auf die Pflanzen und die Bodenoberfläche gespritzt. 3 bis 4 Wochen nach Behandlung der Versuchspflanzen wird die Wirkung der Präparate visuell im Vergleich zu behandelten Kontrollen bonitiert (herbizide Wirkung in Prozent (%): 100 % Wirkung = Pflanzen sind abgestorben, 0 % Wirkung = wie Kontrollpflanzen).

### Verwendung von Safenern

Soll zusätzlich getestet werden, ob Safener die Pflanzenverträglichkeit von Testsubstanzen bei den Kulturpflanzen verbessern können, werden folgende Möglichkeiten für die Anwendung des Safeners verwendet:
- Samen der Kulturpflanzen werden vor der Aussaat mit der Safenersubstanz gebeizt (Angabe der Safenermenge in Prozent bezogen auf das Samengewicht)
- Kulturpflanzen werden vor Anwendung der Testsubstanzen mit dem Safener mit einer bestimmten Hektaraufwandmenge gespritzt (üblicherweise 1 Tag vor Anwendung der Prüfsubstanzen)
- der Safener wird zusammen mit der Testsubstanz als Tankmischung appliziert (Angabe der Safenermenge in g/ha oder als Verhältnis zum Herbizid).

Durch Vergleich der Wirkung von Testsubstanzen auf Kulturpflanzen, welche ohne und mit Safener behandelt wurden, kann die Wirkung der Safenersubstanz beurteilt werden.

### Gefäßversuche mit Getreide im Frein ('Vogelkäfig')

**Mefenpyr 1 Tag vor Herbizidapplikation**

| | Aufwandmenge g ai/ha | Sommergerste n. 28d beobachtet (%) |
|---|---|---|
| Beispiel (I-1-c-2) | 50 | 90 |
| | 25 | 65 |
| Beispiel (I-1-c -2) + Mefenpyr | 50 + 100 | 30 |
| | 25 + 100 | 15 |

| | Aufwandmenge g ai/ha | Sommerweizen n. 10d beobachtet (%) |
|---|---|---|
| Beispiel (I-1-a-4) | 50 | 40 |
| | 25 | 25 |
| Beispiel (I-1-a-4) + Mefenpyr | 50 + 100 | 20 |
| | 25 + 100 | 10 |

| | Aufwandmenge g ai/ha | Sommerweizen n. 28d beobachtet (%) |
|---|---|---|
| Beispiel (I-1-a-3) | 50 | 70 |
| | 25 | 40 |
| Beispiel (I-1-a-3) + Mefenpyr | 50 + 100 | 30 |
| | 25+100 | 20 |

### Gefäßversuche mit Getreide im Gewächshaus und im Freien ('Vogelkäfig')

**Mefenpyr 1 Tag vor Herbizidapplikation**

| | 28 Tage nach Applikation | | |
|---|---|---|---|
| | Aufwandmenge g ai/ha | Sommergerste beobachtet (%) | Sommerweizen beobachtet (%) |
| | 100 | 95 | 75 |
| Beispiel (I-I-a-2) | 50 | 70 | 70 |
| | 25 | 50 | 40 |
| Beispiel (I-1-a-2) + Mefenpyr | 100 + 100 | 50 | 30 |
| | 50 + 100 | 40 | 25 |
| | 25 + 100 | 30 | 20 |

| | | 10 Tage nach Applikation | |
|---|---|---|---|
| | | Aufwandmenge g ai/ha | Sommergerste beobachtet (%) |
| Beispiel (I-1-a-3) | | 50 | 70 |
| | | 25 | 70 |
| | | 12,5 | 60 |
| | | 6,25 | 20 |
| Beispiel (I-1-a-3) + Mefenpyr | | 50 + 100 | 40 |
| | | 25 + 100 | 10 |
| | | 12,5 + 100 | 5 |
| | | 6,25 + 100 | 0 |

| | | 28 Tage nach Applikation | |
|---|---|---|---|
| | | Aufwandmenge g ai/ha | Sommerweizen beobachtet (%) |
| Beispiel (I-1-c-2) | | 50 | 70 |
| | | 25 | 50 |
| | | 12,5 | 30 |
| | | 6,25 | 20 |
| Beispiel (I-1-c-2) + Mefenpyr | | 50 + 100 | 20 |
| | | 25 + 100 | 15 |
| | | 12,5 + 100 | 10 |
| | | 6,25 + 100 | 10 |

| | Aufwandmenge g ai/ha | Sommergerste beobachtet (%) | Sommerweizen beobachtet (%) |
|---|---|---|---|
| | 25 | 85 | |
| Beispiel (I-1-a-5) | 12,5 | 70 | 80 |
| | 6,25 | 30 | 70 |
| | 3,125 | | 30 |
| Beispiel (I-1-a-5) + Mefenpyr | 25 + 100 | 60 | |
| | 12,5 + 100 | 30 | 60 |
| | 6,25 + 100 | 10 | 15 |
| | 3,125 + 100 | | 0 |

| | | 10 Tage nach Applikation | |
|---|---|---|---|
| | Aufwandmenge g ai/ha | Sommergerste beobachtet (%) | Sommerweizen beobachtet (%) |
| Beispiel (I-1-a-7) | 100 | 60 | 50 |
| | 50 | 60 | 50 |
| | 25 | 40 | 40 |
| Beispiel (I-1-a-7) + Mefenpyr | 100 + 100 | 15 | 10 |
| | 50 + 100 | 10 | 5 |
| | 25 + 100 | 5 | 3 |

### Beispiel 7

### Heliothis virescens - Test - Behandlung transgener Pflanzen

| | |
|---|---|
| Lösungsmittel: | 7 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine max) der Sorte Roundup Ready (Warenzeichen der Monsanto Comp. USA) werden durch Tauchen in die Wirkstoffzubereitung der gewünschten Konzentration behandelt und mit der Tabakknospenraupe Heliothis virescens besetzt, solange die Blätter noch feucht sind.

Nach der gewünschten Zeit wird die Abtötung der Insekten bestimmt.

### Beispiel 8

### Grenzkonzentrations-Test / Bodeninsekten - Behandlung transgener Pflanzen

| | |
|---|---|
| Testinsekt: | Diabrotica balteata - Larven im Boden |
| Lösungsmittel: | 7 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird auf den Boden gegossen. Dabei spielt die Konzentration des Wirkstoffs in der Zubereitung praktisch keine Rolle, entscheidend ist allein die Wirkstoffgewichtsmenge pro Volumeneinheit Boden, welche in ppm (mg/l) angegeben wird. Man füllt den Boden in 0,251 Töpfe und lässt diese bei 20°C stehen.

Sofort nach dem Ansatz werden je Topf 5 vorgekeimte Maiskörner der Sorte YIELD GUARD (Warenzeichen von Monsanto Comp., USA) gelegt. Nach 2 Tagen werden in den behandelten Boden die entsprechenden Testinsekten gesetzt. Nach weiteren 7 Tagen wird der Wirkungsgrad des Wirkstoffs durch Auszählen der aufgelaufenen Maispflanzen bestimmt (1 Pflanze = 20 % Wirkung).

## Patentansprüche

1. Verbindungen der Formel (I) 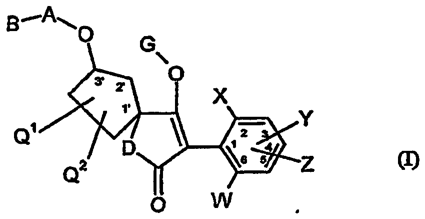 in welcher
W für Wasserstoff, Alkyl, Alkenyl, Alkinyl, Halogen, Alkoxy, Halogenalkyl, Halogenalkoxy oder Cyano steht,
X für Halogen, Alkyl, Alkenyl, Alkinyl, Alkoxy, Alkoxy-alkoxy, Halogenalkyl, Halogenalkoxy oder Cyano steht,
Y in für Wasserstoff, Halogen, Alkyl, Alkenyl, Alkinyl, Alkoxy, Cyano, Halogenalkyl Halogenalkoxy, für jeweils gegebenenfalls substituiertes Phenyl oder Hetaryl steht,
Z für Wasserstoff, Halogen, Alkyl, Halogenalkyl, Cyano, Alkoxy oder Halogenalkoxy steht,
A für eine gegebenenfalls substituierte Alkandiylgruppe oder für gegebenenfalls substituiertes und/oder gegebenenfalls durch ein Heteroatom unterbrochenes Cycloalkyl steht,
B für Wasserstoff oder jeweils gegebenenfalls substituiertes Alkyl, Alkenyl, Alkoxy, Alkoxy-alkoxy, Phenyl, Hetaryl oder für gegebenenfalls substituiertes und/oder gegebenenfalls durch Heteroatome und/oder C=O unterbrochenes Cycloalkyl steht,
oder A für eine Bindung und B für Wasserstoff steht,
D für NH oder Sauerstoff steht,
Q¹ für Wasserstoff für jeweils gegebenefalls substituiertes Alkyl, Alkoxy, Alkoxy-alkyl oder Alkylthioalkyl, für gegebenenfalls substituiertes Cycloalkyl, worin gegebenenfalls eine Methylengruppe durch Heteroatome ersetzt ist oder für gegebenenfalls substituiertes Phenyl, Hetaryl, Phenylalkyl oder Hetarylalkyl steht,
Q² für Wasserstoff oder Alkyl steht,
Q¹ und Q² gemeinsam mit dem Kohlenstoff, an das sie gebunden sind, für einen gegebenenfalls substituierten C₃-C₆ Ring stehen, der gegebenenfalls durch ein Heteroatom unterbrochen sein kann, oder
Q¹ und Q² gemeinsam mit den Kohlenstoffatomen, an die sie gebunden sind, für einen gegebenenfalls substituierten C₃-C₆-Ring stehen, der gegebenenfalls durch ein Hetematom unterbrochen sein kann, oder
G für Wasserstoff (a) oder für eine der Gruppen 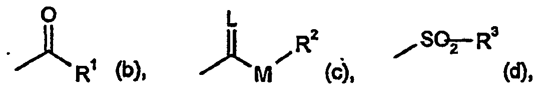 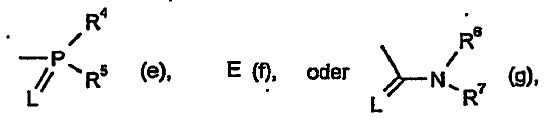 steht,
worin
B für ein Metallion oder ein Ammoniumion steht,
L für Sauerstoff oder Schwefel steht,
M für Sauerstoff oder Schwefel steht,
R¹ für jeweils gegebenenfalls durch Halogen oder Cyano substituiertes Alkyl, Alkenyl, Alkoxyalkyl, Alkylthioalkyl oder Polyalkoxyalkyl oder für jeweils gegebenenfalls durch Halogen, Alkyl oder Alkoxy substituiertes Cycloalkyl oder Heterocyclyl oder für jeweils gegebenenfalls substituiertes Phenyl, Phenylalkyl, Hetaryl, Phenoxyalkyl oder Hetaryloxyalkyl steht
R² für jeweils gegebenenfalls durch Halogen oder Cyano substituiertes Alkyl, Alkenyl, Alkoxyalkyl oder Polyalkoxyalkyl oder für jeweils gegebenenfalls substituiertes Cycloalkyl, Phenyl oder Benzyl steht,
R³, R⁴ und R⁵ unabhängig voneinander für jeweils gegebenenfalls durch Halogen substituiertes Alkyl, Alkoxy, Alkylamino, Dialkylamino, Alkylthio, Alkenylthio oder Cycloalkylthio oder für jeweils gegebenenfalls substituiertes Phenyl, Benzyl, Phenoxy oder Phenylthio stehen,
R⁶ und R⁷ unabhängig voneinander für Wasserstoff, für jeweils gegebenenfalls durch Halogen oder Cyano substituiertes Alkyl, Cycloalkyl, Alkenyl, Alkoxy, Alkoxyalkyl, für jeweils gegebenenfalls substituiertes Phenyl oder Benzyl stehen, oder gemeinsam mit dem N-Atom, an das sie gebunden sind, einen gegebenenfalls Sauerstoff oder Schwefel enthaltenden und gegebenenfalls substituierten Cyclus bilden.

2. Verbindungen der Formel (I) gemäß Anspruch 1, in welcher
W für Wasserstoff C₁-C₆-Alkyl, C₂-C₆-Alkenyl, C₂-C₆-Alkinyl, Halogen, C₁-C₆-Alkoxy, C₁-C₄-Halogenalkyl, C₁-C₄-Halogenalkoxy oder Cyano steht,
X für Halogen, C₁-C₆-Alkyl, C₂-C₆-Alkenyl, C₂-C₆-Alkinyl, C₁-C₆-Alkoxy, C₁-C₆-Alkoxy-C₁-C₄-alkoxy, C₁-C₄ Halogenalkyl, C₁-C₄-Halogenalkoxy oder Cyano steht,
Y für Wasserstoff Halogen, C₁-C₆-Alkyl, C₂-C₆-Alkenyl, C₂-C₆-Alkinyl, C₂-C₆-Alkoxy, Cyano, C₁-C₄-Halogenalkyl, Halogenalkoxy, für durch V¹ und V² substituiertes Phenyl oder Pyridyl steht,
V¹ für Halogen, C₁-C₁₂-Alkyl, C₁-C₆-Alkoxy, C₁-C₄-Halogenalkyl, C₁-C₄-Halogen-alkoxy, Cyano oder Nitro steht,
V² für Wasserstoff Halogen, C₁-C₆-Alkyl, C₁-C₆-Alkoxy oder C₁-C₄-Halogenalkyl steht,
V¹ und V² gemeinsam für C₃-C₄-Alkandiyl stehen, welches gegebenenfalls durch Halogen und/oder C₁-C₂-Alkyl substituiert sein kann und welches gegebenenfalls durch ein oder zwei Sauerstoffatome unterbrochen sein kann,
Z für Wasserstoff, Halogen, C₁-C₆-Alkyl, C₁-C₄-Halogenalkyl, Cyano, C₁-C₆-Alkoxy oder C₁-C₄-Halogenalkoxy steht,
A für eine gegebenenfalls durch C₁-C₄-Alkyl substituierte C₁-C₄-Alkandiylgruppe oder für gegebenenfalls durch C₁-C₄-Alkyl substituiertes C₅-C₈-Cycloalykl steht, in.welchem gegebenenfalls eine Methylgruppe durch Sauerstoff ersetzt ist,
B für Wasserstoff oder für jeweils gegebenenfalls durch Halogen substituiertes C₁-C₈-Alkyl, C₂-C₈-Alkenyl, C₁-C₆-Alkoxy, C₁-C₆-Alkoxy-C₁-C₄-alkoxy, C₁-C₄-Alkoxy-bis-C₁-C₄-alkoxy für gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl für gegebenenfalls durch Halogen, C₁-C₄-Alkyl oder C₁-C₂-Halogenalkyl substituiertes Pyridyl, Pyrimidyl, Thiazolyl oder Thienyl oder für gegebenenfalls durch Halogen C₁-C₄-Alkyl, C₁-C₄-Alkoxy oder C₁-C₂-Halogen-alkyl substituiertes C₃-C₈-Cycloalkyl steht, in welchem gegebenenfalls eine oder zwei nicht direkt benachbarte Methylengruppen durch Sauerstoff ersetzt, zwei Methylengruppen durch den Rest -O-CO- oder drei Methylengruppen durch den Rest-O-CO-O- ersetzt sind,
oder A für eine Bindung und B für Wasserstoff steht,
D für NH oder Sauerstoff steht,
Q¹ für Wasserstoff, oder für jeweils gegebenenfalls durch Halogen substituiertes C₁-C₆-Alkyl, C₁-C₆-Alkoxy, C₁-C₄-Alkoxy-C₁-C₄-alkyl, C₁-C₄-Alkylthio-C₁-C₄-alkyl oder für gegebenenfalls durch Halogen, C₁-C₄-Alkyl oder C₁-C₄-Alkoxy substituiertes C₃-C₆-Cycloalkyl, worin gegebenenfalls eine Methylengruppe durch Sauerstoff ersetzt ist oder für jeweils einfach bis zweifach durch Halogen, C₁-C₄-Halogenalkyl oder C₁-C₄-Halogenalkoxy substituiertes Phenyl, Phenyl-C₁-C₂-alkyl oder Hetaryl steht,
Q² für Wasserstoff oder C₁-C₆-Alkyl steht, oder
Q¹ und Q² gemeinsam mit dem Kohlenstoff, an das sie gebunden sind bevorzugt für einen gegebenenfalls einfach bis zweifach durch Fluor, Chlor, C₁-C₄-Alkyl, C₁-C₄-Alkoxy oder Trifluormethyl substituierten C₃-C₆-Ring stehen, worin gegebenenfalls eine Methylengruppe durch Sauerstoff ersetzt sein kann, oder
Q¹ und Q² gemeinsam mit den Kohlenstoffatomen, an die sie gebunden sind, für eignen Gegebenenfalls einfach bis zweifach durch Fluor, Chlor, C₁-C₄-Alkyl, C₁-C₄-Alkoxy oder Trifluormethyl substituierten C₃-C₆-Ring stehen, worin gegebenenfalls eine Methylengruppe durch Sauerstoff ersetzt sein kann,
G für Wasserstoff (a) oder für eine der Gruppen 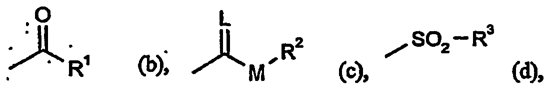 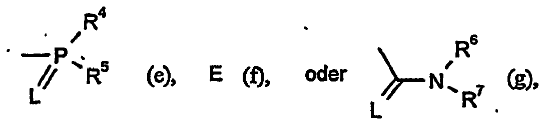 steht,
in welchen
E für ein Metallion oder ein Ammoniumion steht,
L für Sauerstoff oder Schwefel steht und
M für Sauerstoff oder Schwefel steht,
R¹ für jeweils gegebenenfalls durch Halogen oder Cyano substituiertes C₁-C₂₀-Alkyl, C₂-C₂₀-Alkenyl, C₁-C₈-Alkoxy-C₁-C₈-alkyl C₁-C₈-Alkylthio-C₁-C₈-alkyl oder Poly-C₁-C₈-alkoxy-C₁-C₈-alkyl oder für gegebenenfalls durch Halogen, C₁-C₆-Alkyl oder C₁-C₆-Akoxy substituiertes C₃-C₈-Cycloalkyl in welchem gegebenenfalls eine oder zwei nicht direkt benachbarte Methylengruppen durch Sauerstoff und/oder Schwefel ersetzt sind,
für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkylsulfonyl substituiertes Phenyl,
für gegebenenfalls durch Halogen, Nitro. Cyano, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls durch Halogen oder C₁-C₆-Alkyl substituiertes 5- oder 6-gliedriges Hetaryl mit ein oder zwei Heteroatomen aus der Reihe Sauerstoff, Schwefel und Stickstoff,
für gegebenenfalls durch Halogen oder C₁-C₆-Alkyl substituiertes Phenoxy-C₁-C₆-alkyl oder
für gegebenenfalls durch Halogen, Amino oder C₁-C₆-Alkyl substituiertes 5- oder 6-gliedriges Hetaryloxy-C₁-C₆-alkyl steht mit ein oder zwei Heteroatomen aus der Reihe Sauerstoff, Schwefel und Stickstoff,
R² für jeweils gegebenenfalls durch Halogen oder Cyano substituertes C₁-C₂₀-Alkyl C₂-C₂₀-Alkenyl C₁-C₈-Alkoxy-C₂-C₈-alkyl oder Poly-C₁-C₈-alkoxy-C₂-C₈-alkyl,
für gegebenenfalls durch Halogen, C₁-C₆-Alkyl oder C₁-C₆-Alkoxy substituiertes C₃-C₈-Cycloalkyl oder
für jeweils gegebenenfalls durch Halogen, Cyano, Nitro, C₁-C₆-Alkyl C₁-C₆-Alkoxy, C₁-C₆-Halogenalkyl oder C₁-C₆-Halogenalkoxy substituiertes Phenyl oder Benzyl steht,
R³ für gegebenenfalls durch Halogen substituiertes C₁-C₈-Alkyl oder jeweils gegebenenfalls durch Halogen, C₁-C₆-Alkyl, C₁-C₆-Alkoxy, C₁-C₄-Halogenalkyl, C₁-C₄-Haloganalkoxy, Cyano oder Nitro substituiertes Phenyl oder Benzyl steht,
R⁴ and R⁵ unabhängig voneinander für jeweils gegebenenfalls durch Halogen substituiertes C₁-C₈-Alkyl C₁-C₈-Alkoxy, C₁-C₈-Alkylamino, Di-(C₁-C₈-alkyl)amino, C₁-C₈-Alkylthio oder C₃-C₈-Alkenylthio oder für jeweils gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl oder C₁-C₄-Halogenalkyl substituiertes Phenyl, Phenoxy oder Phenylthio stehen,
R⁶ and R⁷ unabhängig voneinander für Wasserstoff, für jeweils gegebaneafalls durch Halogen oder Cyano substituiertes C₁-C₈-Alkyl, C₃-C₈-Cycloalkyl, C₁-C₈-Alkoxy, C₃-C₈-Alkenyl oder C₁-C₈-Alkoxy-C₂-C₈-alkyl, für jeweils gegebenenfalls durch Halogen, C₁-C₈-Alkyl, C₁-C₈-Halogenalkyl oder C₁-C₈-alkoxy substituiertes Phenyl oder Benzyl oder zusammen für einen gegebenenfalls durch C₁-C₆-Alkyl substituierten C₃-C₆-Alkylenrest stehen, in welchem gegebenenfalls eine Methylengruppe durch Sauerstoff oder Schwefel ersetzt ist.

3. Verbindungen der Formel (I) gemäß Anspruch 1, in welcher
W für Wasserstoff, Chlor, Brom, Iod, C₁-C₄-Alkyl, C₂-C₄-Alkenyl, C₂-C₄-Alkinyl, C₁-C₄-Alkoxy, C₁-C₂-Halogenalkyl oder C₁-C₂-Halonalkoxy steht,
X für Chlor, Brom, Iod, C₁-C₄-Alkyl C₂-C₄-Alkenyl, C₂-C₄-Alkinyl, C₁-C₄-Alkoxy, C₁-C₄-Alkoxy-C₁-C₃-alkoxy, C₁-C₂-Halogenalkyl, C₁-C₂-Halogenalkoxy oder Cyano steht,
Y in der 4-Position für Wasserstoff, Fluor, Chlor, Brom, Iod, Methoxy, Ethoxy, Cyano, Trifluormethyl, Difluormethoxy oder Trifluormethoxy steht,
Z für Wasserstoff steht.
W für Wasserstoff Chlor, Brom oder C₁-C₄-Alkyl steht,
X für Chlor, Brom, C₁-C₄-Alkyl, C₁-C₄-Alkoxy, C₁-C₂-Halogenalkyl, C₁-C₂-Halogenalkoxy oder Cyano steht,
Y in der 4-Position für C₂-C₄-Alkenyl, C₂-C₄-Alkinyl oder für den Rest 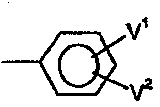 steht,
Z für Wasserstoff steht,
V¹ auch für Fluor, Chlor, C₁-C₄-Alkyl, C₁-C₄-Alkoxy, C₁-C₂-Halogenalkyl oder C₁-C₂-Halogenalkoxy steht,
V² auch für Wasserstoff, Fluor, Chlor, C₁-C₄-Alkyl, C₁-C₄-Alkoxy oder C₁-C₂-Halogenalkyl steht,
V¹ und V² gemeinsam auch für -O-CH₂-O- und -O-CF₂-O- stehen.
W ebenfalls für Wasserstoff, Chlor, Brom oder C₁-C₄-Alkyl steht,
X ebenfalls für Chlor, Brom, C₁-C₄-Alkyl oder C₁-C₂-Halogenalkyl steht,
Y ebenfalls in der 5-Position für C₂-C₄-Alkenyl, C₂-C₄-Alkinyl, für den Rest 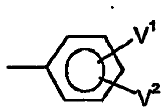 steht,
Z ebenfalls in der 4-Position für Wasserstoff, C₁-C₄-Alkyl oder Chlor steht,
V¹ ebenfalls für Fluor, Chlor, C₁-C₄-Alkyl, C₁-C₄-Alkoxy, C₁-C₂-Halogenalkyl oder C₁-C₂-Halogenalkoxy steht,
V² ebenfalls für Wasserstoff, Fluor, Chlor, C₁-C₄-Alkyl, C₁-C₄-Alkoxy oder C₁-C₂-Halogenalkyl steht,
V¹ und V² gemeinsam ebenfalls für -O-CH₂-O- oder -O-CF₂-O- stehen.
W außerdem für Wasserstoff, C₁-C₄-Alkyl, C₂-C₄-Alkenyl, C₂-C₄-Alkinyl, C₁-C₄-Alkoxy, Chlor, Brom, Iod oder Trifluormethyl steht,
X außerdem für Chlor, Brom, Iod, C₁-C₄-Alkyl, C₂-C₄-Alkenyl, C₂-C₄-Alkinyl, C₁-C₄-Alkoxy, C₁-C₄-Alkoxy-C₁-C₃-alkoxy, C₁-C₂-Halogenalkyl, C₁-C₂-Halogenalkoxy oder Cyano steht,
Y außerdem in der 4-Position für C₁-C₄-Alkyl steht,
Z außerdem für Wasserstoff steht.
W weiterhin für Wasserstoff, Chlor, Brom, Iod, C₁-C₄-Alkyl oder C₁-C₄-Alkoxy steht,
X weiterhin für Chlor, Brom, Iod, C₁-C₄-Alkyl, C₁-C₄-Alkoxy, C₁-C₂-Halogenalkyl, C₁-C₂-Halogenalkoxy oder Cyano steht,
Y weiterhin in der 4-Position für Wasserstoff, Chlor, Brom, C₁-C₄-Alkyl, C₁-C₂-Halogenalkyl oder C₁-C₂-Halogenalkoxy steht,
Z weiterhin in der 3- oder 5-Position für Fluor, Chlor, Brom, Iod, C₁-C₄-Alkyl, C₁-C₂-Halogenalkyl, C₁-C₄-Alkoxy oder C₁-C₂-Halogenalkoxy steht.
A für eine gegebenenfalls durch C₁-C₂-Alkyl substituierte C₁-C₃-Alkandiylgruppe oder für C₅-C₆-Cycloalkyl steht, in welchen gegebenenfalls eine Methylengruppe durch Sauerstoff ersetzt ist.
B für Wasserstoff oder jeweils gegebenenfalls einfach bis dreifach durch Fluor oder Chlor substituiertes C₁-C₆-Alkyl, C₂-C₆-Alkenyl, C₁-C₄-Alkoxy, C₁-C₄-Alkoxy-C₁-C₃-alkoxy, C₁-C₄-Alkoxy-bis-C₁-C₃-alkoxy, für gegebenenfalls einfach bis dreifach durch Fluor, Chlor, Brom, C₁-C₄-Alkyl, C₁-C₄-Alkoxy, C₁-C₂-Halogenalkyl, C₁-C₂-Halogenalkoxy, Cyano oder Nitro substituiertes Phenyl, für gegebenenfalls einfach bis zweifach durch Fluor, Chlor, Brom, Methyl, Ethyl oder Trifluormethyl substituiertes Pyridyl, Pyrimidyl, Thiazolyl oder Thienyl oder für gegebenenfalls einfach bis zweifach durch Fluor, Chlor, Methyl, Methoxy oder Trifluormethyl substituiertes C₃-C₆-Cycloalkyl steht, in welchem gegebenenfalls eine Methylengruppe oder zwei nicht direkt benachbarte Methylengruppen durch Sauerstoff ersetzt sind,
oder A für eine Bindung und B für Wasserstoff steht
D für NH oder Sauerstoff steht.
Q¹ für Wasserstoff, für gegebenenfalls einfach bis dreifach durch Fluor substituiertes C₁-C₄-Alkyl steht,
Q² für Wasserstoff oder C₁-C₄-Alkyl steht.
Q¹ und Q² gemeinsam mit dem Kohlenstoffatom, an das sie gebunden sind, besonders bevorzugt für einen gegebenenfalls einfach durch Fluor, Methyl, Methoxy oder Trifluormethyl substituierten C₃-C₆-Ring stehen, worin gegebenenfalls eine Methylengruppe durch Sauerstoff ersetzt sein kann, oder
Q¹ und Q² gemeinsam mit den Kohlenstoffatomen, an die sie gebunden sind, besonders bevorzugt für einen gegebenenfalls einfach durch Fluor, Methyl, Methoxy oder Trifluormethyl substituierten C₃-C₆-Ring stehen, worin gegebenenfalls eine Methylengruppe durch Sauerstoff ersetzt sein kann.
G für Wasserstoff(a) oder für eine der Gruppen 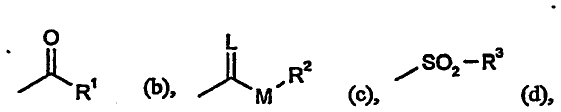 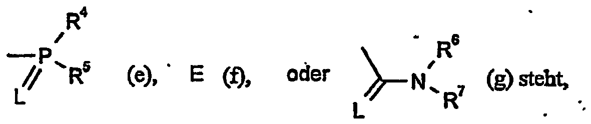 in welchen
B für ein Metallion oder ein Ammoniumion steht,
L für Sauerstoff oder Schwefel steht und
M für Sauerstoff oder Schwefel steht
R¹ für jeweils gegebenenfalls einfach bis dreifach durch Fluor oder Chlor substituiertes C₁-C₁₆-Alkyl, C₂-C₁₆-Alkenyl, C₁-C₆-Alkoxy-C₁-C₄-alkyl C₁-C₆-Alkylthio-C₁-C₄-alkyl oder Poly-C₁-C₆-alkoxy-C₁-C₄-alkyl oder für gegebenenfalls einfach bis zweifach durch Fluor, Chlor, C₁-C₅-Alkyl oder C₁-C₅-Alkoxy substituiertes C₃-C₇-Cycloalkyl, in welchem gegebenenfalls eine oder zwei nicht direkt benachbarte Methylengruppen durch Sauerstoff und/oder Schwefel ersetzt sind,
für gegebenenfalls einfach bis dreifach durch Fluor, Chlor, Brom, Cyano, Nitro, C₁-C₄-Alkyl, C₁-C₄-Alkoxy, C₁-C₃-Halogenalkyl, C₁-C₃-Halogenalkoxy, C₁-C₄-Alkylthio oder C₁-C₄-Alkylsulfonyl substituiertes Phenyl,
für gegebenenfalls einfach bis zweifach durch Fluor, Chlor, Brom, C₁-C₄-Alkyl, C₁-C₄-Alkoxy, C₁-C₃-Halogenalkyl oder C₁-C₃-Halogenalkoxy substituiertes Phenyl-C₁-C₄-alkyl,
für jeweils gegebenenfalls einfach bis zweifach durch Fluor, Chlor, Brom oder C₁-C₄-Alkyl substituiertes Pyrazolyl, Thiazolyl, Pyridyl, Pyrimidyl, Furanyl oder Thienyl,
für gegebenenfalls einfach bis zweifach durch Fluor, Chlor, Brom oder C₁-C₄-Alkyl substituiertes Phenoxy-C₁-C₅-alkyl oder
für jeweils gegebenenfalls einfach bis zweifach durch Fluor, Chlor, Brom, Amino oder C₁-C₄-Alkyl substituiertes Pyridyloxy-C₁-C₅-alkyl, Pyrimidyloxy-C₁-C₅-alkyl oder Thiazolyloxy-C₁-C₅-alkyl steht.
R² für jeweils gegebenenfalls einfach bis dreifach durch Fluor oder Chlor substituiertes C₁-C₁₆-Alkyl, C₂-C₁₆-Alkenyl, C₁-C₆-Alkoxy-C₂-C₆-alkyl oder Poly-C₁-C₆-alkoxy-C₂-C₆-alkyl,
für gegebenenfall einfach bis zweifach durch Fluor, Chlor, C₁-C₄-Alkyl oder C₁-C₄-Alkoxy substituiertes C₃-C₇-Cycloalkyl oder
für jeweils gegebenenfalls einfach bis dreifach durch Fluor, Chlor, Brom, Cyano, Nitro, C₁-C₄-Alkyl, C₁-C₃-Alkoxy, C₁-C₃-Halogenalkyl oder C₁-C₃-Halogenalkoxy substituiertes Phenyl oder Benzyl steht,
R³ für gegebenenfalls einfach bis dreifach durch Fluor oder Chlor substituiertes C₁-C₆-Alkyl oder jeweils gegebenenfalls einfach bis zweifach durch Fluor, Chlor, Brom, C₁-C₄-Alkyl, C₁-C₄-Alkoxy, C₁-C₂-Halogenalkoxy, C₁-C₂-Halogenalkyl, Cyano oder Nitro substituiertes Phenyl oder Benzyl steht.
R⁴ und R⁵ unabhängig voneinander für jeweils gegebenenfalls einfach bis dreifach durch Fluor oder Chlor substituiertes C₁-C₆-Alkyl, C₁-C₆-Alkoxy, C₁-C₆-Alkylamino, Di-(C₁-C₆-alkyl)amino, C₁-C₆-Alkylthio oder C₃-C₄-Alkenylthio oder für jeweils gegebenenfalls einfach bis zweifach durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl oder C₁-C₃-Halogenalkyl substituiertes Phenyl, Phenoxy oder Phenylthio stehen.
R⁶ und R⁷ unabhängig Voneinander für Wasserstoff, für jeweils Gegebenenfalls einfach bis dreifach durch Fluor oder Chlor substituiertes C₁-C₆-Alkyl, C₃-C₆-Cycloalkyl, C₁-C₆-Alkoxy, C₃-C₆-Alkenyl oder C₁-C₆-Alkoxy-C₂-C₆-alkyl für jeweils gegebenenfalls einfach bis dreifach durch Fluor, Chlor, Brom, C₁-C₅-Halogenalkyl, C₁-C₅-Alkyl oder C₁-C₅-Alkoxy substituiertes Phenyl oder Benzyl, oder zusammen für einen gegebenenfalls durch C₁-C₄-Alkyl substituierten C₃-C₆-Alkylenrest stehen, in welchem gegebenenfalls eine Methylengruppe durch Sauerstoff oder Schwefel ersetzt ist.

4. Verbindungen der Formel (I) gemäß Anspruch 1, in welcher
W für Wasserstoff, Chlor, Brom, Iod, Methyl, Ethyl, Methoxy, Ethoxy oder Trifluormethyl steht,
X für Chlor, Brom, Iod, Methyl, Ethyl, Propyl, Methoxy, Ethoxy, Propoxy, Methoxy-ethoxy, Ethoxy-ethoxy, Trifluormethyl, Difluormethoxy, Trifluormethoxy oder Cyano steht,
Y in der 4-Position für Wasserstoff, Chlor, Brom, Iod, Trifluormethyl oder Trifluormethoxy steht,
Z für Wasserstoff steht.
W auch für Wasserstoff, Chlor, Brom, Methyl oder Ethyl steht,
X auch für Chlor, Brom, Methyl, Ethyl, Propyl, Methoxy, Trifluormethyl, Difluormethoxy oder Cyano steht,
Y auch in der 4-Position für Vinyl, Ethinyl, Propinyl oder für den Rest 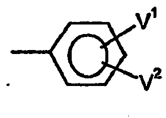 steht,
Z auch für Wasserstoff steht,
V¹ auch für Fluor, Chlor, Methyl, Methoxy, Trifluormethyl oder Trifluormethoxy steht,
V² auch für Wasserstoff, Fluor, Chlor, Methyl, Methoxy oder Trifluormethyl steht.
W ebenfalls für Wasserstoff, Chlor oder Methyl steht,
X. ebenfalls für Chlor, Methyl oder Trifluormethyl steht,
Y ebenfalls in der 5-Position für Vinyl, Ethinyl, Propinyl oder für den Rest 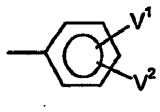 steht,
Z ebenfalls in der 4-Position für Wasserstoff oder Methyl steht,
V¹ ebenfalls für Fluor, Chlor, Methyl, Methoxy, Trifluormethyl oder Trifluormethoxy steht,
V² ebenfalls für Wasserstoff, Fluor, Chlor, Methyl, Methoxy oder Trifluormethyl steht.
W außerdem für Wasserstoff, Methyl, Ethyl, Methoxy, Ethoxy, Chlor, Brom oder Iod steht,
X außerdem für Chlor, Brom, Iod, Methyl, Ethyl, Propyl, Methoxy, Ethoxy, Propoxy, Methoxy-ethoxy, Ethoxy-ethoxy, Trifluormethyl, Difluormethoxy, Trifluormethoxy oder Cyano steht,
Y außerdem in der 4-Position für Methyl oder Ethyl steht,
Z außerdem für Wasserstoff steht.
W weiterhin für Wasserstoff, Chlor, Brom, Iod, Methyl oder Ethyl steht,
X weiterhin für Chlor, Brom, Iod, Methyl, Ethyl, Methoxy, Trifluormethyl, Difluormethoxy oder Trifluormethoxy steht,
Y weiterhin in der 4-Position für Wasserstoff, Chlor, Brom, Iod, Methyl oder Ethyl steht,
Z weiterhin in der 3- oder 5-Position für Fluor, Chlor, Brom, Iod, Methyl, Ethyl, Trifluormethyl oder Trifluormethoxy steht.
A für -CH₂-, -CHCH₃-, -CH₂-CH₂-, -CH₂-CHCH₃-, -CH₂-CH₂-CH₂-steht.
B für Wasserstoff, Methyl, Ethyl, Propyl, iso-Propyl, Butyl, iso-Butyl, C₂-C₄-Alkenyl, Methoxy, Ethoxy, Propoxy, Isopropoxy, Butoxy, Isobutoxy, Methoxy-ethoxy, Ethoxy-ethoxy, für gegebenenfalls einfach bis zweifach durch Fluor, Chlor, Brom, Methyl, Methoxy, Trifluormethyl, Trifluormethoxy, Cyano oder Nitro substituiertes Phenyl, für Cyclopropyl, für Cyclopentyl oder Cyclohexyl steht, in welchem gegebenenfalls eine Methylengruppe durch Sauerstoff ersetzt ist,
oder A für eine Bindung und B für Wasserstoff steht.
D für NH oder Sauerstoff steht.
Q¹ für Wasserstoff, Methyl oder Ethyl steht.
Q² für Wasserstoff, Methyl oder Ethyl steht.
Q¹ und Q² gemeinsam mit dem Kohlenstoffatom, an das sie gebunden sind für Cyclopropyl, Cyclopentyl oder Cyclohexyl stehen, oder
Q¹ und Q² gemeinsam mit den Kohlenstoffatomen an die sie gebunden sind für einen gegebenenfalls durch Sauerstoff unterbrochenen C₅-C₆-Ring stehen.
G für Wasserstoff (a) oder für eine der Gruppen 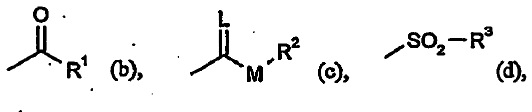 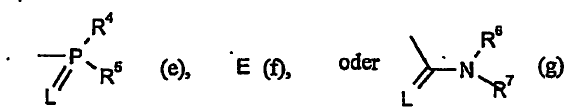 steht, in welchen
E für ein Metallion oder ein Ammoniumion steht,
L für Sauerstoff oder Schwefel steht und
M für Sauerstoff oder Schwefel steht.
R¹ für jeweils gegebenenfalls einfach bis dreifach durch Fluor oder Chlor substituiertes C₁-C₁₀-Alkyl, C₂-C₁₀-Alkenyl, C₁-C₄-Alkoxy-C₁-C₂-alkyl, C₁-C₄-Alkylthio-C₁-C₂-alkyl oder für gegebenenfalls einfach durch Fluor, Chlor, Methyl, Ethyl oder Methoxy substituiertes C₃-C₆-Cycloalkyl,
für gegebenenfalls einfach bis zweifach durch Fluor, Chlor, Brom, Cyano, Nitro, Methyl, Ethyl, n-Propyl, i-Propyl, Methoxy, Ethoxy, Trifluormethyl oder Trifluormethoxy substituiertes Phenyl,
für jeweils gegebenenfalls einfach durch Chlor, Brom oder Methyl substituiertes Furanyl, Thienyl oder Pyridyl steht.
R² für jeweils gegebenenfalls einfach bis dreifach durch Fluor oder Chlor substituiertes C₁-C₁₀-Alkyl, C₂-C₁₀-Alkenyl oder C₁-C₄-Alkoxy-C₂-C₄-alkyl,
für Cyclopentyl oder Cyclohexyl
oder für jeweils gegebenenfalls einfach bis zweifach durch Fluor, Chlor, Cyano, Nitro, Methyl, Ethyl, Methoxy, Trifluormethyl oder Trifluormethoxy substituiertes Phenyl oder Benzyl steht
R³ für jeweils gegebenenfalls einfach bis dreifach durch Fluor oder Chlor substituiertes Methyl, Ethyl, Propyl oder iso-Propyl, oder gegebenenfalls einfach durch Fluor, Chlor, Brom, Methyl, Ethyl, iso-Propyl, tert.-Butyl, Methoxy, Ethoxy, iso-Propoxy, Trifluormethyl, Trifluormethoxy, Cyano oder Nitro substituiertes Phenyl steht.
R⁴ und R⁵ unabhängig voneinander für C₁-C₄-Alkoxy oder C₁-C₄-Alkylthio oder für jeweils gegebenenfalls einfach durch Fluor, Chlor, Brom, Nitro, Cyano, Methyl, Methoxy, Trifluormethyl oder Trifluormethoxy substituiertes Phenyl, Phenoxy oder Phenylthio steht.
R⁶ und R⁷ unabhängig voneinander für Wasserstoff, für C₁-C₄-Alkyl, C₃-C₆-Cycloalkyl, C₁-C₄-Alkoxy, C₃-C₄-Alkenyl oder C₁-C₄-Alkoxy-C₂-C₄-alkyl, für gegebenen-falls einfach bis zweifach durch Fluor, Chlor, Brom, Methyl, Methoxy oder Trifluormethyl substituiertes Phenyl, oder zusammen für einen C₅-C₆-Alkylenrest stehen, in welchem gegebenenfalls eine Methylengruppe durch Sauerstoff oder Schwefel ersetzt ist

5. Verbindungen der Formel (I) gemäß Anspruch 1, in welcher
W für Methyl, Ethyl oder Methoxy steht,
X für Chlor, Methyl, Ethyl oder Methoxy steht,
Y in der 4-Position für Chlor oder Brom steht,
Z für Wasserstoff steht.
W ebenfalls für Wasserstoff steht,
X ebenfalls für Methyl steht,
Y ebenfalls in der 5-Position für den Rest 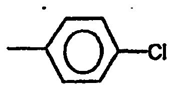 steht,
Z ebenfalls in der 4-Position für Wasserstoff steht.
W außerdem für Methyl oder Ethyl steht,
X außerdem für Chlor, Brom oder Methyl steht,
Y außerdem in der 4-Position für Methyl steht,
Z außerdem für Wasserstoff steht.
A für -CH₂- oder -CH₂-CH₂- steht,
B für Wasserstoff, Methyl, Ethyl, Propyl, Methoxy oder Cyclopropyl steht,
oder A für eine Bindung und B für Wasserstoff steht.
D für NH steht,
Q¹ für Wasserstoff steht,
Q² für Wasserstoff steht,
G für Wasserstoff (a) oder für eine der Gruppen 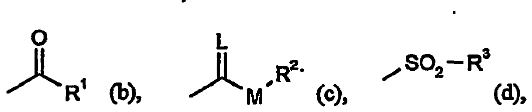 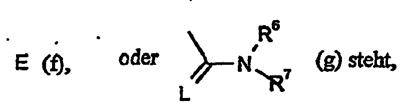 in welchen
E für ein Metallion steht,
L für Sauerstoff steht und
M für Sauerstoff steht.
R¹ für C₁-C₁₀-Alkyl steht,
für gegebenenfalls einfach durch Chlor substituiertes Phenyl steht,
R² für C₁-C₁₀-Alkyl oder C₂-C₁₀-Alkenyl steht, .
R3 für Methyl steht,
R⁶ und R⁷ zusammen für einen C₅-C₆-Alkylenrest stehen, in welchem gegebenenfalls eine Methylengruppe durch Sauerstoff ersetzt ist.

6. Verfahren zur Herstellung von Verbindungen der Formel (I) gemäß Anspruch 1, **dadurch gekennzeichnet, dass** man zum Erhalt von
(A) Verbindungen der Formel (I-1-a) 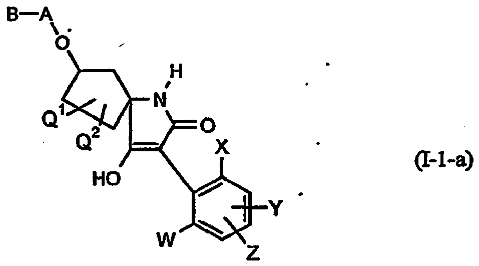 in welcher
A, B, Q¹, Q², W, X, Y und Z die oben angegebenen Bedeutungen haben, Verbindungen der Formel (II) 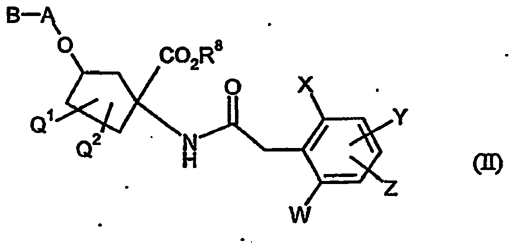 in welcher
A, B, Q¹, Q², W, X, Y und Z die oben angegebenen Bedeutungen haben,
und
R⁸ für Alkyl steht,
in Gegenwart eines Verdünnungsmittels und in Gegenwart einer Base intramolekular kondensiert,
(B) Verbindungen der Formel (I-2-a) 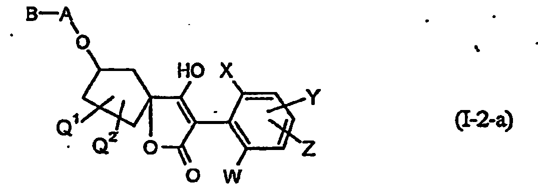 in welcher
A, B, Q¹, Q², W, X, Y und Z die oben angegebenen Bedeutungen haben,
Verbindungen der Formel (III) 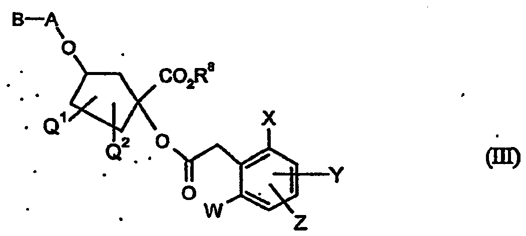 in welcher
A, B, Q¹, Q², W, X, Y, Z und R⁸ die oben angegebenen Bedeutungen haben,
in Gegenwart eines Verdünnungsmittels und in Gegenwart einer Base intramolekular kondensiert,
(C) Verbindungen der oben gezeigten Formeln (I-1-b) bis (I-2-b), in welchen R¹, A,
B, Q¹, Q²,W, X, Y und Z die oben angegebenen Bedeutungen haben, Verbindungen der oben gezeigten Formeln (I-1-a) bis (I-2-a), in welchen A, B, Q¹, Q², W, X, Y und Z die oben angegebenen Bedeutungen haben, jeweils
α) mit Verbindungen der Formel (IV) 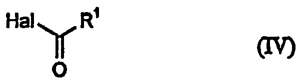 in welcher
R¹ die oben angegebene Bedeutung hat und
Hal für Halogen steht
oder
ß) mit Carbonsäureanhydriden der Formel (V)
R¹-CO-O-CO-R¹ (V)
in welcher
R¹ die oben angegebene Bedeutung hat,
gegebenenfalls in Gegenwart eines Verdünnungsmittels und gegebenenfalls in Gegenwart eines Säurebindemittels umsetzt,
(D) Verbindungen der oben gezeigten Formeln (I-1-c) bis (I-2-c), in welchen R², A, B, Q¹, Q², W, M, X, Y und Z die oben angegebenen Bedeutungen haben und L für Sauerstoff steht, Verbindungen der oben gezeigten Formeln (I-1-a) bis (I-2-a), in welchen A, B, Q¹, Q², W, X, Y und Z die oben angegebenen Bedeutungen haben, jeweils
mit Chloremeisensänreestern oder Chlorameisensäurethioestern der Formel (VI)
R²-M-CO-Cl (VI)
in welcher
R² und M die oben angegebenen Bedeutungen haben,
gegebenenfalls in Gegenwart eines Verdünnungsmittels und gegebenenfalls in Gegenwart eines Säurebindemittels umsetzt,
(B) - Verbindungen der oben gezeigten Formeln (I-1-c) bis (I-2-c), in welchen R², A, B, Q¹, Q², W, M, X, Y und Z die oben angegebenen Bedeutungen haben und L für Schwefel steht, Verbindungen der oben gezeigten Formeln (I-1-a) bis (I-2-a), in welchen A, B, Q¹, Q², W, X, Y und Z die oben angegebenen Bedeutungen haben, jeweils
mit Chlormonothioameisensäureestern oder Chlordithioameisensäureestern der Formel (VII) 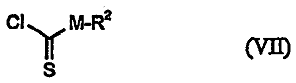 in welcher
M und R² die oben angegebenen Bedeutungen haben,
gegebenenfalls in Gegenwart eines Verdünnungsmittels und gegebenenfalls in gegenwart eines Säurebindemittels umsetzt,
(F) Verbindungen der oben gezeigten Formeln (I-1-d) bis (I-2-d), in welchen R³, A, B, W, Q¹, Q², X, Y und Z die oben angegebenen Bedeutungen haben, Verbindungen der oben gezeigten Formeln (I-1-a) bis (I-2-a), in welchen A, B, Q¹, Q², W, X, Y und Z die oben angegebenen Bedeutungen haben, jeweils mit Sulfonsäurechloriden der Formel (VIII)
R³-SO₂-Cl (VIII)
in welcher
R³ die oben angegebene Bedeutung hat,
gegebenenfalls in Gegenwart eines Verdünnungsmittels und gegebenenfalls in Gegenwart eines Säurebindemittels umsetzt,
(G) Verbindungen der oben gezeigten Formeln (I-1-e) bis (I-2-e), in welchen L, R⁴, R⁵, A, B, Q¹, Q², W, X, Y und Z die oben angegebenen Bedeutungen haben, Verbindungen der oben gezeigten Formeln (I-1-a) bis (I-2-a), in welchen A, B, Q¹, Q², W, X, Y und Z die oben angegebenen Bedeutungen haben, jeweils
mit Phosphorverbindungen der Formel (IX) 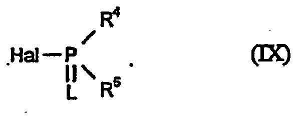 in welcher
L, R⁴ und R⁵ die oben angegebenen Bedeutungen haben und
Hal für Halogen steht,
gegebenenfalls in Gegenwart eines Verdünnungsmittels und gegebenenfalls in Gegenwart eines Säurebindemittels umsetzt,
(H) Verbindungen der oben gezeigten Formeln (I-1-f) bis (I-2-f), in welchen E, A, B, Q¹, Q², W, X, Y und Z die oben angegebenen Bedeutungen haben, Verbindungen der oben gezeigten Formeln (I-1-a) bis (I-2-a), in welchen A, B, Q¹, Q², W, X, Y und Z die oben angegebenen Bedeutungen haben, jeweils mit Metallverbindungen oder Aminen der Formeln (X) oder (XI)
Me(OR¹⁰)ₜ (X) 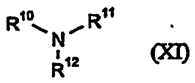
in welchen
Me für ein ein- oder zweiwertiges Metall,
t für die Zahl 1 oder 2 und
R¹⁰, R¹¹, R¹² unabhängig voneinander für Wasserstoff oder Alkyl stehen,
gegebenenfalls in Gegenwart eines Verdünnungsmittels umsetzt,
(I) Verbindungen der oben gezeigten Formeln (I-1-g) bis (I-2-g), in welchen L, R⁶, R⁷, A, B, Q¹, Q², W, X, Y und Z die oben angegebenen Bedeutungen haben, Verbindungen der oben gezeigten Formeln (I-1-a) bis (I-2-a), in welchen A, B, Q¹, Q², W, X, Y und Z die oben angegebenen Bedeutungen haben, jeweils
α) mit Isocyanaten oder Isothiocyanaten der Formel (XII)
R⁶-N=C=L (XII)
in welcher
R⁶ und L die oben angegebenen Bedeutungen haben,
gegebenenfalls in Gegenwart eines Verdünnungsmittels und gegebenenfalls in Gegenwart eines Katalysators umsetzt oder
β) mit Carbamidsäurechloriden oder Thiocarbamidsäurechloriden der Formel (XIII) 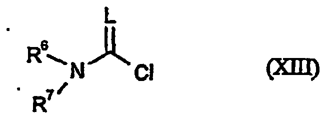 in welcher
L, R⁶ und R⁷ die oben angegebenen Bedeutungen haben,
gegebenenfalls in Gegenwart eines Verdünnungsmittels und gegebenenfalls in Gegenwart eines Säurebindemittels, umsetzt.

7. Mittel enthaltend einen wirksamen Gehalt an einer Wirkstoffkombination umfassend als Komponenten
(a') mindestens eine Verbindung der Formel (I), in welcher A, B, D, G, Q¹, Q², W, X, Y und Z die oben angegebene Bedeutung haben
und
(b') zumindest eine die Kulturpflanzen-Verträglichkeit verbessernde Verbindung aus der folgenden Gruppe von Verbindungen:
4-Dichloracetyl-1-oxa-4-aza-spiro[4.5]-decan (AD-67, MON-4660), 1-Dichloracetyl-hexahydro-3,3,8a-trimethylpyrrolo[1,2-a]-pyrimidin-6(2H)-on (Dicyclonon, BAS-145138), 4-Dichloroacetyl-3,4-dihydro-3-methyl-2H-1,4-benzoxazin (Benoxacor), 5-Chlor-chinolin-8-oxy-essigsäure-(1-methyl-hexylester) (Cloquintocet-mexyl - vgL auch verwandte Verbindungen in EP-A-86750, BP-A-94349, EP-A-191736, EP-A-492366), 3-(2-Chlorbenzyl)-1-(1-methyl-1-phenyl-ethyl)-harnstoff (Cumyluron α-(Cyanomethoximino)-phenylacetonitril (Cyometrinil), 2,4-Dichlor-phenoxyessigsäure (2,4-D), 4-(2,4-Dichlorphenoxy)-buttersäure (2,4-DB), 1-(1-Methyl-1-phenyl-ethyl)-3-(4-methyl-phenyl)-harnstoff (Daimuron, Dymron, 3,6-Dichlor-2-methoxy-benzoesäure (Dicamba), Piperidin-1thiocarbonsäure-S-1-methyl-1-phenyl-ethylester (Dimepiperate), 2,2-Dichlor-N-(2-oxo-2-(2-propenylamino)-ethyl)-N-(2-propenyl)-acetamid (DKA-24), 2,2-Dichlor-N,N-di-2-propenyl-acetamid (Dichlormid), 4,6-Dichlor-2-phenyl-pyrimidin (Fenclorim), 1-(2,4-Dichlorphenyl)-5-trichlormethyl-1H-1,2,4-triazol-3-carbonsäure-ethylester (Fenchlorazole-ethyl - vgl. auch verwandte Verbindungen in EP-A-174562 und EP-A-346620), 2-Chlor-4-trifluormethyl-thiazol-5-carbonsäure-phenylmethylester (Flurazole), 4-Chlor-N-(1,3-dioxolan-2-yl-methoxy)-α-trifluor-acetophenonoxim (Fluxofenim), 3-Dichloracetyl-5-(2-furanyl)-2,2-dimethyl-oxazolidin (Furilazole, MON-13900), Ethyl-4,5-dihydro-5,5-diphenyl-3-isoxazolcarboxylat (Isoxadifen-ethyl - vgL auch verwandte Verbindungen in WO-A-95/07897), 1-(Ethoxycarbonyl)-ethyl-3,6-dichlor-2-methoxybenzoat (Lactidichlor), (4-Chlor-o-tolyloxy)-essigsäure (MCPA), 2-(4-Chlor-o-tolyloxy)-propionsäure (Mecoprop), Diethyl-1-(2,4-dichlor-phenyl)-4,5-dihydro-5-methyl-1H-pyrazol-3,5-dicarboxylat (Mefenpyr-diethyl - vgl. auch verwandte Verbindungen in WO-A-91/07874) 2-Dichlormethyl-2-methyl-1,3-dioxolan (MG-191), 2-Propenyl-1-oxa-4-azaspiro[4.5]decane-4-carbodithioate (MG-838), 1,8-Naphthalsäureanhydrid, α-(1,3-Dioxolan-2-yl-methoximino)-phenylacetonitril (Oxabetrinil), 2,2 Dichlor-N-(1,3-dioxolan-2-yl-methyl)-N-(2-propenyl)-acetamid (PPG-1292), 3-Dichloracetyl-2,2-dimethyl-oxazolidin (R-28725), 3-Dichloracetyl-2,2,5-trimethyl-oxazolidin (R-29148), 4-(4-Chlor-o-tolyl)-buttersäure, 4-(4Chlor-phenoxy)buttersäure, Diphenylmethoxyessigsäure, Diphenylmethoxyessigsäuremethylester, Diphenylmethoxyessigsäure-ethylester, 1-(2-Chlor-phenyl)-5-phenyl-1H-pyrazol-3-carbonsäure-methylester, 1-(2,4-Dichlor-phenyl)-5-methyl-1H-pyrazol-3-carbonsäure-ethylester, 1-(2,4-Dichlor-phenyl)-5-isopropyl-1H-pyrazol-3-carbonsäure-ethylester, 1-(2,4-Dichlor-phenyl)-5-(1,1-dimethyl-ethyl)-1H-pyrazol-3-carbonsäure-ethylester, 1-(2,4-Dichlor-phenyl)-5-phenyl-1H-pyrazol-3-carbonäure-ethylester (vgL auch verwandte Verbindungen in EP-A-269806 und EP-A-333131), 5-(2,4-Dichlor-benzyl)-2-isoxazolin-3-carbonsäure-ethylester, 5-Phenyl-2-isoxazolin-3-carbonsäure-ethylester, 5-(4-Fluorphenyl)-5-phenyl-2-isoxazolin-3-carbonsäure-ethylester (vgl. auch verwandte Verbindungen in WO-A-91/08202), 5-Chlor-chinolin-8-oxy-essigsäure-(1,3-dimethyl-but-1-yl)-ester, 5-Chlor-chinolin-8-oxy-essigsäure-4-allyloxy-butylester, 5-Chlor-chinolin-8-oxy-essigsäure-1-allyloxy-prop-2-yl-ester, 5-Chlor-chinoxalin-8-oxy-essigsäure-methylester, 5-Chlor-chinolin-8-oxy-essigsäure-ethylester, 5-Chlor-chinoxalin-8-oxy-essigsäure-allyl-ester, 5-Chlor-chinolin-8-oxy-essigsäure-2-oxo-prop-1-yl-ester; 5-Chlor-chinolin-8-oxy-malonsäure-diethylester, 5-Chlor-chinoxalin-8-oxy-malonsäure-diallylester, 5-Chlor-chinolin-8-oxy-malonsäure-diethylester (vgl. auch verwandte Verbindungen in EP-A-582198), 4-Carboxy-chroman-4-yl-essigsäure (AC-304415, vgl. EP-A-613618), 4-Chlor-phenoxy-essigsäure, 3,3'-Dimethyl-4-methoxy-benzophenon, 1-Brom-4-chlormethyl-sulfonyl-benzol, 1-[4-(N-2-Methoxybenzoylsulfamoyl)-phenyl]-3-methyl-harnstoff (alias N-(2-Methoxy-benzoyl)-4-[(methylamino-carbonyl)-amino]-benzolsulfonamid), 1-[4-(N-2-Methoxybenzoylsulfamoyl)-phenyl]-3,3-dimethyl-harnstoff, 1-[4-(N-4,5-Dimethylbenzoyl-sulfamoyl)-phenyl]-3-methyl-harnstoff, 1-[4-(N-Naphthylsulfamoyl)-phenyl]-3,3-dimethyl-harnstoff, N-(2-Methoxy-5-methyl-benzoyl)-4-(cyclopropylaminocarbonyl)-benzolsulfonamid,
und/oder eine der folgenden durch allgemeine Formeln definierten Verbindungen
der allgemeinen Formel (IIa) 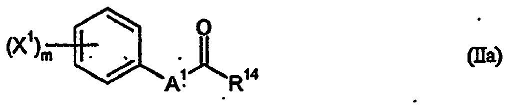 oder der allgemeinen Formel (IIb) 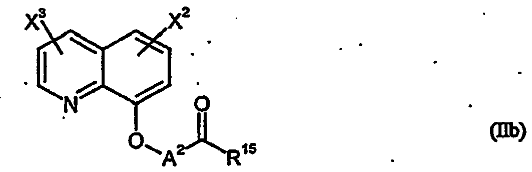 oder der Formel (IIc) 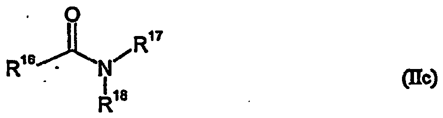 wobei
m für eine Zahl 0, 1, 2, 3, 4 oder 5 steht,
A¹ für eine der nachstehend skizzierten divalenten heterocyclischen. Gruppierungen steht, 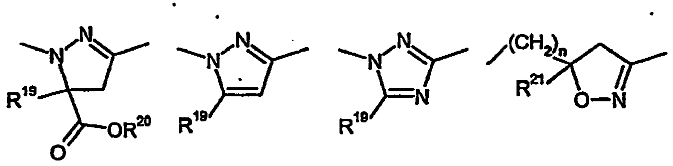
n für eine Zahl 0, 1, 2, 3, 4 oder 5 steht,
A² für gegebenenfalls durch C₁-C₄-Alkyl und/oder C₁-C₄-Alkoxy-carbonyl und/oder C₁C₄-Alkenyloxy-carbonyl substituiertes Alkandiyl mit 1 oder 2 Kohlenstoffatomen steht,
R¹⁴ für Hydroxy, Mercapto, Amino, C₁-C₆-Alkoxy, C₁-C₆-Alkylthio, C₁-C₆-Alkylamino oder Di-(C₁-C₄-alkyl)-amino steht,
R¹⁵ für Hydroxy, Mercapto, Amino, C₁-C₇-Alkoxy, C₁-C₆-Alkenyloxy, C₁-C₆Alkenyloxy-C₁-C₆-alkoxy, C₁-C₆-Alkylthio, C₁-C₆-Alkylamino oder Di-(C₁-C₄-alkyl)amino steht,
R¹⁶ für gegebenenfalls durch Fluor, Chlor und/oder Brom substituiertes C₁-C₄-Alkyl steht,
R¹⁷ für Wasserstoff, jeweils gegebenenfalls durch Fluor, Chlor und/oder Brom substituiertes C₁-C₆-Alkyl, C₂-C₆-Alkenyl oder C₂-C₆-Alkinyl, C₁-C₄-Alkoxy-C₁-C₄-alkyl, Dioxolanyl-C₁-C₄-alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl steht,
R¹⁸ für Wasserstoff, jeweils gegebenenfalls durch Fluor, Chlor und/oder Brom substituiertes C₁-C₆-Alkyl, C₂-C₆-Alkenyl oder C₂-C₆-Alkinyl, C₁-C₄-Alkoxy-C₁-C₄-alkyl, Dioxolanyl-C₁-C₄-alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl steht, R¹⁷ und R¹⁸ auch gemeinsam für jeweils gegebenenfalls durch C₁-C₄Alkyl, Phenyl, Furyl, einen annellierten Benzolring oder durch zwei Substituenten, die gemeinsam mit dem C-Atom, an das sie gebunden sind, einen 5- oder 6-gliedrigen Carboxyclus bilden, substituiertes C₃-C₆-Alkandiyl oder C₂-C₅-Oxaalkandiyl steht,
R¹⁹ für Wasserstoff, Cyano, Halogen, oder für jeweils gegebenenfalls durch Fluor, Chlor und/oder Brom substituiertes C₁-C₄-Alkyl, C₃-C₆-Cycloalkyl oder Phenyl steht,
R²⁰ für Wasserstoff, jeweils gegebenenfalls durch Hydroxy, Cyano, Halogen oder C₁-C₄-Alkoxy substituiertes C₁-C₆-Alkyl, C₃-C₆-Cycloalkyl oder Tri-(C₁-C₄-alkyl)-silyl steht,
R²¹ für Wasserstoff, Cyano, Halogen, oder für jeweils gegebenenfalls durch Fluor, Chlor und/oder Brom substituiertes C₁-C₄-Alkyl, C₃-C₆-Cycloalkyl oder Phenyl steht,
X¹ für Nitro, Cyano, Halogen, C₁-C₄-Alkyl, C₁-C₄-Halogenalkyl, C₁-C₄-Alkoxy oder C₁-C₄-Halogenalkoxy steht,
X² für Wasserstoff, Cyano, Nitro, Halogen, C₁-C₄-Alkyl, C₁-C₄-Halogenalkyl, C₁-C₄-Alkoxy oder C₁-C₄-Halogenalkoxy steht,
X³ für Wasserstoff, Cyano, Nitro, Halogen, C₁-C₄-Alkyl, C₁-C₄-Halogenalkyl, C₁-C₄-Alkoxy oder C₁-C₄-Halogenalkoxy steht,
und/oder die folgenden durch allgemeine Formeln definierten Verbindungen
der allgemeinen Formel (IId) 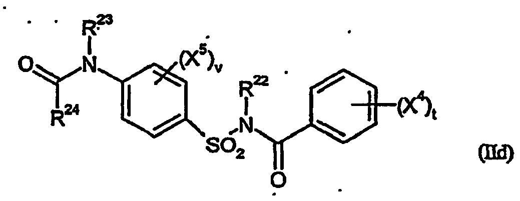 oder der allgemeinen Formel (IIe) 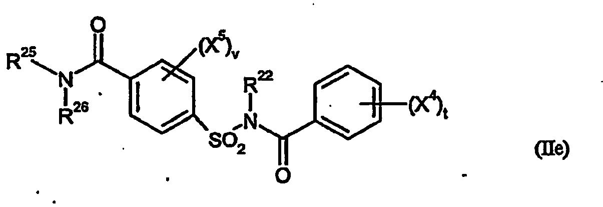 wobei
t für eine Zahl 0, 1, 2, 3, 4 oder 5 steht,
v für eine Zahl 0, 1, 2, 3, 4 oder 5 steht,
R²² für Wasserstoff oder C₁-C₄-Alkyl steht,
R²³ für Wasserstoff oder C₁-C₄₋Alkyl steht,
R²⁴ für Wasserstoff, jeweils gegebenenfalls durch Cyano, Halogen oder C₁-C₄-Alkoxy substituiertes C₁-C₆-Alkyl, C₁-C₆-Alkoxy, C₁-C₆-Alkylthio, C₁-C₆-Alkylamino oder Di-(C₁-C₄-alkyl)-amino, oder jeweils gegebenenfalls durch Cyano, Halogen oder C₁-C₄-Alkyl substituiertes C₃-C₆-Cycloalkyl, C₃-C₆-Cycloalkyloxy, C₃-C₆-Cycloalkylthio oder C₃-C₆-Cycloalkylamino steht,
R²⁵ für Wasserstoff, gegebenenfalls durch Cyano, Hydroxy, Halogen oder C₁-C₄-Alkoxy substituiertes C₁-C₆-Alkyl, jeweils gegebenenfalls durch Cyano oder Halogen substituiertes C₃-C₆-Alkenyl oder C₃-C₆-Alkinyl, oder gegebenenfalls durch Cyano, Halogen oder C₁-C₄-Alkyl substituiertes C₃-C₆-Cycloalkyl steht,
R²⁶ für Wasserstoff, gegebenenfalls durch Cyano, Hydroxy, Halogen oder C₁-C₄-Alkoxy substituiertes C₁-C₆-Alkyl, jeweils gegebenenfalls durch Cyano oder Halogen substituiertes C₃-C₆-Alkenyl oder C₃-C₆-Alkinyl, gegebenenfalls durch Cyano, Halogen oder C₁-C₄-Alkyl substituiertes C₃-C₆-Cycloalkyl, oder gegebenenfalls durch Nitro, Cyano, Halogen, C₁-C₄-Alkyl, C₁-C₄-Halogenalkyl, C₁-C₄-Alkoxy oder C₁-C₄-Halogenalkoxy substituiertes Phenyl steht, oder zusammen mit R²⁵ für jeweils gegebenenfalls durch C₁-C₄-Alkyl substituiertes C₂-C₆-Alkandiyl oder C₂-C₅-Oxaalkandiyl steht,
X⁴ für Nitro, Cyano, Carboxy, Carbamoyl, Formyl, Sulfamoyl, Hydroxy, Amino, Halogen, C₁-C₄-Alkyl, C₁-C₄-Halogenalkyl, C₁-C₄-Alkoxy oder C₁-C₄-Halogenalkoxy steht, und
X⁵ für Nitro, Cyano, Carboxy, Carbamoyl, Formyl, Sulfamoyl, Hydroxy, Amino, Halogen, C₁-C₄-Alkyl, C₁-C₄-Halogenalkyl, C₁-C₄-Alkoxy oder C₁-C₄-Halogenalkoxy steht.

8. Mittel nach Anspruch 7, bei dem die die Kulturpflanzen-Verträglichkeit verbessernde Verbindung aus der folgenden Gruppe von Verbindungen ausgewählt ist:
Cloquintocet-mexyl, Fenchlorazole-ethyl, Isoxadifen-ethyl, Mefenpyr-diethyl, Furilazole, Fenclorim, Cumyluron, Dymron oder die Verbindungen 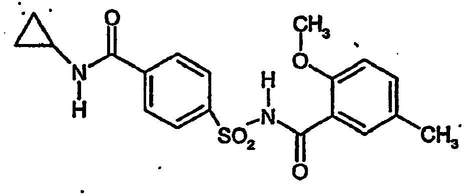 und

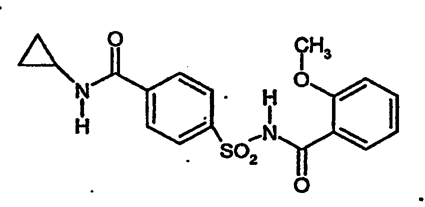

9. Mittel gemäß einem der Ansprüche 7 oder 8, bei denen die Kulturpflanzen-Verträglichkeit verbessernde Verbindung Cloquintocet-mexyl ist.

10. Mittel gemäß einem der Ansprüche 7 oder 8, bei denen die Kulturpflanzen-verträglichkeit verbessernde Verbindung Mefenpyr-diethyl ist.

11. Zusammensetzung umfassend
- mindestens eine Verbindung der Formel (I) gemäß Anspruch 1 oder ein Mittel gemäß Anspruch 7 und
- mindestens ein Salz der Formel (III') 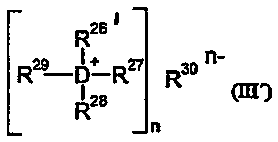 in welcher
D für Stickstoff oder Phosphor steht,
R²⁶', R²⁷, R²⁸ und R²⁹ unabhängig voneinander für Wasserstoff oder jeweils gegebenenfalls substituiertes C₁-C₈-Alkyl oder einfach oder mehrfach, ungesättigtes, gegebenenfalls substituiertes C₁-C₈-Alkylen stehen, wobei die Substituenten aus Halogen, Nitro und Cyano ausgewählt sein können,
n für 1,2,3 oder 4 steht,
R³⁰ für ein anorganisches oder organisches Anion steht.

12. Zusammensetzung gemäß Anspruch 11, **dadurch gekennzeichnet, dass** sie mindestens einen Penetrationsförderer enthält.

13. Verwendung von Verbindungen der Formel (I) gemäß Anspruch 1 zur Herstellung von Schädlingsbekämpfungsmitteln und/oder Herbiziden und/oder Fungiziden.

14. Schädlingsbekämpfungsmittel und/oder Herbizide und/oder Fungizide, **gekennzeichnet durch** einen Gehalt an mindestens einer Verbindung der Formel (I) gemäß Anspruch **I.**

15. Verfahren zur Bekämpfung von tierischen Schädlingen und/oder unerwünschtem Pflanzenbewuchs und/oder Pilzen, **dadurch gekennzeichnet, dass** man Verbindungen der Formel (I) gemäß Anspruch 1 auf Schädlinge und/oder ihren Lebensraum einwirken lässt.

16. Verwendung von Verbindungen der Formel (I) gemäß Anspruch 1 zur Bekämpfung von tierischen Schädlingen und/oder unerwünschtem Pflanzenbewuchs und/oder Pilzen.

17. Verfahren zur Herstellung von Schädlingsbekämpfungsmitteln und/oder Herbiziden und/oder Fungiziden, **dadurch gekennzeichnet, dass** man Verbindungen der Formel (I) gemäß Anspruch 1 mit Streckmitteln und/oder oberflächenaktiven Stoffen vermischt.

18. Verfahren zum Bekämpfen von unerwünschtem Pflanzenwuchs, **dadurch gekennzeichnet, dass** man ein Mittel gemäß Anspruch 7 auf die Pflanzen oder ihre Umgebung einwirken lässt.

19. Verwendung eines Mittels gemäß Anspruch 7 zum Bekämpfen von unerwünschten Pflanzenwuchs.

20. Verfahren zur Bekämpfung von unerwünschtem Pflanzenwuchs, **dadurch gekennzeichnet, dass** man Verbindungen der Formel (I) gemäß Anspruch 1 und die die Kulturpflanzenverträglichkeit verbessernde Verbindung gemäß Anspruch 7 in zeitlich naher Abfolge getrennt auf die Pflanzen oder ihre Umgebung einwirken lässt.

21. Verfahren zur Steigerung der Wirkung von Schädlingsbekämpfungsmitteln und/oder Herbiziden enthaltend einen Wirkstoff der Formel (I) gemäß Anspruch 1 oder ein Mittel gemäß Anspruch 7, **dadurch gekennzeichnet, dass** das anwendungsfertige Mittel (Spritzbrühe) unter Einsatz eines Salzes der Formel (III') gemäß Anspruch 11 zubereitet wird.

22. Verfahren gemäß Anspruch 21, **dadurch gekennzeichnet, dass** die Spritzbrühe unter Einsatz eines Penetrationsförderers zubereitet wird.

23. Verbindungen der Formel (II) 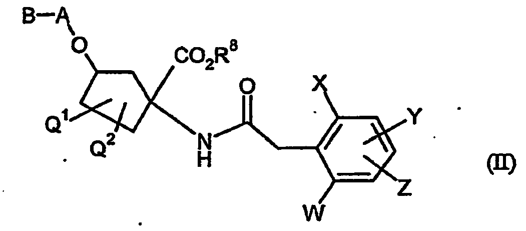 in welcher
A, B, Q¹, Q², W, X, Y, Z und R⁸ die oben angegebenen Bedeutungen haben.

24. Verbindungen der Formel (III) 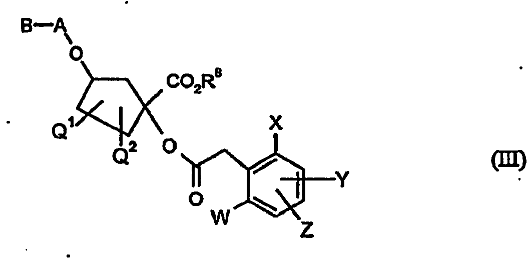 in welcher A, B, Q¹, Q², W, X, Y, Z und R⁸ die oben angegebenen Bedeutungen haben.

25. Verbindungen der Formel (XVI) 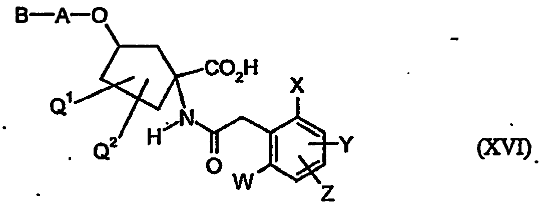 in welcher
A, B, Q¹, Q², W, X, Y und Z die oben angegebenen Bedeutungen haben.

## Claims

1. Compound of the formula (I) 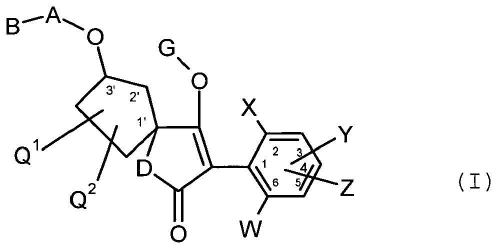 in which
W represents hydrogen, alkyl, alkenyl, alkynyl, halogen, alkoxy, haloalkyl, haloalkoxy or cyano,
X represents halogen, alkyl, alkenyl, alkynyl, alkoxy, alkoxyalkoxy, haloalkyl, haloalkoxy or cyano,
Y represents hydrogen, halogen, alkyl, alkenyl, alkynyl, alkoxy, cyano, haloalkyl, haloalkoxy, represents in each case optionally substituted phenyl or hetaryl,
Z represents hydrogen, halogen, alkyl, haloalkyl, cyano, alkoxy or haloalkoxy,
A represents an optionally substituted alkanediyl group or represents cycloalkyl which is optionally substituted and/or optionally interrupted by a heteroatom,
B represents hydrogen or in each case optionally substituted alkyl, alkenyl, alkoxy, alkoxy-alkoxy, phenyl, hetaryl or represents cycloalkyl which is optionally substituted and/or optionally interrupted by heteroatoms and/or C=O,
or A represents a bond and B represents hydrogen,
D represents NH or oxygen,
Q¹ represents hydrogen, represents in each case optionally substituted alkyl, alkoxy, alkoxyalkyl or alkylthioalkyl, represents in each case optionally substituted cycloalkyl in which optionally one methylene group is replaced by heteroatoms or represents optionally substituted phenyl, hetaryl, phenylalkyl or hetarylalkyl,
Q² represents hydrogen or alkyl,
Q¹ and Q² together with the carbon to which they are attached represent an optionally substituted C₃-C₆-ring which may optionally be interrupted by a heteroatom, or
Q¹ and Q² together with the carbon atoms to which they are attached represent an optionally substituted C₃-C₆-ring which may optionally be interrupted by a heteroatom,
G represents hydrogen (a) or represents one of the groups 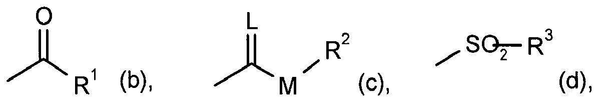 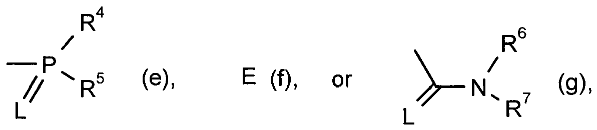 in which
E represents a metal ion or an ammonium ion,
L represents oxygen or sulfur,
M represents oxygen or sulfur,
R¹ represents in each case optionally halogen- or cyano-substituted alkyl, alkenyl, alkoxyalkyl, alkylthioalkyl or polyalkoxyalkyl or represents in each case optionally halogen-, alkyl- or alkoxy-substituted cycloalkyl or heterocyclyl or represents in each case optionally substituted phenyl, phenylalkyl, hetaryl, phenoxyalkyl or hetaryloxyalkyl,
R² represents in each case optionally halogen- or cyano-substituted alkyl, alkenyl, alkoxyalkyl or polyalkoxyalkyl or represents in each case optionally substituted cycloalkyl, phenyl or benzyl,
R³, R⁴ and R⁵ independently of one another represent in each case optionally halogen-substituted alkyl, alkoxy, alkylamino, dialkylamino, alkylthio, alkenylthio or cycloalkylthio or represent in each case optionally substituted phenyl, benzyl, phenoxy or phenylthio,
R⁶ and R⁷ independently of one another represent hydrogen, represent in each case optionally halogen- or cyano-substituted alkyl, cycloalkyl, alkenyl, alkoxy, alkoxyalkyl, represent in each case optionally substituted phenyl or benzyl, or together with the N atom to which they are attached form an optionally substituted cycle which optionally contains oxygen or sulfur.

2. Compound of the formula (I) according to Claim 1 in which
W represents hydrogen, C₁-C₆-alkyl, C₂-C₆-alkenyl, C₂-C₆-alkynyl, halogen, C₁-C₆-alkoxy, C₁-C₄-haloalkyl, C₁-C₄-haloalkoxy or cyano,
X represents hydrogen, C₁-C₆-alkyl, C₂-C₆-alkenyl, C₂-C₆-alkynyl, C₁-C₆-alkoxy, C₁-C₆-alkoxy-C₁-C₄-alkoxy, C₁-C₄-haloalkyl, C₁-C₄-haloalkoxy or cyano,
Y represents hydrogen, halogen, C₁-C₆-alkyl, C₂-C₆-alkenyl, C₂-C₆-alkynyl, C₂-C₆-alkoxy, cyano, C₁-C₄-haloalkyl, haloalkoxy, represents V¹-and V²-substituted phenyl or pyridyl,
V¹ represents halogen, C₁-C₁₂-alkyl, C₁-C₆-alkoxy, C₁-C₄-haloalkyl, C₁-C₄-haloalkoxy, cyano or nitro,
V² represents hydrogen, halogen, C₁-C₆-alkyl, C₁-C₆-alkoxy or C₁-C₄-haloalkyl,
V¹ and V² together represent C₃-C₄-alkanediyl which may optionally be substituted by halogen and/or C₁-C₂-alkyl and which may optionally be interrupted by one or two oxygen atoms,
Z represents hydrogen, halogen, C₁-C₆-alkyl, C₁-C₄-haloalkyl, cyano, C₁-C₆-alkoxy or C₁-C₄-haloalkoxy,
A represents an optionally C₁-C₄-alkyl-substituted C₁-C₄-alkanediyl group or represents optionally C₁-C₄-alkyl-substituted C₅-C₈-cycloalkyl in which optionally one methylene group is replaced by oxygen,
B represents hydrogen or represents in each case optionally halogen-substituted C₁-C₈-alkyl, C₂-C₈-alkenyl, C₁-C₆-alkoxy, C₁-C₆-alkoxy-C₁-C₄-alkoxy, C₁-C₄-alkoxy-bis-C₁-C₄-alkoxy, represents optionally halogen-, C₁-C₆-alkyl-, C₁-C₆-alkoxy-, C₁-C₄-haloalkyl-, C₁-C₄-haloalkoxy-, cyano- or nitro-substituted phenyl, represents optionally halogen-, C₁-C₄-alkyl- or C₁-C₂-haloalkyl-substituted pyridyl, pyrimidyl, thiazolyl or thienyl or represents optionally halogen-, C₁-C₄-alkyl-, C₁-C₄-alkoxy- or C₁-C₂-haloalkyl-substituted C₃-C₈-cycloalkyl in which optionally one or two not directly adjacent methylene groups are replaced by oxygen, two methylene groups are replaced by the radical -O-CO- or three methylene groups are replaced by the radical -O-CO-O-,
or A represents a bond and B represents hydrogen,
D represents NH or oxygen,
Q¹ represents hydrogen or represents in each case optionally halogen-substituted C₁-C₆-alkyl, C₁-C₆-alkoxy, C₁-C₄-alkoxy-C₁-C₄-alkyl, C₁-C₄-alkylthio-C₁-C₄-alkyl or represents optionally halogen-, C₁-C₄-alkyl- or C₁-C₄-alkoxy-substituted C₃-C₆-cycloalkyl in which optionally one methylene group is replaced by oxygen or represents phenyl, phenyl-C₁-C₂-alkyl or hetaryl, each of which is optionally mono- or disubstituted by halogen, C₁-C₄-alkyl, C₁-C₄-alkoxy, C₁-C₄-haloalkyl or C₁-C₄-haloalkoxy,
Q² represents hydrogen or C₁-C₆-alkyl, or
Q¹ and Q² together with the carbon to which they are attached represent a C₃-C₆-ring which is optionally mono- or disubstituted by fluorine, chlorine, C₁-C₄-alkyl, C₁-C₄-alkoxy or trifluoromethyl and in which optionally one methylene group may be replaced by oxygen, or
Q¹ and Q² together with the carbon atoms to which they are attached represent a C₃-C₆-ring which is optionally mono- or disubstituted by fluorine, chlorine, C₁-C₄-alkyl, C₁-C₄-alkoxy or trifluoromethyl and in which optionally one methylene group may be replaced by oxygen.
G represents hydrogen (a) or represents one of the groups 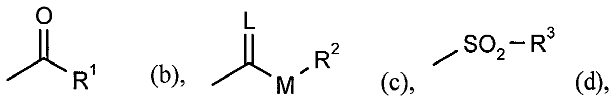 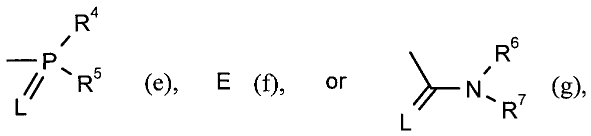 in which
E represents a metal ion or an ammonium ion,
L represents oxygen or sulfur and
M represents oxygen or sulfur,
R¹ represents in each case optionally halogen- or cyano-substituted C₁-C₂₀-alkyl, C₂-C₂₀-alkenyl, C₁-C₈-alkoxy-C₁-C₈-alkyl, C₁-C₈-alkylthio-C₁-C₈-alkyl or poly-C₁-C₈-alkoxy-C₁-C₈-alkyl or represents optionally halogen-, C₁-C₆-alkyl- or C₁-C₆-alkoxy-substituted C₃-C₈-cycloalkyl in which optionally one or two not directly adjacent methylene groups are replaced by oxygen and/or sulfur,
represents optionally halogen-, cyano-, nitro-, C₁-C₆-alkyl-, C₁-C₆-alkoxy-, C₁-C₆-haloalkyl-, C₁-C₆-haloalkoxy-, C₁-C₆-alkylthio- or C₁-C₆-alkylsulfonyl-substituted phenyl,
represents optionally halogen-, nitro-, cyano-, C₁-C₆-alkyl-, C₁-C₆-alkoxy-, C₁-C₆-haloalkyl- or C₁-C₆-haloalkoxy-substituted phenyl-C₁-C₆-alkyl,
represents optionally halogen- or C₁-C₆-alkyl-substituted 5- or 6-membered hetaryl having one or two heteroatoms from the group consisting of oxygen, sulfur and nitrogen,
represents optionally halogen- or C₁-C₆-alkylsubstituted phenoxy-C₁-C₆-alkyl or
represents optionally halogen-, amino- or C₁-C₆-alkyl-substituted 5- or 6-membered hetaryloxy-C₁-C₆-alkyl having one or two heteroatoms from the group consisting of oxygen, sulfur and nitrogen,
R² represents in each case optionally halogen-or cyano-substituted C₁-C₂₀-alkyl, C₂-C₂₀-alkenyl, C₁-C₈-alkoxy-C₂-C₈-alkyl or poly-C₁-C₈-alkoxy-C₂-C₈-alkyl,
represents optionally halogen-, C₁-C₆-alkyl-or C₁-C₆-alkoxy-substituted C₃-C₈-cycloalkyl or
represents in each case optionally halogen-, cyano-, nitro-, C₁-C₆-alkyl-, C₁-C₆-alkoxy-, C₁-C₆-haloalkyl- or C₁-C₆-haloalkoxy-substituted phenyl or benzyl,
R³ represents optionally halogen-substituted C₁-C₈-alkyl or in each case optionally halogen-, C₁-C₆-alkyl-, C₁-C₆-alkoxy-, C₁-C₄-haloalkyl-, C₁-C₄-haloalkoxy-, cyano- or nitro-substituted phenyl or benzyl,
R⁴ and R⁵ independently of one another represent in each case optionally halogen-substituted C₁-C₈-alkyl, C₁-C₈-alkoxy, C₁-C₈-alkylamino, di-(C₁-C₈-alkyl) amino, C₁-C₈-alkylthio or C₃-C₈-alkenylthio or represent in each case optionally halogen-, nitro-, cyano-, C₁-C₄-alkoxy-, C₁-C₄-haloalkoxy-, C₁-C₄-alkylthio-, C₁-C₄-haloalkylthio-, C₁-C₄-alkyl- or C₁-C₄-haloalkyl-substituted phenyl, phenoxy or phenylthio,
R⁶ and R⁷ independently of one another represent hydrogen, represent in each case optionally halogen- or cyano-substituted C₁-C₈-alkyl, C₃-C₈-cycloalkyl, C₁-C₈-alkoxy, C₃-C₈-alkenyl or C₁-C₈-alkoxy-C₂-C₈-alkyl, represent in each case optionally halogen-, C₁-C₈-alkyl-, C₁-C₈-haloalkyl- or C₁-C₈-alkoxy-substituted phenyl or benzyl or together represent an optionally C₁-C₆-alkyl-substituted C₃-C₆-alkylene radical in which optionally one methylene group is replaced by oxygen or sulfur.

3. Compound of the formula (I) according to Claim 1 in which
W represents hydrogen, chlorine, bromine, iodine, C₁-C₄-alkyl, C₂-C₄-alkenyl, C₂-C₄-alkynyl, C₁-C₄-alkoxy, C₁-C₂-haloalkyl or C₁-C₂-haloalkoxy,
X represents chlorine, bromine, iodine, C₁-C₄-alkyl, C₂-C₄-alkenyl, C₂-C₄-alkynyl, C₁-C₄-alkoxy, C₁-C₄-alkoxy-C₁-C₃-alkoxy, C₁-C₂-haloalkyl, C₁-C₂-haloalkoxy or cyano,
Y in the 4-position represents hydrogen, fluorine, chlorine, bromine, iodine, methoxy, ethoxy, cyano, trifluoromethyl, difluoromethoxy or trifluoromethoxy,
Z represents hydrogen.
W represents hydrogen, chlorine, bromine or C₁-C₄-alkyl,
X represents chlorine, bromine, C₁-C₄-alkyl, C₁-C₄-alkoxy, C₁-C₂-haloalkyl, C₁-C₂-haloalkoxy or cyano,
Y in the 4-position represents C₂-C₄-alkenyl, C₂-C₄-alkynyl or represents the radical 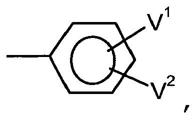
Z represents hydrogen,
V¹ represents fluorine, chlorine, C₁-C₄-alkyl, C₁-C₄-alkoxy, C₁-C₂-haloalkyl or C₁-C₂-haloalkoxy,
V² represents hydrogen, fluorine, chlorine, C₁-C₄-alkyl, C₁-C₄-alkoxy or C₁-C₂-haloalkyl,
V¹ and V² together represent -O-CH₂-O- and -O-CF₂-O-.
W represents hydrogen, chlorine, bromine or C₁-C₄-alkyl,
X represents chlorine, bromine, C₁-C₄-alkyl or C₁-C₂-haloalkyl,
Y in the 5-position represents C₂-C₄-alkenyl, C₂-C₄-alkynyl, represents the radical 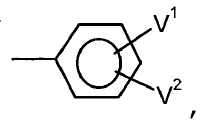
Z in the 4-position represents hydrogen, C₁-C₄-alkyl or chlorine,
V¹ represents fluorine, chlorine, C₁-C₄-alkyl, C₁-C₄-alkoxy, C₁-C₂-haloalkyl or C₁-C₂-haloalkoxy,
V² represents hydrogen, fluorine, chlorine, C₁-C₄-alkyl, C₁-C₄-alkoxy or C₁-C₂-haloalkyl,
V¹ and V² together represent -O-CH₂-O- or -O-CF₂-O-.
W represents hydrogen, C₁-C₄-alkyl, C₂-C₄-alkenyl, C₂-C₄-alkynyl, C₁-C₄-alkoxy, chlorine, bromine, iodine or trifluoromethyl,
X represents chlorine, bromine, iodine, C₁-C₄-alkyl, C₂-C₄-alkenyl, C₂-C₄-alkynyl, C₁-C₄-alkoxy, C₁-C₄-alkoxy-C₁-C₃-alkoxy, C₁-C₂-haloalkyl, C₁-C₂-haloalkoxy or cyano,
Y in the 4-position represents C₁-C₄-alkyl,
Z represents hydrogen.
W represents hydrogen, chlorine, bromine, iodine, C₁-C₄-alkyl or C₁-C₄-alkoxy,
X represents chlorine, bromine, iodine, C₁-C₄-alkyl, C₁-C₄-alkoxy, C₁-C₂-haloalkyl, C₁-C₂-haloalkoxy or cyano,
Y in the 4-position represents hydrogen, chlorine, bromine, C₁-C₄-alkyl, C₁-C₂-haloalkyl or C₁-C₂-haloalkoxy,
Z in the 3- or 5-position represents fluorine, chlorine, bromine, iodine, C₁-C₄-alkyl, C₁-C₂-haloalkyl, C₁-C₄-alkoxy or C₁-C₂-haloalkoxy.
A represents an optionally C₁-C₂-alkyl-substituted C₁-C₃-alkanediyl group or represents C₅-C₆-cycloalkyl in which optionally one methylene group is replaced by oxygen.
B represents hydrogen or C₁-C₆-alkyl, C₂-C₆-alkenyl, C₁-C₄-alkoxy, C₁-C₄-alkoxy-C₁-C₃-alkoxy, C₁-C₄-alkoxy-bis-C₁-C₃-alkoxy, each of which is optionally mono- to trisubstituted by fluorine or chlorine, represents phenyl which is optionally mono- to trisubstituted by fluorine, chlorine, bromine, C₁-C₄-alkyl, C₁-C₄-alkoxy, C₁-C₂-haloalkyl, C₁-C₂-haloalkoxy, cyano or nitro, represents pyridyl, pyrimidyl, thiazolyl or thienyl, each of which is optionally mono- or disubstituted by fluorine, chlorine, bromine, methyl, ethyl or trifluoromethyl or represents C₃-C₆-cycloalkyl which is optionally mono- or disubstituted by fluorine, chlorine, methyl, methoxy or trifluoromethyl, in which optionally one or two not directly adjacent methylene groups are replaced by oxygen,
or A represents a bond and B represents hydrogen.
D represents NH or oxygen.
Q¹ represents hydrogen, represents C₁-C₄-alkyl which is optionally mono- to trisubstituted by fluorine,
Q² represents hydrogen or C₁-C₄-alkyl.
Q¹ and Q² together with the carbon atom to which they are attached represent a C₃-C₆-ring which is optionally monosubstituted by fluorine, methyl, methoxy or trifluoromethyl and in which one methylene group may be replaced by oxygen, or
Q¹ and Q² together with the carbon atoms to which they are attached represent a C₃-C₆-ring which is optionally monosubstituted by fluorine, methyl, methoxy or trifluoromethyl and in which one methylene group may be replaced by oxygen.
G represents hydrogen (a) or represents one of the groups 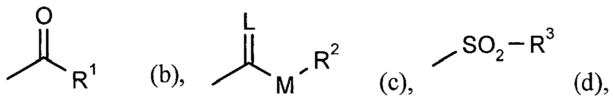 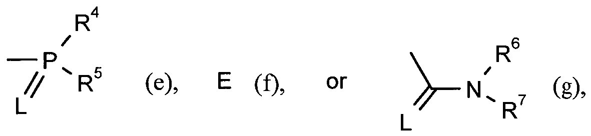 in which
E represents a metal ion or an ammonium ion,
L represents oxygen or sulfur and
M represents oxygen or sulfur.
R¹ represents C₁-C₁₆-alkyl, C₂-C₁₆-alkenyl, C₁-C₆-alkoxy-C₁-C₄-alkyl, C₁-C₆-alkylthio-C₁-C₄-alkyl or poly-C₁-C₆-alkoxy-C₁-C₄-alkyl, each of which is optionally mono- to trisubstituted by fluorine or chlorine, or represents C₃-C₇-cycloalkyl which is optionally mono- to disubstituted by fluorine, chlorine, C₁-C₅-alkyl or C₁-C₅-alkoxy and in which optionally one or two not directly adjacent methylene groups are replaced by oxygen and/or sulfur,
represents phenyl which is optionally mono- to trisubstituted by fluorine, chlorine, bromine, cyano, nitro, C₁-C₄-alkyl, C₁-C₄-alkoxy, C₁-C₃-haloalkyl, C₁-C₃-haloalkoxy, C₁-C₄-alkylthio or C₁-C₄-alkylsulfonyl,
represents phenyl-C₁-C₄-alkyl which is optionally mono- to disubstituted by fluorine, chlorine, bromine, C₁-C₄-alkyl, C₁-C₄-alkoxy, C₁-C₃-haloalkyl or C₁-C₃-haloalkoxy,
represents pyrazolyl, thiazolyl, pyridyl, pyrimidyl, furanyl or thienyl, each of which is optionally mono- to disubstituted by fluorine, chlorine, bromine or C₁-C₄-alkyl,
represents phenoxy-C₁-C₅-alkyl which is optionally mono- to disubstituted by fluorine, chlorine, bromine or C₁-C₄-alkyl or
represents pyridyloxy-C₁-C₅-alkyl, pyrimidyloxy-C₁-C₅-alkyl or thiazolyloxy-C₁-C₅-alkyl, each of which is optionally mono- to disubstituted by fluorine, chlorine, bromine, amino or C₁-C₄-alkyl,
R² represents C₁-C₁₆-alkyl, C₂-C₁₆-alkenyl, C₁-C₆-alkoxy-C₂-C₆-alkyl or poly-C₁-C₆-alkoxy-C₂-C₆-alkyl, each of which is optionally mono- to trisubstituted by fluorine or chlorine,
represents C₃-C₇-cycloalkyl which is optionally mono- to disubstituted by fluorine, chlorine, C₁-C₄-alkyl or C₁-C₄-alkoxy or
represents phenyl or benzyl, each of which is optionally mono- to trisubstituted by fluorine, chlorine, bromine, cyano, nitro, C₁-C₄-alkyl, C₁-C₃-alkoxy, C₁-C₃-haloalkyl or C₁-C₃-haloalkoxy,
R³ represents C₁-C₆-alkyl which is optionally mono- to trisubstituted by fluorine or chlorine or represents phenyl or benzyl, each of which is optionally mono- to disubstituted by fluorine, chlorine, bromine, C₁-C₄-alkyl, C₁-C₄-alkoxy, C₁-C₂-haloalkoxy, C₁-C₂-haloalkyl, cyano or nitro,
R⁴ and R⁵ independently of one another represent C₁-C₆-alkyl, C₁-C₆-alkoxy, C₁-C₆-alkylamino, di-(C₁-C₆-alkyl)amino, C₁-C₆-alkylthio or C₃-C₄-alkenylthio, each of which is optionally mono- to trisubstituted by fluorine or chlorine, or represent phenyl, phenoxy or phenylthio, each of which is optionally mono- to disubstituted by fluorine, chlorine, bromine, nitro, cyano, C₁-C₃-alkoxy, C₁-C₃-haloalkoxy, C₁-C₃-alkylthio, C₁-C₃-haloalkylthio, C₁-C₃-alkyl or C₁-C₃-haloalkyl,
R⁶ and R⁷ independently of one another represent hydrogen, represent C₁-C₆-alkyl, C₃-C₆-cycloalkyl, C₁-C₆-alkoxy, C₃-C₆-alkenyl or C₁-C₆-alkoxy-C₂-C₆-alkyl, each of which is optionally mono- to trisubstituted by fluorine or chlorine, represent phenyl or benzyl, each of which is optionally mono- to trisubstituted by fluorine, chlorine, bromine, C₁-C₅-haloalkyl, C₁-C₅-alkyl or C₁-C₅-alkoxy, or together represent an optionally C₁-C₄-alkyl-substituted C₃-C₆-alkylene radical in which optionally one methylene group is replaced by oxygen or sulfur.

4. Compound of the formula (I) according to Claim 1 in which
W represents hydrogen, chlorine, bromine, iodine methyl, ethyl, methoxy, ethoxy or trifluoromethyl,
X represents chlorine, bromine, iodine, methyl, ethyl, propyl, methoxy, ethoxy, propoxy, methoxyethoxy, ethoxyethoxy, trifluoromethyl, difluoromethoxy, trifluoromethoxy or cyano,
Y in the 4-position represents hydrogen, chlorine, bromine, iodine, trifluoromethyl or trifluoromethoxy,
Z represents hydrogen.
W also represents hydrogen, chlorine, bromine, methyl or ethyl,
X also represents chlorine, bromine, methyl, ethyl, propyl, methoxy, trifluoromethyl, difluoromethoxy or cyano,
Y also in the 4-position represents vinyl, ethynyl, propynyl or represents the radical 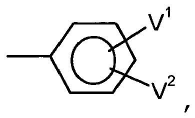
Z also represents hydrogen,
V¹ also represents fluorine, chlorine, methyl, methoxy, trifluoromethyl or trifluoromethoxy,
V² also represents hydrogen, fluorine, chlorine, methyl, methoxy or trifluoromethyl.
W likewise represents hydrogen, chlorine or methyl,
X likewise represents chlorine, methyl or trifluoromethyl,
Y likewise in the 5-position represents vinyl, ethynyl, propynyl or represents the radical 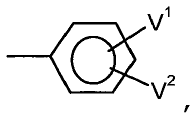
Z likewise in the 4-position represents hydrogen or methyl,
V¹ likewise represents fluorine, chlorine, methyl, methoxy, trifluoromethyl or trifluoromethoxy,
V² likewise represents hydrogen, fluorine, chlorine, methyl, methoxy or trifluoromethyl.
W moreover represents hydrogen, methyl, ethyl, methoxy, ethoxy, chlorine, bromine or iodine,
X moreover represents chlorine, bromine, iodine, methyl, ethyl, propyl, methoxy, ethoxy, propoxy, methoxy-ethoxy, ethoxy-ethoxy, trifluoromethyl, difluoromethoxy, trifluoromethoxy or cyano,
Y moreover in the 4-position represents methyl or ethyl,
Z moreover represents hydrogen.
W furthermore represents hydrogen, chlorine, bromine, iodine, methyl or ethyl,
X furthermore represents chlorine, bromine, iodine, methyl, ethyl, methoxy, trifluoromethyl, difluoromethoxy or trifluoromethoxy,
Y furthermore in the 4-position represents hydrogen, chlorine, bromine, iodine, methyl or ethyl,
Z furthermore in the 3- or 5-position represents fluorine, chlorine, bromine, iodine, methyl, ethyl, trifluoromethyl or trifluoromethoxy.
A represents -CH₂-, -CHCH₃-, -CH₂-CH₂-, -CH₂-CHCH₃-, -CH₂-CH₂-CH₂-.
B represents hydrogen, methyl, ethyl, propyl, isopropyl, butyl, isobutyl, C₂-C₄-alkenyl, methoxy, ethoxy, propoxy, isopropoxy, butoxy, isobutoxy, methoxy-ethoxy, ethoxy-ethoxy, represents phenyl which is optionally mono- or disubstituted by fluorine, chlorine, bromine, methyl, methoxy, trifluoromethyl, trifluoromethoxy, cyano or nitro, represents cyclopropyl, represents cyclopentyl or cyclohexyl in which optionally one methylene group is replaced by oxygen,
or A represents a bond and B represents hydrogen.
D represents NH or oxygen.
Q¹ represents hydrogen, methyl or ethyl.
Q² represents hydrogen, methyl or ethyl.
Q¹ and Q² together with the carbon atom to which they are attached represent cyclopropyl, cyclopentyl or cyclohexyl, or
Q¹ and Q² together with the carbon atoms to which they are attached represent a C₅-C₆-ring which is optionally interrupted by oxygen.
G represents hydrogen (a) or represents one of the groups 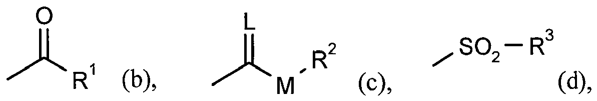 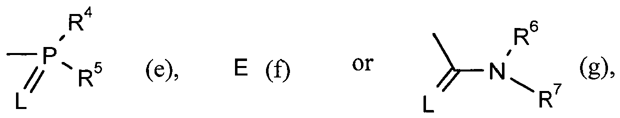 in which
E represents a metal ion or an ammonium ion,
L represents oxygen or sulfur and
M represents oxygen or sulfur.
R¹ represents C₁-C₁₀-alkyl, C₂-C₁₀-alkenyl, C₁-C₄-alkoxy-C₁-C₂-alkyl, C₁-C₄-alkylthio-C₁-C₂-alkyl, each of which is optionally mono- to trisubstituted by fluorine or chlorine, or represents C₃-C₆-cycloalkyl which is optionally monosubstituted by fluorine, chlorine, methyl, ethyl or methoxy,
represents phenyl which is optionally mono- to disubstituted by fluorine, chlorine, bromine, cyano, nitro, methyl, ethyl, n-propyl, isopropyl, methoxy, ethoxy, trifluoromethyl or trifluoromethoxy,
represents furanyl, thienyl or pyridyl, each of which is optionally monosubstituted by chlorine, bromine or methyl,
R² represents C₁-C₁₀-alkyl, C₂-C₁₀-alkenyl or C₁-C₄-alkoxy-C₂-C₄-alkyl, each of which is optionally mono- to trisubstituted by fluorine or chlorine,
represents cyclopentyl or cyclohexyl
or represents phenyl or benzyl, each of which is optionally mono- to disubstituted by fluorine, chlorine, cyano, nitro, methyl, ethyl, methoxy, trifluoromethyl or trifluoromethoxy,
R³ represents methyl, ethyl, propyl or isopropyl, each of which is optionally mono- to trisubstituted by fluorine or chlorine, or represents phenyl which is optionally monosubstituted by fluorine, chlorine, bromine, methyl, ethyl, isopropyl, tert-butyl, methoxy, ethoxy, isopropoxy, trifluoromethyl, trifluoromethoxy, cyano or nitro,
R⁴ and R⁵ independently of one another represent C₁-C₄-alkoxy or C₁-C₄-alkylthio or represent phenyl, phenoxy or phenylthio, each of which is optionally monosubstituted by fluorine, chlorine, bromine, nitro, cyano, methyl, methoxy, trifluoromethyl or trifluoromethoxy,
R⁶ and R⁷ independently of one another represent hydrogen, represent C₁-C₄-alkyl, C₃-C₆-cycloalkyl, C₁-C₄-alkoxy, C₃-C₄-alkenyl or C₁-C₄-alkoxy-C₂-C₄-alkyl, represent phenyl which is optionally mono- to disubstituted by fluorine, chlorine, bromine, methyl, methoxy or trifluoromethyl, or together represent a C₅-C₆-alkylene radical in which optionally one methylene group is replaced by oxygen or sulfur.

5. Compound of the formula (I) according to Claim 1 in which
W represents methyl, ethyl or methoxy,
X represents chlorine, methyl, ethyl or methoxy,
Y in the 4-position represents chlorine or bromine,
Z represents hydrogen.
W likewise represents hydrogen,
X likewise represents methyl,
Y likewise in the 5-position represents the radical 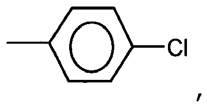
Z in the 4-position represents hydrogen.
W moreover represents methyl or ethyl,
X moreover represents chlorine, bromine or methyl,
Y moreover in the 4-position represents methyl,
Z moreover represents hydrogen.
A represents -CH₂- or -CH₂-CH₂-,
B represents hydrogen, methyl, ethyl, propyl, methoxy or cyclopropyl,
or A represents a bond and B represents hydrogen.
D represents NH.
Q¹ represents hydrogen.
Q² represents hydrogen.
G represents hydrogen (a) or represents one of the groups 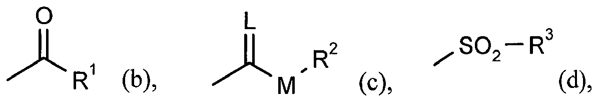 E (f), or 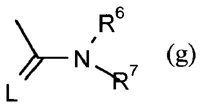 in which
E represents a metal ion,
L represents oxygen and
M represents oxygen.
R¹ represents C₁-C₁₀-alkyl,
represents phenyl which is optionally monosubstituted by chlorine,
R² represents C₁-C₁₀-alkyl or C₂-C₁₀-alkenyl,
R³ represents methyl,
R⁶ and R⁷ together represent a C₅-C₆-alkylene - radical in which optionally one methylene group is replaced by oxygen.

6. Process for preparing compounds of the formula (I) according to Claim 1, **characterized in that** to obtain
(A) compounds of the formula (I-1-a) 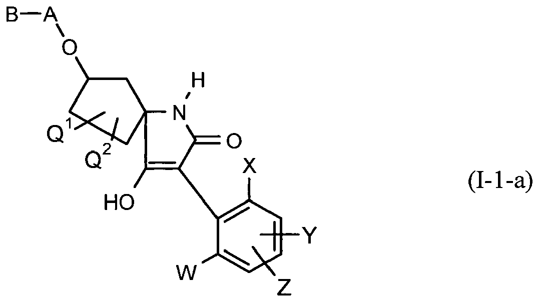 in which
A, B, Q¹, Q², W, X, Y and Z are as defined above,
compounds of the formula (II) 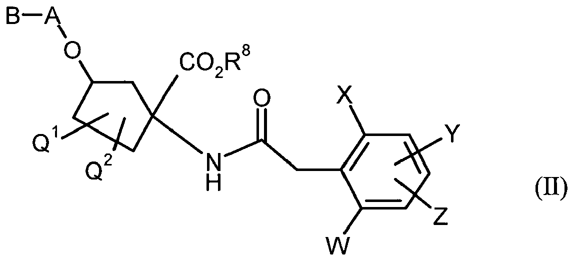 in which
A, B, Q¹, Q², W, X, Y and Z are as defined above,
and
R⁸ represents alkyl,
are condensed intramolecularly in the presence of a diluent and in the presence of a base,
(B) compounds of the formula (I-2-a) 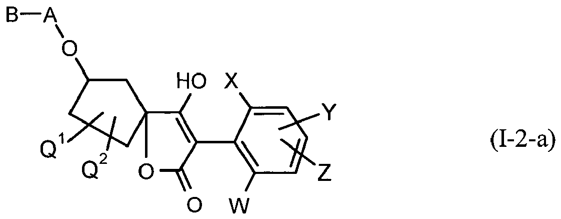 in which
A, B, Q¹, Q², W, X, Y and Z are as defined above,
compounds of the formula (III) 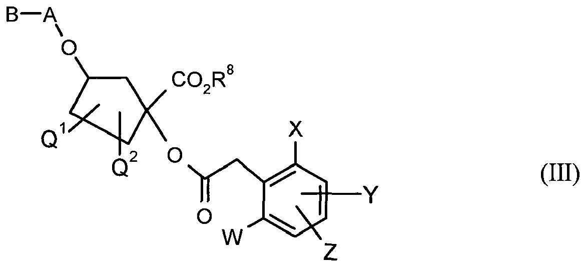 in which
A, B, Q¹, Q², W, X, Y, Z and R⁸ are as defined above,
are condensed intramolecularly in the presence of a diluent and in the presence of a base,
(C) compounds of the formula (I-1-b) to (I-2-b) shown above in which R¹, A, B, Q¹, Q², W, X, Y and Z are as defined above, compounds of the formula (I-1-a) to (I-2-a) shown above in which A, B, Q¹, Q², W, X, Y and Z are as defined above are in each case reacted
α) with compounds of the formula (IV) 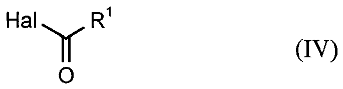 in which
R¹ is as defined above and
Hal represents halogen
or
β) with carboxylic anhydrides of the formula (V)
R¹-CO-O-CO-R¹ (V)
in which
R¹ is as defined above,
if appropriate in the presence of a diluent and if appropriate in the presence of an acid binder,
(D) compounds of the formula (I-1-c) to (I-2-c) shown above in which R², A, B, Q¹, Q², W, M, X, Y and Z are as defined above and L represents oxygen, compounds of the formula (I-1-a) to (I-2-a) shown above in which A, B, Q¹, Q², W, X, Y and Z are as defined above are in each case
reacted with chloroformic esters or chloroformic thioesters of the formula (VI)
R²-M-CO-Cl (VI)
in which
R² and M are as defined above,
if appropriate in the presence of a diluent and if appropriate in the presence of an acid binder,
(E) compounds of the formulae (I-1-c) to (I-2-c) shown above in which R², A, B, Q¹, Q², W, M, X, Y and Z are as defined above and L represents sulfur, compounds of the formulae (I-1-a) to (I-2-a) shown above in which A, B, Q¹, Q², W, X, Y and Z are as defined above are in each case
reacted with chloromonothioformic esters or chlorodithioformic esters of the formula (VII) 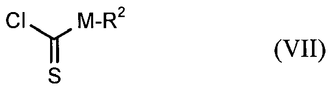 in which
M and R² are as defined above,
if appropriate in the presence of a diluent and if appropriate in the presence of an acid binder,
(F) compounds of the formulae (I-1-d) to (I-2-d) shown above in which R³, A, B, W, Q¹, Q², X, Y and Z are as defined above, compounds of the formulae (I-1-a) to (I-2-a) shown above in which A, B, Q¹, Q², W, X, Y and Z are as defined above are in each case
reacted with sulfonyl chlorides of the formula (VIII)
R³-SO₂-Cl (VIII)
in which
R³ is as defined above,
if appropriate in the presence of a diluent and if appropriate in the presence of an acid binder,
(G) compounds of the formulae (I-1-e) to (I-2-e) shown above in which L, R⁴, R⁵, A, B, Q¹, Q², W, X, Y and Z are as defined above, compounds of the formulae (I-1-a) to (I-2-a) shown above in which A, B, Q¹, Q², W, X, Y and Z are as defined above are in each case
reacted with phosphorus compounds of the formula (IX) 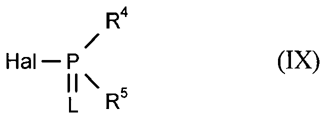 in which
L, R⁴ and R⁵ are as defined above and
Hal represents halogen,
if appropriate in the presence of a diluent and if appropriate in the presence of an acid binder,
(H) compounds of the formulae (I-1-f) to (I-2-f) shown above in which E, A, B, Q¹, Q², W, X, Y and Z are as defined above, compounds of the formulae (I-1-a) to (I-2-a) shown above in which A, B, Q¹, Q², W, X, Y and Z are as defined above are in each case
reacted with metal compounds or amines of the formulae (X) and (XI), respectively
Me(OR¹⁰)ₜ (X) 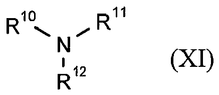
in which
Me represents a mono- or divalent metal,
t represents the number 1 or 2 and
R¹⁰, R¹¹, R¹² independently of one another represent hydrogen or alkyl,
if appropriate in the presence of a diluent,
(I) compounds of the formulae (I-1-g) to (I-2-g) shown above in which L, R⁶, R⁷, A, B, Q¹, Q², W, X, Y and Z are as defined above, compounds of the formulae (I-1-a) to (I-2-a) shown above in which A, B, Q¹, Q², W, X, Y and Z are as defined above are in each case
α) reacted with isocyanates or isothiocyanates of the formula (XII)
R⁶-N=C=L (XII)
in which
R⁶ and L are as defined above,
if appropriate in the presence of a diluent and if appropriate in the presence of a catalyst, or
β) reacted with carbamoyl chlorides or thiocarbamoyl chlorides of the formula (XIII) 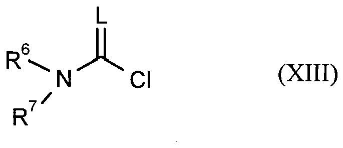 in which
L, R⁶ and R⁷ are as defined above,
if appropriate in the presence of a diluent and if appropriate in the presence of an acid binder.

7. Composition comprising an effective amount of an active compound combination comprising, as components,
(a') at least one compound of the formula (I) in which A, B, D, G, Q¹, Q², W, X, Y and Z are as defined above
and
(b') at least one crop plant compatibility-improving compound from the following group of compounds:
4-dichloroacetyl-1-oxa-4-azaspiro[4.5]decane (AD-67, MON-4660), 1-dichloroacetylhexahydro-3,3,8a-trimethylpyrrolo[1,2-a]pyrimidin-6(2H)-one (dicyclonon, BAS-145138), 4-dichloroacetyl-3,4-dihydro-3-methyl-2H-1,4-benzoxazine (benoxacor), 1-methylhexyl 5-chloroquinoline-8-oxyacetate (cloquintocet-mexyl - cf. also related compounds in EP-A-86750, EP-A-94349, EP-A-191736, EP-A-492366), 3-(2-chlorobenzyl)-1-(1-methyl-1-phenylethyl)urea (cumyluron), α-(cyanomethox-imino)phenylacetonitrile (cyometrinil), 2,4-dichlorophenoxyacetic acid (2,4-D), 4-(2,4-dichlorophenoxy)butyric acid (2,4-DB), 1-(1-methyl-1-phenylethyl)-3-(4-methylphenyl)urea (daimuron, dymron), 3,6-dichloro-2-methoxybenzoic acid (dicamba), S-1-methyl-1-phenylethyl piperidine-1-thiocarboxylate (dimepiperate), 2,2-dichloro-N-(2-oxo-2-(2-propenylamino)ethyl)-N-(2-propenyl)acetamide (DKA-24), 2,2-dichloro-N,N-di-2-propenylacetamide (dichlormid), 4,6-dichloro-2-phenylpyrimidine (fenclorim), ethyl 1-(2,4-dichlorophenyl)-5-trichloromethyl-1H-1,2,4-triazole-3-carboxylate (fenchlorazole-ethyl - cf. also related compounds in EP-A-174562 and EP-A-346620), phenylmethyl 2-chloro-4-trifluoromethylthiazole-5-carboxylate (flurazole), 4-chloro-N-(1,3-dioxolan-2-ylmethoxy)-α-trifluoroacetophenone oxime (fluxofenim), 3-dichloroacetyl-5-(2-furanyl)-2,2-dimethyloxazolidine (furilazole, MON-13900), ethyl 4,5-dihydro-5,5-diphenyl-3-isoxazolecarboxylate (isoxadifen-ethyl - cf. also related compounds in WO-A-95/07897), 1-(ethoxycarbonyl)ethyl 3,6-dichloro-2-methoxybenzoate (lactidichlor), (4-chloro-o-tolyloxy)acetic acid (MCPA), 2-(4-chloro-o-tolyloxy)propionic acid (mecoprop), diethyl 1-(2,4-dichorophenyl)-4,5-dihydro-5-methyl-1H-pyrazole-3,5-dicarboxylate (mefenpyr-diethyl - cf. also related compounds in WO-A-91/07874), 2-dichloromethyl-2-methyl-1,3-dioxolane (MG-191), 2-propenyl-1-oxa-4-azaspiro[4.5]decane-4-carbodithioate (MG-838), 1,8-naphthalic anhydride, α-(1,3-dioxolan-2-ylmethoximino)phenylacetonitrile (oxabetrinil), 2,2-dichloro-N-(1,3-dioxolan-2-ylmethyl)-N-(2-propenyl)acetamide (PPG-1292), 3-dichloroacetyl-2,2-dimethyloxazolidine (R-28725), 3-dichloroacetyl-2,2,5-trimethyloxazolidine (R-29148), 4-(4-chloro-o-tolyl)butyric acid, 4-(4-chlorophenoxy)butyric acid, diphenylmethoxyacetic acid, methyl diphenylmethoxyacetate, ethyl diphenylmethoxyacetate, methyl 1-(2-chlorophenyl)-5-phenyl-1H-pyrazole-3-carboxylate, ethyl 1-(2,4-dichlorophenyl)-5-methyl-1H-pyrazole-3-carboxylate, ethyl 1-(2,4-dichlorophenyl)-5-isopropyl-1H-pyrazole-3-carboxylate, ethyl 1-(2,4-dichlorophenyl)-5-(1,1-dimethylethyl)-1H-pyrazole-3-carboxylate, ethyl 1-(2,4-dichlorophenyl)-5-phenyl-1H-pyrazole-3-carboxylate (cf. also related compounds in EP-A-269806 and EP-A-333131), ethyl 5-(2,4-dichlorobenzyl)-2-isoxazoline-3-carboxylate, ethyl 5-phenyl-2-isoxazoline-3-carboxylate, ethyl 5-(4-fluorophenyl)-5-phenyl-2-isoxazoline-3-carboxylate (cf. also related compounds in WO-A-91/08202), 1,3-dimethylbut-1-yl 5-chloroquinoline-8-oxyacetate, 4-allyloxybutyl 5-chloroquinoline-8-oxyacetate, 1-allyloxyprop-2-yl 5-chloroquinoline-8-oxyacetate, methyl 5-chloroquinoxaline-8-oxyacetate, ethyl 5-chloroquinoline-8-oxyacetate, allyl 5-chloroquinoxaline-8-oxyacetate, 2-oxoprop-1-yl 5-chloroquinoline-8-oxyacetate, diethyl 5-chloroquinoline-8-oxymalonate, diallyl 5-chloroquinoxaline-8-oxymalonate, diethyl 5-chloroquinoline-8-oxymalonate (cf. also related compounds in EP-A-582198), 4-carboxychroman-4-ylacetic acid (AC-304415, cf. EP-A-613618), 4-chlorophenoxyacetic acid, 3,3'-dimethyl-4-methoxybenzophenone, 1-bromo-4-chloromethylsulfonylbenzene, 1-[4-(N-2-methoxybenzoylsulfamoyl)phenyl]-3-methylurea (also known as N-(2-methoxybenzoyl)-4-[(methylaminocarbonyl)amino]benzenesulfonamide), 1-[4-(N-2-methoxybenzoylsulfamoyl)phenyl]-3,3-dimethylurea, 1-[4-(N-4,5-dimethylbenzoyl-sulfamoyl)phenyl]-3-methylurea, 1-[4-(N-naphthylsulfamoyl)phenyl]-3,3-dimethylurea, N-(2-methoxy-5-methylbenzoyl)-4-(cyclopropylaminocarbonyl)benzenesulfonamide, and/or one of the following compounds, defined by general formulae
of the general formula (IIa) 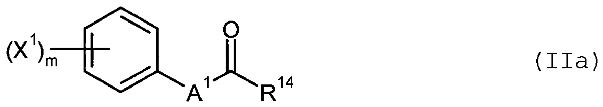 or of the general formula (IIb) 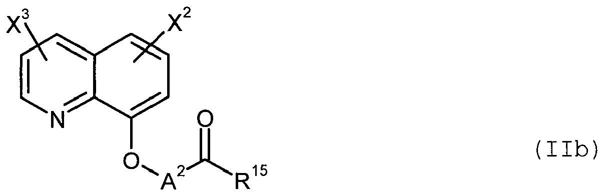 or of the formula (IIc) 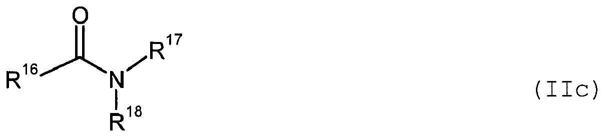 where
m represents a number 0, 1, 2, 3, 4 or 5,
A¹ represents one of the divalent heterocyclic groupings shown below 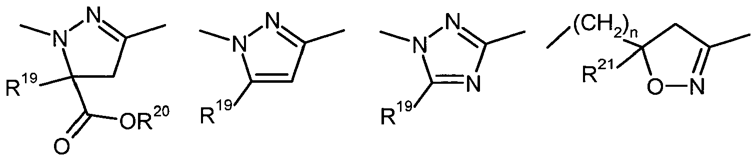
n represents a number 0, 1, 2, 3, 4 or 5,
A² represents optionally C₁-C₄-alkyl- and/or C₁-C₄-alkoxy-carbonyl- and/or C₁-C₄-alkenyloxycarbonyl-substituted alkanediyl having 1 or 2 carbon atoms,
R¹⁴ represents hydroxyl, mercapto, amino, C₁-C₆-alkoxy, C₁-C₆-alkylthio, C₁-C₆-alkylamino or di (C₁-C₄-alkyl) amino,
R¹⁵ represents hydroxyl, mercapto, amino, C₁-C₇-alkoxy, C₁-C₆-alkenyloxy, C₁-C₆-alkenyloxy-C₁-C₆-alkoxy, C₁-C₆-alkylthio, C₁-C₆-alkylamino or di(C₁-C₄-alkyl)-amino,
R¹⁶ represents optionally fluorine-, chlorine- and/or bromine-substituted C₁-C₄-alkyl,
R¹⁷ represents hydrogen, in each case optionally fluorine-, chlorine- and/or bromine-substituted C₁-C₆-alkyl, C₂-C₆-alkenyl or C₂-C₆-alkynyl, C₁-C₄-alkoxy-C₁-C₄-alkyl, dioxolanyl-C₁-C₄-alkyl, furyl, furyl-C₁-C₄-alkyl, thienyl, thiazolyl, piperidinyl, or optionally fluorine-, chlorine- and/or bromine- or C₁-C₄-alkyl-substituted phenyl,
R¹⁸ represents hydrogen, in each case optionally fluorine-, chlorine- and/or bromine-substituted C₁-C₆-alkyl, C₂-C₆-alkenyl or C₂-C₆-alkynyl, C₁-C₄-alkoxy-C₁-C₄-alkyl, dioxolanyl-C₁-C₄-alkyl, furyl, furyl-C₁-C₄-alkyl, thienyl, thiazolyl, piperidinyl, or optionally fluorine-, chlorine- and/or bromine- or C₁-C₄-alkyl-substituted phenyl, R¹⁷ and R¹⁸ also together represent C₃-C₆-alkanediyl or C₂-C₅-oxaalkanediyl, each of which is optionally substituted by C₁-C₄-alkyl, phenyl, furyl, a fused benzene ring or by two substituents which, together with the C atom to which they are attached, form a 5- or 6-membered carbocycle,
R¹⁹ represents hydrogen, cyano, halogen, or represents in each case optionally fluorine-, chlorine- and/or bromine-substituted C₁-C₄-alkyl, C₃-C₆-cycloalkyl or phenyl,
R²⁰ represents hydrogen, in each case optionally hydroxyl-, cyano-, halogen- or C₁-C₄-alkoxy-substituted C₁-C₆-alkyl, C₃-C₆-cycloalkyl or tri-(C₁-C₄-alkyl)silyl,
R²¹ represents hydrogen, cyano, halogen, or represents in each case optionally fluorine-, chlorine- and/or bromine-substituted C₁-C₄-alkyl, C₃-C₆-cycloalkyl or phenyl,
X¹ represents nitro, cyano, halogen, C₁-C₄-alkyl, C₁-C₄-haloalkyl, C₁-C₄-alkoxy or C₁-C₄-haloalkoxy,
X² represents hydrogen, cyano, nitro, halogen, C₁-C₄-alkyl, C₁-C₄-haloalkyl, C₁-C₄-alkoxy or C₁-C₄-haloalkoxy,
X³ represents hydrogen, cyano, nitro, halogen, C₁-C₄-alkyl, C₁-C₄-haloalkyl, C₁-C₄-alkoxy or C₁-C₄-haloalkoxy,
and/or the following compounds, defined by general formulae
of the general formula (IId) 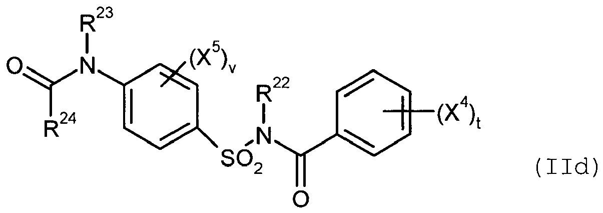 or of the general formula (IIe) 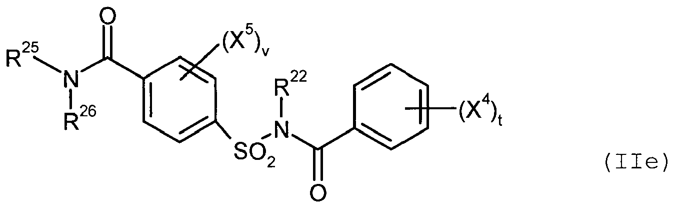 where
t represents a number 0, 1, 2, 3, 4 or 5,
v represents a number 0, 1, 2, 3, 4 or 5,
R²² represents hydrogen or C₁-C₄-alkyl,
R²³ represents hydrogen or C₁-C₄-alkyl,
R²⁴ represents hydrogen, in each case optionally cyano-, halogen- or C₁-C₄-alkoxy-substituted C₁-C₆-alkyl, C₁-C₆-alkoxy, C₁-C₆-alkylthio, C₁-C₆-alkylamino or di(C₁-C₄-alkyl)amino, or in each case optionally cyano-, halogen- or C₁-C₄-alkyl-substituted C₃-C₆-cycloalkyl, C₃-C₆-cycloalkyloxy, C₃-C₆-cycloalkylthio or C₃-C₆-cycloalkylamino,
R²⁵ represents hydrogen, optionally cyano-, hydroxyl-, halogen- or C₁-C₄-alkoxy-substituted C₁-C₆-alkyl, in each case optionally cyano- or halogen-substituted C₃-C₆-alkenyl or C₃-C₆-alkynyl, or optionally cyano-, halogen- or C₁-C₄-alkyl-substituted C₃-C₆-cycloalkyl,
R²⁶ represents hydrogen, optionally cyano-, hydroxyl-, halogen- or C₁-C₄-alkoxy-substituted C₁-C₆-alkyl, in each case optionally cyano- or halogen-substituted C₃-C₆-alkenyl or C₃-C₆-alkynyl, optionally cyano-, halogen- or C₁-C₄-alkyl-substituted C₃-C₆-cycloalkyl, or optionally nitro-, cyano-, halogen-, C₁-C₄-alkyl-, C₁-C₄-haloalkyl-, C₁-C₄-alkoxy- or C₁-C₄-haloalkoxy-substituted phenyl, or together with R²⁵ represents in each case optionally C₁-C₄-alkyl-substituted C₂-C₆-alkanediyl or C₂-C₅-oxaalkanediyl,
X⁴ represents nitro, cyano, carboxyl, carbamoyl, formyl, sulfamoyl, hydroxyl, amino, halogen, C₁-C₄-alkyl, C₁-C₄-haloalkyl, C₁-C₄-alkoxy or C₁-C₄-haloalkoxy, and
X⁵ represents nitro, cyano, carboxyl, carbamoyl, formyl, sulfamoyl, hydroxyl, amino, halogen, C₁-C₄-alkyl, C₁-C₄-haloalkyl, C₁-C₄-alkoxy or C₁-C₄-haloalkoxy.

8. Composition according to Claim 7, **characterized in that** the crop plant compatibility-improving compound is selected from the following group of compounds:
cloquintocet-mexyl, fenchlorazole-ethyl, isoxadifen-ethyl, mefenpyr-diethyl, furilazole, fenclorim, cumyluron, dymron or the compounds 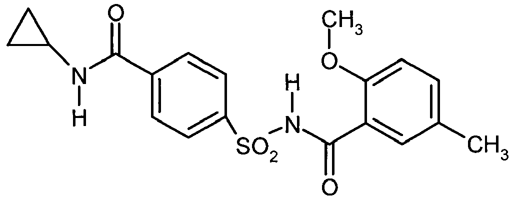 and

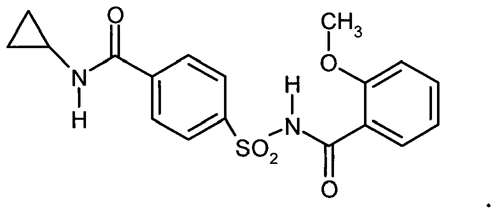

9. Composition according to Claim 7 or 8, **characterized in that** the crop plant compatibility-improving compound is cloquintocet-mexyl.

10. Composition according to Claim 7 or 8, **characterized in that** the crop plant compatibility-improving compound is mefenpyr-diethyl.

11. Composition comprising
- at least one compound of the formula (I) according to Claim 1 or a composition according to Claim 7 and
- at least one salt of the formula (III') 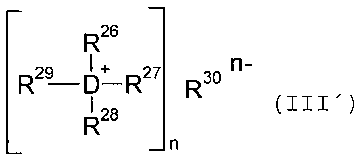 in which
D represents nitrogen or phosphorus,
R²⁶, R²⁷, R²⁸ and R²⁹ independently of one another represent hydrogen or in each case optionally substituted C₁-C₈-alkyl or mono- or polyunsaturated, optionally substituted C₁-C₈-alkylene, the substituents being selectable from halogen, nitro and cyano,
n represents 1, 2, 3 or 4,
R³⁰ represents an organic or inorganic anion.

12. Composition according to Claim 11, **characterized in that** it comprises at least one penetrant.

13. Use of compounds of the formula (I) according to Claim 1 for preparing pesticides and/or herbicides and/or fungicides.

14. Pesticide and/or herbicide and/or fungicide, **characterized in that** it comprises at least one compound of the formula (I) according to Claim 1.

15. Method for controlling animal pests and/or unwanted vegetation and/or fungi, **characterized in that** compounds of the formula (I) according to Claim 1 are allowed to act on pests and/or their habitat.

16. Use of compounds of the formula (I) according to Claim 1 for controlling animal pests and/or unwanted vegetation and/or fungi.

17. Process for preparing pesticides and/or herbicides and/or fungicides, **characterized in that** compounds of the formula (I) according to Claim 1 are mixed with extenders and/or surfactants.

18. Method for controlling unwanted vegetation, **characterized in that** a composition according to Claim 7 is allowed to act on the plants or their habitat.

19. Use of a composition according to Claim 7 for controlling unwanted vegetation.

20. Method for controlling unwanted vegetation, **characterized in that** compounds of the formula (I) according to Claim 1 and the crop plant compatibility-improving compound according to Claim 7 are allowed to act, separately in close temporal succession, on the plants or their surroundings.

21. Method for improving the activity of pesticides and/or herbicides comprising an active compound of the formula (I) according to Claim 1 or a composition according to Claim 7, **characterized in that** the ready-to-use composition (spray liquor) is prepared using a salt of the formula (III') according to Claim 11.

22. Process according to Claim 21, **characterized in that** the spray liquor is prepared using a penetrant.

23. Compound of the formula (II) 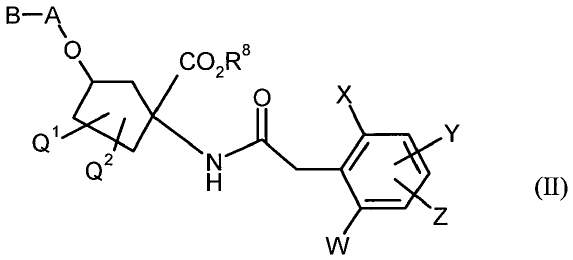 in which
A, B, Q¹, Q², W, X, Y, Z and R⁸ are as defined above.

24. Compound of the formula (III) 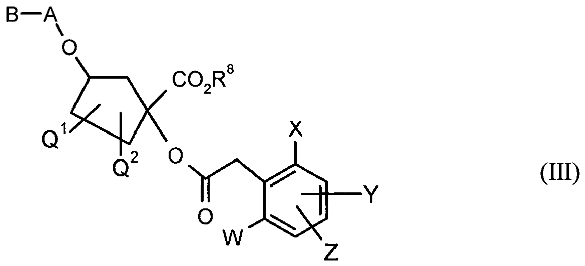 in which
A, B, Q¹, Q², W, X, Y, Z and R⁸ are as defined above.

25. Compound of the formula (XVI) 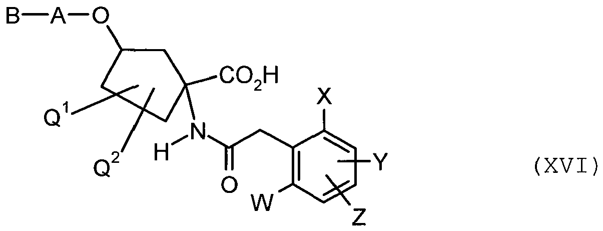 in which
A, B, Q¹, Q², W, X, Y and Z are as defined above.

## Revendications

1. Composés de formule (I) 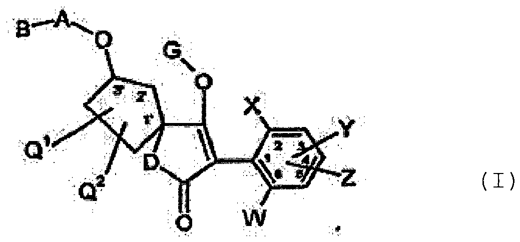 dans laquelle
W représente un atome d'hydrogène ou d'halogène, un groupe alkyle, alcényle, alcynyle, alcoxy, halogénoalkyle, halogénoalcoxy ou cyano,
X représente un atome d'halogène, un groupe alkyle, alcényle, alcynyle, alcoxy, alcoxy-alcoxy, halogénoalkyle, halogénoalcoxy ou cyano,
Y représente un atome d'hydrogène ou d'halogène, un groupe alkyle, alcényle, alcynyle, alcoxy, cyano, halogénoalkyle, halogénoalcoxy, un groupe phényle ou hétéroaryle chacun éventuellement substitué,
Z représente un atome d'hydrogène ou d'halogène, un groupe alkyle, halogénoalkyle, cyano, alcoxy ou halogénoalcoxy,
A représente un groupe alcanediyle éventuellement substitué ou représente un groupe cycloalkyle éventuellement substitué et/ou éventuellement interrompu par un hétéroatome,
B représente un atome d'hydrogène ou un groupe alkyle, alcényle, alcoxy, alcoxy-alcoxy, phényle, hétéroaryle ou un radical cycloalkyle éventuellement substitué et/ou éventuellement interrompue par C=O et/ou des hétéroatomes,
ou A représente une liaison et B représente un atome d'hydrogène,
D représente NH ou un atome d'oxygène,
Q¹ représente un atome d'hydrogène ou un groupe alkyle, alcoxy, alcoxy-alkyle ou alkylthio-alkyle, chacun éventuellement substitué, ou représente un radical cycloalkyle éventuellement substitué, dans lequel un groupe méthylène est éventuellement remplacé par des hétéroatomes, ou représente un radical phényle, hétéroaryle, phényl-alkyle ou hétéroarylalkyle, éventuellement substitué,
Q² représente un atome d'hydrogène ou un groupe alkyle,
Q¹ et Q² représentent ensemble, avec l'atome de carbone auquel ils sont liés, un cycle en C₃-C₆ éventuellement substitué, qui peut éventuellement être interrompu par un hétéroatome,
Q¹ et Q² représentent ensemble, avec les atomes de carbone auxquels ils sont liés, un cycle en C₃-C₆ éventuellement substitué, qui peut éventuellement être interrompu par un hétéroatome, ou
G représente un atome d'hydrogène (a) ou l'un des groupes 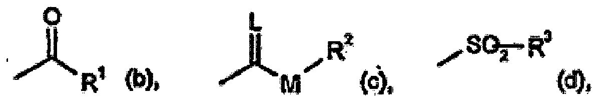 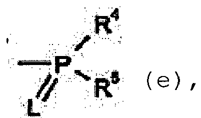 E (f) ou 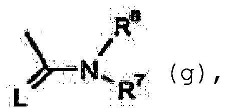 dans lesquels
E représente un ion métallique ou un ion ammonium,
L représente un atome d'oxygène ou de soufre,
M représente un atome d'oxygène ou de soufre,
R¹ représente un groupe alkyle, alcényle, alcoxy-alkyle, alkylthio-alkyle ou polyalcoxy-alkyle chacun éventuellement substitué par halogène ou cyano, ou représente un radical cycloalkyle ou hétérocyclyle éventuellement substitués chacun par halogène, alkyle ou alcoxy, ou représente un radical phényle, phénylalkyle, hétéroaryle, phénoxyalkyle ou hétéroaryloxyalkyle chacun éventuellement substitué,
R² représente un groupe alkyle, alcényle, alcoxyalkyle ou polyalcoxyalkyle chacun éventuellement substitué par halogène ou cyano, ou représente un radical cycloalkyle, phényle ou benzyle chacun éventuellement substitué,
R³, R⁴ et R⁵ chacun indépendamment représentent un groupe alkyle, alcoxy, alkylamino, dialkylamino, alkylthio, alcénylthio ou cycloalkylthio, chacun éventuellement substitué par halogène, ou représentent un radical phényle, benzyle, phénoxy ou phénylthio, chacun éventuellement substitué,
R⁶ et R⁷, indépendamment l'un de l'autre, représentent un atome d'hydrogène, représentent un groupe alkyle, cycloalkyle, alcényle, alcoxy, alcoxy-alkyle, chacun éventuellement substitué par halogène ou cyano, représentent un radical phényle ou benzyle, chacun éventuellement substitué, ou forment ensemble, avec l'atome d'azote auquel ils sont liés, un cycle éventuellement substitué et éventuellement contenant de l'oxygène ou du soufre.

2. Composés de formule (I) selon la revendication 1, dans lesquels
W représente un atome d'hydrogène ou d'halogène, un groupe alkyle en C₁-C₆, alcényle en C₂-C₆, alcynyle en C₂-C₆, alcoxy en C₁-C₆, halogénoalkyle(C₁-C₄), halogénoalcoxy(C₁-C₄) ou cyano,
X représente un atome d'halogène, un groupe alkyle en C₁-C₆, alcényle en C₂-C₆, alcynyle en C₂-C₆, alcoxy en C₁-C₆, alcoxy (C₁-C₆)-alcoxy(C₁-C₄), halogénoalkyle(C₁-C₄), halogénoalcoxy(C₁-C₄) ou cyano,
Y représente un atome d'hydrogène ou d'halogène, un groupe alkyle en C₁-C₆, alcényle en C₂-C₆, alcynyle en C₂-C₆, alcoxy en C₂-C₆, cyano, halogénoalkyle(C₁-C₄), halogénoalcoxy, représente un radical phényle ou pyridyle substitué par V¹ et V²,
V¹ représente un atome d'halogène, un groupe alkyle en C₁-C₁₂, alcoxy en C₁-C₆, halogénoalkyle(C₁-C₄), halogénoalcoxy(C₁-C₄), cyano ou nitro,
V² représente un atome d'hydrogène ou d'halogène, un groupe alkyle en C₁-C₆, alcoxy en C₁-C₆ ou halogénoalkyle(C₁-C₄),
V¹ et V² représentent ensemble un groupe alcane(C₃-C₄)diyle, qui peut éventuellement être substitué par halogène et/ou alkyle en C₁-C₂ et qui peut éventuellement être interrompu par un ou deux atomes d'oxygène,
Z représente un atome d'hydrogène ou d'halogène, un groupe alkyle en C₁-C₆, halogénoalkyle(C₁-C₄), cyano, alcoxy en C₁-C₆ ou halogénoalcoxy(C₁-C₄),
A représente un groupe alcane(C₁-C₄)diyle éventuellement substitué par alkyle en C₁-C₄ ou représente un groupe cycloalkyle en C₅-C₈ éventuellement substitué par alkyle en C₁-C₄, dans lequel un groupe méthylène est éventuellement remplacé par un atome d'oxygène,
B représente un atome d'hydrogène ou un groupe alkyle en C₁-C₈, alcényle en C₂-C₈, alcoxy en C₁-C₆, alcoxy(C₁-C₆)-alcoxy(C₁-C₄), alcoxy(C₁-C₄)-bis-alcoxy(C₁-C₄), chacun éventuellement substitué par halogène, représente un radical phényle éventuellement substitué par halogène, alkyle en C₁-C₆, alcoxy en C₁-C₆, halogénoalkyle(C₁-C₄), halogénoalcoxy(C₁-C₄), cyano ou nitro, représente un radical pyridyle, pyrimidyle, thiazolyle ou thiényle, éventuellement substitué par halogène, alkyle en C₁-C₄ ou halogénoalkyle(C₁-C₂), ou représente un radical cycloalkyle en C₃-C₈ éventuellement substitué par halogène, alkyle en C₁-C₄, alcoxy en C₁-C₄ ou halogénoalkyle(C₁-C₂), dans lequel éventuellement un ou deux groupes méthylène non directement voisins sont remplacés par un atome d'oxygène, deux groupes méthylène sont remplacés par le radical -O-CO- ou trois groupes méthylène sont remplacés par le radical -O-CO-O-,
ou A représente une liaison et B représente un atome d'hydrogène,
D représente NH ou un atome d'oxygène,
Q¹ représente un atome d'hydrogène ou un groupe alkyle en C₁-C₆, alcoxy en C₁-C₆, alcoxy(C₁-C₄)-alkyle(C₁-C₄), alkyl(C₁-C₄)thio-alkyle(C₁-C₄), chacun éventuellement substitué par halogène, ou représente un radical cycloalkyle en C₃-C₆ éventuellement substitué par halogène, alkyle en C₁-C₄ ou alcoxy en C₁-C₄, dans lequel un groupe méthylène est éventuellement remplacé par un atome d'oxygène, ou représente un radical phényle, phényl-alkyle(C₁-C₂) ou hétéroaryle, chacun substitué une ou deux fois par halogène, halogénoalkyle en C₁-C₄ ou halogénoalcoxy en C₁-C₄,
Q² représente un atome d'hydrogène ou un groupe alkyle en C₁-C₆, ou
Q¹ et Q² représentent ensemble, avec l'atome de carbone auquel ils sont liés, de préférence un cycle en C₃-C₆ éventuellement une ou deux fois substitué par fluoro, chloro, alkyle en C₁-C₄, alcoxy en C₁-C₄ ou trifluorométhyle, dans lequel un groupe méthylène peut éventuellement être remplacé par un atome d'oxygène, ou
Q¹ et Q² représentent ensemble, avec les atomes de carbone auxquels ils sont liés, un cycle en C₃-C₆ éventuellement une ou deux fois substitué par fluoro, chloro, alkyle en C₁-C₄, alcoxy en C₁-C₄ ou trifluorométhyle, dans lequel un groupe méthylène peut éventuellement être remplacé par un atome d'oxygène,
G représente un atome d'hydrogène (a) ou l'un des groupes 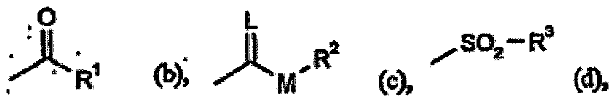 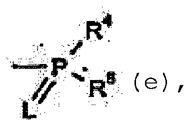 E (f) ou 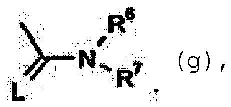 dans lesquels
E représente un ion métallique ou un ion ammonium,
L représente un atome d'oxygène ou de soufre et
M représente un atome d'oxygène ou de soufre,
R¹ représente un groupe alkyle en C₁-C₂₀, alcényle en C₂-C₂₀, alcoxy(C₁-C₈)-alkyle(C₁-C₈), alkyl(C₁-C₈)-thio-alkyle(C₁-C₈) ou poly-alcoxy(C₁-C₈)-alkyle(C₁-C₈) chacun éventuellement substitué par halogène ou cyano, ou représente un radical cycloalkyle en C₃-C₈ éventuellement substitué par halogène, alkyle en C₁-C₆ ou alcoxy en C₁-C₆, dans lequel un ou deux groupes méthylène non directement voisins sont éventuellement remplacés par un atome d'oxygène et/ou un atome de soufre,
représente un radical phényle éventuellement substitué par halogène, cyano, nitro, alkyle en C₁-C₆, alcoxy en C₁-C₆, halogénoalkyle(C₁-C₆), halogénoalcoxy(C₁-C₆), alkyl(C₁-C₆)thio ou alkyl(C₁-C₆)sulfonyle,
représente un radical phényl-alkyle(C₁-C₆) éventuellement substitué par halogène, nitro, cyano, alkyle en C₁-C₆, alcoxy en C₁-C₆, halogénoalkyle(C₁-C₆) ou halogénoalcoxy(C₁-C₆),
représente un radical hétéroaryle à 5 ou 6 chaînons, éventuellement substitué par halogène ou alkyle en C₁-C₆, comportant un ou deux hétéroatomes choisis dans la série constituée par les atomes d'oxygène, de soufre et d'azote,
représente un radical phénoxy-alkyle(C₁-C₆) éventuellement substitué par halogène ou alkyle en C₁-C₆,
représente un radical hétéroaryl(à 5 ou 6 chaînons)oxy-alkyle(C₁-C₆) éventuellement substitué par halogène, amino ou alkyle en C₁-C₆, comportant un ou deux hétéroatomes choisis dans la série constituée par les atomes d'oxygène, de soufre et d'azote,
R² représente un groupe alkyle en C₁-C₂₀, alcényle en C₂-C₂₀, alcoxy(C₁-C₈)-alkyle(C₂-C₈) ou poly(alcoxy(C₁-C₈)-alkyle(C₂-C₈) chacun éventuellement substitué par halogène ou cyano,
représente un radical cycloalkyle en C₃-C₈ éventuellement substitué par halogène, alkyle en C₁-C₆ ou alcoxy en C₁-C₆ ou
représente un radical phényle ou benzyle chacun éventuellement substitué par halogène, cyano, nitro, alkyle en C₁-C₆, alcoxy en C₁-C₆, halogénoalkyle(C₁-C₆) ou halogénoalcoxy(C₁-C₆),
R³ représente un groupe alkyle en C₁-C₈ éventuellement substitué par halogène, ou un radical phényle ou benzyle chacun éventuellement substitué par halogène, alkyle en C₁-C₆, alcoxy en C₁-C₆, halogénoalkyle(C₁-C₄), halogénoalcoxy(C₁-C₄), cyano ou nitro,
R⁴ et R⁵, indépendamment l'un de l'autre, représentent un groupe alkyle en C₁-C₈, alcoxy en C₁-C₈, alkyl (C₁-C₈) amino, di [alkyl(C₁-C₈)]amino, alkyl(C₁-C₈)thio ou alcényl(C₃-C₈)thio, chacun éventuellement substitué par halogène, ou représentent un radical phényle, phénoxy ou phénylthio, chacun éventuellement substitué par halogène, nitro, cyano, alcoxy en C₁-C₄, halogénoalcoxy(C₁-C₄), alkyl(C₁-C₄)thio, halogénoalkyl(C₁-C₄)thio, alkyle en C₁-C₄ ou halogénoalkyle(C₁-C₄),
R⁶ et R⁷, indépendamment l'un de l'autre, représentent un atome d'hydrogène, représentent un groupe alkyle en C₁-C₈, cycloalkyle en C₃-C₈, alcoxy en C₁-C₈, alcényle en C₃-C₈ ou alcoxy(C₁-C₈)-alkyle(C₂-C₈), chacun éventuellement substitué par halogène ou cyano, représentent un radical phényle ou benzyle, chacun éventuellement substitué par halogène, alkyle en C₁-C₈, halogénoalkyle(C₁-C₈) ou alcoxy en C₁-C₈, ou ensemble représentent un radical alkylène en C₃-C₆ éventuellement substitué par alkyle en C₁-C₆, dans lequel éventuellement un groupe méthylène est remplacé par un atome d'oxygène ou de soufre.

3. Composés de formule (I) selon la revendication 1, dans lesquels
W représente un atome d'hydrogène, de chlore, de brome, d'iode, un groupe alkyle en C₁-C₄, alcényle en C₂-C₄, alcynyle en C₂-C₄, alcoxy en C₁-C₄, halogénoalkyle(C₁-C₂) ou halogénoalcoxy(C₁-C₂),
X représente un atome de chlore, de brome, d'iode, un groupe alkyle en C₁-C₄, alcényle en C₂-C₄, alcynyle en C₂-C₄, alcoxy en C₁-C₄, alcoxy(C₁-C₄)-alcoxy(C₁-C₃), halogénoalkyle(C₁-C₂), halogéno-alcoxy(C₁-C₂) ou cyano,
Y en la position 4 représente un atome d'hydrogène, de fluor, de chlore, de brome, d'iode, le groupe méthoxy, éthoxy, cyano, trifluorométhyle, difluorométhoxy ou trifluorométhoxy,
Z représente un atome d'hydrogène.
W représente un atome d'hydrogène, de chlore, de brome, ou un groupe alkyle en C₁-C₄,
X représente un atome de chlore ou de brome, un groupe alkyle en C₁-C₄, alcoxy en C₁-C₄, halogénoalkyle(C₁-C₂), halogénoalcoxy(C₁-C₂) ou cyano,
Y en la position 4 représente un groupe alcényle en C₂-C₄, alcynyle en C₂-C₄ ou le radical 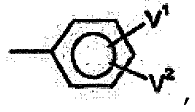
Z représente un atome d'hydrogène,
V¹ représente également un atome de fluor ou de chlore, un groupe alkyle en C₁-C₄, alcoxy en C₁-C₄, halogénoalkyle(C₁-C₂) ou halogénoalcoxy(C₁-C₂),
V² représente également un atome d'hydrogène, de fluor ou de chlore, un groupe alkyle en C₁-C₄, alcoxy en C₁-C₄ ou halogénoalkyle(C₁-C₂),
V¹ et V² également représentent ensemble un groupe -O-CH₂-O- ou -O-CF₂-O-, ou
W représente également un atome d'hydrogène, de chlore ou de brome, ou un groupe alkyle en C₁-C₄,
X représente également un atome de chlore ou de brome, un groupe alkyle en C₁-C₄ ou halogénoalkyle(C₁-C₂),
Y également en la position 5 représente un groupe alcényle en C₂-C₄, alcynyle en C₂-C₄, le radical 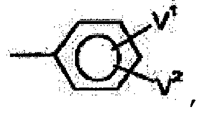
Z également en la position 4 représente un atome d'hydrogène ou de chlore, ou un groupe alkyle en C₁-C₄,
V¹ représente également un atome de fluor ou de chlore, un groupe alkyle en C₁-C₄, alcoxy en C₁-C₄, halogénoalkyle(C₁-C₂) ou halogénoalcoxy(C₁-C₂),
V² représente également un atome d'hydrogène, de fluor ou de chlore, un groupe alkyle en C₁-C₄, alcoxy en C₁-C₄ ou halogénoalkyle(C₁-C₂),
V¹ et V² également forment ensemble un groupe -O-CH₂-O-ou -O-CF₂-O-.
W en outre représente un atome d'hydrogène, un groupe alkyle en C₁-C₄, alcényle en C₂-C₄, alcynyle en C₂-C₄, alcoxy en C₁-C₄, un atome de chlore, de brome, d'iode ou le groupe trifluorométhyle,
X en outre représente un atome de chlore, de brome, d'iode, un groupe alkyle en C₁-C₄, alcényle en C₂-C₄, alcynyle en C₂-C₄, alcoxy en C₁-C₄, alcoxy(C₁-C₄)-alcoxy(C₁-C₃), halogénoalkyle(C₁-C₂), halogénoalcoxy(C₁-C₂) ou cyano,
Y en outre en la position 4 représente un groupe alkyle en C₁-C₄,
Z en outre représente un atome d'hydrogène.
W représente encore un atome d'hydrogène, de chlore, de brome, d'iode, un groupe alkyle en C₁-C₄ ou alcoxy en C₁-C₄,
X représente encore un atome de chlore, de brome, d'iode, un groupe alkyle en C₁-C₄, alcoxy en C₁-C₄, halogénoalkyle(C₁-C₂), halogénoalcoxy(C₁-C₂) ou cyano,
Y en la position 4 représente encore un atome d'hydrogène, de chlore, de brome, un groupe alkyle en C₁-C₄, halogénoalkyle(C₁-C₂) ou halogénoalcoxy(C₁-C₂),
Z en la position 3 ou 5 représente encore un atome de fluor, de chlore, de brome, d'iode, un groupe alkyle en C₁-C₄, halogénoalkyle(C₁-C₂), alcoxy en C₁-C₄ ou halogénoalcoxy(C₁-C₂),
A représente un groupe alcane(C₁-C₃)diyle éventuellement substitué par alkyle en C₁-C₂ ou représente un groupe cycloalkyle en C₅-C₆, dans lequel un groupe méthylène est éventuellement remplacé par un atome d'oxygène,
B représente un atome d'hydrogène ou un groupe alkyle en C₁-C₆, alcényle en C₂-C₆, alcoxy en C₁-C₄, alcoxy(C₁-C₄)-alcoxy(C₁-C₃), alcoxy(C₁-C₄)-bis-alcoxy(C₁-C₃), chacun éventuellement une à trois fois substitué par le fluor ou le chlore, représente un radical phényle éventuellement une à trois fois substitué par le fluor, le chlore, le brome, par alkyle en C₁-C₄, alcoxy en C₁-C₄, halogénoalkyle(C₁-C₂), halogénoalcoxy(C₁-C₂), cyano ou nitro, représente un radical pyridyle, pyrimidyle, thiazolyle ou thiényle, éventuellement une ou deux fois substitué par le fluor, le chlore, le brome, par méthyle, éthyle ou trifluorométhyle, ou représente un radical cycloalkyle en C₃-C₆ éventuellement une ou deux fois substitué par le fluor, le chlore, par méthyle, méthoxy ou trifluorométhyle, dans lequel éventuellement un groupe méthylène ou deux groupes méthylène non directement voisins sont remplacés par un atome d'oxygène,
ou A représente une liaison et B représente un atome d'hydrogène,
D représente NH ou un atome d'oxygène.
Q¹ représente un atome d'hydrogène, représente un groupe alkyle en C₁-C₄ éventuellement une à trois fois substitué par le fluor,
Q² représente un atome d'hydrogène ou un groupe alkyle en C₁-C₄,
Q¹ et Q² représentent ensemble, avec l'atome de carbone auquel ils sont liés, de façon particulièrement préférée un cycle en C₃-C₆ éventuellement une fois substitué par le fluor, par méthyle, méthoxy ou trifluorométhyle, dans lequel un groupe méthylène peut éventuellement être remplacé par un atome d'oxygène, ou
Q¹ et Q² représentent ensemble, avec les atomes de carbone auxquels ils sont liés, de façon particulièrement préférée un cycle en C₃-C₆ éventuellement une fois substitué par le fluor, par méthyle, méthoxy ou trifluorométhyle, dans lequel un groupe méthylène peut éventuellement être remplacé par un atome d'oxygène.
G représente un atome d'hydrogène (a) ou l'un des groupes 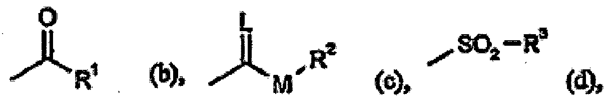 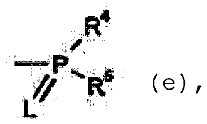 E (f) ou 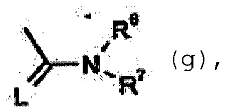 dans lesquels
E représente un ion métallique ou un ion ammonium,
L représente un atome d'oxygène ou de soufre et
M représente un atome d'oxygène ou de soufre,
R¹ représente un groupe alkyle en C₁-C₁₆, alcényle en C₂-C₁₆, alcoxy(C₁-C₆)-alkyle(C₁-C₄), alkyl(C₁-C₆)-thio-alkyle(C₁-C₄) ou poly(alcoxy(C₁-C₆)-alkyle(C₁-C₄), chacun éventuellement une à trois fois substitué par le fluor ou le chlore, ou représente un radical cycloalkyle en C₃-C₇ éventuellement une ou deux fois substitué par le fluor, le chlore, par alkyle en C₁-C₅ ou alcoxy en C₁-C₅, dans lequel un ou deux groupes méthylène non directement voisins sont éventuellement remplacés par un atome d'oxygène et/ou un atome de soufre,
représente un radical phényle éventuellement une à trois fois substitué par le fluor, le chlore, le brome, par cyano, nitro, alkyle en C₁-C₄, alcoxy en C₁-C₄, halogénoalkyle(C₁-C₃), halogénoalcoxy(C₁-C₃), alkyl (C₁-C₄) thio ou alkyl (C₁-C₄) sulfonyle,
représente un radical phényl-alkyle(C₁-C₄) éventuellement une ou deux fois substitué par le fluor, le chlore, le brome, par alkyle en C₁-C₄, alcoxy en C₁-C₄, halogénoalkyle(C₁-C₃) ou halogénoalcoxy(C₁-C₃),
représente un radical pyrazolyle, thiazolyle, pyridyle, pyrimidyle, furanyle ou thiényle, chacun éventuellement une ou deux fois substitué par le fluor, le chlore, le brome ou par alkyle en C₁-C₄,
représente un radical phénoxy-alkyle(C₁-C₅) éventuellement une ou deux fois substitué par le fluor, le chlore, le brome ou par alkyle en C₁-C₄,
représente un radical pyridyloxy-alkyle(C₁-C₅), pyrimidyloxy-alkyle(C₁-C₅) ou thiazolyloxy-alkyle(C₁-C₅), chacun éventuellement une ou deux fois substitué par le fluor, le chlore, le brome, par amino ou alkyle en C₁-C₄,
R² représente un groupe alkyle en C₁-C₁₆, alcényle en C₂-C₁₆, alcoxy(C₁-C₆)-alkyle(C₂-C₆) ou poly-alcoxy(C₁-C₆)-alkyle(C₂-C₆), chacun éventuellement une à trois fois substitué par le fluor ou le chlore,
représente un radical cycloalkyle en C₃-C₇ éventuellement une ou deux fois substitué par le fluor, le chlore, par alkyle en C₁-C₄ ou alcoxy en C₁-C₄ ou
représente un radical phényle ou benzyle chacun éventuellement une à trois fois substitué par le fluor, le chlore, le brome, par cyano, nitro, alkyle en C₁-C₄, alcoxy en C₁-C₃, halogénoalkyle(C₁-C₃) ou halogénoalcoxy(C₁-C₃),
R³ représente un groupe alkyle en C₁-C₆ éventuellement une à trois fois substitué par le fluor ou le chlore, ou un radical phényle ou benzyle chacun éventuellement une ou deux fois substitué par le fluor, le chlore, le brome, par alkyle en C₁-C₄, alcoxy en C₁-C₄, halogénoalcoxy(C₁-C₂), halogénoalkyle(C₁-C₂), cyano ou nitro,
R⁴ et R⁵, indépendamment l'un de l'autre, représentent un groupe alkyle en C₁-C₆, alcoxy en C₁-C₆, alkyl(C₁-C₆) amino, di[alkyl(C₁-C₆)]amino, alkyl (C₁-C₆) thio ou alcényl (C₃-C₄) thio, chacun éventuellement une à trois fois substitué par le fluor ou le chlore, ou représentent un radical phényle, phénoxy ou phénylthio, chacun éventuellement une ou deux fois substitué par le fluor, le chlore, le brome, par nitro, cyano, alcoxy en C₁-C₃, halogénoalcoxy(C₁-C₃), alkyl (C₁-C₃)thio, halogénoalkyl(C₁-C₃)thio, alkyle en C₁-C₃ ou halogénoalkyle(C₁-C₃),
R⁶ et R⁷, indépendamment l'un de l'autre, représentent un atome d'hydrogène, représentent un groupe alkyle en C₁-C₆, cycloalkyle en C₃-C₆, alcoxy en C₁-C₆, alcényle en C₃-C₆ ou alcoxy(C₁-C₆)-alkyle(C₂-C₆), chacun éventuellement une à trois fois substitué par le fluor ou le chlore, représentent un radical phényle ou benzyle, chacun éventuellement une à trois fois substitué par le fluor, le chlore, le brome, par halogénoalkyle(C₁-C₅), alkyle en C₁-C₅ ou alcoxy en C₁-C₅, ou ensemble représentent un radical alkylène en C₃-C₆ éventuellement substitué par alkyle en C₁-C₄, dans lequel éventuellement un groupe méthylène est remplacé par un atome d'oxygène ou de soufre.

4. Composés de formule (I) selon la revendication 1, dans lesquels
W représente un atome d'hydrogène, de chlore, de brome, d'iode, le groupe méthyle, éthyle, méthoxy, éthoxy ou trifluorométhyle,
X représente un atome de chlore, de brome, d'iode, le groupe méthyle, éthyle, propyle, méthoxy, éthoxy, propoxy, méthoxy-éthoxy, éthoxy-éthoxy, trifluorométhyle, difluorométhoxy, trifluorométhoxy ou cyano,
Y en la position 4 représente un atome d'hydrogène, de chlore, de brome, d'iode, le groupe trifluorométhyle ou trifluorométhoxy,
Z représente un atome d'hydrogène.
W représente également un atome d'hydrogène, de chlore ou de brome, le groupe méthyle ou éthyle,
X représente également un atome de chlore ou de brome, le groupe méthyle, éthyle, propyle, méthoxy, trifluorométhyle, difluorométhoxy ou cyano,
Y également en la position 4 représente le radical vinyle, éthynyle, propynyle ou le radical 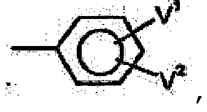
Z représente également un atome d'hydrogène,
V¹ représente également un atome de fluor ou de chlore, le groupe méthyle, méthoxy, trifluorométhyle ou trifluorométhoxy,
V² représente également un atome d'hydrogène, de fluor, de chlore, le groupe méthyle, méthoxy ou trifluorométhyle.
W représente également un atome d'hydrogène, de chlore, ou le groupe méthyle,
X représente également un atome de chlore, le groupe méthyle ou trifluorométhyle,
Y également en la position 5 représente le radical vinyle, éthynyle, propynyle ou le radical 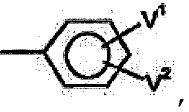
Z également en la position 4 représente un atome d'hydrogène ou le groupe méthyle,
V¹ représente également un atome de fluor, de chlore, le groupe méthyle, méthoxy, trifluorométhyle ou trifluorométhoxy,
V² représente également un atome d'hydrogène, de fluor, de chlore, le groupe méthyle, méthoxy ou trifluorométhyle.
W en outre représente un atome d'hydrogène, le groupe méthyle, éthyle, méthoxy, éthoxy, un atome de chlore, de brome ou d'iode,
X en outre représente un atome de chlore, de brome, d'iode, le groupe méthyle, éthyle, propyle, méthoxy, éthoxy, propoxy, méthoxy-éthoxy, éthoxy-éthoxy, trifluorométhyle, difluorométhoxy, trifluorométhoxy ou cyano,
Y en outre en la position 4 représente le groupe méthyle ou éthyle,
Z en outre représente un atome d'hydrogène.
W représente encore un atome d'hydrogène, de chlore de brome, d'iode, le groupe méthyle ou éthyle,
X représente encore un atome de chlore, de brome, d'iode, le groupe méthyle, éthyle, méthoxy, trifluorométhyle, difluorométhoxy ou trifluorométhoxy,
Y en la position 4 représente encore un atome d'hydrogène, de chlore, de brome, d'iode, le groupe méthyle ou éthyle,
Z en la position 3 ou 5 représente encore un atome de fluor, de chlore, de brome, d'iode, le groupe méthyle, éthyle, trifluorométhyle ou trifluorométhoxy,
A représente un groupe -CH₂-, -CHCH₃-, -CH₂CH₂-, -CH₂-CHCH₃-, -CH₂-CH₂-CH₂-,
B représente un atome d'hydrogène, un groupe méthyle, éthyle, propyle, isopropyle, butyle, isobutyle, alcényle en C₂-C₄, méthoxy, éthoxy, propoxy, isopropoxy, butoxy, isobutoxy, méthoxy-éthoxy, éthoxy-éthoxy, représente un radical phényle éventuellement une ou deux fois substitué par le fluor, le chlore, le brome, par méthyle, méthoxy, trifluorométhyle, trifluorométhoxy, cyano ou nitro, représente un radical cyclopropyle, cyclopentyle ou cyclohexyle, dans lequel éventuellement un groupe méthylène est remplacé par un atome d'oxygène,
ou A représente une liaison et B représente un atome d'hydrogène,
D représente NH ou un atome d'oxygène,
Q¹ représente un atome d'hydrogène, le groupe méthyle ou éthyle,
Q² représente un atome d'hydrogène, le groupe méthyle ou éthyle,
Q¹ et Q² représentent ensemble, avec l'atome de carbone auquel ils sont liés, le groupe cyclopropyle, cyclopentyle ou cyclohexyle, ou
Q¹ et Q² représentent ensemble, avec les atomes de carbone auxquels ils sont liés, un cycle en C₅-C₆ éventuellement interrompu par un atome d'oxygène,
G représente un atome d'hydrogène (a) ou l'un des groupes 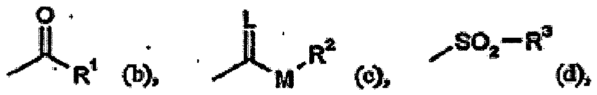 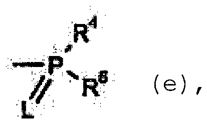 E (f), ou 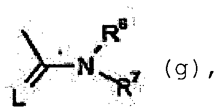 dans lesquels
E représente un ion métallique ou un ion ammonium,
L représente un atome d'oxygène ou de soufre et
M représente un atome d'oxygène ou de soufre,
R¹ représente un groupe alkyle en C₁-C₁₀, alcényle en C₂-C₁₀, alcoxy (C₁-C₄) -alkyle (C₁-C₂), alkyl(C₁-C₄)thio-alkyle(C₁-C₂), chacun éventuellement une à trois fois substitué par le fluor ou le chlore, ou représente un radical cycloalkyle en C₃-C₆ éventuellement une fois substitué par le fluor, le chlore, par méthyle, éthyle ou méthoxy,
représente un radical phényle éventuellement une ou deux fois substitué par le fluor, le chlore, le brome, par cyano, nitro, méthyle, éthyle, n-propyle, isopropyle, méthoxy, éthoxy, trifluorométhyle ou trifluorométhoxy.
représente un radical furanyle, thiényle ou pyridyle, chacun éventuellement une fois substitué par le chlore, le brome ou par méthyle.
R² représente un groupe alkyle en C₁-C₁₀, alcényle en C₂-C₁₀ ou alcoxy (C₁-C₄) -alkyle (C₂-C₄), chacun éventuellement une à trois fois substitué par le fluor ou le chlore,
représente le radical cyclopentyle ou cyclohexyle
ou représente un radical phényle ou benzyle chacun éventuellement une ou deux fois substitué par le fluor ou le chlore, par cyano, nitro, méthyle, éthyle, méthoxy, trifluorométhyle ou trifluorométhoxy.
R³ représente un groupe méthyle, éthyle, propyle ou isopropyle, chacun éventuellement une à trois fois substitué par le fluor ou le chlore, ou un radical phényle éventuellement une fois substitué par le fluor, le chlore, le brome, par méthyle, éthyle, isopropyle, tert-butyle, méthoxy, éthoxy, isopropoxy, trifluorométhyle, trifluorométhoxy, cyano ou nitro.
R⁴ et R⁵, indépendamment l'un de l'autre, représentent un groupe alcoxy en C₁-C₄ ou alkyl(C₁-C₄)thio, ou représentent un radical phényle, phénoxy ou phénylthio, chacun éventuellement une fois substitué par le fluor, le chlore, le brome, par nitro, cyano, méthyle, méthoxy, trifluorométhyle ou trifluorométhoxy.
R⁶ et R⁷, indépendamment l'un de l'autre, représentent un atome d'hydrogène, représentent un groupe alkyle en C₁-C₄, cycloalkyle en C₃-C₆, alcoxy en C₁-C₄, alcényle en C₃-C₄ ou alcoxy(C₁-C₄)-alkyle(C₂-C₄), représentent un radical phényle éventuellement une ou deux fois substitué par le fluor, le chlore, le brome, par méthyle, méthoxy ou trifluorométhyle, ou ensemble représentent un radical alkylène en C₅-C₆, dans lequel éventuellement un groupe méthylène est remplacé par un atome d'oxygène ou de soufre.

5. Composés de formule (I) selon la revendication 1, dans lesquels
W représente le groupe méthyle, éthyle ou méthoxy,
X représente un atome de chlore, le groupe méthyle, éthyle ou méthoxy,
Y en la position 4 représente un atome de chlore ou de brome,
Z représente un atome d'hydrogène.
W représente également un atome d'hydrogène,
X représente également le groupe méthyle,
Y représente également en la position 5 le radical 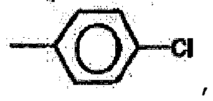
Z représente également en la position 4 un atome d'hydrogène.
W représente en outre le groupe méthyle ou éthyle,
X représente en outre un atome de chlore, de brome ou le groupe méthyle,
Y représente en outre en la position 4 le groupe méthyle,
Z représente en outre un atome d'hydrogène.
A représente -CH₂- ou -CH₂CH₂-,
B représente un atome d'hydrogène, un groupe méthyle, éthyle, propyle, méthoxy ou cyclopropyle,
ou A représente une liaison et B représente un atome d'hydrogène,
D représente NH,
Q¹ représente un atome d'hydrogène,
Q² représente un atome d'hydrogène,
G représente un atome d'hydrogène (a) ou l'un des groupes 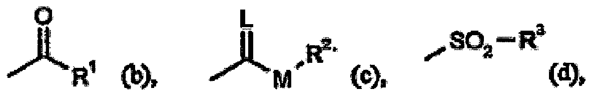 E (f), ou 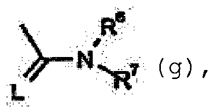 dans lesquels
E représente un ion métallique,
L représente un atome d'oxygène et
M représente un atome d'oxygène.
R¹ représente un groupe alkyle en C₁-C₁₀,
représente un radical phényle éventuellement une fois substitué par le chlore,
R² représente un groupe alkyle en C₁-C₁₀ ou alcényle en C₂-C₁₀,
R³ représente le groupe méthyle,
R⁶ et R⁷ ensemble représentent un radical alkylène en C₅-C₆, dans lequel éventuellement un groupe méthylène est remplacé par un atome d'oxygène.

6. Procédé pour la préparation de composés de formule (I) selon la revendication 1, **caractérisé en ce que**, pour l'obtention
(A) de composés de formule (I-1-a) 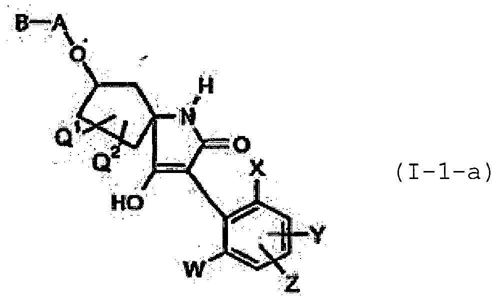 dans laquelle
A, B, Q¹, Q², W, X, Y et Z ont les significations données plus haut,
on condense par condensation intramoléculaire des composés de formule (II) 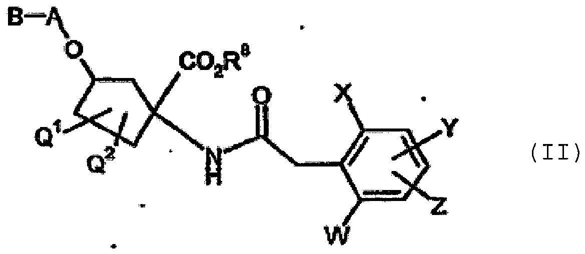 dans laquelle
A, B, Q¹, Q², W, X, Y et Z ont les significations données plus haut,
et
R⁸ représente un groupe alkyle,
en présence d'un diluant et en présence d'une base,
(B) de composés de formule (I-2-a) 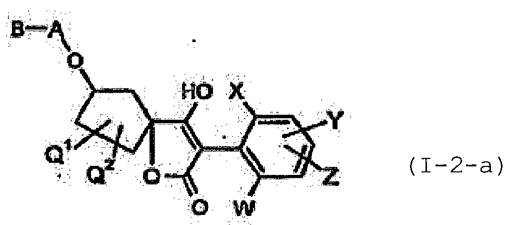 dans laquelle
A, B, Q¹, Q², W, X, Y et Z ont les significations données plus haut,
on condense par condensation intramoléculaire des composés de formule (III) 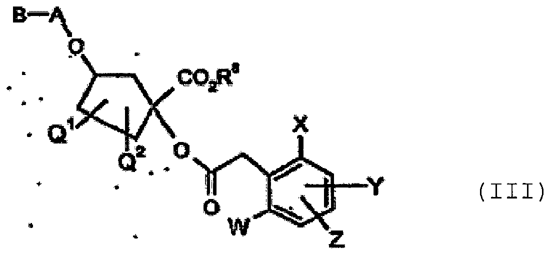 dans laquelle
A, B, Q¹, Q², W, X, Y, Z et R⁸ ont les significations données plus haut,
en présence d'un diluant et en présence d'une base,
(C) de composés de formules (I-1-b) à (I-2-b) indiquées plus haut, dans lesquelles R¹, A, B, Q¹, Q², W, X, Y et Z ont les significations données plus haut, on fait réagir des composés de formule (I-1-a) à (I-2-a) présentées plus haut, dans lesquelles A, B, Q¹, Q², W, X, Y et Z ont les significations indiquées plus haut, respectivement
α) avec des composés de formule (IV) 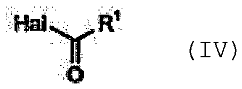 dans laquelle
R¹ a la signification indiquée plus haut et
Hal représente un halogène
ou
β) avec des anhydrides d'acides carboxyliques de formule (V)
R¹-CO-O-CO-R¹ (V)
dans laquelle
R¹ a la signification indiquée plus haut,
éventuellement en présence d'un diluant et éventuellement en présence d'un capteur d'acide ;
(D) de composés de formules (I-1-c) à (I-2-c), dans lesquelles R², A, B, Q¹, Q², W, M, X, Y et Z ont les significations indiquées plus haut et L représente un atome d'oxygène, on fait réagir des composés de formules (I-1-a) à (I-2-a) présentées plus haut, dans lesquelles A, B, Q¹, Q², W, X, Y et Z ont les significations indiquées plus haut, respectivement
avec des esters d'acide chloroformique ou des thioesters d'acide chloroformique de formule (VI)
R²-M-CO-Cl (VI)
dans laquelle
R² et M ont les significations indiquées plus haut, éventuellement en présence d'un diluant et éventuellement en présence d'un capteur d'acide ;
(E) de composés de formules (I-1-c) à (I-2-c) présentées plus haut, dans lesquelles R², A, B, Q¹, Q², W, M, X, Y et Z ont les significations indiquées plus haut et L représente un atome de soufre, on fait réagir des composé de formules (I-1-a) à (I-2-a) présentées plus haut, dans lesquelles A, B, Q¹, Q², W, X, Y et Z ont les significations indiquées plus haut, respectivement avec des esters d'acide chloromonothioformique ou des esters d'acide chlorodithioformique de formule (VII) 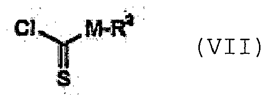 dans laquelle
M et R² ont les significations indiquées plus haut,
éventuellement en présence d'un diluant et éventuellement en présence d'un capteur d'acide ;
(F) de composés de formules (I-1-d) à (I-2-d) présentées plus haut, dans lesquelles R³, A, B, W, Q¹, Q², X, Y et Z ont les significations indiquées plus haut, on fait réagir des composés de formules (I-1-a) à (I-2-a) présentées plus haut, dans lesquelles A, B, Q¹, Q², W, X, Y et Z ont les significations indiquées plus haut, chaque fois
avec des chlorures de sulfonyle de formule (VIII)
R³-SO₂-Cl (VIII)
dans laquelle
R³ a la signification indiquée plus haut,
éventuellement en présence d'un diluant et éventuellement en présence d'un capteur d'acide,
(G) de composés de formules (I-1-e) à (I-2-e) présentées plus haut, dans lesquelles L, R⁴, R⁵, A, B, Q¹, Q², W, X, Y et Z ont les significations indiquées plus haut, on fait réagir des composés de formules (I-1-a) à (I-2-a) présentées plus haut, dans lesquelles A, B, Q¹, Q², W, X, Y et Z ont les significations indiquées plus haut, chaque fois
avec des composés phosphorés de formule (IX) 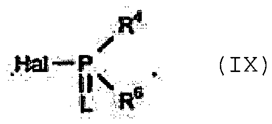 dans laquelle
L, R⁴ et R⁵ ont les significations indiquées plus haut et
Hal représente un halogène,
éventuellement en présence d'un diluant et éventuellement en présence d'un capteur d'acide,
(H) de composés de formules (I-1-f) à (I-2-f), dans lesquelles E, A, B, Q¹, Q², W, X, Y et Z ont les significations indiquées plus haut, on fait réagir des composés de formules (I-1-a) à (I-2-a) présentées plus haut, dans lesquelles A, B, Q¹, Q², W, X, Y et Z ont les significations indiquées plus haut, chaque fois
avec des composés contenant un métal ou des amines de formules (X) ou (XI)
Me(OR¹⁰)ₜ (X) 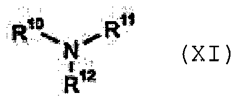
dans lesquelles
Me représente un métal mono- ou divalent,
t représente le nombre 1 ou 2 et
R¹⁰, R¹¹, R¹² représentent chacun indépendamment un atome d'hydrogène ou un groupe alkyle,
éventuellement en présence d'un diluant,
(I) de composés de formules (I-1-g) à (I-2-g) présentées plus haut, dans lesquelles L, R⁶, R⁷, A, B, Q¹, Q², W, X, Y et Z ont les significations indiquées plus haut, on fait réagir des composés de formules (I-1-a) à (I-2-a) présentées plus haut, dans lesquelles A, B, Q¹, Q², W, X, Y et Z ont les significations données plus haut, respectivement
α) avec des isocyanates ou isothiocyanates de formule (XII)
R⁶-N=C=L (XII)
dans laquelle
R⁶ et L ont les significations indiquées plus haut,
éventuellement en présence d'un diluant et éventuellement en présence d'un catalyseur ou
β) avec des chlorures d'acide carbamique ou des chlorures d'acide thiocarbamique de formule (XIII) 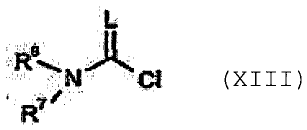 dans laquelle
L, R⁶ et R⁷ ont les significations indiquées plus haut,
éventuellement en présence d'un diluant et éventuellement en présence d'un capteur d'acide.

7. Agent ayant une teneur efficace en une association de substances actives comprenant comme composants
(a') au moins un composé de formule (I), dans lequel A, B, D, G, Q¹, Q², W, X, Y et Z ont la signification donnée plus haut
et
b') au moins un composé améliorant la tolérance par des plantes en culture, choisi dans le groupe suivant de composés :
4-dichloracétyl-1-oxa-4-aza-spiro[4.5]-décane (AD-67, MON-4660), 1-dichloracétyl-hexahydro-3,3,8a-triméthylpyrrolo[1,2-a]-pyrimidin-6(2H)-one (dicyclonone, BAS-145138), 4-dichloracétyl-3,4-dihydro-3-méthyl-2H-1,4-benzoxazine (bénoxacor), 5-chloro-quinoléine-8-oxy-acétate de 1-méthyl-hexyle (cloquintocet-mexyl - voir également les composés apparentés dans EP-A-86750, EP-A-94349, EP-A-191736, EP-A-492366), 3-(2-chlorobenzyl)-1-(1-méthyl-1-phényléthyl)-urée (cumyluron), α-(cyanométhoximino)-phénylacétonitrile (cyométrinil), acide 2,4-dichlorophénoxyacétique (2,4-D), acide 4-(2,4-dichlorophénoxy)-butyrique (2,4-DB), 1-(1-méthyl-1-phényléthyl)-3-(4-méthyl-phényl)-urée (daimuron, dymron), acide 3,6-dichloro-2-méthoxy-benzoïque (dicamba), pipéridine-1-thiocarboxylate de S-1-méthyl-1-phényléthyle (dimépipérate), 2,2-dichloro-N-(2-oxo-2-(2-propénylamino)-éthyl)-N-(2-propényl)-acétamide (DKA-24), 2,2-dichloro-N,N-di-2-propényl-acétamide (dichlormide), 4,6-dichloro-2-phényl-pyrimidine (fenclorim), 1-(2,4-dichlorophényl)-5-trichlorométhyl-1H-1,2,4-triazole-3-carboxylate d'éthyle (fenchlorazole-éthyle - voir également les composés apparentés dans EP-A-174562 et EP-A-346620), 2-chloro-4-trifluorométhyl-thiazole-5-carboxylate de phényl-méthyle (flurazole), 4-chloro-N-(1,3-dioxolan-2-yl-méthoxy)-α-trifluoro-acétophénonoxime (fluxofénim), 3-dichloracétyl-5-(2-furanyl)-2,2-diméthyl-oxazolidine (furilazole, MON-13900), 4,5-dihydro-5,5-diphényl-3-isoxazole-carboxylate d'éthyle (isoxadifène-éthyle - voir également les composés apparentés dans WO-A-95/07897), 3,6-dichloro-2-méthoxybenzoate de 1-(éthoxycarbonyl)-éthyle (lactidichlor), acide (4-chloro-o-tolyloxy)-acétique (MCPA), acide 2-(4-chloro-o-tolyloxy)-propionique (mécoprop), 1-(2,4-dichlorophényl)4,5-dihydro-5-méthyl-1H-pyrazole-3,5-dicarboxylate de diéthyle (méfenpyr-diéthyle - voir également les composés apparentés dans WO-A-91/07874) 2-dichlorométhyl-2-méthyl-1,3-dioxolane (MG-191), 4-carbodithioate de 2-propényl-1-oxa-4-azaspiro-[4.5]décane (MG-838), anhydride d'acide 1,8-naphtalique, α-(1,3-dioxolan-2-yl-méthoximino)-phénylacétonitrile (oxabétrinil), 2,2-dichloro-N-(1,3-dioxolan-2-yl-méthyl)-N-(2-propényl)-acétamide (PPG-1292), 3-dichloracétyl-2,2-diméthyl-oxazolidine (R-28725), 3-dichloracétyl-2,2,5-triméthyl-oxazolidine (R-29148), acide 4-(4-chloro-o-tolyl)-butyrique, acide 4-(4-chloro-phénoxy)-butyrique, acide diphénylméthoxyacétique, diphénylméthoxyacétate de méthyle, diphénylméthoxyacétate d'éthyle, 1-(2-chloro-phényl)-5-phényl-1H-pyrazole-3-carboxylate de méthyle, 1-(2,4-dichlorophényl)-5-méthyl-1H-pyrazole-3-carboxylate d'éthyle, 1-(2,4-dichlorophényl)-5-isopropyl-1H-pyrazole-3-carboxylate d'éthyle, 1-(2,4-dichlorophényl)-5-(1,1-diméthyléthyl)-1H-pyrazole-3-carboxylate d'éthyle, 1-(2,4-dichloro-phényl)-5-phényl-1H-pyrazole-3-carboxylate d'éthyle (voir également les composés apparentés dans EP-A-269806 et EP-A-333131), 5-(2,4-dichlorobenzyl)-2-isoxazoline-3-carboxylate d'éthyle, 5-phényl-2-isoxazoline-3-carboxylate d'éthyle, 5-(4-fluorophényl)-5-phényl-2-isoxazoline-3-carboxylate d'éthyle (voir également les composés apparentés dans WO-A-91/08202), 5-chloro-quinoléine-8-oxy-acétate de 1,3-diméthyl-but-1-yle, 5-chloro-quinoléine-8-oxyacétate de 4-allyloxy-butyle, 5-chloro-quinoléine-8-oxy-acétate de 1-allyloxy-prop-2-yle, 5-chloro-quinoxaline-8-oxy-acétate de méthyle, 5-chloro-quinoléine-8-oxy-acétate d'éthyle, 5-chloro-quinoxaline-8-oxy-acétate d'allyle, 5-chloro-quinoléine-8-oxy-acétate de 2-oxo-prop-1-yle, 5-chloro-quinoléine-8-oxy-malonate de diéthyle, 5-chloro-quinoxaline-8-oxy-malonate de diallyle, 5-chloro-quinoléine-8-oxy-malonate de diéthyle (voir également les composés apparentés dans EP-A-582198), acétate de 4-carboxy-chroman-4-yle (AC-304415, voir EP-A-613618), acide 4-chloro-phénoxy-acétique, 3,3'-diméthyl-4-méthoxy-benzophénone, 1-bromo-4-chlorométhylsulfonyl-benzène, 1-[4-(N-2-méthoxybenzoylsulfamoyl)-phényl]-3-méthyl-urée (alias N-(2-méthoxy-benzoyl)-4-[(méthylamino-carbonyl-amino]-benzène-sulfonamide), 1-[4-(N-2-méthoxybenzoylsulfamoyl)-phényl]-3,3-diméthyl-urée, 1-[4-(N-4,5-diméthyl-benzoylsulfamoyl)-phényl]-3-méthyl-urée, 1-[4-(N-naphtylsulfamoyl)-phényl]3,3-diméthyl-urée, N-(2-méthoxy-5-méthyl-benzoyl)-4-(cyclopropylamino-carbonyl)-benzènesulfonamide,
et/ou l'un des composés définis par des formules générales
de formule générale (IIa) 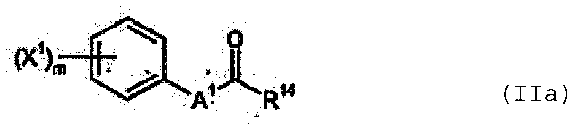
de formule générale (IIb) 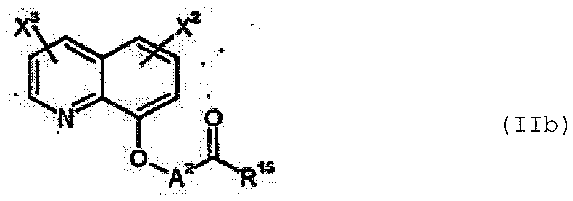
ou de formule (IIc) 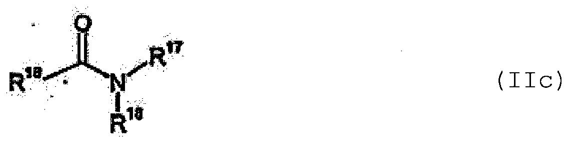 où
m représente un nombre 0, 1, 2, 3, 4 ou 5,
A¹ représente l'un des groupements hétérocycliques divalents schématisés ci-après, 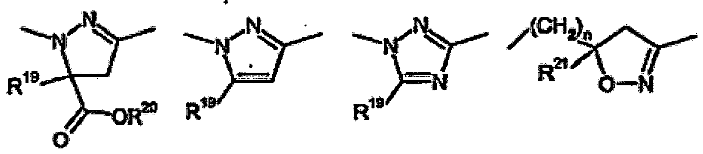
n représente un nombre 0, 1, 2, 3, 4 ou 5,
A² représente un radical alcanediyle ayant 1 ou 2 atomes de carbone, éventuellement substitué par alkyle en C₁-C₄ et/ou alcoxy(C₁-C₄)-carbonyle et/ou alcényl(C₁-C₄)oxy-carbonyle,
R¹⁴ représente un groupe hydroxy, mercapto, amino, alcoxy en C₁-C₆, alkyl(C₁-C₆)thio, alkyl(C₁-C₆) amino ou di[alkyl(C₁-C₄)]amino,
R¹⁵ représente un groupe hydroxy, mercapto, amino, alcoxy en C₁-C₇, alcényl (C₁-C₆) oxy, alcényl (C₁-C₆) oxy-alcoxy (C₁-C₆), alkyl (C₁-C₆)-thio, alkyl(C₁-C₆)amino ou di[alkyl(C₁-C₄)]-amino,
R¹⁶ représente un groupe alkyle en C₁-C₄ éventuellement substitué par le fluor, le chlore et/ou le brome,
R¹⁷ représente un atome d'hydrogène, un groupe alkyle en C₁-C₆, alcényle en C₂-C₆ ou alcynyle en C₂-C₆, chacun éventuellement substitué par le fluor, le chlore et/ou le brome, un groupe alcoxy(C₁-C₄)-alkyle(C₁-C₄), dioxolanyl-alkyle(C₁-C₄), furyle, furyl-alkyle(C₁-C₄), thiényle, thiazolyle, pipéridinyle, ou un radical phényle éventuellement substitué par le fluor, le chlore et/ou le brome ou par alkyle en C₁-C₄,
R¹⁸ représente un atome d'hydrogène, un groupe alkyle en C₁-C₆, alcényle en C₂-C₆ ou alcynyle en C₂-C₆, chacun éventuellement substitué par le fluor, le chlore et/ou le brome, un groupe alcoxy(C₁-C₄)-alkyle(C₁-C₄), dioxolanyl-alkyle(C₁-C₄), furyle, furyl-alkyle (C₁-C₄), thiényle, thiazolyle, pipéridinyle, ou représente un radical phényle éventuellement substitué par le fluor, le chlore et/ou le brome ou par alkyle en C₁-C₄, R¹⁷ et R¹⁸ également représentent ensemble un cycle benzénique condensé, chaque fois éventuellement substitué par alkyle en C₁-C₄, phényle, furyle, ou un radical alcane (C₃-C₆) diyle ou oxa-alcane (C₂-C₅) diyle, substitué par deux substituants qui forment ensemble, avec l'atome de carbone auquel ils sont liés, un carbocycle à 5 ou 6 chaînons,
R¹⁹ représente un atome d'hydrogène ou d'halogène, un groupe cyano, ou représente un groupe alkyle en C₁-C₄, cycloalkyle en C₃-C₆ ou phényle, chacun éventuellement substitué par le fluor, le chlore et/ou le brome,
R²⁰ représente un atome d'hydrogène, un groupe alkyle en C₁-C₆, cycloalkyle en C₃-C₆ ou tri-[alkyl(C₁-C₄)]-silyle, chacun éventuellement substitué par hydroxy, cyano, halogène ou alcoxy en C₁-C₄,
R²¹ représente un atome d'hydrogène, un groupe cyano, un atome d'halogène ou représente un groupe alkyle en C₁-C₄, cycloalkyle en C₃-C₆ ou phényle, chacun éventuellement substitué par le fluor, le chlore et/ou le brome,
X¹ représente un groupe nitro, cyano, un atome d'halogène, un groupe alkyle en C₁-C₄, halogénoalkyle(C₁-C₄), alcoxy en C₁-C₄ ou halogénoalcoxy(C₁-C₄),
X² représente un atome d'hydrogène ou d'halogène, un groupe cyano, nitro, alkyle en C₁-C₄, halogénoalkyle(C₁-C₄), alcoxy en C₁-C₄ ou halogénoalcoxy(C₁-C₄),
X³ représente un atome d'hydrogène ou d'halogène, un groupe cyano, nitro, alkyle en C₁-C₄, halogénoalkyle(C₁-C₄), alcoxy en C₁-C₄ ou halogénoalcoxy(C₁-C₄),
et/ou les composés définis par des formules générales
de formule générale (IId) 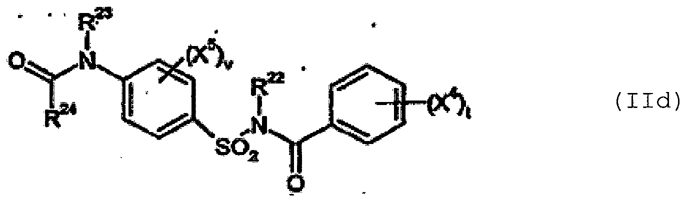 ou de formule générale (IIe) 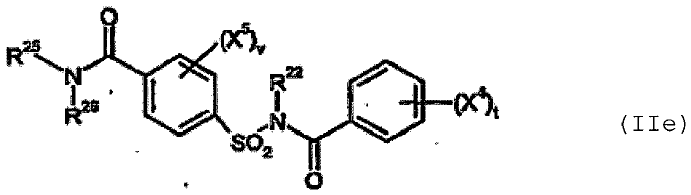 où
t représente un nombre 0, 1, 2, 3, 4 ou 5,
V représente un nombre 0, 1, 2, 3, 4 ou 5,
R²² représente un atome d'hydrogène ou un groupe alkyle en C₁-C₄,
R²³ représente un atome d'hydrogène ou un groupe alkyle en C₁-C₄,
R²⁴ représente un atome d'hydrogène, un groupe alkyle en C₁-C₆, alcoxy en C₁-C₆, alkyl(C₁-C₆)thio, alkyl(C₁-C₆)amino ou di-[alkyl(C₁-C₄)]amino, chacun éventuellement substitué par cyano, halogène ou alcoxy en C₁-C₄, ou un groupe cycloalkyle en C₃-C₆, cycloalkyl(C₃-C₆)oxy, cycloalkyl(C₃-C₆)thio ou cycloalkyl(C₃-C₆)-amino, chacun éventuellement substitué par cyano, halogène ou alcoxy en C₁-C₄,
R²⁵ représente un atome d'hydrogène, un groupe alkyle en C₁-C₆ éventuellement substitué par cyano, hydroxy, halogène ou alcoxy en C₁-C₄, un groupe alcényle en C₃-C₆ ou alcynyle en C₃-C₆, chacun éventuellement substitué par cyano ou halogène, ou un groupe cycloalkyle en C₃-C₆ éventuellement substitué par cyano, halogène ou alkyle en C₁-C₄,
R²⁶ représente un atome d'hydrogène, un groupe alkyle en C₁-C₆ éventuellement substitué par cyano, hydroxy, halogène ou alcoxy en C₁-C₄, un groupe alcényle en C₃-C₆ ou alcynyle en C₃-C₆, chacun éventuellement substitué par cyano ou halogène, un groupe cycloalkyle en C₃-C₆ éventuellement substitué par cyano, halogène ou alkyle en C₁-C₄, ou un groupe phényle éventuellement substitué par nitro, cyano, halogène, alkyle en C₁-C₄, halogénoalkyle(C₁-C₄), alcoxy en C₁-C₄ ou halogénoalcoxy(C₁-C₄), ou conjointement avec R²⁵ représente un groupe alcane(C₂-C₆)diyle ou oxa-alcane(C₂-C₅)diyle chacun éventuellement substitué par alkyle en C₁-C₄.
X⁴ représente un atome d'halogène ou un groupe nitro, cyano, carboxy, carbamoyle, formyle, sulfamoyle, hydroxy, amino, alkyle en C₁-C₄, halogénoalkyle(C₁-C₄), alcoxy en C₁-C₄ ou halogénoalcoxy(C₁-C₄), et
X⁵ représente un atome d'halogène ou un groupe nitro, cyano, carboxy, carbamoyle, formyle, sulfamoyle, hydroxy, amino, alkyle en C₁-C₄, halogénoalkyle(C₁-C₄), alcoxy en C₁-C₄ ou halogénoalcoxy(C₁-C₄).

8. Agent selon la revendication 7, dans lequel le composé améliorant la tolérance par des plantes en culture est choisi dans le groupe suivant de composés :
cloquintocet-mexyl, fenchlorazole-éthyle, isoxadifène-éthyle, méfenpyr-diéthyle, furilazole, fenclorim, cumyluron, dymron ou les composés 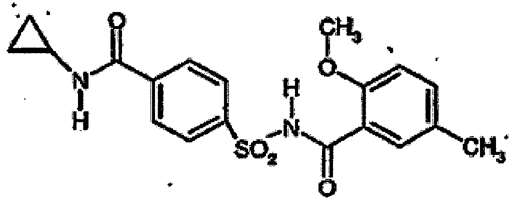
et

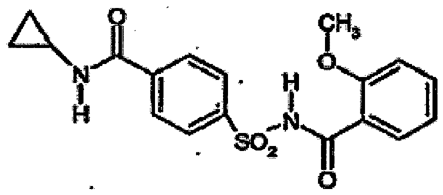

9. Agent selon la revendication 7 ou 8, dans lequel le composé améliorant la tolérance par des plantes en culture est le cloquintocet-mexyl.

10. Agent selon la revendication 7 ou 8, dans lequel le composé améliorant la tolérance par des plantes en culture est le méfenpyr-diéthyle.

11. Composition comprenant
- au moins un composé de formule (I) selon la revendication 1 ou un agent selon la revendication 7 et
- au moins un sel de formule (III') 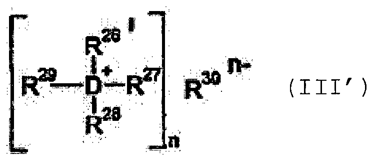 dans laquelle
D représente un atome d'azote ou de phosphore,
R²⁶, R²⁷, R²⁸ et R²⁹ représentent indépendamment les uns des autres un atome d'hydrogène ou un groupe alkyle en C₁-C₈ chaque fois éventuellement substitué ou un groupe alkylène en C₁-C₈ une ou plusieurs insaturé, éventuellement substitué, les substituants pouvant être choisis parmi des atomes d'halogène, les groupes nitro et cyano,
n représente 1, 2, 3 ou 4,
R³⁰ représente un anion organique ou inorganique.

12. Composition selon la revendication 11, **caractérisée en ce qu'**elle contient au moins un agent favorisant la pénétration.

13. Utilisation de composés de formule (I) selon la revendication 1, pour la fabrication de pesticides et/ou d'herbicides et/ou de fongicides.

14. Pesticides et/ou herbicides et/ou fongicides, **caractérisés par** une teneur en au moins un composé de formule (I) selon la revendication 1.

15. Procédé pour la lutte contre des ravageurs animaux et/ou la croissance indésirable de plantes et/ou des champignons, **caractérisé en ce qu'**on fait agir sur les ravageurs et/ou leur habitat des composés de formule (I) selon la revendication 1.

16. Utilisation de composés de formule (I) selon la revendication 1, pour la lutte contre des ravageurs animaux et/ou la croissance indésirable de plantes et/ou des champignons.

17. Procédé pour la fabrication de pesticides et/ou d'herbicides et/ou de fongicides, **caractérisé en ce qu'**on mélange des composés de formule (I) selon la revendication 1 avec des excipients et/ou des substances tensioactives.

18. Procédé pour la lutte contre la croissance indésirable de plantes, **caractérisé en ce qu'**on fait agir sur les plantes ou leur environnement un agent selon la revendication 7.

19. Utilisation d'un agent selon la revendication 7, pour la lutte contre la croissance indésirable de plantes.

20. Procédé pour la lutte contre la croissance indésirable de plantes, **caractérisé en ce qu'**on fait agir sur les plantes ou leur environnement, séparément en proche succession dans le temps des composés de formule (I) selon la revendication 1 et le composé améliorant la tolérance par les plantes en culture selon la revendication 7.

21. Procédé pour augmenter l'action de pesticides et/ou d'herbicides contenant une substance active de formule (I) selon la revendication 1 ou un agent selon la revendication 7, **caractérisé en ce qu'**on prépare le produit prêt à l'emploi (bouillie pulvérisable) en utilisant un sel de formule (III') selon la revendication 11.

22. Procédé selon la revendication 21, **caractérisé en ce qu'**on prépare la bouillie pulvérisable en utilisant un agent favorisant la pénétration.

23. Composés de formule (II) 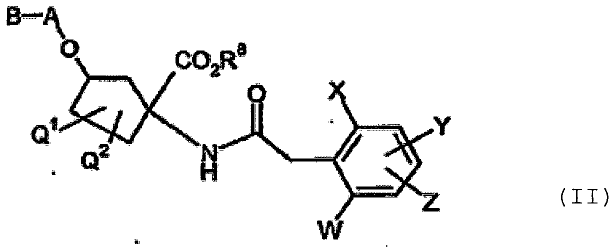 dans laquelle
A, B, Q¹, Q², W, X, Y, Z et R⁸ ont les significations indiquées plus haut.

24. Composés de formule (III) 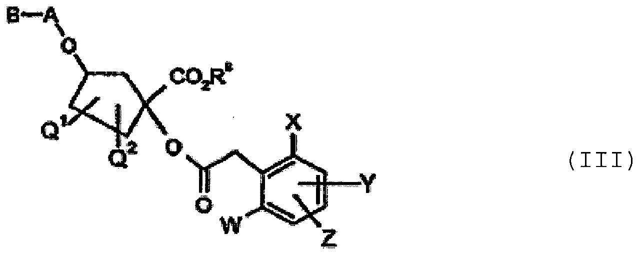 dans laquelle
A, B, Q¹, Q², W, X, Y, Z et R⁸ ont les significations indiquées plus haut.

25. Composés de formule (XVI) 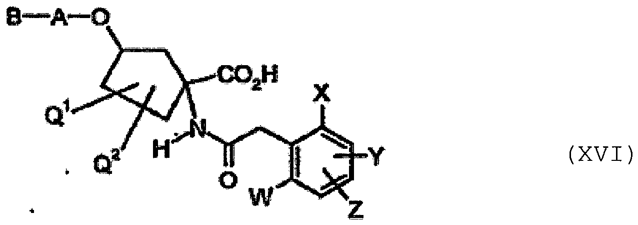 dans laquelle
A, B, Q¹, Q², W, X, Y et Z ont les significations indiquées plus haut.
